# EUROPEAN PATENT APPLICATION

(11) **EP 4 006 139 A1**
(43) Date of publication of application: **01.06.2022**
(21) Application number: 20075015.6
(22) Date of filing: 26.11.2020
(51) Int. Cl.: C12N 1/00

(54) **GENETICALLY ENGINEERED BACTERIUM CAPABLE OF PRODUCING CYTOKININS WITH ISOPRENOID SIDE CHAINS**

(71) Applicant: Acies Bio d.o.o., 1000 Ljubljana (SI)
(72) Inventor: Kogej, Tina, 1000 Ljubljana (SI); Kosec, Gregor, 1000 Ljubljana (SI); Fujs, Stefan, 1000 Ljubljana (SI); Blazic, Marko, 1000 Ljubljana (SI); Horvat, Jaka, 1000 Ljubljana (SI)
(74) Representative: Zacco GmbH

(57) **Abstract**

The present invention generally relates to the biotechnology engineering, and specifically to a genetically engineered bacterium capable of producing cytokinins with isoprenoid side chains (isoprenoid cytokinins), and the preparation and application thereof.

SEQUENCE LISTING

<110>  Acies Bio d.o.o.

<120>  Genetically engineered bacterium capable of producing cytokinins
       with isoprenoid side chains

<130>  P49904EP00

<160>  95

<170>  PatentIn version 3.5

<210>  1
<211>  243
<212>  PRT
<213>  Agrobacterium tumefaciens

<400>  1

Met Leu Leu His Leu Ile Tyr Gly Pro Thr Cys Ser Gly Lys Thr Asp
1               5                   10                  15

Met Ala Ile Gln Ile Ala Gln Glu Thr Gly Trp Pro Val Val Ala Leu
            20                  25                  30

Asp Arg Val Gln Cys Cys Pro Gln Ile Ala Thr Gly Ser Gly Arg Pro
        35                  40                  45

Leu Glu Ser Glu Leu Gln Ser Thr Arg Arg Ile Tyr Leu Asp Ser Arg
    50                  55                  60

Pro Leu Thr Glu Gly Ile Leu Asp Ala Glu Ser Ala His Arg Arg Leu
65                  70                  75                  80

Ile Phe Glu Val Asp Trp Arg Lys Ser Glu Glu Gly Leu Ile Leu Glu
                85                  90                  95

Gly Gly Ser Ile Ser Leu Leu Asn Cys Met Ala Lys Ser Pro Phe Trp
            100                 105                 110

Arg Ser Gly Phe Gln Trp His Val Lys Arg Leu Arg Leu Gly Asp Ser
        115                 120                 125

Asp Ala Phe Leu Thr Arg Ala Lys Gln Arg Val Ala Glu Met Phe Ala
    130                 135                 140

Ile Arg Glu Asp Arg Pro Ser Leu Leu Glu Glu Leu Ala Glu Leu Trp
145                 150                 155                 160

Asn Tyr Pro Ala Ala Arg Pro Ile Leu Glu Asp Ile Asp Gly Tyr Arg
                165                 170                 175

Cys Ala Ile Arg Phe Ala Arg Lys His Asp Leu Ala Ile Ser Gln Leu
            180                 185                 190

Pro Asn Ile Asp Ala Gly Arg His Val Glu Leu Ile Glu Ala Ile Ala
        195                 200                 205

Asn Glu Tyr Leu Glu His Ala Leu Ser Gln Glu Arg Asp Phe Pro Gln
    210                 215                 220

Trp Pro Glu Asp Gly Ala Gly Gln Pro Val Cys Pro Val Thr Leu Thr
225                 230                 235                 240

Arg Ile Arg

<210>  2
<211>  240
<212>  PRT
<213>  Agrobacterium tumefaciens

<400>  2

Met Asp Leu Arg Leu Ile Phe Gly Pro Thr Cys Thr Gly Lys Thr Ser
1               5                   10                  15

Thr Ala Val Ala Leu Ala Gln Gln Thr Gly Leu Pro Val Leu Ser Leu
            20                  25                  30

Asp Arg Val Gln Cys Cys Pro Gln Leu Ser Thr Gly Ser Gly Arg Pro
        35                  40                  45

Thr Val Glu Glu Leu Lys Gly Thr Ser Arg Leu Tyr Leu Asp Asp Arg
    50                  55                  60

Pro Leu Val Lys Gly Ile Ile Ala Ala Lys Gln Ala His Glu Arg Leu

65                  70                  75                  80

Met Gly Glu Val Tyr Asn Tyr Glu Ala His Gly Gly Leu Ile Leu Glu
                85                  90                  95

Gly Gly Ser Ile Ser Leu Leu Lys Cys Met Ala Gln Ser Ser Tyr Trp
            100                 105                 110

Ser Ala Asp Phe Arg Trp His Ile Ile Arg His Glu Leu Ala Asp Glu
        115                 120                 125

Glu Thr Phe Met Asn Val Ala Lys Ala Arg Val Lys Gln Met Leu Arg
    130                 135                 140

Pro Ala Ala Gly Leu Ser Ile Ile Gln Glu Leu Val Asp Leu Trp Lys
145                 150                 155                 160

Glu Pro Arg Leu Arg Pro Ile Leu Lys Glu Ile Asp Gly Tyr Arg Tyr
                165                 170                 175

Ala Met Leu Phe Ala Ser Gln Asn Gln Ile Thr Ser Asp Met Leu Leu
            180                 185                 190

Gln Leu Asp Ala Asp Met Glu Asp Lys Leu Ile His Gly Ile Ala Gln
        195                 200                 205

Glu Tyr Leu Ile His Ala Arg Arg Gln Glu Gln Lys Phe Pro Arg Val
    210                 215                 220

Asn Ala Ala Ala Tyr Asp Gly Phe Glu Gly His Pro Phe Gly Met Tyr
225                 230                 235                 240

<210>  3
<211>  234
<212>  PRT
<213>  Pseudomonas savastanoi

<400>  3

Met Lys Ile Tyr Leu Ile Trp Gly Ala Thr Cys Thr Gly Lys Thr Glu
1               5                   10                  15

His Ser Ile Lys Leu Ser Lys Ser Thr Gly Trp Pro Val Ile Val Leu
                20                  25                  30

Asp Arg Val Gln Cys Cys Phe Asp Ile Ala Thr Gly Ser Gly Arg Pro
            35                  40                  45

His Pro Glu Glu Leu Gln Ser Thr Arg Arg Ile Tyr Leu Asp Asn Arg
    50                  55                  60

Arg Ile Ser Glu Gly Val Ile Ser Ala Glu Glu Ala Asn Asp Arg Leu
65                  70                  75                  80

Lys Leu Glu Val Asn Lys His Ile Asp Ser Gly Gly Val Ile Leu Glu
                85                  90                  95

Gly Gly Ser Ile Ser Leu Leu Lys Leu Ile Ser Lys Asp Pro Tyr Trp
            100                 105                 110

Cys Asp Arg Phe Ile Trp Ser Gln His Arg Met Arg Leu Gln Asp Thr
        115                 120                 125

Asp Val Phe Met Asp Lys Ala Lys Ala Arg Val Arg Arg Met Leu Val
    130                 135                 140

Gly Ser Thr Glu Thr Thr Gly Leu Leu Asp Glu Leu Val Ala Ala Gln
145                 150                 155                 160

Ser Asp Leu Asn Ala Lys Leu Ala Ile Gln Asp Ile Asp Gly Tyr Arg
                165                 170                 175

Tyr Ile Met Asn Tyr Ala Gln Ala Arg Arg Leu Ser Ile Thr Gln Leu
            180                 185                 190

Leu Asn Val Met Thr Gly Asp Met Lys Glu Glu Leu Ile Asn Gly Ile
        195                 200                 205

Ala Leu Glu Tyr Tyr Glu His Ala Lys Trp Gln Glu Arg Asp Phe Pro
    210                 215                 220

Ala Glu Trp Leu Ala Glu Arg Ser Thr Arg
225                 230

<210>  4
<211>  236
<212>  PRT
<213>  Erwinia herbicola

<400>  4

Met Arg Gly Asn Val Tyr Leu Leu Trp Gly Ala Thr Thr Thr Gly Lys
1               5                   10                  15

Thr Ala Phe Ser Val Ser Val Ala Lys Glu Arg Gly Trp Ser Val Ile
            20                  25                  30

Ala Leu Asp Arg Phe Gln Gly Tyr His Glu Ile Ser Thr Gly Ser Gly
        35                  40                  45

Ala Pro Thr Asn Asn Glu Leu Glu Lys Thr Glu Arg Ile Tyr Ile Thr
    50                  55                  60

Pro Gln Arg Gly Leu Ala Asp Gly Ile Ile Thr Ala Lys Asp Tyr Asn
65                  70                  75                  80

Tyr Trp Leu Lys Ser Arg Val Leu Ser Leu Pro Glu Gly Lys Ser Asp
                85                  90                  95

Phe Ile Ile Glu Gly Gly Ser Val Ser Leu Leu Lys Glu Met Val Ser
            100                 105                 110

Asp Glu Phe Trp Ala Asp Phe Leu Trp Glu Ile Lys Ile Phe Arg Ala
        115                 120                 125

Pro Ser Lys Asp Val Phe Ile Lys Arg Ala Lys Arg Arg Ile Thr Asp
    130                 135                 140

Met Leu His Pro Glu Asp Gly Arg Pro Ser Ile Met Asp Glu Val Ser
145                 150                 155                 160

Ser Phe Phe Met Asn His Lys Thr Ile His Pro Leu Glu Asp Ile Asp
                165                 170                 175

Gly Tyr Arg Thr Ile Ile Asp Tyr Cys Arg Cys Lys Lys Ile Gly Phe

180                 185                 190

Asp Lys Leu Lys Ile Met Thr Thr Asp Gln Glu Lys Ile Ile Ile Ser
        195                 200                 205

Gly Ile Ala Glu Glu Tyr Tyr Ala His Ala Leu Trp Gln Glu Gln Glu
    210                 215                 220

Thr Pro Cys Phe Pro His Ser Trp Ser Arg Arg Trp
225                 230                 235

<210>  5
<211>  255
<212>  PRT
<213>  Rhodococcus fascians

<400>  5

Met Lys Glu Ser Thr Met Ala Gln Thr Gln Ala Arg Phe Asp Arg Val
1               5                   10                  15

Arg Trp Glu Pro Gly Val Tyr Ala Ile Val Gly Ala Thr Gly Ile Gly
            20                  25                  30

Lys Ser Ala Glu Ala Ser Lys Leu Ala Leu Ser His Ser Ala Pro Ile
        35                  40                  45

Val Val Ala Asp Arg Ile Gln Cys Tyr Ser Asp Leu Leu Val Thr Ser
    50                  55                  60

Gly Arg Ala Phe Asp Ala Lys Val Glu Gly Leu Asn Arg Val Trp Leu
65                  70                  75                  80

Asp Asn Arg Thr Ile His Gln Gly Asn Phe Asp Pro Asp Glu Ala Phe
                85                  90                  95

Asp Arg Leu Ile Lys Val Leu Thr Ser Tyr Val Asp Arg Gly Glu Ala
            100                 105                 110

Val Val Met Glu Gly Gly Ser Ile Ser Leu Ile Leu Arg Phe Ala Gln
        115                 120                 125

Thr Ile Ser Asn Leu Pro Phe Pro Ala Val Val Asn Val Met Pro Ile
    130                 135                 140

Pro Asp Arg Gln His Tyr Phe Ala Gln Gln Cys Ala Arg Ala Arg Gln
145                 150                 155                 160

Met Leu Arg Gly Asp Ser Thr Gly Arg Asn Leu Leu Thr Glu Leu Ala
                165                 170                 175

Glu Ala Trp Val Leu Gly Asp Gln His Asn Phe Ile Ala Ser Val Ala
            180                 185                 190

Gly Leu Asp Cys Val Leu Asp Trp Cys Ala Thr His Ser Val Thr Pro
        195                 200                 205

Glu Glu Leu Ala Asn Arg Asp Leu Thr Thr Glu Val Leu Asp Glu Leu
    210                 215                 220

Ala Ala Ser Met Gly Gly Arg Tyr Val Glu His Gly Val Leu Gln Gln
225                 230                 235                 240

Glu Ile Phe Leu Arg Thr Phe Gly Ala Pro Gly Val Thr Ala Arg
                245                 250                 255

<210>  6
<211>  243
<212>  PRT
<213>  Agrobacterium tumefaciens

<400>  6

Met Ile Leu His Leu Ile Tyr Gly Pro Thr Cys Ser Gly Lys Thr Asp
1               5                   10                  15

Met Ala Ile Gln Ile Ala Gln Glu Thr Gly Trp Pro Val Val Ala Leu
            20                  25                  30

Asp Arg Val Gln Cys Cys Pro Gln Ile Ala Thr Gly Ser Gly Arg Pro
        35                  40                  45

Leu Glu Ser Glu Leu Gln Ser Thr Arg Arg Ile Tyr Leu Asp Ser Arg
    50                  55                  60

Pro Leu Thr Glu Gly Ile Leu Asp Ala Glu Ser Ala His Arg Arg Leu
65                  70                  75                  80

Ile Phe Glu Val Asp Trp Arg Lys Ser Glu Asp Gly Leu Ile Leu Glu
                85                  90                  95

Gly Gly Ser Ile Ser Leu Leu Asn Cys Met Ala Lys Ser Pro Phe Trp
            100                 105                 110

Arg Ser Gly Phe Gln Trp His Val Lys Arg Leu Arg Leu Gly Asp Ser
        115                 120                 125

Asp Ala Phe Leu Thr Arg Ala Lys Gln Arg Val Ala Glu Met Phe Ala
    130                 135                 140

Ile Arg Glu Asp Arg Pro Ser Leu Leu Glu Glu Leu Ala Glu Leu Trp
145                 150                 155                 160

Asn Tyr Pro Ala Ala Arg Pro Ile Leu Glu Asp Ile Asp Gly Tyr Arg
                165                 170                 175

Cys Ala Ile Arg Phe Ala Arg Lys His Asp Leu Ala Ile Ser Gln Leu
            180                 185                 190

Pro Asn Ile Asp Ala Gly Arg His Val Glu Leu Ile Glu Ala Ile Ala
        195                 200                 205

Asn Glu Tyr Leu Glu His Ala Leu Ser Gln Glu Arg Asp Phe Pro Gln
    210                 215                 220

Trp Pro Glu Asp Gly Ala Gly Gln Pro Val Cys Pro Val Thr Leu Thr
225                 230                 235                 240

Arg Ile Arg

<210>  7
<211>  238
<212>  PRT
<213>  Ralstonia solanacearum

<400>  7

Met Pro Val Arg Leu Tyr Leu Ile Trp Gly Ala Thr Thr Thr Gly Lys
1               5                   10                  15

Thr Ala Gln Ser Val Ala Leu Ala Arg Ser Ala Gly Ala Pro Val Ile
            20                  25                  30

Ser Leu Asp Arg Val Gln Cys Cys His Glu Leu Ala Val Gly Ser Gly
        35                  40                  45

Arg Pro Ser Pro Ser Glu Leu Leu Gly Thr Arg Arg Glu Tyr Leu Cys
    50                  55                  60

Glu Arg Glu Val Ser Arg Gly Val Val Ser Ala Ala Glu Ala Asn Gln
65                  70                  75                  80

Leu Leu Leu Asp Lys Val Ala Arg Tyr Ala Thr Gln Glu Arg Ala Leu
                85                  90                  95

Ile Leu Glu Gly Gly Ser Val Ser Leu Ile Asn Ala Met Ile Arg Asp
            100                 105                 110

Ala Arg Trp Ser Glu Arg Gly Glu Trp Ile Leu Arg Arg Ile Pro Val
        115                 120                 125

Pro Gly Arg Ala Ala Phe Met Ala Ala Ala Arg Lys Arg Val Arg Glu
    130                 135                 140

Met Leu Asp Pro Pro Pro Gly Gln Ala Gly Ile Leu Asp Glu Leu Gln
145                 150                 155                 160

Gly Leu Trp Gly Tyr Pro Arg Asn His Ala Val Leu Glu Asp Ile Asp
                165                 170                 175

Gly Tyr Arg Gln Ile Ile Arg Tyr Ala Asn Ala Leu Gln Val Pro Ile
            180                 185                 190

Cys Arg Ile Thr Ser Ile Asp Pro Asn Ala Lys Ala Leu Leu Ile Glu
        195                 200                 205

Arg Ile Ala Gln Glu Tyr Trp Glu His Ala Leu Trp Gln Glu Gln Glu
    210                 215                 220

Phe Leu Gly Ile Pro Ala Ser Trp Met Arg Ala Asp Asp Ala
225                 230                 235

<210>  8
<211>  249
<212>  PRT
<213>  Xanthomonas oryzae

<400>  8

Met Ser Cys Ile Leu His Leu Ile Trp Gly Pro Thr Cys Ser Gly Lys
1               5                   10                  15

Thr Glu Val Ala Val Ala Leu Ala Glu Gln Thr Gly Trp Pro Val Ile
            20                  25                  30

Ala Leu Asp Arg Val Gln Cys Cys Pro Glu Met Ser Thr Cys Ser Gly
        35                  40                  45

Arg Pro Leu Pro Ala Glu Leu Arg Ala Thr Arg Arg Ile Tyr Leu Ala
    50                  55                  60

Ser Arg Ala Val Arg Glu Gly Ile Ile Ala Ala Asp Glu Ala His Ala
65                  70                  75                  80

Leu Leu Val Asp Leu Val Asp Arg Arg Ser Ala Ala Gly Gly Val Ile
                85                  90                  95

Leu Glu Gly Gly Ser Ile Ser Leu Ile Asn Arg Met Ile Leu Asp Pro
            100                 105                 110

Tyr Trp Ala Asn Asp Trp Gln Trp Arg Ser His Arg Leu Leu Leu Gly
        115                 120                 125

Glu Cys Ala Asp Phe Leu Asp Arg Ala Leu Arg Arg Val Glu Lys Met
    130                 135                 140

Leu Arg Thr Gly Met Gly Arg Pro Ser Leu Leu Glu Glu Leu Val Ala
145                 150                 155                 160

Leu Trp Pro Asp Pro Ala Leu Arg Pro Thr Ile Glu Asp Val Asp Gly
                165                 170                 175

Tyr Arg Tyr Ser Ile Arg Phe Ala Arg Gln Trp Asn Val Pro Val Gly
            180                 185                 190

Gln Leu Leu Ser Ile Gly Asp Glu Met Lys Gln Arg Leu Ile His Gly
        195                 200                 205

Ile Ala Glu Glu Tyr Leu Gly His Ala Gln Trp Gln Glu Arg Asp Leu
    210                 215                 220

Leu Thr Leu Pro Ala Asn Trp Gln Ala Gln Asp Val Ser Met Gly Gly
225                 230                 235                 240

Ser Lys Ser Gly Leu His His Arg Leu
                245

<210>  9
<211>  243
<212>  PRT
<213>  Neorhizobium sp. NCHU2750

<400>  9

Met Leu Leu His Leu Ile Tyr Gly Pro Thr Cys Ser Gly Lys Thr Asp
1               5                   10                  15

Met Ala Ile Gln Ile Ala Gln Glu Thr Gly Trp Pro Val Val Ala Leu
            20                  25                  30

Asp Arg Val Gln Cys Cys Pro Gln Ile Ala Thr Gly Ser Gly Arg Pro
        35                  40                  45

Leu Glu Ser Glu Leu Gln Ser Thr Arg Arg Ile Tyr Leu Asp Ser Arg
    50                  55                  60

Pro Leu Thr Glu Gly Ile Leu Asp Ala Glu Ser Ala His Arg Arg Leu
65                  70                  75                  80

Ile Phe Glu Val Asp Trp Arg Lys Ser Glu Glu Gly Leu Ile Leu Glu
                85                  90                  95

Gly Gly Ser Ile Ser Leu Leu Asn Cys Met Ala Lys Ser Pro Phe Trp
            100                 105                 110

Arg Ser Gly Phe Gln Trp His Val Lys Arg Leu Arg Leu Gly Asp Ser
        115                 120                 125

Asp Ala Phe Leu Thr Arg Ala Lys Gln Arg Val Ala Glu Met Phe Ala
    130                 135                 140

Ile Arg Glu Asp Arg Pro Ser Leu Leu Glu Glu Leu Ala Glu Leu Trp
145                 150                 155                 160

Asn Tyr Pro Ala Ala Arg Pro Ile Leu Glu Asp Ile Asp Gly Tyr Arg
                165                 170                 175

Cys Ala Ile Arg Phe Ala Arg Lys His Asp Leu Ala Ile Ser Gln Leu
            180                 185                 190

Pro Asn Ile Asp Ala Gly Arg His Val Glu Leu Ile Glu Ala Ile Ala
        195                 200                 205

Asn Glu Tyr Leu Glu His Ala Leu Ser Gln Glu Arg Asp Phe Pro Gln
    210                 215                 220

Trp Pro Glu Asp Gly Ala Gly Gln Pro Val Cys Pro Val Thr Leu Thr
225                 230                 235                 240

Arg Ile Arg

<210>  10
<211>  237
<212>  PRT
<213>  Streptomyces hainanensis

<400>  10

Met His Ile Ile Ala Gly Pro Thr Gly Ile Gly Lys Ser Ala Ala Ala
1               5                   10                  15

Thr Glu Leu Ala Arg Ala Thr Gly Ala Pro Ile Val Val Ala Asp Arg

20                  25                  30

Ile Gln Cys Phe Pro Glu Leu Ala Thr Thr Ser Ala Arg Ala Gly Gly
        35                  40                  45

Glu Ala Ala Asp Val Glu Arg Ile Trp Leu Ala Glu Arg Ala Val Ala
    50                  55                  60

Asp Gly Asp Phe Ala Val Ala Asp Ala Leu Glu Ala Leu Ile Gly Thr
65                  70                  75                  80

Leu Arg Gly Leu Arg Glu Arg His Pro Phe Val Ile Val Glu Gly Gly
                85                  90                  95

Ser Ile Ser Leu Leu Trp Asp Phe Ala Glu Leu Leu Pro Glu Leu Gly
            100                 105                 110

Phe Arg Ala Thr Val Arg Leu Met His Ile Ala Asp Arg Arg Glu Tyr
        115                 120                 125

Val Ala Gly Leu Thr Arg Arg Ala Arg Gly Met Leu Val Pro Glu Gly
    130                 135                 140

Ala Ala Thr Ser Ala Ile Glu Glu Leu Ala Ile Ala Trp Lys Ser Val
145                 150                 155                 160

Glu Gln Arg Ser Phe Val Ala Ser Ile Asn Gly Phe Lys Gly Ile Leu
                165                 170                 175

Glu Trp Cys Ala Thr His Ser Phe Ala Val Glu Ser Leu Ala Ser Ala
            180                 185                 190

Ala Leu Thr Glu Glu Gln Val Gly Glu Leu Ser Arg Met Val Ala Leu
        195                 200                 205

Arg His Ala Glu His Gly Val Leu Gln Asp Arg Ile Phe Ser Met Val
    210                 215                 220

Phe Asp Gly Trp Cys Gly Glu Pro Asp Ala Val Thr Asp
225                 230                 235

<210>  11
<211>  256
<212>  PRT
<213>  Streptomyces ipomoeae

<400>  11

Met Ala Asp Thr Gly Thr His Val Glu Thr Ser Pro Leu Val His Leu
1               5                   10                  15

Ile Ala Gly Pro Thr Gly Val Gly Lys Ser Ala Ala Ala Thr Glu Leu
            20                  25                  30

Ala Arg Glu Thr Gly Ala Pro Val Val Val Ala Asp Arg Leu Gln Cys
        35                  40                  45

Phe Thr Asp Leu Ala Thr Ala Ser Ala Arg Ala Gly Ala Glu Val Pro
    50                  55                  60

Asp Val Gln Arg Tyr Trp Leu Gly Asp Arg Thr Val Ala Asp Gly Asp
65                  70                  75                  80

Leu Ser Ala Ser Ala Ala Thr Asp Ala Leu Val Ala Leu Val Glu Arg
                85                  90                  95

Leu Ala Ile Arg His Pro Leu Val Ile Val Glu Gly Gly Ser Ile Ser
            100                 105                 110

Leu Leu Arg Glu Leu Ser Glu Arg Arg Ala Ala Leu Ser Trp Arg Leu
        115                 120                 125

Thr Val Arg Leu Met Pro Leu Pro Asp Arg Ser Asp Tyr Val Ala Ala
    130                 135                 140

Leu Thr Arg Arg Ala Arg Thr Met Leu Ala Pro Pro Pro Pro Glu Arg
145                 150                 155                 160

Gly Leu Leu Asp Glu Leu Ala Thr Leu Trp Arg Asp Pro His Arg Arg
                165                 170                 175

Tyr Phe Ala Ala Ser Val Asn Gly Phe Glu Thr Val Leu Glu Cys Cys
            180                 185                 190

Ala Lys Tyr Ser Leu Asp Val Lys Thr Leu His Lys Arg Asn Leu Pro
        195                 200                 205

Glu His Ile Leu Ala Arg Met Ala Ala Leu Ile Ala Glu Arg His Ala
    210                 215                 220

Glu His Gly Ile Leu Gln His Gln Val Phe Thr Glu Ile Phe Ala Asp
225                 230                 235                 240

Arg Ile Ser Gly Ser Arg Cys Asp Leu Gly Ala Leu Glu Arg Val Ala
                245                 250                 255

<210>  12
<211>  257
<212>  PRT
<213>  Streptomyces turgidiscabies

<400>  12

Met Glu Lys Val Leu Lys Met Thr Ala Arg Ile Arg Thr Ala Arg Gly
1               5                   10                  15

Arg Gly Thr Ile Gly Val Arg Ala Leu Val Gly Pro Thr Gly Val Gly
            20                  25                  30

Lys Ser Ala Ala Ala Thr Ala Leu Ala Arg Asp Thr Gly Ala Pro Val
        35                  40                  45

Leu Val Ala Asp Arg Ile Gln Cys Phe Thr Asp Leu Val Val Thr Ser
    50                  55                  60

Gly Arg Ala Ala Asp Ala His Val Leu Glu Val Asp Arg Ile Trp Leu
65                  70                  75                  80

Ser Asp Arg Thr Val Ser Asp Gly Asp Tyr Ala Leu Gly Glu Ala Ser
                85                  90                  95

Glu Asp Leu Leu Ser Arg Leu Gly Ser Leu Val Thr Asp Arg Gly Leu
            100                 105                 110

Val Val Leu Glu Gly Gly Ser Ile Ser Leu Leu Leu Arg Phe Ala Glu

115                 120                 125

Leu Met Glu Asn Leu Pro Tyr Ser Val Ser Val Asp Leu Leu Pro Ile
    130                 135                 140

Phe Asp Lys Ser Tyr Tyr Ser Ala Arg Gln Ile Ala Arg Ala Arg Lys
145                 150                 155                 160

Met Leu Leu Pro Asp Ser Ser Gly Arg Ser Leu Leu Thr Glu Phe Ala
                165                 170                 175

Ala Ala Trp Arg Thr Pro Gly Gln Arg Arg Phe Ala Ala Ser Val Ser
            180                 185                 190

Gly Leu Asp Ser Ile Cys Glu Trp Cys Ala Lys His Ser Val Glu Pro
        195                 200                 205

Glu Glu Val Ala Asp Val Asp Leu Ser Glu Lys Met Leu Gly Glu Leu
    210                 215                 220

Ser Thr Met Leu Gly Glu Arg Tyr Ala Ala His Gly Phe Thr Gln Glu
225                 230                 235                 240

Arg Leu Phe Leu Lys Thr Phe Asp His His Val Arg Asn Ser Pro Asp
                245                 250                 255

Ala

<210>  13
<211>  256
<212>  PRT
<213>  Streptomyces turgidiscabies

<400>  13

Met Gly Glu Ile Pro Ala Asp Cys Thr Val Arg Pro Tyr Val His Leu
1               5                   10                  15

Ile Ala Gly Pro Thr Gly Ala Gly Lys Ser Ala Val Ala Asn Asp Leu
            20                  25                  30

Ala Arg Val Thr Gly Ala Pro Val Val Val Ala Asp Arg Leu Gln Cys
        35                  40                  45

Phe Thr Asp Leu Ala Thr Thr Ser Ala Arg Ala Gly Ala Gln Ala Pro
    50                  55                  60

Gly Val Arg Arg Tyr Trp Leu Ala Asp Arg Thr Val Ala Asp Gly Asp
65                  70                  75                  80

Leu Asp Ala Ala Ala Ala Ala Asp Ser Leu Phe Gly Leu Val Glu Arg
                85                  90                  95

Leu Gly Ser Arg His Pro Phe Val Ile Val Glu Gly Gly Ser Ile Ser
            100                 105                 110

Leu Leu Lys Val Leu Ala Ala Arg Cys Ala Ala Leu Pro Trp Gln Leu
        115                 120                 125

Ser Val Arg Val Leu Pro Leu Pro Ala Tyr Gly Asp Tyr Ile Glu Ala
    130                 135                 140

Leu Thr Cys Arg Ala Arg Thr Met Leu Ala Pro Pro Thr Pro Glu Arg
145                 150                 155                 160

Ser Leu Leu Glu Glu Leu Ala Val Leu Trp Ala Asp Pro Arg His Arg
                165                 170                 175

Arg Phe Ala Ala Ser Val Asn Gly Phe Glu Ala Val Leu Glu Cys Cys
            180                 185                 190

Ala Lys Tyr Ser Leu Asn Val Gly Thr Ile His Lys Gln Ala Pro Pro
        195                 200                 205

Asp Pro Ile Arg Ser Gln Met Ala Ser Leu Ile Ala Leu Arg His Ala
    210                 215                 220

Glu His Gly Ile Leu Gln Ser Arg Val Phe Thr Glu Thr Phe Gly Ser
225                 230                 235                 240

Arg Leu Pro Glu Gln Ile Leu Asp Pro Ser Val Leu Gly Arg Ala Ala
                245                 250                 255

<210>  14
<211>  243
<212>  PRT
<213>  Agrobacterium rhizogenes

<400>  14

Met Leu Leu Tyr Leu Ile Tyr Gly Pro Thr Cys Ser Gly Lys Thr Asp
1               5                   10                  15

Ile Ala Ile Gln Ile Ala Gln Lys Thr Gly Trp Pro Val Val Ala Leu
            20                  25                  30

Asp Arg Val Gln Cys Cys Pro Gln Ile Ala Thr Gly Ser Gly Arg Pro
        35                  40                  45

Leu Pro Ser Glu Leu Gln Ser Thr Arg Arg Ile Tyr Leu Asp Ser Arg
    50                  55                  60

Arg Leu Thr Lys Gly Ile Ile Asp Ala Glu Gly Ala His Arg Arg Leu
65                  70                  75                  80

Ile Leu Glu Val Asp Trp Gln Glu Ser Glu Glu Gly Leu Ile Leu Glu
                85                  90                  95

Gly Gly Ser Val Ser Leu Leu Asn Cys Met Ala Lys Ser Pro Tyr Trp
            100                 105                 110

Lys Ser Gly Phe Gln Trp His Val Lys Arg Leu Arg Leu Gly Asp Ser
        115                 120                 125

Asp Ala Phe Leu Ala Arg Ala Lys Gln Arg Val Thr Glu Met Phe Ala
    130                 135                 140

Ile Arg Glu Asp Arg Pro Ser Leu Leu Glu Glu Leu Ala Glu Leu Trp
145                 150                 155                 160

Asn Tyr Pro Ala Thr Arg Pro Ile Leu Glu Asp Ile Asp Gly Tyr Arg
                165                 170                 175

Cys Ala Ile Arg Phe Ala Arg Lys His Asp Leu Ala Ile Asn Gln Leu

180                 185                 190

Pro Asp Ile Asp Ala Glu Arg Gln Gln Asp Leu Ile Glu Ala Ile Ala
        195                 200                 205

Lys Glu Tyr Leu Glu His Ala Ile Met Gln Glu Arg Asp Phe Pro Gln
    210                 215                 220

Trp Pro Glu Asp Gly Ala Arg Gln Pro Val Gly Pro Ala Thr Leu Met
225                 230                 235                 240

Arg Ile Gln

<210>  15
<211>  240
<212>  PRT
<213>  Agrobacterium tumefaciens

<400>  15

Met Asp Leu His Leu Ile Phe Gly Pro Thr Cys Thr Gly Lys Thr Thr
1               5                   10                  15

Thr Ala Ile Ala Leu Ala Gln Gln Thr Gly Leu Pro Val Leu Ser Leu
            20                  25                  30

Asp Arg Val Gln Cys Cys Pro Gln Leu Ser Thr Gly Ser Gly Arg Pro
        35                  40                  45

Thr Val Glu Glu Leu Lys Gly Thr Thr Arg Leu Tyr Leu Asp Asp Arg
    50                  55                  60

Pro Leu Val Glu Gly Ile Ile Ala Ala Lys Gln Ala His His Arg Leu
65                  70                  75                  80

Ile Glu Glu Val Tyr Asn His Glu Ala Asn Gly Gly Leu Ile Leu Glu
                85                  90                  95

Gly Gly Ser Thr Ser Leu Leu Asn Cys Met Ala Arg Asn Ser Tyr Trp
            100                 105                 110

Ser Ala Asp Phe Arg Trp His Ile Ile Arg His Lys Leu Pro Asp Gln
        115                 120                 125

Glu Thr Phe Met Lys Ala Ala Lys Ala Arg Val Lys Gln Met Leu His
    130                 135                 140

Pro Ala Ala Gly His Ser Ile Ile Gln Glu Leu Val Tyr Leu Trp Asn
145                 150                 155                 160

Glu Pro Arg Leu Arg Pro Ile Leu Lys Glu Ile Asp Gly Tyr Arg Tyr
                165                 170                 175

Ala Met Leu Phe Ala Ser Gln Asn Gln Ile Thr Ala Asp Met Leu Leu
            180                 185                 190

Gln Leu Asp Ala Asn Met Glu Gly Lys Leu Ile Asn Gly Ile Ala Gln
        195                 200                 205

Glu Tyr Phe Ile His Ala Arg Gln Gln Glu Gln Lys Phe Pro Gln Val
    210                 215                 220

Asn Ala Ala Ala Phe Asp Gly Phe Glu Gly His Pro Phe Gly Met Tyr
225                 230                 235                 240

<210>  16
<211>  240
<212>  PRT
<213>  Agrobacterium tumefaciens

<400>  16

Met Asp Leu Arg Leu Ile Phe Gly Pro Thr Cys Thr Gly Lys Thr Ser
1               5                   10                  15

Thr Ala Val Ala Leu Ala Gln Gln Thr Gly Leu Pro Val Leu Ser Leu
            20                  25                  30

Asp Arg Val Gln Cys Cys Pro Gln Leu Ser Thr Gly Ser Gly Arg Pro
        35                  40                  45

Thr Val Glu Glu Leu Lys Gly Thr Ser Arg Leu Tyr Leu Asp Asp Arg
    50                  55                  60

Pro Leu Val Lys Gly Ile Ile Ala Ala Lys Gln Ala His Glu Arg Leu
65                  70                  75                  80

Met Gly Glu Val Tyr Asn Tyr Glu Ala His Gly Gly Leu Ile Leu Glu
                85                  90                  95

Gly Gly Ser Ile Ser Leu Leu Lys Cys Met Ala Gln Ser Ser Tyr Trp
            100                 105                 110

Ser Ala Asp Phe Arg Trp His Ile Ile Arg His Glu Leu Ala Asp Glu
        115                 120                 125

Glu Thr Phe Met Asn Val Ala Lys Ala Arg Val Lys Gln Met Leu Arg
    130                 135                 140

Pro Ala Ala Gly Leu Ser Ile Ile Gln Glu Leu Val Asp Leu Trp Lys
145                 150                 155                 160

Glu Pro Arg Leu Arg Pro Ile Leu Lys Glu Ile Asp Gly Tyr Arg Tyr
                165                 170                 175

Ala Met Leu Phe Ala Ser Gln Asn Gln Ile Thr Ser Asp Met Leu Leu
            180                 185                 190

Gln Leu Asp Ala Asp Met Glu Asp Lys Leu Ile His Gly Ile Ala Gln
        195                 200                 205

Glu Tyr Leu Ile His Ala Arg Arg Gln Glu Gln Lys Phe Pro Arg Val
    210                 215                 220

Asn Ala Ala Ala Tyr Asp Gly Phe Glu Gly His Pro Phe Gly Met Tyr
225                 230                 235                 240

<210>  17
<211>  240
<212>  PRT
<213>  Agrobacterium vitis

<400>  17

Met Asp Leu Arg Leu Ile Phe Gly Pro Thr Cys Thr Gly Lys Thr Ser
1               5                   10                  15

Thr Ala Ile Ala Leu Ala Gln Gln Thr Gly Leu Pro Val Leu Ser Leu
            20                  25                  30

Asp Arg Val Gln Cys Cys Pro Gln Leu Ser Thr Gly Ser Gly Arg Pro
        35                  40                  45

Thr Val Glu Glu Leu Lys Gly Thr Thr Arg Leu Tyr Leu Asp Asp Arg
    50                  55                  60

Pro Leu Val Lys Gly Ile Ile Ala Ala Glu Gln Ala His Glu Arg Leu
65                  70                  75                  80

Ile Ala Glu Val Tyr Asn Tyr Glu Ala His Gly Gly Leu Ile Leu Glu
                85                  90                  95

Gly Gly Ser Ile Ser Leu Leu Lys Cys Met Ala Gln Ser Gly Tyr Trp
            100                 105                 110

Ser Ala Asp Phe Arg Trp His Ile Ile Arg His Lys Leu Ala Asp Glu
        115                 120                 125

Glu Thr Phe Met Lys Ala Ala Lys Ala Arg Val Lys Gln Met Leu Cys
    130                 135                 140

Pro Ala Ile Gly Pro Ser Leu Ile Gln Glu Leu Val Tyr Leu Trp Asn
145                 150                 155                 160

Glu Pro Arg Leu Arg Pro Ile Leu Lys Glu Ile Asp Gly Tyr Arg Tyr
                165                 170                 175

Ala Met Leu Phe Ala Ser Gln Asn Arg Ile Thr Pro Asp Met Leu Leu
            180                 185                 190

Gln Leu Asp Ala Asp Met Glu Gly Lys Leu Ile His Gly Ile Ala Gln
        195                 200                 205

Glu Tyr Leu Ile His Ala Arg Arg Gln Glu His Glu Phe Pro Pro Val
    210                 215                 220

Ser Ala Ala Ala Phe Glu Gly Phe Glu Gly Pro Pro Phe Gly Ala Tyr
225                 230                 235                 240

<210>  18
<211>  236
<212>  PRT
<213>  Agrobacterium vitis

<400>  18

Met Glu Ala His Leu Ile Phe Gly Pro Thr Ser Thr Gly Lys Thr Ser
1               5                   10                  15

Val Ala Ile Ala Leu Ala Lys Arg Thr Gly Phe Pro Val Ile Val Leu
            20                  25                  30

Asp Arg Ile Gln Cys Tyr Ser Gln Leu Ser Val Gly Gly Gly Arg Pro
        35                  40                  45

Ser Ala Ala Glu Phe Gln Gly Thr Arg Arg Ile Tyr Leu Ile Glu Gly
    50                  55                  60

Ser Leu Asp Glu Gly Val Ile Ser Ala Glu Arg Ala His Glu Cys Leu
65                  70                  75                  80

Val Ala Ala Val Glu Ala His Lys Pro Glu Gly Gly Val Ile Leu Glu
                85                  90                  95

Gly Gly Ser Ile Ser Leu Phe Lys Arg Met Ala Gln Ser Ser Tyr Trp
            100                 105                 110

Asn Cys Gly Phe Thr Trp His Val Thr Arg Leu His Leu Gly Gly Glu
        115                 120                 125

Glu Ile Phe Leu Ala Ala Ala Lys Lys Arg Ile Asn Gln Met Met Gln
    130                 135                 140

Pro Asp Glu Gln Gly Asn Ser Phe Leu Gly Glu Leu Val Ser Val Trp
145                 150                 155                 160

Lys Thr Thr Ala Leu Arg Ala Thr Leu Glu Gly Ile Cys Gly Tyr Arg
                165                 170                 175

Tyr Ala Ile Glu Phe Ala Gly Lys Gln Gly Leu Glu Met Asp Ala Leu
            180                 185                 190

Thr Ser Leu Asn Arg Arg Gln Leu Glu Gln Leu Val His Gly Met Ala
        195                 200                 205

His Glu Tyr Leu Cys Tyr Ala Arg Gln Gln Glu Gln Glu Leu Pro Leu
    210                 215                 220

Pro Ser Leu Ala Gly Gly Glu Gly Pro Pro Phe Gln
225                 230                 235

<210>  19
<211>  240
<212>  PRT
<213>  Halomonas anticariensis

<400>  19

Met Arg Arg His Tyr Ile Leu Gly Thr Thr Ala Ser Gly Lys Thr Ser
1               5                   10                  15

Ala Ser Ile Ser Leu Ala Glu Met Val Asn Ala Pro Val Val Val Leu
            20                  25                  30

Asp Arg Ile Gln Cys Phe Gln Glu Leu Trp Thr Val Ser Ser Arg Pro
        35                  40                  45

Gln Asp Ser Glu Leu Gly Ala Thr Lys Arg Phe Tyr Leu Asp Asp Arg
    50                  55                  60

Glu Ile Ser His Gly Asn Leu Gly Val Glu Asp Ala Tyr Thr Lys Leu
65                  70                  75                  80

Val Asn Ile Val Asp Lys Leu Ser Asn Tyr His Ser Phe Val Ile Leu
                85                  90                  95

Glu Gly Gly Ser Val Ser Leu Cys Arg Leu Ile Val Glu Arg Ala Glu
            100                 105                 110

Leu Cys Arg Gly Ser Thr Val Glu Ile Arg Trp Asn Asn Pro Asp Asp
        115                 120                 125

Pro Ala Tyr Val Gln Lys Ile Arg Glu Arg Val Val Gln Met Met Glu
    130                 135                 140

Pro Arg Val Asp Arg Arg Ser Ile Val Asp Glu Leu Lys Tyr Ala Trp
145                 150                 155                 160

Asp Tyr Lys Glu Gln Arg Asp Phe Ile Thr Ser Ile Cys Gly Phe Asp
                165                 170                 175

Gly Ile Leu Ser Trp Cys Asp Asp Asn Asn Phe His Ile Glu Asp Val
            180                 185                 190

Asp Phe Glu Cys Leu Pro Leu Ser Ala Ile Gly Glu Ile Thr Asp Arg
        195                 200                 205

Val Ala Glu Ser His Arg Lys Tyr Ala Arg Thr Gln Phe Glu Met Leu
    210                 215                 220

Asn Ile Met Glu Gln Ala Met Ile Ala Thr Gly Val Lys Val Val Arg
225                 230                 235                 240

<210>  20
<211>  233
<212>  PRT
<213>  Lonsdalea quercina

<400>  20

Met Asp Thr Thr Leu His Leu Ile Trp Gly Pro Thr Ser Thr Gly Lys
1               5                   10                  15

Thr Ala Arg Ser Val Ala Ile Ala Glu Lys Thr Gly Ala Pro Val Leu
            20                  25                  30

Val Leu Asp Arg Ile Gln Cys Cys Pro Glu Val Thr Met Gly Ser Gly
        35                  40                  45

Arg Pro Thr Glu Gln Glu Leu Arg Gly Thr Glu Arg Val Tyr Leu Ser
    50                  55                  60

Ser Arg Pro Ile Glu Gln Ser Ile Ile Ser Ala Pro Gln Ala Val Glu

65                  70                  75                  80

Arg Leu His Gln Leu Val Asp His Phe Ala Lys Ile His Pro Leu Ile
                85                  90                  95

Ile Leu Glu Gly Gly Ser Val Ser Ile Leu Gln Glu Met Val Lys His
            100                 105                 110

Gln Thr Trp Gly Gln Gln Tyr Ala Trp Thr Phe Glu Pro Ile Pro Met
        115                 120                 125

Pro Ser Ala Glu His Phe Leu Asn Arg Ala Arg Lys Arg Ile Trp Glu
    130                 135                 140

Met Phe Tyr Pro Gly Gly Gly Leu Lys Ser Leu Leu Asp Glu Met Val
145                 150                 155                 160

Gln Ile Trp Ala His Pro Glu Tyr Arg Asp Thr Leu Ala Asp Ile Asp
                165                 170                 175

Gly Tyr Arg Cys Ile Ile Ser Tyr Leu Arg Gln Arg Gly Arg Arg Val
            180                 185                 190

Asp Glu Ile Pro Arg Met Ser Leu His Glu His Lys Gln Met Ala Glu
        195                 200                 205

Asp Ile Ala Gln Glu Tyr Trp Gln His Ala Val Trp Gln Thr Asp Asn
    210                 215                 220

Phe Pro Glu Ile Pro Pro Cys Trp Arg
225                 230

<210>  21
<211>  238
<212>  PRT
<213>  Ralstonia solanacearum

<400>  21

Met Pro Ile Arg Leu Tyr Leu Ile Trp Gly Ala Thr Thr Thr Gly Lys
1               5                   10                  15

Thr Ala Gln Ser Val Ala Leu Ala Arg Ala Thr Gly Ala Pro Val Ile
            20                  25                  30

Ser Leu Asp Arg Val Gln Cys Cys His Glu Leu Ala Val Gly Ser Gly
        35                  40                  45

Arg Pro Leu Pro Ser Glu Leu Leu Gly Thr Arg Arg Glu Tyr Leu Cys
    50                  55                  60

Asp Arg Ser Val Ser Ser Gly Val Ile Pro Ala Ala Asp Ala Asn Gln
65                  70                  75                  80

Leu Leu Phe Asp Lys Val Val Arg Tyr Ser Ala His Glu Arg Ala Leu
                85                  90                  95

Ile Leu Glu Gly Gly Ser Val Ser Leu Ile Asn Ala Met Ile Arg Asp
            100                 105                 110

Ala Arg Trp Ser Ala Leu Gly Glu Trp Ala Leu Arg Arg Ile Val Leu
        115                 120                 125

Pro Gly Arg Ala Val Phe Ile Ala Gln Ala Ile Lys Arg Val Arg Asp
    130                 135                 140

Met Leu Glu Pro Pro Pro Gly His Ala Ser Ile Leu Asp Glu Leu Glu
145                 150                 155                 160

Gly Leu Trp Ala His Pro Gly Asn His Ala Val Leu Glu Asp Val Asp
                165                 170                 175

Gly Tyr Arg Gln Ile Ile Arg Tyr Ala Arg Ala Ser Gln Val Pro Ile
            180                 185                 190

Asp Arg Ile Thr Ser Ile Asp Arg Ser Thr Met Ala Thr Leu Val Glu
        195                 200                 205

Arg Ile Ala Gln Glu Tyr Trp Glu His Ala Leu Trp Gln Glu Gln Glu
    210                 215                 220

Phe Leu Gly Ile Pro Ala Thr Trp Arg Arg Ala Asp Asp Ala
225                 230                 235

<210>  22
<211>  239
<212>  PRT
<213>  Rhizobium radiobacter

<400>  22

Met Asp Leu Arg Leu Ile Phe Gly Pro Thr Cys Thr Gly Lys Thr Ser
1               5                   10                  15

Thr Ala Ile Ala Leu Ala Gln Gln Thr Gly Leu Pro Val Leu Ser Leu
            20                  25                  30

Asp Arg Val Gln Cys Cys Pro Gln Leu Ser Thr Gly Ser Gly Arg Pro
        35                  40                  45

Thr Val Glu Glu Leu Lys Gly Thr Thr Arg Leu Tyr Leu Asp Asp Arg
    50                  55                  60

Pro Leu Val Lys Gly Ile Ile Thr Ala Lys Gln Ala His Glu Arg Leu
65                  70                  75                  80

Ile Ala Glu Val His Asn His Glu Ala Lys Gly Gly Leu Ile Leu Glu
                85                  90                  95

Gly Gly Ser Ile Ser Leu Leu Arg Cys Met Ala Gln Ser Arg Tyr Trp
            100                 105                 110

Asn Ala Asp Phe Arg Trp His Ile Ile Arg Asn Glu Leu Ala Asp Glu
        115                 120                 125

Glu Ser Phe Met Ser Val Ala Lys Thr Arg Val Lys Gln Met Leu Arg
    130                 135                 140

Pro Ser Ala Gly Leu Ser Ile Ile Gln Glu Leu Val Gln Leu Trp Arg
145                 150                 155                 160

Glu Pro Arg Leu Arg Pro Ile Leu Glu Gly Ile Asp Gly Tyr Arg Tyr
                165                 170                 175

Ala Leu Leu Phe Ala Thr Gln Asn Gln Ile Thr Pro Asp Met Leu Leu

            180                 185                 190

Gln Leu Asp Ala Asp Met Glu Asn Lys Leu Ile His Gly Ile Ala Gln
        195                 200                 205

Glu Phe Leu Ile His Ala Arg Arg Gln Glu Gln Lys Phe Pro Leu Val
    210                 215                 220

Gly Ala Thr Ala Val Glu Ala Phe Glu Gly Pro Pro Phe Arg Met
225                 230                 235

<210> 23
<211> 252
<212> PRT
<213> Rhizobium tumorigenes

<400> 23

Met Leu Ile Tyr Leu Ile Tyr Gly Pro Thr Cys Ser Gly Lys Thr Asp
1               5                   10                  15

Leu Ala Ile Gln Ile Ala Arg Glu Thr Gly Trp Pro Val Ile Ala Leu
            20                  25                  30

Asp Arg Val Gln Cys Cys Pro Gln Ile Ala Thr Gly Ser Gly Arg Pro
        35                  40                  45

Leu Glu Ser Glu Leu Gln Ser Thr Gln Arg Ile Tyr Leu Asp Ser Arg
    50                  55                  60

Pro Leu Thr Glu Gly Val Met Glu Ala Glu Ala Ala His Leu Gln Leu
65                  70                  75                  80

Ile Ser Ala Ile Glu Asp Arg Lys Ser Ser Pro Gly Leu Ile Leu Glu
                85                  90                  95

Gly Gly Ser Ile Ser Leu Leu Asn Cys Met Ala Lys Asp Ser Tyr Trp
            100                 105                 110

Asn Ala Gly Phe Arg Trp His Val Arg Arg Leu Arg Leu Pro Thr Pro
        115                 120                 125

Asp Val Phe Leu Pro Arg Ala Arg Leu Arg Val Thr Glu Met Phe Ala
    130                 135                 140

Ile Lys Asp Glu Arg Pro Ser Leu Leu Glu Glu Leu Ala Asp Leu Trp
145                 150                 155                 160

Asn Gln Glu Val Leu Arg Pro Val Leu Glu Asp Ile Asp Gly Tyr Arg
                165                 170                 175

Cys Ala Ile Gln Phe Ala Arg Glu Asn Asn Ile Glu Ile Gly Glu Leu
            180                 185                 190

Leu His Leu Asp Pro Met Arg Arg Glu Ala Leu Ile Ala Ala Ile Ala
        195                 200                 205

Glu Glu Tyr Leu Glu His Ala Arg Trp Gln Glu Arg Glu Phe Pro Asp
    210                 215                 220

Trp Gln Glu Gly Gly Asn Val Gly Leu Thr Pro Leu Pro Ala Ser His
225                 230                 235                 240

Asp Thr Gly Ser Pro Arg Asp Leu Arg Ser Leu Gly
                245                 250

<210>  24
<211>  236
<212>  PRT
<213>  Streptomyces bingchenggensis

<400>  24

Met His Val Ile Ala Gly Pro Thr Gly Val Gly Lys Ser Ala Ala Ala
1               5                   10                  15

Thr Ala Leu Ala Arg Ile Thr Gly Ala Pro Ile Val Val Ala Asp Arg
            20                  25                  30

Ile Gln Cys Phe Thr Asp Leu Ala Thr Thr Ser Ala Arg Ala Gly Glu
        35                  40                  45

Glu Thr Val Glu Val Glu Arg Ser Trp Leu Gly Asp Arg Thr Val Ala
    50                  55                  60

Asp Gly Asp Tyr Pro Pro Glu Glu Ala Ala Asp Ala Leu Val Arg Arg
65                  70                  75                  80

Leu Ala Glu Leu Gly Glu Arg His Arg Phe Val Ile Val Glu Gly Gly
                85                  90                  95

Ser Ile Ser Leu Leu Arg Asp Phe Ala Glu Arg Leu Pro Asp Leu Pro
            100                 105                 110

Tyr Arg Leu Thr Val Arg Leu Leu His Ile Pro Asp Arg Glu Gly Tyr
        115                 120                 125

Val Val Arg Leu Ala Arg Arg Ala Arg Arg Met Leu Ala Pro Gly Gly
    130                 135                 140

Ser Gln Thr Ser Met Leu Glu Glu Leu Ala Ala Leu Trp Glu Ser Pro
145                 150                 155                 160

Asp His Arg Phe Phe Ala Ala Ser Val Asn Gly Leu Glu Ala Ile Leu
                165                 170                 175

Glu Trp Cys Ala Thr Tyr Ser Val Arg Val Glu Ser Leu Ala Ser Tyr
            180                 185                 190

Glu Leu Ser Glu Ala Gln Leu Glu Lys Met Ala Trp Met Val Ala Glu
        195                 200                 205

Arg His Ala Glu His Gly Ile Leu Gln Glu Arg Ile Phe Ser Lys Val
    210                 215                 220

Phe Asp Gly Ile Pro Ser Ala Val Tyr Gly Pro Ala
225                 230                 235

<210>  25
<211>  246
<212>  PRT
<213>  Streptomyces glauciniger

<400>  25

Met Ile Ser Leu His Leu Ile Leu Gly Pro Thr Gly Val Gly Lys Thr
1               5                   10                  15

Ser Arg Ser Val Ala Leu Ala Leu Arg His Glu Thr Pro Val Leu Val
            20                  25                  30

Leu Asp Arg Val Gln Cys His Pro Gln Leu Ala Ile Gly Ser Gly Arg
        35                  40                  45

Pro Thr Gln Val Glu Leu Gln Asp Thr Val Arg Tyr Tyr Leu Thr Glu
    50                  55                  60

Arg Pro Leu His His Gly Val Ile Thr Ala Ala Asp Ala Val Asp Arg
65                  70                  75                  80

Leu Ile His His Arg Lys Met Ile His Asp Ser Gly Val Asp Arg Leu
                85                  90                  95

Ile Leu Glu Gly Gly Ser Ile Ser Ile Leu Ala Glu Leu Leu Thr Arg
            100                 105                 110

Thr Asp Trp Tyr Glu Asp Ala Thr Met Ala Val Glu Cys Trp Leu Glu
        115                 120                 125

Ala Ser Ala Ser Ala Tyr Glu Ala Arg Val Thr Glu Arg Val Glu His
    130                 135                 140

Met Leu Gly Tyr Ser Asp His Ser Gly Arg Thr Met Leu Asp Glu Leu
145                 150                 155                 160

Ala Glu Leu Trp Leu His Pro Glu Ala Arg Pro Arg Leu His Ser Ile
                165                 170                 175

Ala Gly Tyr Arg Glu Val Ile Asp Leu Cys Ala Ala Glu Gly Leu Asp
            180                 185                 190

Pro Met Tyr Leu Arg Gly Leu Ala Ala Arg Leu Arg Arg Thr Gln Cys
        195                 200                 205

Ala Thr Ala Ile Thr Ser Ala His Leu Asp Tyr Ala Arg Arg Gln Arg
    210                 215                 220

Leu Ala Leu Ala Thr Ala Leu Pro Glu Leu Val Gln Arg Gly Phe Glu
225                 230                 235                 240

Val Thr Leu Cys Glu Ile
                245

<210>  26
<211>  294
<212>  PRT
<213>  Streptomyces hainanensis

<400>  26

Met Ala Arg Ser Gly Arg Arg Ser Leu Ala Arg Ser Glu Arg Ser Arg
1               5                   10                  15

Thr Ser Leu Asn Val Ser Thr His Arg Pro Ile Gly Arg Pro Arg Ser
            20                  25                  30

Arg Ala Pro Ile Ala Thr Arg Glu Glu Pro Thr Val Arg His Ile Ala
        35                  40                  45

Ala Glu Val Pro Leu Thr Val Arg Leu Ile Val Gly Pro Thr Gly Val
    50                  55                  60

Gly Lys Ser Ala Ala Ala Ser Asp Val Ala Arg Arg Thr Gly Ala Pro
65                  70                  75                  80

Val Val Val Ala Asp Arg Ile Gln Cys Phe Thr Asp Leu Ala Thr Thr
                85                  90                  95

Ser Ala Arg Ala Gly Ala Asp Glu Pro Asp Val Thr Arg Ala Trp Leu
            100                 105                 110

Gly Asp Arg Thr Val Ala Asp Gly Asp Tyr Pro Ala Pro Glu Ala Ala
        115                 120                 125

Ala Ala Leu Val Arg Ala Val Arg Arg Val Ala Arg Pro Gly Arg Pro
    130                 135                 140

Val Val Val Glu Gly Gly Ser Ile Ser Leu Leu Ala Tyr Leu Val Asp
145                 150                 155                 160

His His Leu Pro Val Leu Pro Trp Arg Phe Thr Val Thr Ser Leu Thr
                165                 170                 175

Arg Pro Ala Pro Ser Arg Tyr Leu Ala Ala Leu Thr Ala Arg Ala His
            180                 185                 190

Ala Met Leu Arg Pro Glu Pro Pro Leu Ala Gly Leu Leu Asp Glu Leu
        195                 200                 205

Ala Ala Leu Trp Arg Val Pro Gly His Arg Trp Phe Ala Ala Ser Val
    210                 215                 220

Asn Gly Phe Glu Ala Ala Leu Glu Trp Cys Ala Lys Tyr Gly Leu Asp
225                 230                 235                 240

Pro Thr Glu Pro Thr Leu Val Gly Leu Pro Pro Arg Leu Val Asp Glu
                245                 250                 255

Leu Ala Leu Leu Ile Ala Glu Arg His Ala Glu His Gly Ala Asp Gln
            260                 265                 270

Glu Arg Ala Phe Ala Ala Leu Phe Ala Pro Ala Ala Arg Ala Gly Val
        275                 280                 285

Arg Leu Gly Asp Ala Ala
    290

<210>  27
<211>  256
<212>  PRT
<213>  Streptomyces sp. NRRL WC-3618

<400>  27

Met Ala Glu Ile Ser Asn Asp Arg Thr Ala Arg Pro Phe Val His Leu
1               5                   10                  15

Ile Ala Gly Pro Thr Gly Ala Gly Lys Ser Ala Ala Ala Asn Glu Leu
            20                  25                  30

Ala Arg Val Thr Gly Ala Pro Val Val Val Ala Asp Arg Leu Gln Cys
        35                  40                  45

Phe Thr Asp Leu Val Thr Thr Ser Ala Arg Ala Gly Ala Glu Ala Arg
    50                  55                  60

Gly Val Arg Arg Tyr Trp Leu Ala Asp Arg Thr Val Ala Asp Gly Asp
65                  70                  75                  80

Leu Gly Ala Val Ala Ala Ala Asp Ser Leu Val Gly Leu Val Glu Arg
                85                  90                  95

Leu Gly Arg Gln His Arg Phe Val Ile Val Glu Gly Gly Ser Ile Ser
            100                 105                 110

Leu Leu Lys Ala Leu Ala Glu Arg Arg Ala Thr Leu Pro Trp Gln Leu
        115                 120                 125

Ser Val Arg Val Leu Pro Leu Pro Ala Tyr Gly Asp Tyr Val Glu Ala
    130                 135                 140

Leu Thr Arg Arg Ala Gln Thr Met Ile Ala Pro Pro Thr Pro Asp Pro
145                 150                 155                 160

Ser Leu Leu Glu Glu Leu Ala Thr Leu Trp Ala Asp Pro Arg Leu Arg
                165                 170                 175

Arg Phe Ala Ala Ser Val Asn Gly Phe Glu Ala Val Leu Glu Ile Cys
            180                 185                 190

Ala Lys Tyr Ser Leu Asp Val Glu Thr Ile His Lys Gln Val Pro Pro
        195                 200                 205

Ala Pro Val Leu Ser Gln Met Ala Ser Leu Ile Ala Leu Arg His Ala
    210                 215                 220

Glu His Gly Ile Leu Gln Ser Tyr Val Phe Thr Glu Ile Phe Glu Glu
225                 230                 235                 240

Gln Ile Pro Val Gln Val Ser Asp Pro Gly Val Leu Val Arg Ala Ala
                245                 250                 255

<210>  28
<211>  239
<212>  PRT
<213>  Trinickia caryophylli

<400>  28

Met Thr Ile Arg Leu Tyr Met Ile Trp Gly Pro Thr Thr Thr Gly Lys
1               5                   10                  15

Thr Ala Leu Ser Val Ala Leu Ala Arg Met Val Gly Val Pro Val Leu
            20                  25                  30

Ser Leu Asp Arg Val Gln Cys Cys His Glu Ile Ala Val Gly Ser Gly
        35                  40                  45

Arg Pro Ser Pro Glu Glu Leu Glu Gly Thr Ala Arg Glu Tyr Leu Cys
    50                  55                  60

His Arg Arg Val Ala Asp Gly Val Val Ser Ala Ala Glu Ala Asn Arg
65                  70                  75                  80

Leu Leu Lys Ala Lys Val Leu Asp Tyr Ser Arg His Leu Pro Ala Leu
                85                  90                  95

Ile Leu Glu Gly Gly Ser Val Ser Leu Phe Asn Glu Met Thr Ala Asp
            100                 105                 110

Asp Ser Trp Ala His Ile Ala Glu Trp Val Leu Gln Arg Val Pro Leu
        115                 120                 125

Pro Ala Gln Asp Val Phe Met Lys Arg Ala Arg Gly Arg Ala Arg Ala
    130                 135                 140

Met Leu Asp Ala Pro Ala Gly Glu Arg Ser Met Leu Asp Glu Leu Val
145                 150                 155                 160

Asp Leu Trp Arg Asp Pro Arg Thr His Ala Val Leu Glu Asp Ile Asp
                165                 170                 175

Gly Tyr Arg Gln Leu Ile Gly Tyr Ala Arg Ala Ala Gly Ile Pro Val
            180                 185                 190

Val Glu Leu Ser Asn Ala Ala Pro Glu Ile Phe Asp Asp Trp Val Glu
        195                 200                 205

Leu Ile Ala Arg Glu Tyr Trp Glu His Ala Cys Trp Gln Glu Gln Arg
    210                 215                 220

Phe Ala Ser Pro Pro Thr Cys Trp Arg His Val Ala His Ala Trp
225                 230                 235

<210>  29
<211>  238
<212>  PRT
<213>  Xanthomonas albilineans

<400>  29

Met Asn Gly Ala Leu Tyr Leu Ile Trp Gly Pro Thr Cys Ser Gly Lys
1               5                   10                  15

Thr Ala Leu Ala Val Ala Leu Ala Glu Gln Ser Gly Trp Pro Val Ile
            20                  25                  30

Ala Leu Asp Arg Ile Gln Cys Tyr Pro Glu Leu Ala Thr Gly Ser Gly
        35                  40                  45

Arg Pro Leu Ser Ser Glu Leu Arg Gly Thr Arg Arg Ile Tyr Leu Ala
    50                  55                  60

Ser Arg Ala Val Arg Glu Gly Ile Ile Ala Ala Asp Glu Ala His Ala
65                  70                  75                  80

Leu Leu Lys Asp Met Val Gly Arg His Ser Ala Ala Gly Gly Val Ile
                85                  90                  95

Leu Glu Gly Gly Ser Ile Ser Leu Ile Asn Arg Met Ile Ala Asp Pro
            100                 105                 110

Tyr Trp Ala Ser Gly Trp Gln Trp His Ser Arg Arg Leu Arg Leu Gly
        115                 120                 125

Asp Pro Ala Ala Phe Leu Asp Arg Ala Leu Arg Arg Val Glu Gln Met
    130                 135                 140

Leu Arg Thr His Gln Glu His Pro Ser Leu Leu Glu Glu Leu Val Ala
145                 150                 155                 160

Leu Trp Pro Asp Pro Ala Leu Arg Pro Ile Leu Glu Asp Val Asp Gly
                165                 170                 175

Tyr Arg Tyr Ala Ile Arg Phe Ala Arg His Trp Ser Leu Pro Val Ala
            180                 185                 190

Asp Leu Leu Ser Met Arg Asp Glu Met Lys Gln Arg Leu Ile Arg Gly
        195                 200                 205

Ile Ala Glu Glu Tyr Leu Glu His Ala Gln Trp Gln Glu Arg Asp Leu
    210                 215                 220

Leu Ala Leu Pro Ala Ser Trp Gln Ala Gln Glu Leu Ser Thr
225                 230                 235

<210>  30
<211>  238
<212>  PRT
<213>  Chondromyces crocatus

<400>  30

Met Lys Ala His Cys Ile Val Gly Ala Thr Gly Thr Gly Lys Thr Ala
1               5                   10                  15

Arg Ala Thr Ser Leu Ala Arg Gln Thr Gly Ala Pro Val Leu Val Leu
            20                  25                  30

Asp Arg Phe Gln Val Phe Glu Asp Leu Ala Ser Gly Thr Gly Arg Pro
        35                  40                  45

Leu Ser Arg Glu Val Ala Gly Thr Thr Arg Leu Tyr Leu Gly His Arg
    50                  55                  60

Tyr Met Met Asp Gly Glu Leu Thr Arg Gln Asp Ala Leu Leu Asp Phe
65                  70                  75                  80

Leu Arg Thr Val Gly Lys Leu Ala Val Asp His Asp Phe Ile Ile Leu

85                  90                  95

Glu Gly Gly Ser Ile Ser Leu Asn Glu Ala Leu Ile Glu Glu Ser Leu
            100                 105                 110

Leu Gly Thr Thr Gly Val Thr Val Glu Arg Met Arg Met Thr Ser Val
        115                 120                 125

Lys Ala Tyr Glu Gln Gly Leu Arg Arg Arg Ala Arg Met Met Leu Thr
    130                 135                 140

Pro Ser Ala Glu Arg Pro Ser Met Ile Glu Glu Leu Ala Arg Gly Trp
145                 150                 155                 160

Asp Asn Pro Arg Leu Arg Ala Phe Leu Ser Ser Ile Thr Gly Tyr Asp
                165                 170                 175

Ala Ile Val Asp His Cys Glu Gln Ala Gly Ile Glu Pro Ala Glu Leu
            180                 185                 190

Ala Ala Gly Pro Val Asp Pro Val Leu Ile Asp Glu Val Val Leu Ser
        195                 200                 205

His Met Lys Tyr Ala Ala Lys Gln Thr Ala Ala Phe Asn Gln Leu Phe
    210                 215                 220

Pro Glu Gln Ser Ser Phe Pro Gly Cys Gln Pro Cys Thr Ala
225                 230                 235

<210>  31
<211>  239
<212>  PRT
<213>  Dickeya dianthicola

<400>  31

Met Ser Thr Ile Leu Tyr Leu Leu Trp Gly Pro Thr Ser Thr Gly Lys
1               5                   10                  15

Thr Ser His Ser Val Ile Leu Ala Lys Lys Tyr Gly Phe Pro Val Ile
            20                  25                  30

Ala Leu Asp Arg Phe Gln Gly Tyr Gln Glu Ile Lys Thr Gly Ser Gly
        35                  40                  45

Ala Pro Asp Gln Ala Glu Leu Gln Gly Thr Glu Arg Ile Tyr Ile Thr
    50                  55                  60

Pro Ser His His Leu Thr Glu Ser Val Val Ser Ser Glu Glu Ala His
65                  70                  75                  80

Asn Ile Leu Lys Lys Lys Ile Ser Ile Leu Leu Asn Arg Asn Ser Ser
                85                  90                  95

Val Ile Leu Glu Gly Gly Ser Val Ser Leu Leu Thr Lys Ile Val Met
            100                 105                 110

Asp Asp Tyr Trp Ser Gln Phe Ser Trp Arg Ile Arg Arg Phe His Met
        115                 120                 125

Ile Ser Lys Lys Asp Phe Ile Glu Lys Ala Gln Lys Arg Val Thr Ser
    130                 135                 140

Met Ile Tyr Pro Gln Asn Gly Gly Ser Ser Ile Leu Lys Glu Thr Ser
145                 150                 155                 160

Leu Phe Phe Arg Lys Asn Leu Thr Ile Glu Pro Leu Glu Asp Ile Asp
                165                 170                 175

Gly Tyr Arg Lys Ile Ile Gln Tyr Cys Lys Glu Lys Asn Ile Asp Phe
            180                 185                 190

Asp Ser Leu Asp Lys Leu Asn Ser Asn Glu Lys Asp Asn Leu Ile Asp
        195                 200                 205

Ile Ile Ser Asn Glu Tyr Tyr Glu His Ala Leu Trp Gln Glu Lys Asn
    210                 215                 220

Phe Pro Gly Phe Pro Ala Ser Trp Ser Trp Glu Thr Leu Lys Asn
225                 230                 235

<210>  32
<211>  244

<212>  PRT
<213>  Nostoc sp.

<400>  32

Met Arg Leu His Ile Ile Leu Gly Pro Thr Ser Val Gly Lys Thr Asp
1               5                   10                  15

Arg Ser Val Val Leu Ala Lys Gln Thr Lys Ala Pro Val Ile Val Leu
            20                  25                  30

Asp Arg Ile Gln Ile Tyr Gln Glu Ile Ala Thr Gly Ser Gly Arg Pro
        35                  40                  45

Leu Ile Asp Glu Leu Glu Gly Thr Thr Arg Ile Tyr Leu Glu Glu Arg
    50                  55                  60

Gln Leu Ala Asp Gly Asn Leu Asn Thr Leu Glu Ser Leu Ser Leu Ala
65                  70                  75                  80

Leu Gln His Ile Asp Arg Leu Ser Ser Gln His Lys Leu Leu Ile Leu
                85                  90                  95

Glu Gly Gly Ser Ile Ser Leu Cys Thr Ala Leu Trp Lys Ser Arg Ile
            100                 105                 110

Leu Glu Asn Tyr Gln Thr Thr Ile Glu Tyr Val Lys Val Glu Asn Glu
        115                 120                 125

Glu Leu Tyr Gln Ser Arg Leu Trp Arg Arg Met Gln Asn Ala Leu Ile
    130                 135                 140

Ser Asn Pro Arg Arg Pro Ser Leu Ile Glu Glu Leu Ser Arg Val Trp
145                 150                 155                 160

Gln Asp Pro His Lys Leu Ala Leu Val Arg Thr Val Val Gly Tyr Asp
                165                 170                 175

Val Leu Ile His Trp Cys Gln Lys Tyr Gly Leu Ser Pro Asp Gln Met
            180                 185                 190

Trp Lys Ser Phe Gln Asp Asn Ser Phe Tyr Ile Asn Leu Met Gln Glu

195                 200                 205

Met Phe Leu Ala Tyr Met Gln Tyr Ser Gln Asn Gln Arg Gln Ala Phe
    210                 215                 220

Asp Gln Leu Ala Val Glu Tyr Lys Gln Gln Gln Ala Leu Leu Ala Ser
225                 230                 235                 240

Thr Val Thr Leu

<210>  33
<211>  283
<212>  PRT
<213>  Dictyostelium discoideum

<400>  33

Met Lys Ile Leu Leu Ile Ile Gly Ser Thr Gly Val Gly Lys Thr Asp
1               5                   10                  15

Leu Ser Ile Asn Tyr Ser Lys Lys Tyr Asn Ala Pro Val Val Val Leu
            20                  25                  30

Asp Arg Ile Gln Cys Phe Pro Glu Leu Ser Ile Thr Ser Gly Arg Pro
        35                  40                  45

Asp Glu Ser Glu Tyr Phe Gly Ser Lys Arg Ile Tyr Leu Thr Asp Leu
    50                  55                  60

Leu Val Glu Pro Gly Asn Glu Asn Ile Lys Lys Thr Phe Tyr Val Asn
65                  70                  75                  80

Lys Leu Ile Asn Ile Leu Asn Glu Ile Lys Asn Asn Tyr Asp Thr Gln
                85                  90                  95

Asn Leu Pro Asn Glu Lys Gly Tyr Gly Cys Ile Phe Glu Gly Gly Ser
            100                 105                 110

Ile Ser Leu Leu Lys Glu Leu Leu Thr Lys Ile Asn Lys Leu Pro Tyr
        115                 120                 125

Lys Ile Thr Cys Val Ile Tyr Ile Arg Pro Ser Asp Ser Ile Asp Asn
    130                 135                 140

His Lys Leu Tyr Lys Ala Lys Ile Phe Lys Arg Val Ser Gln Met Leu
145                 150                 155                 160

Phe Pro Thr Glu Glu Gly Asn Asp Ser Gln Ile Leu Glu Val Lys Arg
                165                 170                 175

Ile Leu Asn Lys Gly Lys Thr Val Asn Ala Gln Gly Glu Ile Asn Leu
            180                 185                 190

Glu Tyr Tyr Glu Lys Ile Lys Gln Val Leu Ile Ser Leu Val Gly Leu
        195                 200                 205

Val Gly Ile Glu Asp Val Ile His Phe Leu Asp Lys Gln Tyr Asp Gln
    210                 215                 220

Lys Asn Ile Thr Ser Lys Leu Asp Pro Asn Tyr Leu Asn Glu Ile Gln
225                 230                 235                 240

Ser Gln Leu Ile Glu Thr Ile Thr Leu Ala His Tyr Asn Tyr Ala Leu
                245                 250                 255

Ser Gln Ile Glu Leu Ile Asp Ser Leu Ile Lys Gln Leu Pro Lys Ser
            260                 265                 270

Ile Glu Tyr Cys Leu Lys Asn Ile Glu Ile Asn
        275                 280

<210>  34
<211>  195
<212>  PRT
<213>  Corynebacterium glutamicum

<400>  34

Met Thr Ser Leu Phe Asp Ala Pro Thr Leu Gln Arg Val Thr Val Phe
1               5                   10                  15

Thr Gly Ser Ala Leu Gly Ser Ser Ser Leu Tyr Thr Gln Ala Ala Gln
            20                  25                  30

Thr Leu Ala Lys Thr Ala Val Asp Arg Gly Ile Asp Leu Val Tyr Gly
        35                  40                  45

Gly Gly Lys Val Gly Leu Met Gly Ile Val Ala Asp Ala Phe Leu Glu
    50                  55                  60

Ser Gly Gly Glu Ala Phe Gly Val Ile Thr Glu Ser Leu Met Lys Gly
65                  70                  75                  80

Glu Leu Gly His Glu Lys Leu Thr Glu Leu Glu Ile Val Pro Asp Met
                85                  90                  95

His Ile Arg Lys Arg Arg Met Ala Glu Leu Gly Asp Gly Phe Ile Ala
            100                 105                 110

Met Pro Gly Gly Ala Gly Thr Leu Glu Glu Leu Phe Glu Val Trp Thr
        115                 120                 125

Trp Gln Gln Leu Gly Ile His Gln Lys Pro Val Ala Leu Tyr Asp Val
    130                 135                 140

Asp Gly Phe Trp Gln Pro Leu Leu Glu Met Leu Glu Gln Met Thr Gln
145                 150                 155                 160

Arg Gly Phe Ile Lys Arg Asp Phe Phe Glu Cys Leu Ile Val Glu Ser
                165                 170                 175

Asp Pro His Ala Leu Leu Lys Ala Met Gln Thr Trp Thr Pro Pro Ala
            180                 185                 190

Pro Lys Trp
        195

<210>  35
<211>  191
<212>  PRT
<213>  Bacillus subtilis

<400>  35

Met Lys Thr Ile Cys Val Phe Ala Gly Ser Asn Pro Gly Gly Asn Glu
1               5                   10                  15

Ala Tyr Lys Arg Lys Ala Ala Glu Leu Gly Val Tyr Met Ala Glu Gln
            20                  25                  30

Gly Ile Gly Leu Val Tyr Gly Gly Ser Arg Val Gly Leu Met Gly Thr
        35                  40                  45

Ile Ala Asp Ala Ile Met Glu Asn Gly Gly Thr Ala Ile Gly Val Met
    50                  55                  60

Pro Ser Gly Leu Phe Ser Gly Glu Val Val His Gln Asn Leu Thr Glu
65                  70                  75                  80

Leu Ile Glu Val Asn Gly Met His Glu Arg Lys Ala Lys Met Ser Glu
                85                  90                  95

Leu Ala Asp Gly Phe Ile Ser Met Pro Gly Gly Phe Gly Thr Tyr Glu
            100                 105                 110

Glu Leu Phe Glu Val Leu Cys Trp Ala Gln Ile Gly Ile His Gln Lys
        115                 120                 125

Pro Ile Gly Leu Tyr Asn Val Asn Gly Tyr Phe Glu Pro Met Met Lys
    130                 135                 140

Met Val Lys Tyr Ser Ile Gln Glu Gly Phe Ser Asn Glu Ser His Leu
145                 150                 155                 160

Lys Leu Ile His Ser Ser Ser Arg Pro Asp Glu Leu Ile Glu Gln Met
                165                 170                 175

Gln Asn Tyr Ser Tyr Pro Ile Leu Glu Lys Lys Trp Thr Glu Ile
            180                 185                 190

<210>  36
<211>  256
<212>  PRT
<213>  Corynebacterium glutamicum

<400>  36

Met Ala Pro Lys Gln Thr Pro Ser Pro Glu Lys Asn Arg Asn Leu Val

1               5                   10                  15

Gly Pro Val Leu Gln Arg Arg Gln Thr Glu Gly Thr Phe Asp Gln Arg
            20                  25                  30

Leu Leu Glu Met Arg Ala Asp His Asn Trp Lys His Ala Asp Pro Trp
        35                  40                  45

Arg Val Leu Arg Ile Gln Ser Glu Phe Val Ala Gly Phe Asp Ala Leu
    50                  55                  60

His Glu Met Pro Lys Ala Val Thr Val Phe Gly Ser Ala Arg Ile Lys
65                  70                  75                  80

Glu Asp His Pro Tyr Tyr Lys Ala Gly Val Glu Leu Gly Glu Lys Leu
                85                  90                  95

Val Ala Ala Asp Tyr Ala Val Val Thr Gly Gly Gly Pro Gly Leu Met
            100                 105                 110

Glu Ala Pro Asn Lys Gly Ala Ser Glu Ala Asn Gly Leu Ser Val Gly
        115                 120                 125

Leu Gly Ile Glu Leu Pro His Glu Gln His Leu Asn Pro Tyr Val Asp
    130                 135                 140

Leu Gly Leu Asn Phe Arg Tyr Phe Phe Ala Arg Lys Thr Met Phe Leu
145                 150                 155                 160

Lys Tyr Ser Gln Ala Phe Val Cys Leu Pro Gly Gly Phe Gly Thr Leu
                165                 170                 175

Asp Glu Leu Phe Glu Val Leu Cys Met Val Gln Thr Gly Lys Val Thr
            180                 185                 190

Asn Phe Pro Ile Val Leu Ile Gly Thr Glu Phe Trp Ala Gly Leu Val
        195                 200                 205

Asp Trp Ile Arg His Arg Leu Val Glu Glu Gly Met Ile Asp Glu Lys
    210                 215                 220

Asp Val Asp Arg Met Leu Val Thr Asp Asp Leu Asp Gln Ala Val Lys
225                 230                 235                 240

Phe Ile Val Asp Ala His Ala Gly Leu Asp Val Ala Arg Arg His Asn
                245                 250                 255

<210>  37
<211>  195
<212>  PRT
<213>  Pseudomonas aeruginosa

<400>  37

Met Thr Leu Arg Ser Val Cys Val Phe Cys Gly Ala Ser Pro Gly Ala
1               5                   10                  15

Ser Pro Val Tyr Gln Glu Ala Ala Val Ala Leu Gly Arg His Leu Ala
            20                  25                  30

Glu Arg Gly Leu Thr Leu Val Tyr Gly Gly Gly Ala Val Gly Leu Met
        35                  40                  45

Gly Thr Val Ala Asp Ala Ala Leu Ala Ala Gly Gly Glu Val Ile Gly
    50                  55                  60

Ile Ile Pro Gln Ser Leu Gln Glu Ala Glu Ile Gly His Lys Gly Leu
65                  70                  75                  80

Thr Arg Leu Glu Val Val Asp Gly Met His Ala Arg Lys Ala Arg Met
                85                  90                  95

Ala Glu Leu Ala Asp Ala Phe Ile Ala Leu Pro Gly Gly Leu Gly Thr
            100                 105                 110

Leu Glu Glu Leu Phe Glu Val Trp Thr Trp Gly Gln Leu Gly Tyr His
        115                 120                 125

Ala Lys Pro Leu Gly Leu Leu Glu Val Asn Gly Phe Tyr Asp Pro Leu
    130                 135                 140

Leu Thr Phe Leu Asp His Leu Val Asp Glu Arg Phe Val Arg Ala Glu
145                 150                 155                 160

His Arg Gly Met Leu Gln Arg Gly Ala Ser Pro Glu Ala Leu Leu Asp
                165                 170                 175

Ala Leu Ala Ala Trp Thr Pro Ser Val Ala Pro Lys Trp Val Asp Arg
            180                 185                 190

Thr Pro Gln
        195

<210>  38
<211>  256
<212>  PRT
<213>  Rhodococcus fascians

<400>  38

Met Asn Ser Val Asp Asp Pro Gly Ser Tyr Glu Gln Ile Gly Ser Glu
1               5                   10                  15

Lys Pro Ala Lys His Lys Gly Ser Val Val Leu Arg Arg Gly Arg Asn
            20                  25                  30

Thr Glu Thr Ser Thr Ser Asp Gln Arg Leu Leu Asp Glu Arg Gly Ser
        35                  40                  45

Gly Asn Trp Ile His Ser Asp Thr Trp Arg Val Leu Arg Ile Gln Ser
    50                  55                  60

Glu Phe Val Glu Gly Phe Gly Ala Leu Ala Glu Val Pro Arg Ala Val
65                  70                  75                  80

Thr Val Phe Gly Ser Ala Arg Thr Pro Val Asp His Pro Glu Tyr Glu
                85                  90                  95

Arg Ala Arg Glu Leu Gly Ala Ala Leu Ala Arg Glu Gly Phe Ala Val
            100                 105                 110

Ile Thr Gly Gly Gly Pro Gly Val Met Glu Gly Ala Asn Arg Gly Ala
        115                 120                 125

Ser Glu Ala Asp Gly Tyr Ser Ile Gly Leu Gly Ile Glu Leu Pro Phe

130                 135                 140

Glu Gln Gly Leu Asn Asp Trp Val Asp Leu Gly Val Asn Phe Arg Tyr
145                 150                 155                 160

Phe Phe Val Arg Lys Thr Met Phe Val Lys Tyr Ser Gln Ala Phe Val
                165                 170                 175

Cys Leu Pro Gly Gly Phe Gly Thr Leu Asp Glu Leu Phe Glu Ala Leu
            180                 185                 190

Thr Leu Val Gln Thr Ala Lys Val Thr Arg Phe Pro Ile Val Leu Leu
        195                 200                 205

Gly Lys Asp Phe Trp Ser Gly Leu Leu Asp Trp Ile Lys Thr Thr Leu
    210                 215                 220

Val Lys Glu Gly Lys Val Ser Glu Lys Asp Leu Asp Leu Ile His Cys
225                 230                 235                 240

Thr Asp Ser Val Asp Glu Ala Val Arg Ile Val Arg Glu Ala Gln Gln
                245                 250                 255

<210>  39
<211>  198
<212>  PRT
<213>  Rhodococcus fascians

<400>  39

Met Asn Leu Arg Pro Met Pro Ala Thr Thr Val Ser Ala Gln Ala Arg
1               5                   10                  15

Pro Thr Pro Lys Ser Val Thr Val Phe Cys Gly Ala Met Pro Gly Arg
            20                  25                  30

Gly Thr Lys Tyr Gly Gln Leu Ala Glu Gly Met Gly Arg Ala Ile Ala
        35                  40                  45

Arg Ser Lys Leu Arg Leu Val Tyr Gly Gly Ala Arg Val Gly Leu Met
    50                  55                  60

Gly Thr Leu Ala Asn Ala Ala Leu Asp Ser Gly Gly Thr Val Val Gly
65                  70                  75                  80

Val Ile Pro Glu Ser Phe Thr Ala Ile Pro Glu Ala Ala His His Gly
                85                  90                  95

Leu Thr Glu Leu His Val Val His Asp Met His Gln Arg Lys Ala Leu
            100                 105                 110

Met Ala Glu Leu Gly Asp Ala Phe Ile Ala Leu Pro Gly Gly Val Gly
        115                 120                 125

Thr Ala Glu Glu Phe Phe Glu Val Leu Thr Trp Ser His Leu Gly Leu
    130                 135                 140

His Asn Lys Pro Cys Val Leu Leu Asn Asp Asn Glu Tyr Tyr Arg Pro
145                 150                 155                 160

Leu Leu Ser Tyr Ile Glu His Ala Ala Val Glu Gly Phe Ile Thr Pro
                165                 170                 175

Ala Thr Arg Ser Arg Val Ile Val Cys Lys Asp Ile Glu Gly Ala Ile
            180                 185                 190

Ala Ala Ile Arg Ser Pro
        195

<210>  40
<211>  187
<212>  PRT
<213>  Corynebacterium stationis

<400>  40

Met Lys Ser Val Ala Ile Tyr Cys Gly Ser Ser Met Gly Ser Ser Pro
1               5                   10                  15

Arg Tyr Ala Glu Val Ala Lys Glu Met Gly Glu Glu Leu Ala Arg Arg
            20                  25                  30

Asn Leu Arg Val Val Tyr Gly Gly Gly Asn Val Gly Leu Met Gly Thr
        35                  40                  45

Val Ala Asp Ala Ala Leu Ala Ala Gly Gly Glu Val Val Gly Val Ile
    50                  55                  60

Pro Arg Gln Leu Ile Asn Arg Glu Met Ala His Ala Gly Leu Thr Asp
65                  70                  75                  80

Leu Glu Val Val Asp Thr Met Ala Gln Arg Lys Thr Arg Met Glu Glu
                85                  90                  95

Leu Ala Asp Ala Phe Val Cys Leu Pro Gly Gly Val Gly Thr Leu Glu
            100                 105                 110

Glu Val Val Glu Val Leu Thr Met Gln Gln Leu Gly His Val Gln Gly
        115                 120                 125

Pro Val Gly Leu Val Asn Val Asp Gly Phe Trp Glu Pro Phe Val Arg
    130                 135                 140

Thr Leu Glu Ser Met Val Ala Cys Gly Phe Val Gln Ser Arg Tyr Ile
145                 150                 155                 160

Asp Ala Ile Ala Leu Ser Ser Asp Pro Ala Glu Ile Leu Asp Glu Phe
                165                 170                 175

Ala Gly Trp Ser Pro Ile Gly Ala Lys Trp Ser
            180                 185

<210>  41
<211>  275
<212>  PRT
<213>  Corynebacterium stationis

<400>  41

Met Ala Pro Leu Arg Ser Asn Lys Asp Gly Lys Pro Arg Val Pro Arg
1               5                   10                  15

Thr Thr Glu Ser Ala Glu Thr Asn Arg Met Leu Arg Gly Pro Met Leu
            20                  25                  30

Leu Arg Thr Asp Gly Asn Gln Thr Ser Thr Tyr Asp Gln Arg Leu Leu
        35                  40                  45

Glu Ser Gly Ala Asp His Ala Trp Lys His Ala Asp Pro Trp Arg Val
    50                  55                  60

Leu Arg Ile Gln Gly Glu Phe Val Ala Gly Phe Asp Ala Leu Ser Asp
65                  70                  75                  80

Leu Pro Lys Ala Val Thr Val Tyr Gly Ser Ala Arg Thr Lys Glu Asp
                85                  90                  95

Thr Pro Glu Tyr Ala Gln Gly Ile Asp Leu Gly Ala Lys Leu Val Glu
            100                 105                 110

His Asp Tyr Ala Val Val Thr Gly Gly Gly Pro Gly Ile Met Glu Ala
        115                 120                 125

Ala Asn Arg Gly Ala Tyr Glu Ala Gly Gly Leu Ser Val Gly Leu Gly
    130                 135                 140

Ile Glu Leu Pro Phe Glu Gln Gly Leu Asn Lys Tyr Val Asp Leu Gly
145                 150                 155                 160

Ile Asn Phe Arg Tyr Phe Phe Ala Arg Lys Thr Met Phe Leu Lys Tyr
                165                 170                 175

Ser Gln Ala Phe Val Cys Leu Pro Gly Gly Met Gly Thr Met Asp Glu
            180                 185                 190

Leu Phe Glu Met Leu Cys Met Val Gln Thr Gly Lys Val Thr Asn Tyr
        195                 200                 205

Pro Ile Val Leu Ile Gly Thr Glu Tyr Trp Ser Gly Leu Val Glu Trp
    210                 215                 220

Met Ala Asn Thr Leu Val Ala Glu Gly Tyr Ile Asn Pro Gln Asp Met
225                 230                 235                 240

Asp Leu Phe Leu Leu Thr Asp Ser Val Asp Glu Ala Val Glu His Ile
                245                 250                 255

Val Ala Ala His Lys Glu Met Thr Asp Glu Arg Val Ser Arg Leu Asn
            260                 265                 270

His Asp Ala
        275

<210>  42
<211>  252
<212>  PRT
<213>  Streptomyces coelicolor

<400>  42

Met Ala Thr Gly Asn Pro Glu Gly Lys Lys Arg Pro Pro Glu Glu Gln
1               5                   10                  15

Arg Leu Gly Pro Val Leu Arg Arg Arg Gly Gln Val Gln Glu Ser Thr
            20                  25                  30

Thr Asp Gln Arg Leu Leu Asp Glu Arg Ala Pro Thr Asp Trp Val His
        35                  40                  45

Thr Asp Pro Trp Arg Val Leu Arg Ile Gln Ser Glu Phe Ile Glu Gly
    50                  55                  60

Phe Gly Thr Leu Ala Glu Leu Pro Pro Ala Ile Ser Val Phe Gly Ser
65                  70                  75                  80

Ala Arg Thr Pro Ala Asp Ser Pro Glu Tyr Asp Ala Gly Val Arg Leu
                85                  90                  95

Gly Arg Gly Leu Val Glu Ala Gly Phe Ala Val Ile Thr Gly Gly Gly
            100                 105                 110

Pro Gly Ala Met Glu Ala Ala Asn Lys Gly Ala Leu Glu Ala Lys Gly
        115                 120                 125

Thr Ser Val Gly Leu Gly Ile Glu Leu Pro Phe Glu Gln Gly Leu Asn
    130                 135                 140

Pro Tyr Val Asp Ile Gly Leu Asn Phe Arg Tyr Phe Phe Val Arg Lys
145                 150                 155                 160

Met Met Phe Val Lys Tyr Ala Gln Gly Phe Val Val Leu Pro Gly Gly
                165                 170                 175

Leu Gly Thr Leu Asp Glu Leu Phe Glu Ala Leu Thr Leu Val Gln Thr
            180                 185                 190

Gln Lys Val Thr Arg Phe Pro Ile Val Leu Phe Gly Ser Glu Tyr Trp
        195                 200                 205

Gly Gly Leu Val Asp Trp Leu Arg Gly Thr Leu Val Ala Gln Gly Lys
    210                 215                 220

Ala Ala Glu Lys Asp Leu Met Leu Phe His Val Thr Asp Asp Val Asp
225                 230                 235                 240

Glu Ala Val Ala Leu Val Ser Lys Glu Ala Gly Arg
                245                 250

<210>  43
<211>  215
<212>  PRT
<213>  Arabidopsis thaliana

<400>  43

Met Glu Ile Lys Gly Glu Ser Met Gln Lys Ser Lys Phe Arg Arg Ile
1               5                   10                  15

Cys Val Phe Cys Gly Ser Ser Gln Gly Lys Lys Ser Ser Tyr Gln Asp
            20                  25                  30

Ala Ala Val Asp Leu Gly Asn Glu Leu Val Ser Arg Asn Ile Asp Leu
        35                  40                  45

Val Tyr Gly Gly Gly Ser Ile Gly Leu Met Gly Leu Val Ser Gln Ala
    50                  55                  60

Val His Asp Gly Gly Arg His Val Ile Gly Ile Ile Pro Lys Thr Leu
65                  70                  75                  80

Met Pro Arg Glu Leu Thr Gly Glu Thr Val Gly Glu Val Arg Ala Val
                85                  90                  95

Ala Asp Met His Gln Arg Lys Ala Glu Met Ala Lys His Ser Asp Ala
            100                 105                 110

Phe Ile Ala Leu Pro Gly Gly Tyr Gly Thr Leu Glu Glu Leu Leu Glu
        115                 120                 125

Val Ile Thr Trp Ala Gln Leu Gly Ile His Asp Lys Pro Val Gly Leu
    130                 135                 140

Leu Asn Val Asp Gly Tyr Tyr Asn Ser Leu Leu Ser Phe Ile Asp Lys
145                 150                 155                 160

Ala Val Glu Glu Gly Phe Ile Ser Pro Thr Ala Arg Glu Ile Ile Val
                165                 170                 175

Ser Ala Pro Thr Ala Lys Glu Leu Val Lys Lys Leu Glu Glu Tyr Ala
            180                 185                 190

Pro Cys His Glu Arg Val Ala Thr Lys Leu Cys Trp Glu Met Glu Arg
        195                 200                 205

Ile Gly Tyr Ser Ser Glu Glu
    210                 215

<210>  44
<211>  306
<212>  PRT
<213>  Arabidopsis thaliana

<400>  44

Met Gly Val Ser Met Leu Asp Ser Ser Trp Ser Phe Arg Leu Ser Ser
1               5                   10                  15

Lys Met Ser Pro Ser Pro Thr Gln Lys Leu Ala Ser Ser Leu Thr Glu
            20                  25                  30

Arg Asp Pro Ile Ser Leu Asn Leu Arg Phe Asp His Ser Ile Arg Ala
        35                  40                  45

Tyr Ser Ala Arg Ala Leu Lys Cys Arg Ser Val Lys Ser Glu Asp Phe

50                  55                  60

Val Glu Arg Ser Ser Pro Phe Glu Val Lys Lys Glu Leu Glu Ile Cys
65                  70                  75                  80

Phe Asp Leu Val His Arg Leu Gly Arg Gly Ile Val Tyr Leu Gly Ser
                85                  90                  95

Ala Arg Ile Pro Pro Asn His Ser His Tyr Leu Gln Ala Gln Glu Leu
            100                 105                 110

Ser Arg Glu Ala Ala Thr Leu Leu Asp Cys Thr Thr Trp Ser Gly Ala
        115                 120                 125

Gly Pro Gly Leu Met Asp Ala Val Thr Glu Gly Ala Leu Glu Ala Glu
    130                 135                 140

Lys Pro Val Gly Gly Ile Lys Ile Glu Lys Glu Ala Gly Glu Trp Thr
145                 150                 155                 160

Ala Ser Lys Phe His Pro Tyr Leu Pro Pro Gln Asn Tyr His Thr Cys
                165                 170                 175

Arg Phe Phe Ser Ala Arg Lys His Gly Leu Val Asp Ala Val Ile Arg
            180                 185                 190

Asn Asn Val Ser Glu Lys Thr Ala Ile Ile Ala Leu Pro Gly Gly Ile
        195                 200                 205

Gly Thr Leu Asp Glu Met Phe Glu Ile Leu Ala Leu Ile Gln Leu Glu
    210                 215                 220

Arg Ile Gly Ser Ala Leu Pro Val Pro Phe Ile Val Met Asn Tyr Asp
225                 230                 235                 240

Ala Phe Tyr Ser Lys Leu Leu Glu Phe Ile Glu Thr Cys Glu Asn Leu
                245                 250                 255

Gly Thr Val Ser Lys Gly Glu Val Ser Ala Leu Trp Lys Val Cys Asn
            260                 265                 270

Asn Asn Phe Glu Ala Leu Thr Tyr Leu Ala Glu Phe Tyr Asp Leu Pro
        275                 280                 285

Asp Gly Ser Ala Lys Leu Glu Thr Glu Leu Arg Lys Thr Thr Pro Thr
    290                 295                 300

Arg Gly
305

<210>  45
<211>  194
<212>  PRT
<213>  Burkholderia sp. TNe-862

<400>  45

Met Lys Ala Val Cys Val Tyr Cys Gly Ser Ala Glu Gly Ala Asn Pro
1               5                   10                  15

Ile Tyr Ala Glu Ala Ala Gln Ala Phe Gly Arg Ala Leu Val Ala Ala
            20                  25                  30

Asn Leu Ala Leu Val Tyr Gly Gly Gly Lys Val Gly Leu Met Gly Val
        35                  40                  45

Ile Ala Asp Thr Val Met Ala Ala Gly Gly Arg Ala Ile Gly Val Ile
    50                  55                  60

Pro Glu Leu Leu Val Asp Lys Glu Val Gly His Thr Gly Leu Thr Glu
65                  70                  75                  80

Leu His Val Val Pro Asp Met His His Arg Lys Lys Met Met Ala Asp
                85                  90                  95

Phe Ser Asp Ala Phe Val Ala Met Pro Gly Gly Ala Gly Thr Leu Glu
            100                 105                 110

Glu Leu Phe Glu Val Tyr Thr Trp Ala Gln Leu Gly Tyr His Arg Lys
        115                 120                 125

Pro Val Gly Val Leu Asn Ile Gly Gly Phe Tyr Asp Pro Leu Val Ala
    130                 135                 140

Leu Leu Asp His Thr Val Arg Glu Gly Phe Met Arg Gln Thr Tyr Arg
145                 150                 155                 160

Glu Met Leu Gln Ile Asp Gly Asp Pro Ala Ala Leu Ile Ala Arg Leu
                165                 170                 175

Lys Arg Tyr His Ala Pro Ala Lys Asp Lys Trp Ala Asp Leu Arg Glu
            180                 185                 190

Asn Val

<210>  46
<211>  194
<212>  PRT
<213>  Paraburkholderia sp. SOS3

<400>  46

Met Lys Ala Val Cys Val Tyr Cys Gly Ser Ala Lys Gly Ala Asn Pro
1               5                   10                  15

Ile Tyr Ala Glu Ala Ala His Ala Phe Gly Arg Ala Leu Val Asp Ala
            20                  25                  30

Asn Leu Ala Leu Val Tyr Gly Gly Gly Lys Val Gly Leu Met Gly Met
        35                  40                  45

Ile Ala Asp Ser Val Met Ala Ala Gly Gly Arg Ala Ile Gly Val Ile
    50                  55                  60

Pro Glu Leu Leu Val Asp Lys Glu Ile Gly His His Gly Leu Thr Glu
65                  70                  75                  80

Leu His Val Val Ser Asp Met His Glu Arg Lys Lys Leu Met Ala Asp
                85                  90                  95

Leu Ser Asp Ala Phe Val Ala Leu Pro Gly Gly Ala Gly Thr Leu Glu
            100                 105                 110

Glu Leu Phe Glu Val Tyr Thr Trp Ala Gln Leu Gly Tyr His Arg Lys

        115                 120                 125

Pro Val Gly Ile Leu Asn Ile Ala Gly Phe Tyr Asp Pro Leu Ile Ala
    130                 135                 140

Met Leu Glu His Thr Val Arg Glu Gly Phe Met Gln Gln Thr Tyr Phe
145                 150                 155                 160

Glu Thr Leu Gln Ile Asp Asn Glu Ala Ala Ala Leu Leu Gly Lys Leu
                165                 170                 175

Gln His Tyr Arg Pro Leu Ala Gln Asp Lys Trp Ser Gln His Arg Asp
            180                 185                 190

Lys Val

<210> 47
<211> 195
<212> PRT
<213> Pseudomonas sp. HAR-UPW-AIA-41

<400> 47

Met Pro Leu Gln Ser Ile Cys Val Phe Cys Gly Ala Ser Val Gly His
1               5                   10                  15

Asp Pro Ala Tyr Gln Gln Ala Ala Glu Ala Leu Gly Arg Thr Leu Ala
            20                  25                  30

Glu Arg Gly Leu Arg Leu Ile Tyr Gly Gly Gly Glu Val Gly Leu Met
        35                  40                  45

Gly Lys Leu Ala Asp Ala Val Leu Ala Ala Gly Gly Glu Val Thr Gly
    50                  55                  60

Ile Ile Pro Gln Ser Leu Gln Arg Ser Glu Ile Gly His Lys Gly Leu
65                  70                  75                  80

Thr Arg Leu Glu Val Val Asp Gly Met His Ala Arg Lys Ala Arg Met
                85                  90                  95

Ala Glu Leu Ser Asp Ala Phe Ile Ala Leu Pro Gly Gly Leu Gly Thr
            100                 105                 110

Leu Glu Glu Leu Phe Glu Val Trp Thr Trp Ala Gln Leu Gly Tyr His
        115                 120                 125

Thr Lys Pro Ile Gly Leu Leu Asp Val Ala Gly Phe Tyr Arg Gly Leu
    130                 135                 140

Ser Ala Phe Leu Asp Gln Thr Val Ser Ala Gly Phe Ile Arg Pro Pro
145                 150                 155                 160

His Arg Gly Leu Leu His Gln Ala Glu Asp Pro Ala Gln Leu Leu Asp
                165                 170                 175

Leu Leu Gln Gln Gln Gln Gly Ser Val Thr Pro Lys Trp Asp Ala Arg
            180                 185                 190

Gln Pro Ile
        195

<210>  48
<211>  195
<212>  PRT
<213>  Pseudomonas putida

<400>  48

Met Pro Val Arg Ser Val Cys Val Phe Cys Gly Ala Ser Met Gly Ala
1               5                   10                  15

Asn Pro Ala Tyr Arg Glu Ala Ala Val Ala Leu Gly Gln Ala Ile Ala
            20                  25                  30

Arg Arg Gly Leu Thr Leu Val Tyr Gly Gly Gly Ala Val Gly Leu Met
        35                  40                  45

Gly Val Val Ala Asp Ala Ala Met Ala Ala Gly Gly Glu Val Val Gly
    50                  55                  60

Ile Ile Pro Gln Ser Leu Leu Asp Ala Glu Val Gly His Lys Gly Leu
65                  70                  75                  80

Thr Arg Leu Glu Val Val Asp Gly Met His Ala Arg Lys Ala Arg Met
                85                  90                  95

Ala Glu Leu Ser Asp Ala Phe Ile Ala Leu Pro Gly Gly Leu Gly Thr
            100                 105                 110

Leu Glu Glu Leu Phe Glu Val Trp Thr Trp Gly Gln Leu Gly Tyr His
        115                 120                 125

Ala Lys Pro Leu Gly Leu Leu Asp Val Asn Gly Phe Tyr Asp Lys Leu
    130                 135                 140

Gly Gly Phe Leu Asp His Ile Val Glu Glu Gly Phe Val Arg Pro Gln
145                 150                 155                 160

His Arg Ala Met Leu Leu Leu Gly Gln Gln Pro Asp Glu Leu Leu Glu
                165                 170                 175

Gly Met Asp Ser Phe Val Ala Pro Val Ala Pro Lys Trp Val Asp Lys
            180                 185                 190

Gln Pro Asp
        195

<210>  49
<211>  203
<212>  PRT
<213>  Bradyrhizobium sp. Leo170

<400>  49

Met Gln Asn Gln Asn Ala Lys Ala Leu Lys Ser Val Cys Ile Phe Cys
1               5                   10                  15

Gly Ser Ser Pro Gly Arg Asp Pro Ile Tyr Leu Gln Thr Ala Val Ala
            20                  25                  30

Thr Gly Gln Ala Leu Ala Lys Ala Gly Val Thr Ile Val Tyr Gly Gly
        35                  40                  45

Gly Lys Val Gly Leu Met Gly Ala Val Ala Asp Ala Ala Leu Ala Ala
    50                  55                  60

Gly Gly Arg Val Val Gly Val Ile Pro Arg Ala Leu Val Glu Arg Glu
65                  70                  75                  80

Ile Gly His Thr Gly Leu Ser Gln Leu His Ile Val Glu Thr Met His
                85                  90                  95

Glu Arg Lys Thr Met Met Ala Gln Leu Ser Asp Gly Phe Ile Ala Leu
            100                 105                 110

Pro Gly Gly Ala Gly Thr Leu Glu Glu Leu Phe Glu Glu Trp Thr Trp
        115                 120                 125

Ala Gln Leu Gly Ile His Gln Lys Pro Cys Gly Leu Leu Asn Val Lys
    130                 135                 140

Asp Tyr Phe Ala Pro Leu Ile Ala Met Val Glu Arg Met Val Ala Glu
145                 150                 155                 160

Gly Phe Thr Ile Ala Ala Tyr Ala Glu Met Leu Ile Ile Glu Thr Lys
                165                 170                 175

Val Asp Ala Leu Leu Ser Arg Phe Gly Thr Tyr Gln Pro Pro Pro Arg
            180                 185                 190

Lys Trp Leu Arg Thr Glu Ala Ala Ala Lys Pro
        195                 200

<210>  50
<211>  200
<212>  PRT
<213>  Rhodococcus hoagii

<400>  50

Met Arg Ile Thr Val Phe Met Gly Ser Ala Thr Gly His Asp Pro Ala
1               5                   10                  15

His Leu Gln Thr Ala Thr Gln Phe Gly Arg Asp Leu Ala Ala Ala Gly
            20                  25                  30

Ile Gly Leu Val Tyr Gly Gly Gly Arg Val Gly Leu Met Gly Ala Val

35                  40                  45

Ala Asp Ala Val Val Ala Gly Gly Gly Glu Thr Val Gly Val Ile Pro
    50                  55                  60

Arg His Leu Ala Asp Lys Glu Ile Ala His Pro Gly Leu Ser Thr Leu
65                  70                  75                  80

Glu Val Val Glu Ser Met His Ala Arg Lys Gln Arg Met Ala Glu Leu
                85                  90                  95

Ala Asp Ala Phe Val Val Leu Pro Gly Gly Ala Gly Thr Leu Asp Glu
            100                 105                 110

Phe Phe Glu Ile Trp Thr Trp Gln Gln Leu Gly Leu His Asp Lys Pro
        115                 120                 125

Val Phe Leu Phe Asp Ala Asp Gly Phe Trp Gln Pro Leu Val Gly Leu
    130                 135                 140

Leu Asp His Leu Val Asp Ala Gly Phe Val Ala Ala Tyr Gln Arg Glu
145                 150                 155                 160

Ala Leu Leu Val Ala Ala Asp Leu Asp Asp Val Arg Thr Ala Met Thr
                165                 170                 175

Thr Trp Ala Pro Pro Ala Pro Lys Trp Ser Gly Thr Ala Glu Pro Ala
            180                 185                 190

Asn Asp Thr Asp Leu Glu Arg Ala
        195                 200

<210>  51
<211>  185
<212>  PRT
<213>  Streptomyces gilvigriseus

<400>  51

Met Arg Val Ala Val Phe Thr Gly Ser Ser Phe Gly Ser Ser Pro Ala
1               5                   10                  15

Phe Ala Arg Ala Ala Glu Glu Leu Gly Arg Gly Leu Ala Glu Ala Gly
            20                  25                  30

Met Gly Ile Val Tyr Gly Gly Gly His Val Gly Leu Met Gly Ala Val
        35                  40                  45

Ala Asp Ala Ala Leu Ala Ala Gly Gly Glu Val Ser Gly Val Ile Pro
    50                  55                  60

Arg Ala Leu Ala Glu Arg Glu Leu Gln His Glu Gly Leu Thr Arg Leu
65                  70                  75                  80

Arg Ile Val Glu Thr Met His Glu Arg Lys Ala Ala Met Ala Ala Glu
                85                  90                  95

Ala Asp Ala Phe Val Ala Leu Pro Gly Gly Ala Gly Thr Leu Glu Glu
            100                 105                 110

Leu Phe Glu Ala Trp Thr Trp Gln His Leu Gly Tyr His Ala Lys Pro
        115                 120                 125

Val Val Leu Tyr Asp Val Asp Gly Phe Trp Gln Pro Leu Arg Ala Met
    130                 135                 140

Val Ser Gly Met Val Glu Ser Gly Phe Ile Arg Ala Glu Phe Arg Asp
145                 150                 155                 160

Thr Leu Pro Thr Ala Gly Ser Val Ala Glu Leu Leu Gly Leu Leu Lys
                165                 170                 175

Asp Trp Arg Ala Pro Gly Gly Lys Trp
            180                 185

<210>  52
<211>  195
<212>  PRT
<213>  Pseudomonas fluorescens

<400>  52

Met Ser Ile Ala Ser Val Cys Val Phe Cys Gly Ala Ser Thr Gly Thr
1               5                   10                  15

Asp Pro Ala Tyr Arg Glu Ala Ala Gln Ala Leu Gly Arg Ala Leu Ala
            20                  25                  30

Glu Arg Lys Leu Thr Leu Val Tyr Gly Gly Gly Ala Val Gly Leu Met
        35                  40                  45

Gly Ile Val Ala Asp Ala Ala Leu Ala Ala Gly Gly Glu Val Ile Gly
    50                  55                  60

Ile Ile Pro Gln Ser Leu Lys Asp Lys Glu Ile Gly His Ser Gly Leu
65                  70                  75                  80

Thr Arg Leu Glu Val Val Asp Gly Met His Ala Arg Lys Ala Arg Met
                85                  90                  95

Ala Glu Leu Ser Asp Ala Phe Ile Ala Leu Pro Gly Gly Leu Gly Thr
            100                 105                 110

Leu Glu Glu Leu Phe Glu Val Trp Thr Trp Gly Gln Leu Gly Tyr His
        115                 120                 125

Gly Lys Pro Leu Gly Leu Leu Glu Val Asn Gly Phe Tyr Ser Lys Leu
    130                 135                 140

Thr Ala Phe Leu Asp His Ile Val Gly Glu Gly Phe Val Arg Ala Pro
145                 150                 155                 160

His Arg Asp Met Leu Gln Val Ser Glu Ser Ala Gln Asp Leu Leu Asp
                165                 170                 175

Ala Leu Asp Glu Trp Gln Pro Ser Val Gln Pro Lys Trp Ala Glu Gln
            180                 185                 190

Lys Pro Ser
        195

<210>  53
<211>  195
<212>  PRT
<213>  Pseudomonas oryzihabitans

<400>  53

Met Ser Leu Lys Ala Val Cys Val Phe Cys Gly Ala Lys Ser Gly Ala
1               5                   10                  15

Ser Pro Val Phe Glu Gln Ala Ala Ala Gln Leu Gly Arg Ala Leu Ala
            20                  25                  30

Glu Arg Gly Leu Thr Leu Val Tyr Gly Gly Gly Lys Val Gly Leu Met
        35                  40                  45

Gly Val Val Ala Asp Ala Ala Leu Ala Ala Gly Gly Glu Val Ile Gly
    50                  55                  60

Ile Ile Pro Gln Ser Leu Lys Thr Ala Glu Leu Gly His Thr Gly Cys
65                  70                  75                  80

Thr Arg Leu Glu Val Val Asp Gly Met His Ala Arg Lys Ala Arg Met
                85                  90                  95

Ala Glu Leu Ala Asp Ala Phe Ile Ala Leu Pro Gly Gly Leu Gly Thr
            100                 105                 110

Leu Glu Glu Leu Phe Glu Val Trp Thr Trp Gly Gln Leu Gly Tyr His
        115                 120                 125

Ala Lys Pro Met Gly Leu Leu Glu Val Asp Gly Phe Tyr Ala Gln Leu
    130                 135                 140

Glu Gly Phe Leu Asp His Leu Val Ala Gln Gly Phe Val Ala Ala Pro
145                 150                 155                 160

His Arg Ala Met Leu Gln Val Glu Asp Ser Pro Ile Ala Leu Leu Asp
                165                 170                 175

Ala Leu Ala Ala Trp Gln Pro Thr Ala Pro Pro Arg Trp Ala Glu Pro
            180                 185                 190

Ala Pro Arg
        195

<210>  54
<211>  189
<212>  PRT
<213>  Amycolatopsis rhizosphaerae

<400>  54

Met Arg Arg Ile Cys Val Phe Cys Gly Ser Ser Ser Gly Asn Asp Ala
1               5                   10                  15

Val Tyr Ala Glu Gln Ala Ala Ala Leu Gly Lys Leu Leu Ala Glu Arg
            20                  25                  30

Gly Ile Gly Leu Val Tyr Gly Gly Ala Ser Val Gly Ile Met Gly Val
        35                  40                  45

Val Ala Asp Ala Ala Leu Ala Ala Gly Gly Glu Val Ile Gly Val Ile
    50                  55                  60

Pro Lys His Leu Met Arg Val Glu Ile Gly His Ala Asp Leu Thr Glu
65                  70                  75                  80

Leu His Val Thr Ala Gly Met His Glu Arg Lys Ala Lys Met Ala Glu
                85                  90                  95

Leu Ser Asp Gly Phe Leu Ala Leu Pro Gly Gly Ala Gly Thr Leu Glu
            100                 105                 110

Glu Leu Ala Glu Val Trp Thr Trp Ala Gln Leu Gly Leu His His Lys
        115                 120                 125

Pro Ile Gly Leu Val Asp Val Ala Gly Tyr Tyr Arg Pro Leu Arg Glu
    130                 135                 140

Phe Ala Asp His Met Val Ala Glu Gly Phe Leu Arg Pro Gln His Arg
145                 150                 155                 160

Asp Leu Ile Phe Ile Asp Pro Asp Pro Val Val Leu Leu Asp Ala Phe
                165                 170                 175

Glu Ser Tyr Glu Pro Pro Thr Val Ala Lys Trp Ile Gly
            180                 185

<210>  55
<211>  193
<212>  PRT
<213>  Corynebacterium provencense

<400>  55

Met Thr Ala Ala Ala Pro Val Arg Lys Val Ala Leu Tyr Leu Gly Ser
1               5                   10                  15

Ala Thr Gly Asp Ser Pro Ala Tyr Arg Glu Thr Thr Val Arg Thr Val
            20                  25                  30

Glu Val Leu Ala Asp Ala Gly Cys Glu Leu Val Tyr Gly Gly Gly Ser
        35                  40                  45

Val Gly Leu Met Gly Val Ala Ala Asp Thr Ala Leu Ala Ala Gly Met
    50                  55                  60

Pro Val His Gly Val Ile Thr Arg His Leu Met Asp Gly Glu Thr Gly
65                  70                  75                  80

His Pro Gly Leu Thr Asp Leu Glu Val Val Asp Thr Met Thr Arg Arg
                85                  90                  95

Lys Glu Arg Met Ala Glu Ile Ala Asp Ala Phe Ile Val Leu Pro Gly
            100                 105                 110

Gly Ile Gly Thr Leu Glu Glu Phe Phe Glu Val Trp Val Ala Gln Val
        115                 120                 125

Leu Gly Ile His Thr Arg Pro Val Ala Leu Val Asp Val Asp Asn Tyr
    130                 135                 140

Trp Gln Pro Leu Thr Asp Met Ile Asp His Met Val Ser Arg Gly Phe
145                 150                 155                 160

Leu Ser Ala Val His Arg Asp His Leu Ile Arg Val Asn Asp Pro Glu
                165                 170                 175

Met Ile Pro Thr Ala Leu Asp Val Trp Thr Pro Ala Ala Ala Lys Trp
            180                 185                 190

Ser

<210>  56
<211>  195
<212>  PRT
<213>  Erwinia typographi

<400>  56

Met Asn Thr Val Gly Ile Phe Cys Gly Ser Ser Glu Gly His Ser Thr
1               5                   10                  15

Ala Tyr Ile Asp Ala Ala Arg His Val Gly Lys Tyr Leu Ala Glu Gln
            20                  25                  30

Gly Ile Ser Ile Val Tyr Gly Gly Gly Arg Val Gly Leu Met Gly Ala
        35                  40                  45

Val Ala Asp Ala Ala Leu Ala Tyr Gly Gly His Val Thr Gly Val Ile
    50                  55                  60

Pro Lys Ser Leu Leu Glu Arg Glu Ile Ala His Thr Gly Leu Ser Glu
65                  70                  75                  80

Leu Leu Val Val Asp Asn Met His Gln Arg Lys Asn Met Met Ala Glu
                85                  90                  95

Leu Ser Ser Gly Phe Ile Ala Leu Pro Gly Gly Ala Gly Thr Ala Glu
            100                 105                 110

Glu Ile Phe Glu Gln Trp Thr Trp Ala Gln Leu Gly Ile His Asp Lys
        115                 120                 125

Pro Leu Ala Phe Leu Asn Val Asn His Phe Tyr Asp Pro Leu Thr Val
    130                 135                 140

Met Val Gln Arg Met Val Ser Asp Gly Phe Met Lys Gln Asp Tyr Ala
145                 150                 155                 160

Asn Met Leu Arg Phe Ser Glu Asn Leu His Asp Ile Ile Asp Tyr Phe

                165                 170                 175

Lys Thr Tyr Thr Pro Pro Val Ser Lys Trp Thr Thr Arg Asn Glu Asn
            180                 185                 190

His His Val
        195

<210> 57
<211> 194
<212> PRT
<213> Paraburkholderia sp. BL10I2N1

<400> 57

Met Lys Ser Val Cys Val Tyr Cys Gly Ser Ser Asp Gly Val Lys Pro
1               5                   10                  15

Leu Tyr Ala Glu Ala Ala Arg Ala Phe Gly Arg Ala Ile Val Glu Ala
            20                  25                  30

Arg Leu Ala Leu Val Tyr Gly Gly Gly Lys Val Gly Leu Met Gly Val
        35                  40                  45

Ile Ala Asp Glu Val Met Ala Ala Gly Gly Arg Ala Ile Gly Val Ile
    50                  55                  60

Pro Glu Leu Leu Val Asn Lys Glu Val Gly His Glu Gly Leu Thr Glu
65                  70                  75                  80

Leu His Val Val Pro Asp Met His Gln Arg Lys Lys Met Met Ala Asp
                85                  90                  95

Leu Ser Asp Ala Phe Ile Ala Met Pro Gly Gly Ala Gly Thr Leu Glu
            100                 105                 110

Glu Leu Phe Glu Val Tyr Thr Trp Ala Gln Leu Gly Tyr His Gln Lys
        115                 120                 125

Pro Val Ala Val Leu Asn Ile Asp Gly Tyr Tyr Asp Pro Leu Ile Ser
    130                 135                 140

Met Leu Gln His Thr Val Gln Glu Gly Phe Met Arg Gln Thr Tyr Leu
145                 150                 155                 160

Asp Ile Leu Gln Ile Asp Ser Asp Pro Val Ser Leu Ile Ser Lys Leu
                165                 170                 175

Gln Arg Tyr Leu Pro Pro Ala Arg Asp Lys Trp Ala Asp Asn Arg Glu
            180                 185                 190

Ala Val

<210>  58
<211>  195
<212>  PRT
<213>  Pseudomonas putida

<400>  58

Met Ser Leu Arg Ser Val Cys Val Phe Cys Gly Ala Ser Thr Gly Ala
1               5                   10                  15

Ser Pro Val Tyr Arg Glu Ala Ala Ile Ala Leu Gly Gln Ala Ile Ala
            20                  25                  30

Gly Arg Gly Leu Thr Leu Val Tyr Gly Gly Gly Ala Val Gly Leu Met
        35                  40                  45

Gly Ile Val Ala Asp Ala Ala Met Ala Ala Gly Gly Glu Val Ile Gly
    50                  55                  60

Ile Ile Pro Val Ser Leu Gln Arg Ala Glu Ile Ala His Pro Gly Leu
65                  70                  75                  80

Thr Arg Leu Glu Val Val Asp Gly Met His Ala Arg Lys Ala Arg Met
                85                  90                  95

Ala Glu Leu Ser Asp Ala Phe Ile Ala Leu Pro Gly Gly Leu Gly Thr
            100                 105                 110

Phe Glu Glu Leu Phe Glu Val Trp Thr Trp Gly Gln Leu Gly Tyr His
        115                 120                 125

Gly Lys Pro Leu Gly Leu Leu Glu Val Asn Gly Phe Tyr Glu Lys Leu
    130                 135                 140

Ser Gly Phe Leu Asp His Val Val Glu Gln Gly Phe Val Arg Pro Gln
145                 150                 155                 160

His Arg Ser Met Leu Gln Met Asn Glu Ala Pro Asp Ala Leu Leu Asp
                165                 170                 175

Ala Leu Asp Ala Trp Glu Pro Val Val Gln Pro Lys Trp Val Asp Gln
            180                 185                 190

Pro Pro Ser
        195

<210>  59
<211>  199
<212>  PRT
<213>  Acinetobacter baylyi

<400>  59

Met Val Lys Lys Phe Met Asn Ser Val Cys Ile Phe Cys Gly Ser Ala
1               5                   10                  15

Val Gly Thr Asp Pro Val Phe Ile Gln Ala Ala Gln Ser Thr Gly Lys
            20                  25                  30

Arg Ile Ala Glu Gln Gly Lys Thr Leu Val Tyr Gly Gly Gly Arg Ser
        35                  40                  45

Gly Leu Met Gly Val Val Ala Asp Ser Ala Leu Gln Ala Gly Gly His
    50                  55                  60

Val Ile Gly Val Ile Pro Thr Ala Leu Val Asp Arg Glu Leu Ala His
65                  70                  75                  80

Pro His Leu Thr Glu Leu His Val Val Lys Asn Met His Glu Arg Lys
                85                  90                  95

Thr Lys Met Ala Glu Leu Ser Asp Gly Phe Ile Ala Leu Pro Gly Gly
            100                 105                 110

Ala Gly Thr Leu Glu Glu Ile Phe Glu Gln Trp Thr Trp Ala Gln Leu
        115                 120                 125

Gly Ile His Gln Lys Pro Cys Gly Phe Leu Asn Ile Lys Gly Phe Tyr
    130                 135                 140

Asp Asp Leu Leu Lys Met Ile Gln Gly Thr Val Asp Tyr Gly Phe Thr
145                 150                 155                 160

Gln Ser Arg Phe Ala Glu Lys Leu Ile Ala Ser Asp Asp Ile Glu Ile
                165                 170                 175

Ile Leu Asn Gln Phe Glu His Tyr Leu Ala Pro Thr Pro Lys Trp Gly
            180                 185                 190

Asp His Ala Lys Val His Pro
        195

<210>  60
<211>  185
<212>  PRT
<213>  Arcobacter mytili

<400>  60

Met Lys Ile Ala Val Phe Cys Gly Ser Ser Met Gly Asn Asn Ile Lys
1               5                   10                  15

Tyr Leu Asn Ala Thr Lys Ala Leu Gly Lys Phe Phe Ala Gln Asn Asn
            20                  25                  30

Ile Asp Val Val Tyr Gly Gly Gly Lys Val Gly Leu Met Gly Ala Ile
        35                  40                  45

Ala Asp Ser Val Met Glu Asn Gly Gly Lys Val Tyr Gly Val Ile Pro
    50                  55                  60

Glu Lys Leu Lys Ser Lys Glu Leu Ala His Thr Gly Ile Thr Glu Leu
65                  70                  75                  80

His Val Val Ser Thr Met His Glu Arg Lys Ala Met Met Ala Glu Leu

                85                  90                  95

Ala Asp Ala Phe Val Thr Leu Pro Gly Gly Ala Gly Thr Leu Glu Glu
            100                 105                 110

Ile Phe Glu Ala Trp Thr Trp Ala Gln Leu Gly Tyr His Asn Lys Pro
        115                 120                 125

Cys Ala Phe Tyr Asn Val Asp Gly Phe Tyr Asp Lys Leu Phe Glu Leu
    130                 135                 140

Ile Asn Asn Met Cys Glu Cys Glu Phe Leu Lys Pro Glu Tyr Ser Asn
145                 150                 155                 160

Met Leu Ile Lys Thr Asp Asn Lys Glu Glu Leu Leu Glu Ala Ile Lys
                165                 170                 175

Asn Tyr Lys Ala Pro Leu Asn Lys Trp
            180                 185

<210> 61
<211> 189
<212> PRT
<213> Blastococcus endophyticus

<400> 61

Met Arg Ile Ala Val Phe Thr Gly Ser Gln Ser Gly Pro Glu Ser His
1               5                   10                  15

Arg Thr Ala Ala Ala Ala Leu Ala Gly Asp Leu Ala Arg Ala Gly Val
            20                  25                  30

Gly Ile Val Tyr Gly Gly Gly His Val Gly Leu Met Gly Val Val Ala
        35                  40                  45

Asp Ala Ala Leu Ala Ala Gly Gly Glu Val Val Gly Val Ile Pro Gln
    50                  55                  60

His Leu Val Asp Asp Glu Leu Ala His Pro Arg Leu Pro Arg Leu Glu
65                  70                  75                  80

Ile Val Ser Ser Met His Glu Arg Lys Ala Arg Met Ala Glu Leu Ser
                85                  90                  95

Asp Ala Phe Val Ala Leu Pro Gly Ala Ala Gly Thr Leu Glu Glu Leu
            100                 105                 110

Phe Glu Ala Trp Thr Trp Gly Met Leu Gly Leu His Ala Lys Pro Thr
        115                 120                 125

Val Leu Val Asp Val Asp Gly Phe Trp Gln Pro Leu Leu Ala Gln Leu
    130                 135                 140

Arg Arg Met Ala Asp Asp Gly Tyr Leu Asp Arg Arg Arg Leu Asp Ala
145                 150                 155                 160

Leu Gly Val Val Thr Asp Ala Ala Gly Leu Leu Ala Phe Val Glu Gly
                165                 170                 175

Tyr Glu His Pro Pro Arg Lys Trp Thr Pro Thr Pro Ala
            180                 185

<210>  62
<211>  194
<212>  PRT
<213>  Burkholderia sp. OLGA172

<400>  62

Met Lys Ser Val Cys Val Tyr Cys Gly Ser Ser Asp Gly Val Arg Pro
1               5                   10                  15

Leu Tyr Ala Glu Ala Ala Arg Ala Phe Gly Arg Ala Leu Val Gln Ala
            20                  25                  30

Asp Leu Ala Leu Val Tyr Gly Gly Gly Lys Val Gly Leu Met Gly Val
        35                  40                  45

Ile Ala Asp Thr Val Met Ala Glu Gly Gly Arg Ala Val Gly Val Ile
    50                  55                  60

Pro Glu Leu Leu Val Asn Lys Glu Val Gly His Asn Gly Leu Thr Glu
65                  70                  75                  80

Leu His Val Val Pro Asp Met His His Arg Lys Lys Met Met Ala Asp
                85                  90                  95

Leu Ser Asp Ala Phe Val Ala Met Pro Gly Gly Ala Gly Thr Leu Glu
            100                 105                 110

Glu Leu Phe Glu Val Tyr Thr Trp Ala Gln Leu Gly Tyr His Gln Lys
        115                 120                 125

Pro Val Ala Leu Leu Asn Ile Asp Gly Phe Tyr Asp Pro Leu Ile Ala
    130                 135                 140

Leu Leu Lys His Thr Val Asp Glu Gly Phe Met Arg Gln Thr Tyr Phe
145                 150                 155                 160

Asp Ile Leu Gln Arg Asp Ala Asp Pro Val Ala Leu Ile Asp Lys Leu
                165                 170                 175

Gln Arg Tyr Lys Pro Pro Ala Ser Asp Lys Trp Ala Ile Lys Arg Asp
            180                 185                 190

Ala Val

<210>  63
<211>  633
<212>  PRT
<213>  Bacillus subtilis

<400>  63

Met Asp Leu Leu Ser Ile Gln Asp Pro Ser Phe Leu Lys Asn Met Ser
1               5                   10                  15

Ile Asp Glu Leu Glu Lys Leu Ser Asp Glu Ile Arg Gln Phe Leu Ile
            20                  25                  30

Thr Ser Leu Ser Ala Ser Gly Gly His Ile Gly Pro Asn Leu Gly Val
        35                  40                  45

Val Glu Leu Thr Val Ala Leu His Lys Glu Phe Asn Ser Pro Lys Asp
    50                  55                  60

Lys Phe Leu Trp Asp Val Gly His Gln Ser Tyr Val His Lys Leu Leu
65                  70                  75                  80

Thr Gly Arg Gly Lys Glu Phe Ala Thr Leu Arg Gln Tyr Lys Gly Leu
                85                  90                  95

Cys Gly Phe Pro Lys Arg Ser Glu Ser Glu His Asp Val Trp Glu Thr
            100                 105                 110

Gly His Ser Ser Thr Ser Leu Ser Gly Ala Met Gly Met Ala Ala Ala
        115                 120                 125

Arg Asp Ile Lys Gly Thr Asp Glu Tyr Ile Ile Pro Ile Ile Gly Asp
    130                 135                 140

Gly Ala Leu Thr Gly Gly Met Ala Leu Glu Ala Leu Asn His Ile Gly
145                 150                 155                 160

Asp Glu Lys Lys Asp Met Ile Val Ile Leu Asn Asp Asn Glu Met Ser
                165                 170                 175

Ile Ala Pro Asn Val Gly Ala Ile His Ser Met Leu Gly Arg Leu Arg
            180                 185                 190

Thr Ala Gly Lys Tyr Gln Trp Val Lys Asp Glu Leu Glu Tyr Leu Phe
        195                 200                 205

Lys Lys Ile Pro Ala Val Gly Gly Lys Leu Ala Ala Thr Ala Glu Arg
    210                 215                 220

Val Lys Asp Ser Leu Lys Tyr Met Leu Val Ser Gly Met Phe Phe Glu
225                 230                 235                 240

Glu Leu Gly Phe Thr Tyr Leu Gly Pro Val Asp Gly His Ser Tyr His
                245                 250                 255

Glu Leu Ile Glu Asn Leu Gln Tyr Ala Lys Lys Thr Lys Gly Pro Val
            260                 265                 270

Leu Leu His Val Ile Thr Lys Lys Gly Lys Gly Tyr Lys Pro Ala Glu
        275                 280                 285

Thr Asp Thr Ile Gly Thr Trp His Gly Thr Gly Pro Tyr Lys Ile Asn
    290                 295                 300

Thr Gly Asp Phe Val Lys Pro Lys Ala Ala Ala Pro Ser Trp Ser Gly
305                 310                 315                 320

Leu Val Ser Gly Thr Val Gln Arg Met Ala Arg Glu Asp Gly Arg Ile
                325                 330                 335

Val Ala Ile Thr Pro Ala Met Pro Val Gly Ser Lys Leu Glu Gly Phe
            340                 345                 350

Ala Lys Glu Phe Pro Asp Arg Met Phe Asp Val Gly Ile Ala Glu Gln
        355                 360                 365

His Ala Ala Thr Met Ala Ala Ala Met Ala Met Gln Gly Met Lys Pro
    370                 375                 380

Phe Leu Ala Ile Tyr Ser Thr Phe Leu Gln Arg Ala Tyr Asp Gln Val
385                 390                 395                 400

Val His Asp Ile Cys Arg Gln Asn Ala Asn Val Phe Ile Gly Ile Asp
                405                 410                 415

Arg Ala Gly Leu Val Gly Ala Asp Gly Glu Thr His Gln Gly Val Phe
            420                 425                 430

Asp Ile Ala Phe Met Arg His Ile Pro Asn Met Val Leu Met Met Pro
        435                 440                 445

Lys Asp Glu Asn Glu Gly Gln His Met Val His Thr Ala Leu Ser Tyr
    450                 455                 460

Asp Glu Gly Pro Ile Ala Met Arg Phe Pro Arg Gly Asn Gly Leu Gly
465                 470                 475                 480

Val Lys Met Asp Glu Gln Leu Lys Thr Ile Pro Ile Gly Thr Trp Glu
                485                 490                 495

Val Leu Arg Pro Gly Asn Asp Ala Val Ile Leu Thr Phe Gly Thr Thr
            500                 505                 510

Ile Glu Met Ala Ile Glu Ala Ala Glu Glu Leu Gln Lys Glu Gly Leu
        515                 520                 525

Ser Val Arg Val Val Asn Ala Arg Phe Ile Lys Pro Ile Asp Glu Lys
    530                 535                 540

Met Met Lys Ser Ile Leu Lys Glu Gly Leu Pro Ile Leu Thr Ile Glu
545                 550                 555                 560

Glu Ala Val Leu Glu Gly Gly Phe Gly Ser Ser Ile Leu Glu Phe Ala
                565                 570                 575

His Asp Gln Gly Glu Tyr His Thr Pro Ile Asp Arg Met Gly Ile Pro
            580                 585                 590

Asp Arg Phe Ile Glu His Gly Ser Val Thr Ala Leu Leu Glu Glu Ile
        595                 600                 605

Gly Leu Thr Lys Gln Gln Val Ala Asn Arg Ile Arg Leu Leu Met Pro
    610                 615                 620

Pro Lys Thr His Lys Gly Ile Gly Ser
625                 630

<210>  64
<211>  620
<212>  PRT
<213>  Escherichia coli

<400>  64

Met Ser Phe Asp Ile Ala Lys Tyr Pro Thr Leu Ala Leu Val Asp Ser
1               5                   10                  15

Thr Gln Glu Leu Arg Leu Leu Pro Lys Glu Ser Leu Pro Lys Leu Cys
            20                  25                  30

Asp Glu Leu Arg Arg Tyr Leu Leu Asp Ser Val Ser Arg Ser Ser Gly

35                  40                  45

His Phe Ala Ser Gly Leu Gly Thr Val Glu Leu Thr Val Ala Leu His
    50                  55                  60

Tyr Val Tyr Asn Thr Pro Phe Asp Gln Leu Ile Trp Asp Val Gly His
65                  70                  75                  80

Gln Ala Tyr Pro His Lys Ile Leu Thr Gly Arg Arg Asp Lys Ile Gly
                85                  90                  95

Thr Ile Arg Gln Lys Gly Gly Leu His Pro Phe Pro Trp Arg Gly Glu
            100                 105                 110

Ser Glu Tyr Asp Val Leu Ser Val Gly His Ser Ser Thr Ser Ile Ser
        115                 120                 125

Ala Gly Ile Gly Ile Ala Val Ala Ala Glu Lys Glu Gly Lys Asn Arg
    130                 135                 140

Arg Thr Val Cys Val Ile Gly Asp Gly Ala Ile Thr Ala Gly Met Ala
145                 150                 155                 160

Phe Glu Ala Met Asn His Ala Gly Asp Ile Arg Pro Asp Met Leu Val
                165                 170                 175

Ile Leu Asn Asp Asn Glu Met Ser Ile Ser Glu Asn Val Gly Ala Leu
            180                 185                 190

Asn Asn His Leu Ala Gln Leu Leu Ser Gly Lys Leu Tyr Ser Ser Leu
        195                 200                 205

Arg Glu Gly Gly Lys Lys Val Phe Ser Gly Val Pro Pro Ile Lys Glu
    210                 215                 220

Leu Leu Lys Arg Thr Glu Glu His Ile Lys Gly Met Val Val Pro Gly
225                 230                 235                 240

Thr Leu Phe Glu Glu Leu Gly Phe Asn Tyr Ile Gly Pro Val Asp Gly
                245                 250                 255

His Asp Val Leu Gly Leu Ile Thr Thr Leu Lys Asn Met Arg Asp Leu
            260                 265                 270

Lys Gly Pro Gln Phe Leu His Ile Met Thr Lys Lys Gly Arg Gly Tyr
        275                 280                 285

Glu Pro Ala Glu Lys Asp Pro Ile Thr Phe His Ala Val Pro Lys Phe
    290                 295                 300

Asp Pro Ser Ser Gly Cys Leu Pro Lys Ser Ser Gly Gly Leu Pro Ser
305                 310                 315                 320

Tyr Ser Lys Ile Phe Gly Asp Trp Leu Cys Glu Thr Ala Ala Lys Asp
                325                 330                 335

Asn Lys Leu Met Ala Ile Thr Pro Ala Met Arg Glu Gly Ser Gly Met
            340                 345                 350

Val Glu Phe Ser Arg Lys Phe Pro Asp Arg Tyr Phe Asp Val Ala Ile
        355                 360                 365

Ala Glu Gln His Ala Val Thr Phe Ala Ala Gly Leu Ala Ile Gly Gly
    370                 375                 380

Tyr Lys Pro Ile Val Ala Ile Tyr Ser Thr Phe Leu Gln Arg Ala Tyr
385                 390                 395                 400

Asp Gln Val Leu His Asp Val Ala Ile Gln Lys Leu Pro Val Leu Phe
                405                 410                 415

Ala Ile Asp Arg Ala Gly Ile Val Gly Ala Asp Gly Gln Thr His Gln
            420                 425                 430

Gly Ala Phe Asp Leu Ser Tyr Leu Arg Cys Ile Pro Glu Met Val Ile
        435                 440                 445

Met Thr Pro Ser Asp Glu Asn Glu Cys Arg Gln Met Leu Tyr Thr Gly
    450                 455                 460

Tyr His Tyr Asn Asp Gly Pro Ser Ala Val Arg Tyr Pro Arg Gly Asn

465                 470                 475                 480

Ala Val Gly Val Glu Leu Thr Pro Leu Glu Lys Leu Pro Ile Gly Lys
                485                 490                 495

Gly Ile Val Lys Arg Arg Gly Glu Lys Leu Ala Ile Leu Asn Phe Gly
            500                 505                 510

Thr Leu Met Pro Glu Ala Ala Lys Val Ala Glu Ser Leu Asn Ala Thr
        515                 520                 525

Leu Val Asp Met Arg Phe Val Lys Pro Leu Asp Glu Ala Leu Ile Leu
    530                 535                 540

Glu Met Ala Ala Ser His Glu Ala Leu Val Thr Val Glu Glu Asn Ala
545                 550                 555                 560

Ile Met Gly Gly Ala Gly Ser Gly Val Asn Glu Val Leu Met Ala His
                565                 570                 575

Arg Lys Pro Val Pro Val Leu Asn Ile Gly Leu Pro Asp Phe Phe Ile
            580                 585                 590

Pro Gln Gly Thr Gln Glu Glu Met Arg Ala Glu Leu Gly Leu Asp Ala
        595                 600                 605

Ala Gly Met Glu Ala Lys Ile Lys Ala Trp Leu Ala
    610                 615                 620

<210>  65
<211>  633
<212>  PRT
<213>  Corynebacterium stationis

<400>  65

Met Gly Ile Leu Asn Lys Val Lys Ser Pro Gln Asp Leu Lys Lys Leu
1               5                   10                  15

Ser Asp Asp Lys Val Glu Glu Leu Ala Ala Glu Ile Arg Gln Phe Leu
            20                  25                  30

Ile Asp Lys Val Ser Val Thr Gly Gly His Leu Gly Pro Asn Leu Gly
        35                  40                  45

Val Val Glu Leu Thr Ile Ala Leu His Arg Val Phe Asp Ser Pro Arg
    50                  55                  60

Asp Pro Ile Val Phe Asp Thr Ser His Gln Ser Tyr Val His Lys Ile
65                  70                  75                  80

Leu Thr Gly Arg Ala Asp Gln Phe Asp Thr Leu Arg Gln Lys Asp Gly
                85                  90                  95

Leu Ser Gly Tyr Thr Asp Arg Gly Glu Ser Glu His Asp Trp Thr Glu
            100                 105                 110

Ser Ser His Ala Ser Ala Ala Ile Ser Val Ile Asp Gly Leu Ser Lys
        115                 120                 125

Ala Phe Cys Ile Lys Gly Glu Ser Arg Arg Asn Ala Val Ala Val Val
    130                 135                 140

Gly Asp Gly Ala Leu Thr Gly Gly Met Cys Trp Glu Ala Leu Asn Asn
145                 150                 155                 160

Ile Ser Ala Asn Lys Glu Arg Asn Ala Val Ile Val Val Asn Asp Asn
                165                 170                 175

Gly Arg Ser Tyr Ser Pro Thr Ile Gly Gly Leu Ser Glu Asn Leu Ser
            180                 185                 190

Arg Ile Arg Thr Gln His Gly Tyr Asp Glu Leu Met Glu His Gly Lys
        195                 200                 205

Lys Thr Leu Lys Ser Leu Gly Trp Val Gly Asn Arg Thr Phe Asp Ala
    210                 215                 220

Leu Arg Ala Val Lys Glu Gly Val Lys Ser Ser Ile Leu Pro Thr Glu
225                 230                 235                 240

Met Phe Pro Glu Leu Gly Met Lys Tyr Ile Gly Pro Ile Asn Gly His
                245                 250                 255

Asp Leu Asp Ala Leu Asp His Ala Leu Ser Tyr Ala Arg Asp Tyr Glu
            260                 265                 270

Gly Pro Ile Ile Val His Val Val Thr Glu Lys Gly His Gly Phe Ala
        275                 280                 285

Pro Ala Val Asn Glu Pro Gln Asp Gln Met His Ser Thr Gly Ala Ile
    290                 295                 300

Asp Pro Val Thr Gly Val Pro Lys Gly Lys Ser Gln Pro Gly Trp Thr
305                 310                 315                 320

Ala Val Phe Ser Glu Glu Leu Ile Glu Ala Ala Lys Lys Arg Asp Asp
                325                 330                 335

Ile Val Ala Ile Thr Ala Ala Met Ala Gly Pro Thr Gly Leu Ile Pro
            340                 345                 350

Phe Ala Glu Gln Phe Pro Asn Arg Phe Phe Asp Val Gly Ile Ala Glu
        355                 360                 365

Gln His Ala Met Ala Ser Ala Ser Gly Leu Ala Leu Gly Gly Leu His
    370                 375                 380

Pro Val Val Ala Ile Tyr Ser Thr Phe Leu Asn Arg Ala Phe Asp Gln
385                 390                 395                 400

Leu Leu Met Asp Ile Ala Leu Leu Lys Gln Pro Val Thr Ile Val Leu
                405                 410                 415

Asp Arg Ala Gly Val Thr Gly Ser Asp Gly Ala Ser His Asn Gly Val
            420                 425                 430

Trp Asp Met Ala Leu Thr Thr Ile Ile Pro Gly Ile His Val Ala Ala
        435                 440                 445

Pro Arg Asp Gly Gln Arg Leu Arg Glu Leu Phe Gln Glu Ser Leu Glu
    450                 455                 460

Ile Lys Ser Gly Pro Ser Val Val Arg Phe Pro Lys Gly Asn Leu Leu
465                 470                 475                 480

Glu Asp Met Asp Ala Val Ala Thr Thr Glu Asp Gly Val Asp Ile Ile
                485                 490                 495

Phe Glu Ser Glu Gln Glu Arg Phe Ala Asp Ala Glu Ala Lys Lys Val
            500                 505                 510

Leu Val Ile Ser Ile Gly Ala Met Ala Ala Arg Ser Leu Gly Ala Ala
        515                 520                 525

Gln Ile Leu Glu Asp Lys Gly Met Ala Val Thr Val Val Asp Pro Arg
    530                 535                 540

Trp Leu Cys Pro Val Ala Pro Ser Leu Ile Glu Met Ala Asp Glu His
545                 550                 555                 560

Asp Ile Val Val Val Ala Glu Asp Gly Met Met Arg Ala Gly Val Gly
                565                 570                 575

Ser Leu Phe Asp Glu Ala Phe Ser Ala Ala Glu Val Asp Thr Pro Leu
            580                 585                 590

Arg Arg Val Ala Phe Pro Ser Ile Phe Pro Lys His Gly Ser Arg Gly
        595                 600                 605

Glu Val Leu Glu Glu Val Gly Met Asp Ala Asp Gly Ile Ala Ala Ala
    610                 615                 620

Val Thr Glu Trp Val Asp Asp Leu His
625                 630

<210>  66
<211>  642
<212>  PRT
<213>  Streptomyces avermitilis

<400>  66

Met Pro Leu Leu Thr Arg Ile Arg Gly Pro Arg Asp Leu Asp Arg Leu
1               5                   10                  15

Ser Leu Glu Gln Leu Asp Gln Leu Ala Glu Glu Ile Arg Thr Phe Leu
        20                  25                  30

Val Asp Ala Val Ser Lys Thr Gly Gly His Leu Gly Pro Asn Leu Gly
    35                  40                  45

Val Val Glu Leu Thr Ile Ala Leu His Arg Val Phe Asp Ser Pro Lys
    50                  55                  60

Asp Arg Val Leu Phe Asp Thr Gly His Gln Ser Tyr Val His Lys Leu
65                  70                  75                  80

Leu Thr Gly Arg Gln Asp Phe Ser Lys Leu Lys Met Lys Gly Gly Leu
                85                  90                  95

Ser Gly Tyr Pro Ser Gln Ala Glu Ser Glu His Asp Val Ile Glu Asn
            100                 105                 110

Ser His Ala Ser Thr Val Leu Gly Trp Ala Asp Gly Leu Ala Lys Ala
        115                 120                 125

Asn Gln Val Leu Glu Arg Asp Asp His Val Val Ala Val Ile Gly Asp
    130                 135                 140

Gly Ala Leu Thr Gly Gly Met Ala Trp Glu Ala Leu Asn Asn Ile Ala
145                 150                 155                 160

Ala Ala Lys Asp Arg Pro Leu Val Ile Val Val Asn Asp Asn Glu Arg
                165                 170                 175

Ser Tyr Ala Pro Thr Ile Gly Gly Leu Ala Asn His Leu Ala Thr Leu
            180                 185                 190

Arg Thr Thr Asp Gly Tyr Glu Arg Phe Leu Ala Arg Gly Lys Asp Leu
        195                 200                 205

Leu Glu Arg Thr Pro Val Val Gly Arg Pro Leu Tyr Glu Thr Leu His
    210                 215                 220

Gly Ala Lys Lys Gly Leu Lys Asp Phe Ile Ala Pro Gln Gly Met Phe

225                 230                 235                 240

Glu Asp Leu Gly Leu Lys Tyr Val Gly Pro Ile Asp Gly His Asp Ile
                245                 250                 255

Glu Ala Leu Glu Ser Ala Leu Ala Arg Ala Lys Arg Phe Gly Gly Pro
            260                 265                 270

Val Ile Val His Cys Leu Thr Glu Lys Gly Arg Gly Tyr Gln Pro Ala
        275                 280                 285

Leu Gln Asp Glu Ala Asp Arg Phe His Ala Val Gly Lys Ile His Pro
    290                 295                 300

Asp Thr Gly Leu Pro Ile Ala Ser Ser Gly Ala Asp Trp Thr Ser Val
305                 310                 315                 320

Phe Gly Glu Glu Met Val Lys Leu Gly Gln Glu Arg Lys Asp Ile Val
                325                 330                 335

Ala Ile Thr Ala Ala Met Leu Gln Pro Val Gly Leu Asp Lys Phe Ala
            340                 345                 350

Lys Val Phe Pro Lys Arg Val Tyr Asp Val Gly Ile Ala Glu Gln His
        355                 360                 365

Ala Ala Val Ser Ala Ala Gly Leu Ala Thr Gly Gly Leu His Pro Val
    370                 375                 380

Phe Ala Val Tyr Ala Thr Phe Leu Asn Arg Ala Phe Asp Gln Val Leu
385                 390                 395                 400

Met Asp Val Ala Leu His Lys Cys Gly Val Thr Phe Val Leu Asp Arg
                405                 410                 415

Ala Gly Val Thr Gly Thr Asp Gly Ala Ser His Asn Gly Met Trp Asp
            420                 425                 430

Met Ser Ile Leu Gln Val Val Pro Gly Leu Arg Leu Ala Ala Pro Arg
        435                 440                 445

Asp Ala Asp Gln Val Arg Ala Gln Leu Arg Glu Ala Val Asp Val Asp
    450                 455                 460

Asp Ala Pro Thr Val Val Arg Phe Ser Lys Gly Ala Val Gly Pro Ala
465                 470                 475                 480

Val Pro Ala Val Gly Arg Ile Gly Gly Met Asp Val Leu Arg Glu Pro
                485                 490                 495

Gly Thr Asp Ala Pro Asp Val Leu Leu Val Ser Val Gly Ala Leu Ala
            500                 505                 510

Pro Met Cys Leu Glu Ile Ala Gly Leu Leu Asp Lys Gln Gly Ile Ser
        515                 520                 525

Ala Thr Val Val Asp Pro Arg Trp Val Lys Pro Val Asp Glu Ala Met
    530                 535                 540

Ala Pro Leu Ala Glu Arg His Arg Val Val Val Thr Val Glu Asp Asn
545                 550                 555                 560

Ser Arg Val Gly Gly Val Gly Ser Ala Ile Ala Gln Ala Leu Arg Asp
                565                 570                 575

Ala Gly Val Asp Val Pro Leu Arg Asp Phe Gly Ile Pro Pro Arg Phe
            580                 585                 590

Leu Asp His Ala Ser Arg Gly Glu Val Met Ala Glu Ile Gly Leu Thr
        595                 600                 605

Ala Pro Asp Ile Ala Arg Gln Val Thr Gly Leu Val Ser Lys Leu Asp
    610                 615                 620

Gly Arg Phe Glu Arg Pro Thr Ala Glu Ile Asp Ser Val Glu Pro Ala
625                 630                 635                 640

Arg Asp

<210>  67

<211>  656
<212>  PRT
<213>  Streptomyces coelicolor

<400>  67

Met Thr Ile Leu Glu Asn Ile Arg Gly Pro Arg Asp Leu Lys Ala Leu
1               5                   10                  15

Ser Glu Ala Glu Leu Gly Glu Leu Ser Asp Glu Ile Arg Asp Phe Leu
            20                  25                  30

Val His Ala Val Ala Arg Thr Gly Gly His Leu Gly Pro Asn Leu Gly
        35                  40                  45

Val Val Glu Leu Thr Val Ala Leu His Arg Val Phe Glu Ser Pro Asp
    50                  55                  60

Asp Arg Ile Leu Trp Asp Thr Gly His Gln Ser Tyr Val His Lys Leu
65                  70                  75                  80

Leu Thr Gly Arg Gln Asp Phe Ser Lys Leu Arg Ser Lys Gly Gly Leu
                85                  90                  95

Ser Gly Tyr Pro Ser Arg Glu Glu Ser Ala His Asp Val Ile Glu Asn
            100                 105                 110

Ser His Ala Ser Thr Val Leu Gly Trp Ala Asp Gly Leu Ala Lys Ala
        115                 120                 125

Arg Gln Val Gln Gly Glu Arg Ser His Val Val Ala Val Ile Gly Asp
    130                 135                 140

Gly Ala Leu Thr Gly Gly Met Ala Trp Glu Ala Leu Asn Asn Ile Ala
145                 150                 155                 160

Ala Ala Arg Asp Arg Pro Leu Ile Ile Val Val Asn Asp Asn Glu Arg
                165                 170                 175

Ser Tyr Ala Pro Thr Ile Gly Gly Leu Ala Asn His Leu Ala Thr Leu
            180                 185                 190

Arg Thr Thr Asp Gly Tyr Glu Arg Ala Leu Ala Trp Gly Lys Asp Val
        195                 200                 205

Leu Gln Arg Thr Pro Val Val Gly His Thr Val Tyr Glu Ala Leu His
    210                 215                 220

Gly Ala Lys Lys Gly Phe Lys Asp Ala Phe Ala Pro Gln Gly Leu Phe
225                 230                 235                 240

Glu Asp Leu Gly Leu Lys Tyr Leu Gly Pro Ile Asp Gly His Asp Ile
                245                 250                 255

Gly Ala Val Glu Ser Ala Leu Arg Arg Ala Lys Arg Phe His Gly Pro
            260                 265                 270

Val Leu Val His Cys Leu Thr Glu Lys Gly Arg Gly Tyr Glu Pro Ala
        275                 280                 285

Leu Arg Asp Glu Glu Asp His Phe His Thr Val Gly Val Met Asp Pro
    290                 295                 300

Leu Thr Cys Glu Pro Leu Gly Pro Ala Gly Gly Pro Ser Trp Thr Ser
305                 310                 315                 320

Val Phe Gly Glu Glu Ile Val Arg Ile Gly Glu Glu Arg Ala Asp Val
                325                 330                 335

Val Ala Ile Thr Ala Ala Met Leu His Pro Val Gly Leu Ala Pro Phe
            340                 345                 350

Ala Glu Arg Phe Pro Asp Arg Val Trp Asp Val Gly Ile Ala Glu Gln
        355                 360                 365

His Ala Ala Val Ser Ala Ala Gly Leu Ala Thr Gly Gly Leu His Pro
    370                 375                 380

Val Val Ala Val Tyr Ala Thr Phe Leu Asn Arg Ala Phe Asp Gln Leu
385                 390                 395                 400

Leu Met Asp Val Ala Leu His Arg Cys Gly Val Thr Phe Val Leu Asp
                405                 410                 415

Arg Ala Gly Val Thr Gly Val Asp Gly Ala Ser His Asn Gly Met Trp
            420                 425                 430

Asp Met Ser Val Leu Gln Val Val Pro Gly Leu Arg Ile Ala Ala Pro
        435                 440                 445

Arg Asp Ala Asp Gln Leu Arg Thr Gln Leu Arg Glu Ala Val Ala Val
    450                 455                 460

Asp Asp Ala Pro Thr Leu Leu Arg Phe Pro Lys Glu Ser Val Gly Pro
465                 470                 475                 480

Ala Val Pro Ala Val Asp Arg Ile Gly Gly Leu Asp Val Leu His Thr
                485                 490                 495

Ala Asp Arg Ser Glu Val Leu Leu Val Ala Val Gly Val Met Ala Pro
            500                 505                 510

Val Cys Leu Gly Ala Ala Glu Leu Leu Glu Ala Arg Gly Ile Gly Cys
        515                 520                 525

Thr Val Val Asp Pro Arg Trp Val Lys Pro Val Asp Pro Ala Leu Ala
    530                 535                 540

Pro Leu Ala Ala Arg His Arg Leu Val Ala Val Val Glu Asp Asn Ser
545                 550                 555                 560

Arg Ala Ala Gly Val Gly Ser Ala Val Ala Leu Ala Leu Gly Asp Ala
                565                 570                 575

Glu Val Asp Val Pro Val Arg Arg Phe Gly Ile Pro Glu Gln Phe Leu
            580                 585                 590

Ala His Ala Lys Arg Ala Glu Val Leu Ala Asp Ile Gly Leu Thr Pro
        595                 600                 605

Val Asp Val Ala Gly Arg Ile Ala Ala Ser Leu Ala Leu Ala Glu Pro
    610                 615                 620

Ala Gly Asp Arg Ala Gly Gly Pro Ala Val Glu Gln Pro Gly Asp Gly
625                 630                 635                 640

Arg Met Ser Gly Asp Gly Arg Ile Val Met Pro Ala Gln Gly Glu Asn
                645                 650                 655

<210>  68
<211>  629
<212>  PRT
<213>  Deinococcus radiodurans

<400>  68

Met Asn Glu Leu Pro Gly Thr Ser Asp Thr Pro Leu Leu Asp Gln Ile
1               5                   10                  15

His Gly Pro Lys Asp Leu Lys Arg Leu Ser Arg Glu Gln Leu Pro Ala
            20                  25                  30

Leu Thr Glu Glu Leu Arg Gly Glu Ile Val Arg Val Cys Ser Arg Gly
        35                  40                  45

Gly Leu His Leu Ala Ser Ser Leu Gly Ala Val Asp Ile Ile Thr Ala
    50                  55                  60

Leu His Tyr Val Leu Asp Ser Pro Arg Asp Arg Ile Leu Phe Asp Val
65                  70                  75                  80

Gly His Gln Ala Tyr Ala His Lys Ile Leu Thr Gly Arg Arg Asp Gln
                85                  90                  95

Met Ala Asp Ile Lys Lys Glu Gly Gly Ile Ser Gly Phe Thr Lys Val
            100                 105                 110

Ser Glu Ser Glu His Asp Ala Ile Thr Val Gly His Ala Ser Thr Ser
        115                 120                 125

Leu Ala Asn Ala Leu Gly Met Ala Leu Ala Arg Asp Ala Gln Gly Lys
    130                 135                 140

Asp Phe His Val Ala Ala Val Ile Gly Asp Gly Ser Leu Thr Gly Gly
145                 150                 155                 160

Met Ala Leu Ala Ala Leu Asn Thr Ile Gly Asp Met Gly Arg Lys Met
                165                 170                 175

Leu Ile Val Leu Asn Asp Asn Glu Met Ser Ile Ser Glu Asn Val Gly
            180                 185                 190

Ala Met Asn Lys Phe Met Arg Gly Leu Gln Val Gln Lys Trp Phe Gln
        195                 200                 205

Glu Gly Glu Gly Ala Gly Lys Lys Ala Val Glu Ala Val Ser Lys Pro
    210                 215                 220

Leu Ala Asp Phe Met Ser Arg Ala Lys Asn Ser Thr Arg His Phe Phe
225                 230                 235                 240

Asp Pro Ala Ser Val Asn Pro Phe Ala Ala Met Gly Val Arg Tyr Val
                245                 250                 255

Gly Pro Val Asp Gly His Asn Val Gln Glu Leu Val Trp Leu Leu Glu
            260                 265                 270

Arg Leu Val Asp Leu Asp Gly Pro Thr Ile Leu His Ile Val Thr Thr
        275                 280                 285

Lys Gly Lys Gly Leu Ser Tyr Ala Glu Ala Asp Pro Ile Tyr Trp His
    290                 295                 300

Gly Pro Ala Lys Phe Asp Pro Ala Thr Gly Glu Tyr Val Pro Ser Ser
305                 310                 315                 320

Ala Tyr Ser Trp Ser Ala Ala Phe Gly Glu Ala Val Thr Glu Trp Ala
                325                 330                 335

Lys Thr Asp Pro Arg Thr Phe Val Val Thr Pro Ala Met Arg Glu Gly
            340                 345                 350

Ser Gly Leu Val Glu Phe Ser Arg Val His Pro His Arg Tyr Leu Asp
        355                 360                 365

Val Gly Ile Ala Glu Glu Val Ala Val Thr Thr Ala Ala Gly Met Ala

370                 375                 380

Leu Gln Gly Met Arg Pro Val Val Ala Ile Tyr Ser Thr Phe Leu Gln
385                 390                 395                 400

Arg Ala Tyr Asp Gln Val Leu His Asp Val Ala Ile Glu His Leu Asn
                405                 410                 415

Val Thr Phe Cys Ile Asp Arg Ala Gly Ile Val Gly Ala Asp Gly Ala
            420                 425                 430

Thr His Asn Gly Val Phe Asp Leu Ser Phe Leu Arg Ser Ile Pro Gly
        435                 440                 445

Val Arg Ile Gly Leu Pro Lys Asp Ala Ala Glu Leu Arg Gly Met Leu
    450                 455                 460

Lys Tyr Ala Gln Thr His Asp Gly Pro Phe Ala Ile Arg Tyr Pro Arg
465                 470                 475                 480

Gly Asn Thr Ala Gln Val Pro Ala Gly Thr Trp Pro Asp Leu Lys Trp
                485                 490                 495

Gly Glu Trp Glu Arg Leu Lys Gly Gly Asp Asp Val Val Ile Leu Ala
            500                 505                 510

Gly Gly Lys Ala Leu Asp Tyr Ala Leu Lys Ala Ala Glu Asp Leu Pro
        515                 520                 525

Gly Val Gly Val Val Asn Ala Arg Phe Val Lys Pro Leu Asp Glu Glu
    530                 535                 540

Met Leu Arg Glu Val Gly Gly Arg Ala Arg Ala Leu Ile Thr Val Glu
545                 550                 555                 560

Asp Asn Thr Val Val Gly Gly Phe Gly Gly Ala Val Leu Glu Ala Leu
                565                 570                 575

Asn Ser Met Asn Leu His Pro Thr Val Arg Val Leu Gly Ile Pro Asp
            580                 585                 590

Glu Phe Gln Glu His Ala Thr Ala Glu Ser Val His Ala Arg Ala Gly
        595                 600                 605

Ile Asp Ala Pro Ala Ile Arg Thr Val Leu Ala Glu Leu Gly Val Asp
    610                 615                 620

Val Pro Ile Glu Val
625

<210>  69
<211>  620
<212>  PRT
<213>  Artificial Sequence

<220>
<223>  Mutant Escherichia coli enzyme DXS (Y392F)

<400>  69

Met Ser Phe Asp Ile Ala Lys Tyr Pro Thr Leu Ala Leu Val Asp Ser
1               5                   10                  15

Thr Gln Glu Leu Arg Leu Leu Pro Lys Glu Ser Leu Pro Lys Leu Cys
            20                  25                  30

Asp Glu Leu Arg Arg Tyr Leu Leu Asp Ser Val Ser Arg Ser Ser Gly
        35                  40                  45

His Phe Ala Ser Gly Leu Gly Thr Val Glu Leu Thr Val Ala Leu His
    50                  55                  60

Tyr Val Tyr Asn Thr Pro Phe Asp Gln Leu Ile Trp Asp Val Gly His
65                  70                  75                  80

Gln Ala Tyr Pro His Lys Ile Leu Thr Gly Arg Arg Asp Lys Ile Gly
                85                  90                  95

Thr Ile Arg Gln Lys Gly Gly Leu His Pro Phe Pro Trp Arg Gly Glu
            100                 105                 110

Ser Glu Tyr Asp Val Leu Ser Val Gly His Ser Ser Thr Ser Ile Ser
        115                 120                 125

Ala Gly Ile Gly Ile Ala Val Ala Ala Glu Lys Glu Gly Lys Asn Arg
    130                 135                 140

Arg Thr Val Cys Val Ile Gly Asp Gly Ala Ile Thr Ala Gly Met Ala
145                 150                 155                 160

Phe Glu Ala Met Asn His Ala Gly Asp Ile Arg Pro Asp Met Leu Val
                165                 170                 175

Ile Leu Asn Asp Asn Glu Met Ser Ile Ser Glu Asn Val Gly Ala Leu
            180                 185                 190

Asn Asn His Leu Ala Gln Leu Leu Ser Gly Lys Leu Tyr Ser Ser Leu
        195                 200                 205

Arg Glu Gly Gly Lys Lys Val Phe Ser Gly Val Pro Pro Ile Lys Glu
    210                 215                 220

Leu Leu Lys Arg Thr Glu Glu His Ile Lys Gly Met Val Val Pro Gly
225                 230                 235                 240

Thr Leu Phe Glu Glu Leu Gly Phe Asn Tyr Ile Gly Pro Val Asp Gly
                245                 250                 255

His Asp Val Leu Gly Leu Ile Thr Thr Leu Lys Asn Met Arg Asp Leu
            260                 265                 270

Lys Gly Pro Gln Phe Leu His Ile Met Thr Lys Lys Gly Arg Gly Tyr
        275                 280                 285

Glu Pro Ala Glu Lys Asp Pro Ile Thr Phe His Ala Val Pro Lys Phe
    290                 295                 300

Asp Pro Ser Ser Gly Cys Leu Pro Lys Ser Ser Gly Gly Leu Pro Ser
305                 310                 315                 320

Tyr Ser Lys Ile Phe Gly Asp Trp Leu Cys Glu Thr Ala Ala Lys Asp
                325                 330                 335

Asn Lys Leu Met Ala Ile Thr Pro Ala Met Arg Glu Gly Ser Gly Met

            340                 345                 350

Val Glu Phe Ser Arg Lys Phe Pro Asp Arg Tyr Phe Asp Val Ala Ile
        355                 360                 365

Ala Glu Gln His Ala Val Thr Phe Ala Ala Gly Leu Ala Ile Gly Gly
    370                 375                 380

Tyr Lys Pro Ile Val Ala Ile Phe Ser Thr Phe Leu Gln Arg Ala Tyr
385                 390                 395                 400

Asp Gln Val Leu His Asp Val Ala Ile Gln Lys Leu Pro Val Leu Phe
                405                 410                 415

Ala Ile Asp Arg Ala Gly Ile Val Gly Ala Asp Gly Gln Thr His Gln
            420                 425                 430

Gly Ala Phe Asp Leu Ser Tyr Leu Arg Cys Ile Pro Glu Met Val Ile
        435                 440                 445

Met Thr Pro Ser Asp Glu Asn Glu Cys Arg Gln Met Leu Tyr Thr Gly
    450                 455                 460

Tyr His Tyr Asn Asp Gly Pro Ser Ala Val Arg Tyr Pro Arg Gly Asn
465                 470                 475                 480

Ala Val Gly Val Glu Leu Thr Pro Leu Glu Lys Leu Pro Ile Gly Lys
                485                 490                 495

Gly Ile Val Lys Arg Arg Gly Glu Lys Leu Ala Ile Leu Asn Phe Gly
            500                 505                 510

Thr Leu Met Pro Glu Ala Ala Lys Val Ala Glu Ser Leu Asn Ala Thr
        515                 520                 525

Leu Val Asp Met Arg Phe Val Lys Pro Leu Asp Glu Ala Leu Ile Leu
    530                 535                 540

Glu Met Ala Ala Ser His Glu Ala Leu Val Thr Val Glu Glu Asn Ala
545                 550                 555                 560

Ile Met Gly Gly Ala Gly Ser Gly Val Asn Glu Val Leu Met Ala His
                565                 570                 575

Arg Lys Pro Val Pro Val Leu Asn Ile Gly Leu Pro Asp Phe Phe Ile
            580                 585                 590

Pro Gln Gly Thr Gln Glu Glu Met Arg Ala Glu Leu Gly Leu Asp Ala
        595                 600                 605

Ala Gly Met Glu Ala Lys Ile Lys Ala Trp Leu Ala
    610                 615                 620

<210>  70
<211>  633
<212>  PRT
<213>  Artificial Sequence

<220>
<223>  Mutant Bacillus subtilis enzyme DXS (Y389F)

<400>  70

Met Asp Leu Leu Ser Ile Gln Asp Pro Ser Phe Leu Lys Asn Met Ser
1               5                   10                  15

Ile Asp Glu Leu Glu Lys Leu Ser Asp Glu Ile Arg Gln Phe Leu Ile
            20                  25                  30

Thr Ser Leu Ser Ala Ser Gly Gly His Ile Gly Pro Asn Leu Gly Val
        35                  40                  45

Val Glu Leu Thr Val Ala Leu His Lys Glu Phe Asn Ser Pro Lys Asp
    50                  55                  60

Lys Phe Leu Trp Asp Val Gly His Gln Ser Tyr Val His Lys Leu Leu
65                  70                  75                  80

Thr Gly Arg Gly Lys Glu Phe Ala Thr Leu Arg Gln Tyr Lys Gly Leu
                85                  90                  95

Cys Gly Phe Pro Lys Arg Ser Glu Ser Glu His Asp Val Trp Glu Thr
            100                 105                 110

Gly His Ser Ser Thr Ser Leu Ser Gly Ala Met Gly Met Ala Ala Ala
        115                 120                 125

Arg Asp Ile Lys Gly Thr Asp Glu Tyr Ile Ile Pro Ile Ile Gly Asp
    130                 135                 140

Gly Ala Leu Thr Gly Gly Met Ala Leu Glu Ala Leu Asn His Ile Gly
145                 150                 155                 160

Asp Glu Lys Lys Asp Met Ile Val Ile Leu Asn Asp Asn Glu Met Ser
                165                 170                 175

Ile Ala Pro Asn Val Gly Ala Ile His Ser Met Leu Gly Arg Leu Arg
            180                 185                 190

Thr Ala Gly Lys Tyr Gln Trp Val Lys Asp Glu Leu Glu Tyr Leu Phe
        195                 200                 205

Lys Lys Ile Pro Ala Val Gly Gly Lys Leu Ala Ala Thr Ala Glu Arg
    210                 215                 220

Val Lys Asp Ser Leu Lys Tyr Met Leu Val Ser Gly Met Phe Phe Glu
225                 230                 235                 240

Glu Leu Gly Phe Thr Tyr Leu Gly Pro Val Asp Gly His Ser Tyr His
                245                 250                 255

Glu Leu Ile Glu Asn Leu Gln Tyr Ala Lys Lys Thr Lys Gly Pro Val
            260                 265                 270

Leu Leu His Val Ile Thr Lys Lys Gly Lys Gly Tyr Lys Pro Ala Glu
        275                 280                 285

Thr Asp Thr Ile Gly Thr Trp His Gly Thr Gly Pro Tyr Lys Ile Asn
    290                 295                 300

Thr Gly Asp Phe Val Lys Pro Lys Ala Ala Ala Pro Ser Trp Ser Gly
305                 310                 315                 320

Leu Val Ser Gly Thr Val Gln Arg Met Ala Arg Glu Asp Gly Arg Ile

                325                 330                 335

Val Ala Ile Thr Pro Ala Met Pro Val Gly Ser Lys Leu Glu Gly Phe
            340                 345                 350

Ala Lys Glu Phe Pro Asp Arg Met Phe Asp Val Gly Ile Ala Glu Gln
        355                 360                 365

His Ala Ala Thr Met Ala Ala Ala Met Ala Met Gln Gly Met Lys Pro
    370                 375                 380

Phe Leu Ala Ile Phe Ser Thr Phe Leu Gln Arg Ala Tyr Asp Gln Val
385                 390                 395                 400

Val His Asp Ile Cys Arg Gln Asn Ala Asn Val Phe Ile Gly Ile Asp
                405                 410                 415

Arg Ala Gly Leu Val Gly Ala Asp Gly Glu Thr His Gln Gly Val Phe
            420                 425                 430

Asp Ile Ala Phe Met Arg His Ile Pro Asn Met Val Leu Met Met Pro
        435                 440                 445

Lys Asp Glu Asn Glu Gly Gln His Met Val His Thr Ala Leu Ser Tyr
    450                 455                 460

Asp Glu Gly Pro Ile Ala Met Arg Phe Pro Arg Gly Asn Gly Leu Gly
465                 470                 475                 480

Val Lys Met Asp Glu Gln Leu Lys Thr Ile Pro Ile Gly Thr Trp Glu
                485                 490                 495

Val Leu Arg Pro Gly Asn Asp Ala Val Ile Leu Thr Phe Gly Thr Thr
            500                 505                 510

Ile Glu Met Ala Ile Glu Ala Ala Glu Glu Leu Gln Lys Glu Gly Leu
        515                 520                 525

Ser Val Arg Val Val Asn Ala Arg Phe Ile Lys Pro Ile Asp Glu Lys
    530                 535                 540

Met Met Lys Ser Ile Leu Lys Glu Gly Leu Pro Ile Leu Thr Ile Glu
545                 550                 555                 560

Glu Ala Val Leu Glu Gly Gly Phe Gly Ser Ser Ile Leu Glu Phe Ala
                565                 570                 575

His Asp Gln Gly Glu Tyr His Thr Pro Ile Asp Arg Met Gly Ile Pro
            580                 585                 590

Asp Arg Phe Ile Glu His Gly Ser Val Thr Ala Leu Leu Glu Glu Ile
        595                 600                 605

Gly Leu Thr Lys Gln Gln Val Ala Asn Arg Ile Arg Leu Leu Met Pro
    610                 615                 620

Pro Lys Thr His Lys Gly Ile Gly Ser
625                 630

<210>  71
<211>  1451
<212>  DNA
<213>  Artificial Sequence

<220>
<223>  Synthetic DNA fragment tzs-LOG (IPT-LOG)

<400>  71
cgcagcatac gcagcgaaat cagcatcacc ggagaatccc aacgaagcca actagtatgt      60

tattacacct gatctacggt cctacatgta gtggtaaaac tgatatggct atacaaatcg     120

cgcaagaaac aggttggccc gtcgttgctc ttgatcgtgt gcagtgctgt ccacagattg     180

cgacaggatc cggcagacct ctggaaagcg agttacaatc tacccggcgg atttatttag     240

actcacgccc tttgacagaa ggcattcttg acgcggaatc cgctcatcgc cgtcttatat     300

tcgaggtaga ttggaggaaa tcggaagaag gcttaatcct cgaaggaggg tccatctcgc     360

ttctaaattg tatggctaag tctccatttt ggagatctgg attccagtgg cacgtcaagc     420

gcctgagact tggggattca gatgcgtttc tgacgcgagc aaaacagcgt gtggctgaga     480

tgtttgctat tagagaagat cggccaagtt tattggagga gctcgccgag ctgtggaatt     540

acccggcagc tcgcccgatt ttggaagaca ttgacggcta tcgatgtgcc attagatttg     600

caaggaaaca cgatctcgcc atatcacagc tgccgaacat cgatgccggc cggcatgttg 660

aactgatcga agccattgca aacgaatatc ttgaacatgc gctaagccaa gaacgcgact 720

ttcctcaatg gccggaggac ggcgcaggac aaccagtatg cccggttacc ttaacgagaa 780

tccgttaaaa aagaggggag ggaaacatta tgatgacatc gctttttgac gcgcctacgc 840

ttcaacgcgt aacggttttc actgggagcg ccttgggctc ctctagtctt tatactcagg 900

ctgctcaaac cctggctaaa accgcggtag accgagggat tgatctggtt tatggcggtg 960

gaaaagtcgg cctcatgggt attgttgctg atgcattcct tgaaagcggg ggtgaggcat 1020

tcggcgtgat aacagagtca ctgatgaaag gagaattagg ccatgagaaa ctgacggagt 1080

tggaaatcgt gcccgacatg catattcgta aaaggcggat ggcggaactt ggggatggat 1140

tcattgcgat gcctggagga gccggcaccc tggaagaact gtttgaagtc tggacatggc 1200

agcagttagg aattcatcaa aagccggttg cactctatga cgtcgatggc ttctggcaac 1260

cgttattgga aatgttggaa caaatgacac agagaggatt tatcaagaga gatttttttg 1320

aatgcctcat cgtcgaatca gatccgcacg cattattaaa ggccatgcag acatggacac 1380

ctccagcccc aaaatggtaa attaattcta gagtcgatcc ccgggttcgc cagcaatgac 1440

taccggcagc c 1451

<210> 72
<211> 1442
<212> DNA
<213> Artificial Sequence

<220>
<223> Synthetic DNA fragment tmr-LOG (IPT-LOG)

<400> 72
cgcagcatac gcagcgaaat cagcatcacc ggagaatccc aacgaagcca actagtatgg 60

atttaagact gattttcggt ccaacctgta cgggaaagac ttccaccgca gttgcgttag 120

ctcaacagac aggcctcccg gttttatcat tggatcgggt tcagtgttgc ccgcaactgt 180

ccacgggctc tggccgaccc acagtggagg aactaaaagg aacatcgcgg ttatatttgg 240

atgaccgacc attggtgaaa ggtataatag ctgcgaaaca ggcacacgag agattgatgg 300

gcgaagtgta caactatgaa gcgcatgggg ggctgatttt ggagggagga agtatctcac 360

tgttaaaatg catggctcaa tcaagctatt ggagcgctga tttccgctgg catattataa 420

gacacgaatt ggcagatgaa gaaacattta tgaatgtcgc gaaagcacgg gtaaagcaga 480

tgttacgtcc tgccgcaggc cttagcatca ttcaagagtt agtggatctt tggaaggagc 540

cgcgccttag acctatcctg aaagaaatag atggctatcg ttacgctatg ctctttgcat 600

ctcagaatca gattacttct gatatgctgc tgcaacttga cgcggacatg gaagacaaac 660

tcattcatgg tattgcccaa gaatacctta ttcatgcgag gcgccaagaa caaaagtttc 720

ccagagtaaa cgccgccgcc tatgacgggt ttgaaggcca tccgtttgga atgtattaaa 780

aaagagggga gggaaacatt atgatgacat cgctttttga cgcgcctacg cttcaacgcg 840

taacggtttt cactgggagc gccttgggct cctctagtct ttatactcag gctgctcaaa 900

ccctggctaa aaccgcggta gaccgaggga ttgatctggt ttatggcggt ggaaaagtcg 960

gcctcatggg tattgttgct gatgcattcc ttgaaagcgg gggtgaggca ttcggcgtga 1020

taacagagtc actgatgaaa ggagaattag gccatgagaa actgacggag ttggaaatcg 1080

tgcccgacat gcatattcgt aaaaggcgga tggcggaact tggggatgga ttcattgcga 1140

tgcctggagg agccggcacc ctggaagaac tgtttgaagt ctggacatgg cagcagttag 1200

gaattcatca aaagccggtt gcactctatg acgtcgatgg cttctggcaa ccgttattgg 1260

aaatgttgga acaaatgaca cagagaggat ttatcaagag agattttttt gaatgcctca 1320

tcgtcgaatc agatccgcac gcattattaa aggccatgca gacatggaca cctccagccc 1380

caaaatggta aattaattct agagtcgatc cccgggttcg ccagcaatga ctaccggcag 1440

cc 1442

<210> 73
<211> 958
<212> DNA
<213> Artificial Sequence

<220>
<223> Erythromycin selectable marker

<400> 73
gagggattcg tcatgttggt attccaaatg cgttatgtag ataaaacatc tactgttttg 60

aaacagacta aaaacagtga ttacgcagat aaataaatac gttagattaa ttcctaccag 120

tgactaatct tatgactttt taaacagata actaaaatta caaacaaatc gtttaacttc 180

tgtatttgtt tatagatgta atcacttcag gagtgattac atgaacaaaa atataaaata 240

ttctcaaaac tttttaacga gtgaaaaagt actcaaccaa ataataaaac aattgaattt 300

aaaagaaacc gataccgttt acgaaattgg aacaggtaaa gggcatttaa cgacgaaact 360

ggctaaaata agtaaacagg taacgtctat tgaattagac agtcatctat tcaacttatc 420

gtcagaaaaa ttaaaactga acattcgtgt cactttaatt caccaagata ttctacagtt 480

tcaattccct aacaaacaga ggtataaaat tgttgggaat attccttacc atttaagcac 540

acaaattatt aaaaaagtgg tttttgaaag ccatgcgtct gacatctatc tgattgttga 600

agaaggattc tacaagcgta ccttggatat tcaccgaaca ctagggttgc tcttgcacac 660

tcaagtctcg attcagcaat tgcttaagct gccagcggaa tgctttcatc ctaaaccaaa 720

agtaaacagt gtcttaataa aacttacccg ccataccaca gatgttccag ataaatattg 780

gaagctatat acgtactttg tttcaaaatg ggtcaatcga gaatatcgtc aactgtttac 840

taaaaatcag tttcatcaag caatgaaaca cgccaaagta aacaatttaa gtaccgttac 900

ttatgagcaa gtattgtcta tttttaatag ttatctatta tttaacggga ggaaataa 958

<210> 74
<211> 22
<212> DNA
<213> Artificial Sequence

<220>
<223> Primer TwistBio For

<400> 74
gaagtgccat tccgcctgac ct 22

<210> 75
<211> 21
<212> DNA
<213> Artificial Sequence

<220>
<223> Primer TwistBio Rev

<400> 75
cactgagcct ccacctagcc t 21

<210> 76
<211> 1680
<212> DNA
<213> Artificial Sequence

<220>
<223>  Initial fragment amyE

<400>  76
gaagtgccat tccgcctgac cttcgccttt gcggtagtgg tgcttacgat gtacgacagg      60

gggattcccc atacattctt cgcttggctg aaaatgattc ttctttttat cgtctgcggc     120

ggcgttctgt ttctgcttcg gtatgtgatt gtgaagctgg cttacagaag agcggtaaaa     180

gaagaaataa aaaagaaatc atcttttttg tttggaaagc gagggaagcg ttcacagttt     240

cgggcagctt tttttatagg aacattgatt tgtattcact ctgccaagtt gttttgatag     300

agtgattgtg ataattttaa atgtaagcgt taacaaaatt ctccagtctt cacatcggtt     360

tgaaaggagg aagcggaaga atgaagtaag agggattttt gactccgaag taagtcttca     420

aaaaatcaaa taaggagtgt caagaatgtt tgcaaaacga ttcaaaacct ctttactgcc     480

gttattcgct ggatttttat tgctgtttca tttggttctg gcaggaccgg cggctgcgag     540

tgctgaaacg gcgaacaaat cgaatgagct tacagcaccg tcgatcaaaa gcggaaccat     600

tcttcatgca tggaattggt cgttcaatac gttaaaacac aatatgaagg atattcatga     660

tgcaggatat acagccattc agacatctcc gattaaccaa gtaaaggaag ggaatcaagg     720

agataaaagc atgtcgaact ggtactggct gtatcagccg acatcgtatc aaattggcaa     780

ccgttactta ggtactgaac aagaatttaa agaaatgtgt gcagccgctg aagaatatgg     840

cataaaggtc attgttgacg cggtcatcaa tcataccacc agtgattatg ccgcgatttc     900

caatgaggtt aagagtattc caaactggac acatggaaac acacaaatta aaaactggtc     960

tgatcgatgg gatgtcacgc agaattcatt gctcgggctg tatgactgga atacacaaaa    1020

tacacaagta cagtcctatc tgaaacggtt cttagacagg gcattgaatg acggggcaga    1080

cggttttcga tttgatgccg ccaaacatat agagcttcca gatgatggca gttacggcag    1140

tcaattttgg ccgaatatca caaatacatc tgcagagttc caatacggag aaatcctgca    1200

ggatagtgcc tccagagatg ctgcttatgc gaattatatg gatgtgacag cgtctaacta    1260

tgggcattcc ataaggtccg ctttaaagaa tcgtaatctg ggcgtgtcga atatctccca    1320

ctatgcatct gatgtgtctg cggacaagct agtgacatgg gtagagtcgc atgatacacg    1380

ggtgcgcatg atcgtatggt tcactgtcca ccaaccaaaa ctgtgctcag taccgccaat    1440

atttctccct tggggggtac aaagaggtgt ccctagaaga gatccacgct gtgtaaaaat    1500

tttacaaaaa ggtattgact ttccctacag ggtgtgtaat aatttaatta caggcggggg 1560

caaccccgct cagtacctag agcgtaaaag aggggaggga aacactagtt ggcttcgttg 1620

ggattctccg gtgatgctga tttcgctgcg tatgctgcga ggctaggtgg aggctcagtg 1680

<210> 77
<211> 32
<212> DNA
<213> Artificial Sequence

<220>
<223> Primer For for end fragment with erythromycin selectable marker

<400> 77
aaatctagaa attaagaagg agggattcgt ca 32

<210> 78
<211> 44
<212> DNA
<213> Artificial Sequence

<220>
<223> Primer Rev for end fragment with erythromycin selectable marker

<400> 78
cactgagcct ccacctagcc taatactcat ggcttgtgac cgct 44

<210> 79
<211> 1911
<212> DNA
<213> Artificial Sequence

<220>
<223> End fragment containing erythromycin selectable marker

<400> 79
gaagtgccat tccgcctgac ctcacggtag tcattgctgg cgaacgcgtc tagaaattaa 60

gaaggaggga ttcgtcatgt tggtattcca aatgcgttat gtagataaaa catctactgt 120

tttgaaacag actaaaaaca gtgattacgc agataaataa atacgttaga ttaattccta 180

ccagtgacta atcttatgac tttttaaaca gataactaaa attacaaaca aatcgtttaa 240

cttctgtatt tgtttataga tgtaatcact tcaggagtga ttacatgaac aaaaatataa 300

aatattctca aaacttttta acgagtgaaa aagtactcaa ccaaataata aaacaattga 360

atttaaaaga aaccgatacc gtttacgaaa ttggaacagg taaagggcat ttaacgacga 420

aactggctaa aataagtaaa caggtaacgt ctattgaatt agacagtcat ctattcaact 480

tatcgtcaga aaaattaaaa ctgaacattc gtgtcacttt aattcaccaa gatattctac 540

agtttcaatt ccctaacaaa cagaggtata aaattgttgg gaatattcct taccatttaa 600

gcacacaaat tattaaaaaa gtggtttttg aaagccatgc gtctgacatc tatctgattg 660

ttgaagaagg attctacaag cgtaccttgg atattcaccg aacactaggg ttgctcttgc 720

acactcaagt ctcgattcag caattgctta agctgccagc ggaatgcttt catcctaaac 780

caaaagtaaa cagtgtctta ataaaactta cccgccatac cacagatgtt ccagataaat 840

attggaagct atatacgtac tttgtttcaa aatgggtcaa tcgagaatat cgtcaactgt 900

ttactaaaaa tcagtttcat caagcaatga aacacgccaa agtaaacaat ttaagtaccg 960

ttacttatga gcaagtattg tctattttta atagttatct attatttaac gggaggaaat 1020

aattctatga gtcgcttttg taaatttgga aagttacacg ttactaaagg gaatgtagat 1080

ggatcctttt aatggagtgt acgccaagcg cggtcaattg gcataacgtc aacaaggcaa 1140

agcgcccggg catgaatctg ctgtcatcca tgcaaatgat tgcccacggc tcggacagcg 1200

ttctctattt ccaataccgc aaatcacggg ggtcatcaga aaaattacac ggagcggttg 1260

tggatcatga caatagcccg aagaaccgcg tctttcaaga agtggccaag gtaggcgaga 1320

cattggaacg gctgtccgaa gttgtcggaa cgaagaggcc ggctcaaacc gcgattttat 1380

atgactggga aaatcattgg gcgctcgagg atgctcaggg gtttgcgaag gcgacaaaac 1440

gttatccgca aacgcttcag cagcattacc gcacattctg ggaacacgat atccctgtcg 1500

acgtcatcac gaaagaacaa gacttttcac catataaact gctgatcgtc ccgatgctgt 1560

atttaatcag cgaggacacc gtttcccgtt taaaagcgtt tacggctgac ggcggcacct 1620

tagtcatgac gtatatcagc ggggttgtga atgagcatga cttaacatac acaggcggat 1680

ggcatccgga tcttcaagct atatttggag ttgagcctct tgaaacggac accctgtatc 1740

cgaaggatcg aaacgctgtc agctaccgca gccaaatata tgaaatgaag gattatgcaa 1800

ccgtgattga tgtaaagaca gcttcagtgg aagcggtgta tcaagaagat ttttatgcgc 1860

gcacgccagc ggtcacaagc catgagtatt aggctaggtg gaggctcagt g 1911

<210> 80
<211> 20
<212> DNA

<213>  Artificial Sequence

<220>
<223>  Primer For for IPT-LOG operon

<400>  80
gcggtagtgg tgcttacgat                                                   20


<210>  81
<211>  25
<212>  DNA
<213>  Artificial Sequence

<220>
<223>  Primer Rev for IPT-LOG operon

<400>  81
aggatccatc tacattccct ttagt                                             25


<210>  82
<211>  3968
<212>  DNA
<213>  Artificial Sequence

<220>
<223>  Complete synthetic trans-zeatin operon containing IPT-LOG (with
       TZS)

<400>  82
gcggtagtgg tgcttacgat gtacgacagg gggattcccc atacattctt cgcttggctg      60

aaaatgattc ttctttttat cgtctgcggc ggcgttctgt ttctgcttcg gtatgtgatt     120

gtgaagctgg cttacagaag agcggtaaaa gaagaaataa aaaagaaatc atcttttttg     180

tttggaaagc gagggaagcg ttcacagttt cgggcagctt tttttatagg aacattgatt     240

tgtattcact ctgccaagtt gttttgatag agtgattgtg ataattttaa atgtaagcgt     300

taacaaaatt ctccagtctt cacatcggtt tgaaaggagg aagcggaaga atgaagtaag     360

agggattttt gactccgaag taagtcttca aaaaatcaaa taaggagtgt caagaatgtt     420

tgcaaaacga ttcaaaacct ctttactgcc gttattcgct ggatttttat tgctgtttca     480

tttggttctg gcaggaccgg cggctgcgag tgctgaaacg gcgaacaaat cgaatgagct     540

tacagcaccg tcgatcaaaa gcggaaccat tcttcatgca tggaattggt cgttcaatac     600

gttaaaacac aatatgaagg atattcatga tgcaggatat acagccattc agacatctcc     660

gattaaccaa gtaaaggaag ggaatcaagg agataaaagc atgtcgaact ggtactggct     720

gtatcagccg acatcgtatc aaattggcaa ccgttactta ggtactgaac aagaatttaa 780

agaaatgtgt gcagccgctg aagaatatgg cataaaggtc attgttgacg cggtcatcaa 840

tcataccacc agtgattatg ccgcgatttc caatgaggtt aagagtattc caaactggac 900

acatggaaac acacaaatta aaaactggtc tgatcgatgg gatgtcacgc agaattcatt 960

gctcgggctg tatgactgga atacacaaaa tacacaagta cagtcctatc tgaaacggtt 1020

cttagacagg gcattgaatg acggggcaga cggttttcga tttgatgccg ccaaacatat 1080

agagcttcca gatgatggca gttacggcag tcaattttgg ccgaatatca caaatacatc 1140

tgcagagttc caatacggag aaatcctgca ggatagtgcc tccagagatg ctgcttatgc 1200

gaattatatg gatgtgacag cgtctaacta tgggcattcc ataaggtccg ctttaaagaa 1260

tcgtaatctg ggcgtgtcga atatctccca ctatgcatct gatgtgtctg cggacaagct 1320

agtgacatgg gtagagtcgc atgatacacg ggtgcgcatg atcgtatggt tcactgtcca 1380

ccaaccaaaa ctgtgctcag taccgccaat atttctccct tggggggtac aaagaggtgt 1440

ccctagaaga gatccacgct gtgtaaaaat tttacaaaaa ggtattgact ttccctacag 1500

ggtgtgtaat aatttaatta caggcggggg caaccccgct cagtacctag agcgtaaaag 1560

aggggaggga aacactagta tgttattaca cctgatctac ggtcctacat gtagtggtaa 1620

aactgatatg gctatacaaa tcgcgcaaga aacaggttgg cccgtcgttg ctcttgatcg 1680

tgtgcagtgc tgtccacaga ttgcgacagg atccggcaga cctctggaaa gcgagttaca 1740

atctacccgg cggatttatt tagactcacg ccctttgaca gaaggcattc ttgacgcgga 1800

atccgctcat cgccgtctta tattcgaggt agattggagg aaatcggaag aaggcttaat 1860

cctcgaagga gggtccatct cgcttctaaa ttgtatggct aagtctccat tttggagatc 1920

tggattccag tggcacgtca agcgcctgag acttggggat tcagatgcgt ttctgacgcg 1980

agcaaaacag cgtgtggctg agatgtttgc tattagagaa gatcggccaa gtttattgga 2040

ggagctcgcc gagctgtgga attacccggc agctcgcccg attttggaag acattgacgg 2100

ctatcgatgt gccattagat ttgcaaggaa acacgatctc gccatatcac agctgccgaa 2160

catcgatgcc ggccggcatg ttgaactgat cgaagccatt gcaaacgaat atcttgaaca 2220

tgcgctaagc caagaacgcg actttcctca atggccggag gacggcgcag gacaaccagt 2280

atgcccggtt accttaacga gaatccgtta aaaaagaggg gagggaaaca ttatgatgac 2340

atcgcttttt gacgcgccta cgcttcaacg cgtaacggtt ttcactggga gcgccttggg 2400

ctcctctagt ctttatactc aggctgctca aaccctggct aaaaccgcgg tagaccgagg 2460

gattgatctg gtttatggcg gtggaaaagt cggcctcatg ggtattgttg ctgatgcatt 2520

ccttgaaagc gggggtgagg cattcggcgt gataacagag tcactgatga aaggagaatt 2580

aggccatgag aaactgacgg agttggaaat cgtgcccgac atgcatattc gtaaaaggcg 2640

gatggcggaa cttggggatg gattcattgc gatgcctgga ggagccggca ccctggaaga 2700

actgtttgaa gtctggacat ggcagcagtt aggaattcat caaaagccgg ttgcactcta 2760

tgacgtcgat ggcttctggc aaccgttatt ggaaatgttg gaacaaatga cacagagagg 2820

atttatcaag agagattttt ttgaatgcct catcgtcgaa tcagatccgc acgcattatt 2880

aaaggccatg cagacatgga cacctccagc cccaaaatgg taaattaatt ctagaaatta 2940

agaaggaggg attcgtcatg ttggtattcc aaatgcgtta tgtagataaa acatctactg 3000

ttttgaaaca gactaaaaac agtgattacg cagataaata aatacgttag attaattcct 3060

accagtgact aatcttatga ctttttaaac agataactaa aattacaaac aaatcgttta 3120

acttctgtat ttgtttatag atgtaatcac ttcaggagtg attacatgaa caaaaatata 3180

aaatattctc aaaacttttt aacgagtgaa aaagtactca accaaataat aaaacaattg 3240

aatttaaaag aaaccgatac cgtttacgaa attggaacag gtaaagggca tttaacgacg 3300

aaactggcta aaataagtaa acaggtaacg tctattgaat tagacagtca tctattcaac 3360

ttatcgtcag aaaaattaaa actgaacatt cgtgtcactt taattcacca agatattcta 3420

cagtttcaat tccctaacaa acagaggtat aaaattgttg ggaatattcc ttaccattta 3480

agcacacaaa ttattaaaaa agtggttttt gaaagccatg cgtctgacat ctatctgatt 3540

gttgaagaag gattctacaa gcgtaccttg gatattcacc gaacactagg gttgctcttg 3600

cacactcaag tctcgattca gcaattgctt aagctgccag cggaatgctt tcatcctaaa 3660

ccaaaagtaa acagtgtctt aataaaactt acccgccata ccacagatgt tccagataaa 3720

tattggaagc tatatacgta ctttgtttca aaatgggtca atcgagaata tcgtcaactg 3780

tttactaaaa atcagtttca tcaagcaatg aaacacgcca aagtaaacaa tttaagtacc 3840

gttacttatg agcaagtatt gtctattttt aatagttatc tattatttaa cgggaggaaa 3900

taattctatg agtcgctttt gtaaatttgg aaagttacac gttactaaag ggaatgtaga 3960

tggatcct                                                          3968

<210>  83
<211>  3968
<212>  DNA
<213>  Artificial Sequence

<220>
<223>  Complete synthetic trans-zeatin operon containing IPT-LOG (with
       TMR)

<400>  83
gcggtagtgg tgcttacgat gtacgacagg gggattcccc atacattctt cgcttggctg      60

aaaatgattc ttctttttat cgtctgcggc ggcgttctgt ttctgcttcg gtatgtgatt     120

gtgaagctgg cttacagaag agcggtaaaa gaagaaataa aaaagaaatc atcttttttg     180

tttggaaagc gagggaagcg ttcacagttt cgggcagctt tttttatagg aacattgatt     240

tgtattcact ctgccaagtt gttttgatag agtgattgtg ataattttaa atgtaagcgt     300

taacaaaatt ctccagtctt cacatcggtt tgaaaggagg aagcggaaga atgaagtaag     360

agggattttt gactccgaag taagtcttca aaaaatcaaa taaggagtgt caagaatgtt     420

tgcaaaacga ttcaaaacct ctttactgcc gttattcgct ggatttttat tgctgtttca     480

tttggttctg gcaggaccgg cggctgcgag tgctgaaacg gcgaacaaat cgaatgagct     540

tacagcaccg tcgatcaaaa gcggaaccat tcttcatgca tggaattggt cgttcaatac     600

gttaaaacac aatatgaagg atattcatga tgcaggatat acagccattc agacatctcc     660

gattaaccaa gtaaaggaag ggaatcaagg agataaaagc atgtcgaact ggtactggct     720

gtatcagccg acatcgtatc aaattggcaa ccgttactta ggtactgaac aagaatttaa     780

agaaatgtgt gcagccgctg aagaatatgg cataaaggtc attgttgacg cggtcatcaa     840

tcataccacc agtgattatg ccgcgatttc caatgaggtt aagagtattc caaactggac     900

acatggaaac acacaaatta aaaactggtc tgatcgatgg gatgtcacgc agaattcatt     960

gctcgggctg tatgactgga atacacaaaa tacacaagta cagtcctatc tgaaacggtt    1020

cttagacagg gcattgaatg acggggcaga cggttttcga tttgatgccg ccaaacatat    1080

agagcttcca gatgatggca gttacggcag tcaattttgg ccgaatatca caaatacatc    1140

tgcagagttc caatacggag aaatcctgca ggatagtgcc tccagagatg ctgcttatgc    1200

gaattatatg gatgtgacag cgtctaacta tgggcattcc ataaggtccg ctttaaagaa 1260

tcgtaatctg ggcgtgtcga atatctccca ctatgcatct gatgtgtctg cggacaagct 1320

agtgacatgg gtagagtcgc atgatacacg ggtgcgcatg atcgtatggt tcactgtcca 1380

ccaaccaaaa ctgtgctcag taccgccaat atttctccct tggggggtac aaagaggtgt 1440

ccctagaaga gatccacgct gtgtaaaaat tttacaaaaa ggtattgact ttccctacag 1500

ggtgtgtaat aatttaatta caggcggggg caaccccgct cagtacctag agcgtaaaag 1560

aggggaggga aacactagta tgttattaca cctgatctac ggtcctacat gtagtggtaa 1620

aactgatatg gctatacaaa tcgcgcaaga aacaggttgg cccgtcgttg ctcttgatcg 1680

tgtgcagtgc tgtccacaga ttgcgacagg atccggcaga cctctggaaa gcgagttaca 1740

atctacccgg cggatttatt tagactcacg ccctttgaca gaaggcattc ttgacgcgga 1800

atccgctcat cgccgtctta tattcgaggt agattggagg aaatcggaag aaggcttaat 1860

cctcgaagga gggtccatct cgcttctaaa ttgtatggct aagtctccat tttggagatc 1920

tggattccag tggcacgtca agcgcctgag acttggggat tcagatgcgt ttctgacgcg 1980

agcaaaacag cgtgtggctg agatgtttgc tattagagaa gatcggccaa gtttattgga 2040

ggagctcgcc gagctgtgga attacccggc agctcgcccg attttggaag acattgacgg 2100

ctatcgatgt gccattagat ttgcaaggaa acacgatctc gccatatcac agctgccgaa 2160

catcgatgcc ggccggcatg ttgaactgat cgaagccatt gcaaacgaat atcttgaaca 2220

tgcgctaagc caagaacgcg actttcctca atggccggag gacggcgcag gacaaccagt 2280

atgcccggtt accttaacga gaatccgtta aaaaagaggg gagggaaaca ttatgatgac 2340

atcgcttttt gacgcgccta cgcttcaacg cgtaacggtt ttcactggga gcgccttggg 2400

ctcctctagt ctttatactc aggctgctca aaccctggct aaaaccgcgg tagaccgagg 2460

gattgatctg gtttatggcg gtggaaaagt cggcctcatg ggtattgttg ctgatgcatt 2520

ccttgaaagc gggggtgagg cattcggcgt gataacagag tcactgatga aaggagaatt 2580

aggccatgag aaactgacgg agttggaaat cgtgcccgac atgcatattc gtaaaaggcg 2640

gatggcggaa cttggggatg gattcattgc gatgcctgga ggagccggca ccctggaaga 2700

actgtttgaa gtctggacat ggcagcagtt aggaattcat caaaagccgg ttgcactcta 2760

tgacgtcgat ggcttctggc aaccgttatt ggaaatgttg gaacaaatga cacagagagg 2820

atttatcaag agagattttt ttgaatgcct catcgtcgaa tcagatccgc acgcattatt 2880

aaaggccatg cagacatgga cacctccagc cccaaaatgg taaattaatt ctagaaatta 2940

agaaggaggg attcgtcatg ttggtattcc aaatgcgtta tgtagataaa acatctactg 3000

ttttgaaaca gactaaaaac agtgattacg cagataaata aatacgttag attaattcct 3060

accagtgact aatcttatga ctttttaaac agataactaa aattacaaac aaatcgttta 3120

acttctgtat ttgtttatag atgtaatcac ttcaggagtg attacatgaa caaaaatata 3180

aaatattctc aaaacttttt aacgagtgaa aaagtactca accaaataat aaaacaattg 3240

aatttaaaag aaaccgatac cgtttacgaa attggaacag gtaaagggca tttaacgacg 3300

aaactggcta aaataagtaa acaggtaacg tctattgaat tagacagtca tctattcaac 3360

ttatcgtcag aaaaattaaa actgaacatt cgtgtcactt taattcacca agatattcta 3420

cagtttcaat tccctaacaa acagaggtat aaaattgttg ggaatattcc ttaccattta 3480

agcacacaaa ttattaaaaa agtggttttt gaaagccatg cgtctgacat ctatctgatt 3540

gttgaagaag gattctacaa gcgtaccttg gatattcacc gaacactagg gttgctcttg 3600

cacactcaag tctcgattca gcaattgctt aagctgccag cggaatgctt tcatcctaaa 3660

ccaaaagtaa acagtgtctt aataaaactt acccgccata ccacagatgt tccagataaa 3720

tattggaagc tatatacgta ctttgtttca aaatgggtca atcgagaata tcgtcaactg 3780

tttactaaaa atcagtttca tcaagcaatg aaacacgcca aagtaaacaa tttaagtacc 3840

gttacttatg agcaagtatt gtctattttt aatagttatc tattatttaa cgggaggaaa 3900

taattctatg agtcgctttt gtaaatttgg aaagttacac gttactaaag ggaatgtaga 3960

tggatcct 3968

<210> 84
<211> 1080
<212> DNA
<213> Artificial Sequence

<220>
<223> synthetic DNA fragment of dxs part1

<400> 84
gaagtgccat tccgcctgac ctcgcagcat acgcagcgaa atcagcatca ccggagaatc 60

ccaacgaagc caactagtat ggacttgttg tccattcaag acccgtcttt cctaaagaat 120

atgagcattg acgagcttga aaaattgtcc gatgagatcc gccaatttct catcacatct 180

ctatcggcct cgggcggaca cattggaccg aatctggggg tcgtggaact gacagtagcc 240

ctccacaagg agttcaactc acccaaagac aaatttctct gggacgtggg gcaccaaagc 300

tacgttcata aattgcttac tggtcgtggc aaagagttcg ccacattgcg ccagtacaaa 360

ggactctgcg gattccctaa acgctcggaa tccgagcacg atgtgtggga aacaggtcat 420

tcttctactt ctctcagcgg cgccatggga atggccgctg cacgagatat taaagggact 480

gatgagtaca taatcccaat aataggtgac ggcgctttaa caggaggaat ggccctagag 540

gccttgaacc acatagggga tgagaagaaa gatatgatag tcattttgaa tgataatgag 600

atgtctattg ctcccaacgt tggagcaata catagcatgt tagggcggtt acgtactgca 660

ggaaagtacc agtgggtaaa agatgaactt gaatacctct tcaagaagat acccgctgta 720

ggaggcaagc ttgcagccac ggccgagaga gtgaaggatt ctcttaaata tatgttggtt 780

tcagggatgt tctttgaaga attgggattc acgtaccttg gtccggttga cggccattct 840

taccacgagt taattgaaaa tttacaatac gcgaaaaaaa cgaaggggcc tgttctgctg 900

cacgttatta ccaaaaaagg aaaaggttat aaacctgcgg aaaccgatac aattggcacg 960

tggcacggca ccgggcctta taaaatcaac acgggggact ttgtcaagcc gaaagctgcc 1020

gcgcctagtt ggtccggcct ggttagtggg acggtacagc gaatggcagg ctaggtggag 1080

<210> 85
<211> 1094
<212> DNA
<213> Artificial Sequence

<220>
<223> Synthetic DNA fragment of dxs part2

<400> 85
gaagtgccat tccgcctgac ctaatcaaca cgggggactt tgtcaagccg aaagctgccg 60

cgcctagttg gtccggcctg gttagtggga cggtacagcg aatggcccgg gaagatggga 120

ggattgtcgc tatcacaccg gcaatgccag taggtagcaa gttagaaggt tttgcgaaag 180

aatttccgga taggatgttt gacgtaggta ttgctgagca gcatgcggcg acgatggctg 240

cggcaatggc aatgcagggc atgaagcctt ttttagctat ttattcaaca tttctgcaaa 300

gagcgtatga ccaagtggta catgacattt gtcggcaaaa tgctaatgtc tttattggga 360

ttgatcgcgc agggttagtg ggtgcagacg gggaaacgca tcaaggagtg tttgacatcg 420

cgtttatgcg acatatccca aatatggtcc ttatgatgcc aaaagacgaa aacgagggtc 480

aacacatggt gcatacagct cttagttatg acgaaggacc tatcgcgatg agatttccga 540

gagggaatgg attaggcgtc aaaatggatg agcagcttaa aacaattccg attggaacat 600

gggaagtctt acgtccagga aatgacgcgg ttatccttac atttggcact accattgaga 660

tggcgattga ggcagcagaa gaactccaga aggagggcct gtcagtccgt gttgttaatg 720

cgagatttat taaaccgatt gacgaaaaga tgatgaaaag tatcctgaaa gaaggcctgc 780

cgatacttac catcgaggaa gctgttctgg aaggaggatt tggctcatca atccttgaat 840

ttgcacatga tcagggcgaa tatcacacac cgatcgatcg aatgggcatc ccggatcgat 900

ttatcgagca tggctccgtt acagcactgc ttgaagagat cggcttaacg aaacagcagg 960

tggcaaacag aatcaggtta cttatgccgc cgaagacaca taagggcatc ggctcataaa 1020

aaagagggga gggaaacatt aatgacgacc tggctaacga gtctcgccga tctaggctag 1080

gtggaggctc agtg 1094

<210> 86
<211> 2040
<212> DNA
<213> Artificial Sequence

<220>
<223> Joined synthetic gene dxs complete sequence

<400> 86
gaagtgccat tccgcctgac ctcgcagcat acgcagcgaa atcagcatca ccggagaatc 60

ccaacgaagc caactagtat ggacttgttg tccattcaag acccgtcttt cctaaagaat 120

atgagcattg acgagcttga aaaattgtcc gatgagatcc gccaatttct catcacatct 180

ctatcggcct cgggcggaca cattggaccg aatctggggg tcgtggaact gacagtagcc 240

ctccacaagg agttcaactc acccaaagac aaatttctct gggacgtggg gcaccaaagc 300

tacgttcata aattgcttac tggtcgtggc aaagagttcg ccacattgcg ccagtacaaa 360

ggactctgcg gattccctaa acgctcggaa tccgagcacg atgtgtggga aacaggtcat 420

tcttctactt ctctcagcgg cgccatggga atggccgctg cacgagatat taaagggact 480

gatgagtaca taatcccaat aataggtgac ggcgctttaa caggaggaat ggccctagag 540

gccttgaacc acatagggga tgagaagaaa gatatgatag tcattttgaa tgataatgag 600

atgtctattg ctcccaacgt tggagcaata catagcatgt tagggcggtt acgtactgca 660

ggaaagtacc agtgggtaaa agatgaactt gaatacctct tcaagaagat acccgctgta 720

ggaggcaagc ttgcagccac ggccgagaga gtgaaggatt ctcttaaata tatgttggtt 780

tcagggatgt tctttgaaga attgggattc acgtaccttg gtccggttga cggccattct 840

taccacgagt taattgaaaa tttacaatac gcgaaaaaaa cgaaggggcc tgttctgctg 900

cacgttatta ccaaaaaagg aaaaggttat aaacctgcgg aaaccgatac aattggcacg 960

tggcacggca ccgggcctta taaaatcaac acgggggact ttgtcaagcc gaaagctgcc 1020

gcgcctagtt ggtccggcct ggttagtggg acggtacagc gaatggcccg ggaagatggg 1080

aggattgtcg ctatcacacc ggcaatgcca gtaggtagca agttagaagg ttttgcgaaa 1140

gaatttccgg ataggatgtt tgacgtaggt attgctgagc agcatgcggc gacgatggct 1200

gcggcaatgg caatgcaggg catgaagcct tttttagcta tttattcaac atttctgcaa 1260

agagcgtatg accaagtggt acatgacatt tgtcggcaaa atgctaatgt ctttattggg 1320

attgatcgcg cagggttagt gggtgcagac ggggaaacgc atcaaggagt gtttgacatc 1380

gcgtttatgc gacatatccc aaatatggtc cttatgatgc caaaagacga aaacgagggt 1440

caacacatgg tgcatacagc tcttagttat gacgaaggac ctatcgcgat gagatttccg 1500

agagggaatg gattaggcgt caaaatggat gagcagctta aaacaattcc gattggaaca 1560

tgggaagtct tacgtccagg aaatgacgcg gttatcctta catttggcac taccattgag 1620

atggcgattg aggcagcaga agaactccag aaggagggcc tgtcagtccg tgttgttaat 1680

gcgagattta ttaaaccgat tgacgaaaag atgatgaaaa gtatcctgaa agaaggcctg 1740

ccgatactta ccatcgagga agctgttctg gaaggaggat ttggctcatc aatccttgaa 1800

tttgcacatg atcagggcga atatcacaca ccgatcgatc gaatgggcat cccggatcga 1860

tttatcgagc atggctccgt tacagcactg cttgaagaga tcggcttaac gaaacagcag 1920

gtggcaaaca gaatcaggtt acttatgccg ccgaagacac ataagggcat cggctcataa 1980

aaaagagggg agggaaacat taatgacgac ctggctaacg agtctcgccg atctaggcta 2040

<210> 87
<211> 768
<212> DNA

<213>  Artificial Sequence

<220>
<223>  Spectinomycin selectable marker

<400>  87
gtgaggcgaa tatatttgaa tacatacgaa caaatcaata aagtgaaaaa aatacttcgg      60

aaacatttaa aaaataacct tattggtact tacatgtttg gatcaggagt tgagagtgga     120

ctcaaaccaa atagtgatct tgacttttta gtcgtcgtat ctgaaccatt gacagatcaa     180

agtaaagaaa tacttataca aaaaattaga cctatttcaa aaaaaatagg agataaaagc     240

aacttacgat atattgaatt aacaattatt attcagcaag aaatggtacc gtggaatcat     300

cctcccaaac aagaatttat ttatggagaa tggttacaag agctttatga acaaggatac     360

attcctcaga aggaattaaa ttcagattta accataatgc tttaccaagc aaaacgaaaa     420

aataaaagaa tatacggaaa ttatgactta gaggaattac tacctgatat tccattttct     480

gatgtgagaa gagccattat ggattcgtca gaggaactta tagataatta tcaggatgat     540

gaaaccaact ctatattaac tttatgccgc atgattttaa ctatggacac gggtaaaatc     600

ataccaaaag atattgcggg aaatgcagtg gctgaatctt ctccattaga acatagggag     660

agaattttgt tagcagttcg tagttatctt ggagagaata ttgaatggac taatgaaaat     720

gtaaatctta cgataaacta tcttaataac cgccttaaga aactttaa                  768


<210>  88
<211>  1503
<212>  DNA
<213>  Artificial Sequence

<220>
<223>  Initial fragment lacA

<400>  88
gaagtgccat tccgcctgac ctgcctttta atcccggcaa cagcttaatc agtacatcca      60

tcattccgaa gcatccgaca ttcgatcatt acaaggaatt atttgcgggc aaggaaagcc     120

ttcaatatgt gcagtggtat gtcaactcta tgaagatcag cctgtttaca atggcagggt     180

ctttgctctg tgtgacgttt acggcctatg cgttttcgcg ctttcggttt aaaggaggga     240

aatacgcttt aacgctcttt ttattgctgc agatgattcc tcagttttca gctttaattg     300

ccttgtttgt gctggcgcaa atcttgggaa tgatcaatag ccactggctg ctaatcttgc     360

tttatatcgg cggcctgatc ccgatgaata cgtatttgat gaaagggtac atggattcca 420

ttccgatgga tttagacgaa agcgccaaga ttgacggagc cagcagcacc agaatcttct 480

tccagatcat tctgccatta tcaaaaccga tggcggcagt cgtggccatg aacggcttta 540

ccggtccgct cggagatttt gtgctgtcct caaccatatt gagaacgcct gaatcatata 600

cattgcccgt cggtctattc aatttagtga atgatgtcat gggggccagc tatacgacat 660

ttgcggccgg agccctgctt atcagcatac cggttgccgt catctttatt atgctgcaaa 720

agaattttgt gtccggatta accgcaggcg gaacgaaggg ctaagagaac aaggaggaga 780

atgtgatgtc aaagcttgaa aaaacgcacg taacaaaagc aaaatttatg ctccatgggg 840

gagactacaa ccccgatcag tggctggatc ggcccgatat tttagctgac gatatcaaac 900

tgatgaagct ttctcatacg aatacgtttt ctgtcggcat ttttgcatgg agcgcacttg 960

agccggagga gggcgtatat caatttgaat ggctggatga tatttttgag cggattcaca 1020

gtataggcgg ccgggtcata ttagcaacgc cgagcggagc ccgtccggcc tggctgtcgc 1080

aaacctatcc ggaagttttg cgcgtcaatg cctcccgcgt caaacagctg cacggcggaa 1140

ggcacaacca ctgcctcaca tctaaagtct accgagaaaa aacacggcac atcaaccgct 1200

acgggtgcgc atgatcgtat ggttcactgt ccaccaacca aaactgtgct cagtaccgcc 1260

aatatttctc ccttgggggg tacaaagagg tgtccctaga agagatccac gctgtgtaaa 1320

aattttacaa aaaggtattg actttcccta cagggtgtgt aataatttaa ttacaggcgg 1380

gggcaacccc gctcagtacc tagagcgtaa aagaggggag ggaaacacta gttggcttcg 1440

ttgggattct ccggtgatgc tgatttcgct gcgtatgctg cgaggctagg tggaggctca 1500

gtg 1503

<210> 89
<211> 2172
<212> DNA
<213> Artificial Sequence

<220>
<223> End fragment with spectinomycin selectable marker

<400> 89
gaagtgccat tccgcctgac ctcacggtag tcattgctgg cgaacgcgtc tagagctccg 60

gcataaaatt aactaaaata attattatgt agataaaaaa tttagaagcc aatgaaatct 120

ataaataaac taaattaagt ttatttaatt aacaactatg gatataaaat aggtactaat 180

caaaatagtg aggcgaatat atttgaatac atacgaacaa atcaataaag tgaaaaaaat 240

acttcggaaa catttaaaaa ataaccttat tggtacttac atgtttggat caggagttga 300

gagtggactc aaaccaaata gtgatcttga ctttttagtc gtcgtatctg aaccattgac 360

agatcaaagt aaagaaatac ttatacaaaa aattagacct atttcaaaaa aaataggaga 420

taaaagcaac ttacgatata ttgaattaac aattattatt cagcaagaaa tggtaccgtg 480

gaatcatcct cccaaacaag aatttattta tggagaatgg ttacaagagc tttatgaaca 540

aggatacatt cctcagaagg aattaaattc agatttaacc ataatgcttt accaagcaaa 600

acgaaaaaat aaaagaatat acggaaatta tgacttagag gaattactac ctgatattcc 660

attttctgat gtgagaagag ccattatgga ttcgtcagag gaacttatag ataattatca 720

ggatgatgaa accaactcta tattaacttt atgccgcatg attttaacta tggacacggg 780

taaaatcata ccaaaagata ttgcgggaaa tgcagtggct gaatcttctc cattagaaca 840

tagggagaga attttgttag cagttcgtag ttatcttgga gagaatattg aatggactaa 900

tgaaaatgta aatcttacga taaactatct taataaccgc cttaagaaac tttaacccgg 960

gcgcgttgct ttcgatgaag ttttaccgcc atcaccagac cctcaatcgt atgtgtttac 1020

tgccaatact ggagacagca cggtttcggt tattgatgca gatttaaaca ctgttgtaaa 1080

aacaatccct ttttctgacg tgccaactaa tctaggcgtt actttcgata aagcattcac 1140

gtatgtcctc catggaaata ccaaccttgt ctcagtgatt gataataaaa cattaaccat 1200

cataaatacc attactgttg gaggagggcc gagaaaaatc gaatttgatc caacagatga 1260

gttcgcatac gtgatggccg ccggatctat atatgtgatt aacacggcct ctcaatcggt 1320

cattgatgtc attccgatac ccggcgcttt agactttgct cttgatccga atggacagta 1380

tgtctatacc gctaatggaa gcagttggtc ggttgataaa tatgatgtaa atactgggca 1440

gttggtagaa agtatcatag atacatttga atttcccagt ttaattacaa ctccttacgc 1500

tggaaatttt gcgtacgtat tgaatggtga gctgtggccg aaaggtgtaa cggagattag 1560

tttgtcacca ttaagcaggg gaggggactt cagccgttta tttgaaacgc tgaggacgat 1620

tgtattttct ctcgatagta ctagagctta ttttcaagag ccatatagtg aaccgtttct 1680

catcaataac ttatacgtag ttaatacggc cagacagagg atcattgcaa acgtgtctct 1740

ccccggcgcc tttgacctcg ccgtaacgcc ggacaaacag tatatttatg cggcacagcc 1800

caacgacaat gctgtgaccg tgtaccgcac aagtgattat acagccgtga cggtgattcc 1860

ggttggcgcg ggtccgtctg cgattgcgat gtagctattt tatagatcaa gagaagcggt 1920

gctgtgataa gagaatcgtt tataaataaa tgatccgatt tgctgaatgg cagccaggat 1980

aatcagaaac agcagttcac ttatagagaa actgagcatc caccaaatcc cggctgctgc 2040

cagtgctccc gcaaacactt ttctgaacag gctgccgtgc cggaaaagct gccgcaaata 2100

cgttcgcatg caaaacaata aatacaggat cagtagcatc gcaagtccca taggctaggt 2160

ggaggctcag tg 2172

<210> 90
<211> 4245
<212> DNA
<213> Artificial Sequence

<220>
<223> Complete synthetic dxs operon

<400> 90
taatcccggc aacagcttaa tcagtacatc catcattccg aagcatccga cattcgatca 60

ttacaaggaa ttatttgcgg gcaaggaaag ccttcaatat gtgcagtggt atgtcaactc 120

tatgaagatc agcctgttta caatggcagg gtctttgctc tgtgtgacgt ttacggccta 180

tgcgttttcg cgctttcggt ttaaagggag gaaatacgct ttaacgctct ttttattgct 240

gcagatgatt cctcagtttt cagctttaat tgccttgttt gtgctggcgc aaatcttggg 300

aatgatcaat agccactggc tgctaatctt gctttatatc ggcggcctga tcccgatgaa 360

tacgtatttg atgaaagggt acatggattc cattccgatg gatttagacg aaagcgccaa 420

gattgacgga gccagcagca ccagaatctt cttccagatc attctgccat tatcaaaacc 480

gatggcggca gtcgtggcca tgaacggctt taccggtccg ctcggagatt ttgtgctgtc 540

ctcaaccata ttgagaacgc ctgaatcata tacattgccc gtcggtctat tcaatttagt 600

gaatgatgtc atgggggcca gctatacgac atttgcggcc ggagccctgc ttatcagcat 660

accggttgcc gtcatcttta ttatgctgca aaagaatttt gtgtccggat taaccgcagg 720

cggaacgaag ggctaagaga acaaggagga gaatgtgatg tcaaagcttg aaaaaacgca 780

cgtaacaaaa gcaaaattta tgctccatgg gggagactac aaccccgatc agtggctgga 840

tcggcccgat attttagctg acgatatcaa actgatgaag ctttctcata cgaatacgtt 900

ttctgtcggc atttttgcat ggagcgcact tgagccggag gagggcgtat atcaatttga 960

atggctggat gatatttttg agcggattca cagtataggc ggccgggtca tattagcaac 1020

gccgagcgga gcccgtccgg cctggctgtc gcaaacctat ccggaagttt tgcgcgtcaa 1080

tgcctcccgc gtcaaacagc tgcacggcgg aaggcacaac cactgcctca catctaaagt 1140

ctaccgagaa aaaacacggc acatcaaccg ctacgggtgc gcatgatcgt atggttcact 1200

gtccaccaac caaaactgtg ctcagtaccg ccaatatttc tcccttgggg ggtacaaaga 1260

ggtgtcccta gaagagatcc acgctgtgta aaaattttac aaaaaggtat tgactttccc 1320

tacagggtgt gtaataattt aattacaggc gggggcaacc ccgctcagta cctagagcgt 1380

aaaagagggg agggaaacac tagtatggac ttgttgtcca ttcaagaccc gtctttccta 1440

aagaatatga gcattgacga gcttgaaaaa ttgtccgatg agatccgcca atttctcatc 1500

acatctctat cggcctcggg cggacacatt ggaccgaatc tgggggtcgt ggaactgaca 1560

gtagccctcc acaaggagtt caactcaccc aaagacaaat ttctctggga cgtggggcac 1620

caaagctacg ttcataaatt gcttactggt cgtggcaaag agttcgccac attgcgccag 1680

tacaaaggac tctgcggatt ccctaaacgc tcggaatccg agcacgatgt gtgggaaaca 1740

ggtcattctt ctacttctct cagcggcgcc atgggaatgg ccgctgcacg agatattaaa 1800

gggactgatg agtacataat cccaataata ggtgacggcg ctttaacagg aggaatggcc 1860

ctagaggcct tgaaccacat aggggatgag aagaaagata tgatagtcat tttgaatgat 1920

aatgagatgt ctattgctcc caacgttgga gcaatacata gcatgttagg gcggttacgt 1980

actgcaggaa agtaccagtg ggtaaaagat gaacttgaat acctcttcaa gaagataccc 2040

gctgtaggag gcaagcttgc agccacggcc gagagagtga aggattctct taaatatatg 2100

ttggtttcag ggatgttctt tgaagaattg ggattcacgt accttggtcc ggttgacggc 2160

cattcttacc acgagttaat tgaaaattta caatacgcga aaaaaacgaa ggggcctgtt 2220

ctgctgcacg ttattaccaa aaaaggaaaa ggttataaac ctgcggaaac cgatacaatt 2280

ggcacgtggc acggcaccgg gccttataaa atcaacacgg gggactttgt caagccgaaa 2340

gctgccgcgc ctagttggtc cggcctggtt agtgggacgg tacagcgaat ggcccgggaa 2400

gatgggagga ttgtcgctat cacaccggca atgccagtag gtagcaagtt agaaggtttt 2460

gcgaaagaat ttccggatag gatgtttgac gtaggtattg ctgagcagca tgcggcgacg 2520

atggctgcgg caatggcaat gcagggcatg aagccttttt tagctattta ttcaacattt 2580

ctgcaaagag cgtatgacca agtggtacat gacatttgtc ggcaaaatgc taatgtcttt 2640

attgggattg atcgcgcagg gttagtgggt gcagacgggg aaacgcatca aggagtgttt 2700

gacatcgcgt ttatgcgaca tatcccaaat atggtcctta tgatgccaaa agacgaaaac 2760

gagggtcaac acatggtgca tacagctctt agttatgacg aaggacctat cgcgatgaga 2820

tttccgagag ggaatggatt aggcgtcaaa atggatgagc agcttaaaac aattccgatt 2880

ggaacatggg aagtcttacg tccaggaaat gacgcggtta tccttacatt tggcactacc 2940

attgagatgg cgattgaggc agcagaagaa ctccagaagg agggcctgtc agtccgtgtt 3000

gttaatgcga gatttattaa accgattgac gaaaagatga tgaaaagtat cctgaaagaa 3060

ggcctgccga tacttaccat cgaggaagct gttctggaag gaggatttgg ctcatcaatc 3120

cttgaatttg cacatgatca gggcgaatat cacacaccga tcgatcgaat gggcatcccg 3180

gatcgattta tcgagcatgg ctccgttaca gcactgcttg aagagatcgg cttaacgaaa 3240

cagcaggtgg caaacagaat caggttactt atgccgccga agacacataa gggcatcggc 3300

tcataaatta attctagagc tccggcataa aattaactaa aataattatt atgtagataa 3360

aaaatttaga agccaatgaa atctataaat aaactaaatt aagtttattt aattaacaac 3420

tatggatata aaataggtac taatcaaaat agtgaggcga atatatttga atacatacga 3480

acaaatcaat aaagtgaaaa aaatacttcg gaaacattta aaaaataacc ttattggtac 3540

ttacatgttt ggatcaggag ttgagagtgg actcaaacca aatagtgatc ttgacttttt 3600

agtcgtcgta tctgaaccat tgacagatca aagtaaagaa atacttatac aaaaaattag 3660

acctatttca aaaaaaatag gagataaaag caacttacga tatattgaat taacaattat 3720

tattcagcaa gaaatggtac cgtggaatca tcctcccaaa caagaattta tttatggaga 3780

atggttacaa gagctttatg aacaaggata cattcctcag aaggaattaa attcagattt 3840

aaccataatg ctttaccaag caaaacgaaa aaataaaaga atatacggaa attatgactt 3900

agaggaatta ctacctgata ttccattttc tgatgtgaga agagccatta tggattcgtc 3960

agaggaactt atagataatt atcaggatga tgaaaccaac tctatattaa ctttatgccg 4020

catgatttta actatggaca cgggtaaaat cataccaaaa gatattgcgg gaaatgcagt 4080

ggctgaatct tctccattag aacataggga gagaattttg ttagcagttc gtagttatct 4140

tggagagaat attgaatgga ctaatgaaaa tgtaaatctt acgataaact atcttaataa 4200

ccgccttaag aaactttaac ccgggcgcgt tgctttcgat gaagt 4245

<210> 91
<211> 27
<212> DNA
<213> Artificial Sequence

<220>
<223> Primer For for dxs operon

<400> 91
taatcccggc aacagcttaa tcagtac 27

<210> 92
<211> 20
<212> DNA
<213> Artificial Sequence

<220>
<223> Primer Rev for dxs operon

<400> 92
acttcatcga aagcaacgcg 20

<210> 93
<211> 399
<212> PRT
<213> Rhodococcus fascians

<400> 93

Met Ala Gly Thr Ala Asp Leu Pro Leu Glu Met Arg Arg Asn Gly Leu
1               5                   10                  15

Asn Pro Thr Glu Glu Leu Ala Gln Val Arg Asp Arg Asp Gly Val Ile
            20                  25                  30

Pro Val Gly Glu Leu Tyr Gly Ala Pro Ala Phe Leu Val Cys Arg Tyr
        35                  40                  45

Glu Asp Val Arg Arg Ile Phe Ala Asp Ser Asn Arg Phe Ser Asn Ala
    50                  55                  60

His Thr Pro Met Phe Ala Ile Pro Ser Gly Gly Asp Val Ile Glu Asp
65                  70                  75                  80

Glu Leu Ala Ala Met Arg Ala Gly Asn Leu Ile Gly Leu Asp Pro Pro
                85                  90                  95

Asp His Thr Arg Leu Arg His Ile Leu Ala Ala Glu Phe Ser Val His
            100                 105                 110

Arg Leu Ser Arg Leu Gln Pro Arg Ile Ala Glu Ile Val Asp Ser Ala
        115                 120                 125

Leu Asp Gly Leu Glu Gln Ala Gly Gln Pro Ala Asp Leu Met Asp Arg
    130                 135                 140

Tyr Ala Leu Pro Val Ser Leu Leu Val Leu Cys Glu Leu Leu Gly Val
145                 150                 155                 160

Pro Tyr Ala Asp Arg Asp Glu Leu Arg Asp Arg Thr Ala Arg Leu Leu
                165                 170                 175

Asp Leu Ser Ala Ser Ala Glu Gln Arg Ala Val Ala Gln Arg Glu Asp
            180                 185                 190

Arg Arg Tyr Met Ala Thr Leu Val Thr Arg Ala Gln Glu Gln Pro Gly
        195                 200                 205

Asp Asp Leu Leu Gly Ile Leu Ala Arg Lys Ile Gly Asp Asn Leu Ser
    210                 215                 220

Thr Asp Glu Leu Ile Ser Ile Ile Ser Leu Ile Met Leu Gly Gly His
225                 230                 235                 240

Glu Thr Thr Ala Ser Met Ile Gly Leu Ser Val Leu Ala Leu Leu His
                245                 250                 255

His Pro Glu Gln Ala Ala Met Met Ile Glu Asp Pro Asn Cys Val Asn
            260                 265                 270

Ser Gly Ile Glu Glu Leu Leu Arg Trp Leu Ser Val Ala His Ser Gln
        275                 280                 285

Pro Pro Arg Met Ala Val Thr Glu Val Gln Ile Ala Gly Val Thr Ile
    290                 295                 300

Pro Ala Gly Ser Phe Val Ile Pro Ser Leu Leu Ala Ala Asn Arg Asp
305                 310                 315                 320

Ser Asn Leu Thr Asp Arg Pro Asp Asp Leu Asp Ile Thr Arg Gly Val
                325                 330                 335

Ala Gly His Leu Ala Phe Gly His Gly Val His Phe Cys Leu Gly His
            340                 345                 350

Ser Leu Ala Arg Met Thr Leu Arg Thr Ala Val Pro Ala Val Leu Arg
        355                 360                 365

Arg Phe Pro Asp Leu Ala Leu Ser Pro Ser His Asp Val Arg Leu Arg
    370                 375                 380

Ser Ala Ser Ile Val Leu Gly Leu Glu Glu Leu Gln Leu Thr Trp
385                 390                 395

<210>  94
<211>  512
<212>  PRT
<213>  Arabidopsis thaliana

<400>  94

Met Met Val Thr Leu Val Leu Lys Tyr Val Leu Val Ile Val Met Thr
1               5                   10                  15

Leu Ile Leu Arg Val Leu Tyr Asp Ser Ile Cys Cys Tyr Phe Leu Thr
            20                  25                  30

Pro Arg Arg Ile Lys Lys Phe Met Glu Arg Gln Gly Ile Thr Gly Pro
        35                  40                  45

Lys Pro Arg Leu Leu Thr Gly Asn Ile Ile Asp Ile Ser Lys Met Leu
    50                  55                  60

Ser His Ser Ala Ser Asn Asp Cys Ser Ser Ile His His Asn Ile Val

65                  70                  75                  80

Pro Arg Leu Leu Pro His Tyr Val Ser Trp Ser Lys Gln Tyr Gly Lys
                85                  90                  95

Arg Phe Ile Met Trp Asn Gly Thr Glu Pro Arg Leu Cys Leu Thr Glu
            100                 105                 110

Thr Glu Met Ile Lys Glu Leu Leu Thr Lys His Asn Pro Val Thr Gly
        115                 120                 125

Lys Ser Trp Leu Gln Gln Gln Gly Thr Lys Gly Phe Ile Gly Arg Gly
    130                 135                 140

Leu Leu Met Ala Asn Gly Glu Ala Trp His His Gln Arg His Met Ala
145                 150                 155                 160

Ala Pro Ala Phe Thr Arg Asp Arg Leu Lys Gly Tyr Ala Lys His Met
                165                 170                 175

Val Glu Cys Thr Lys Met Met Ala Glu Arg Leu Arg Lys Glu Val Gly
            180                 185                 190

Glu Glu Val Glu Ile Gly Glu Glu Met Arg Arg Leu Thr Ala Asp Ile
        195                 200                 205

Ile Ser Arg Thr Glu Phe Gly Ser Ser Cys Asp Lys Gly Lys Glu Leu
    210                 215                 220

Phe Ser Leu Leu Thr Val Leu Gln Arg Leu Cys Ala Gln Ala Thr Arg
225                 230                 235                 240

His Leu Cys Phe Pro Gly Ser Arg Phe Leu Pro Ser Lys Tyr Asn Arg
                245                 250                 255

Glu Ile Lys Ser Leu Lys Thr Glu Val Glu Arg Leu Leu Met Glu Ile
            260                 265                 270

Ile Asp Ser Arg Lys Asp Ser Val Glu Ile Gly Arg Ser Ser Ser Tyr
        275                 280                 285

Gly Asp Asp Leu Leu Gly Leu Leu Leu Asn Gln Met Asp Ser Asn Lys
    290                 295                 300

Asn Asn Leu Asn Val Gln Met Ile Met Asp Glu Cys Lys Thr Phe Phe
305                 310                 315                 320

Phe Thr Gly His Glu Thr Thr Ser Leu Leu Leu Thr Trp Thr Leu Met
                325                 330                 335

Leu Leu Ala His Asn Pro Thr Trp Gln Asp Asn Val Arg Asp Glu Val
            340                 345                 350

Arg Gln Val Cys Gly Gln Asp Gly Val Pro Ser Val Glu Gln Leu Ser
        355                 360                 365

Ser Leu Thr Ser Leu Asn Lys Val Ile Asn Glu Ser Leu Arg Leu Tyr
    370                 375                 380

Pro Pro Ala Thr Leu Leu Pro Arg Met Ala Phe Glu Asp Ile Lys Leu
385                 390                 395                 400

Gly Asp Leu Ile Ile Pro Lys Gly Leu Ser Ile Trp Ile Pro Val Leu
                405                 410                 415

Ala Ile His His Ser Asn Glu Leu Trp Gly Glu Asp Ala Asn Glu Phe
            420                 425                 430

Asn Pro Glu Arg Phe Thr Thr Arg Ser Phe Ala Ser Ser Arg His Phe
        435                 440                 445

Met Pro Phe Ala Ala Gly Pro Arg Asn Cys Ile Gly Gln Thr Phe Ala
    450                 455                 460

Met Met Glu Ala Lys Ile Ile Leu Ala Met Leu Val Ser Lys Phe Ser
465                 470                 475                 480

Phe Ala Ile Ser Glu Asn Tyr Arg His Ala Pro Ile Val Val Leu Thr
                485                 490                 495

Ile Lys Pro Lys Tyr Gly Val Gln Leu Val Leu Lys Pro Leu Asp Leu

500                 505                 510

<210>  95
<211>  518
<212>  PRT
<213>  Arabidopsis thaliana

<400>  95

Met Leu Leu Thr Ile Leu Lys Ser Leu Leu Val Ile Phe Val Thr Thr
1               5                   10                  15

Ile Leu Arg Val Leu Tyr Asp Thr Ile Ser Cys Tyr Trp Leu Thr Pro
            20                  25                  30

Arg Arg Ile Lys Lys Ile Met Glu Gln Gln Gly Val Thr Gly Pro Lys
        35                  40                  45

Pro Arg Pro Leu Thr Gly Asn Ile Leu Glu Ile Ser Ala Met Val Ser
    50                  55                  60

Gln Ser Ala Ser Lys Asp Cys Asp Ser Ile His His Asp Ile Val Gly
65                  70                  75                  80

Arg Leu Leu Pro His Tyr Val Ala Trp Ser Lys Gln Tyr Gly Lys Arg
                85                  90                  95

Phe Ile Val Trp Asn Gly Thr Asp Pro Arg Leu Cys Leu Thr Glu Thr
            100                 105                 110

Glu Leu Ile Lys Glu Leu Leu Met Lys His Asn Gly Val Ser Gly Arg
        115                 120                 125

Ser Trp Leu Gln Gln Gln Gly Thr Lys Asn Phe Ile Gly Arg Gly Leu
    130                 135                 140

Leu Met Ala Asn Gly Gln Asp Trp His His Gln Arg His Leu Ala Ala
145                 150                 155                 160

Pro Ala Phe Thr Gly Glu Arg Leu Lys Gly Tyr Ala Arg His Met Val
                165                 170                 175

Glu Cys Thr Ser Lys Leu Val Glu Arg Leu Arg Lys Glu Val Gly Glu
            180                 185                 190

Gly Ala Asn Glu Val Glu Ile Gly Glu Glu Met His Lys Leu Thr Ala
        195                 200                 205

Asp Ile Ile Ser Arg Thr Lys Phe Gly Ser Ser Phe Glu Lys Gly Lys
    210                 215                 220

Glu Leu Phe Asn His Leu Thr Val Leu Gln Arg Arg Cys Ala Gln Ala
225                 230                 235                 240

Thr Arg His Leu Cys Phe Pro Gly Ser Arg Phe Leu Pro Ser Lys Tyr
                245                 250                 255

Asn Arg Glu Ile Lys Ser Leu Lys Lys Glu Val Glu Arg Leu Leu Ile
            260                 265                 270

Glu Ile Ile Gln Ser Arg Arg Asp Cys Ala Glu Met Gly Arg Ser Ser
        275                 280                 285

Thr His Gly Asp Asp Leu Leu Gly Leu Leu Leu Asn Glu Met Asp Ile
    290                 295                 300

Asp Lys Asn Asn Asn Asn Asn Asn Asn Asn Leu Gln Leu Ile Met Asp
305                 310                 315                 320

Glu Cys Lys Thr Phe Phe Phe Ala Gly His Glu Thr Thr Ala Leu Leu
                325                 330                 335

Leu Thr Trp Thr Thr Met Leu Leu Ala Asp Asn Pro Thr Trp Gln Glu
            340                 345                 350

Lys Val Arg Glu Glu Val Arg Glu Val Phe Gly Arg Asn Gly Leu Pro
        355                 360                 365

Ser Val Asp Gln Leu Ser Lys Leu Thr Ser Leu Ser Lys Val Ile Asn
    370                 375                 380

Glu Ser Leu Arg Leu Tyr Pro Pro Ala Thr Leu Leu Pro Arg Met Ala
385                 390                 395                 400

Phe Glu Asp Leu Lys Leu Gly Asp Leu Thr Ile Pro Lys Gly Leu Ser
                405                 410                 415

Ile Trp Ile Pro Val Leu Ala Ile His His Ser Glu Glu Leu Trp Gly
            420                 425                 430

Lys Asp Ala Asn Gln Phe Asn Pro Glu Arg Phe Gly Gly Arg Pro Phe
        435                 440                 445

Ala Ser Gly Arg His Phe Ile Pro Phe Ala Ala Gly Pro Arg Asn Cys
    450                 455                 460

Ile Gly Gln Gln Phe Ala Leu Met Glu Ala Lys Ile Ile Leu Ala Thr
465                 470                 475                 480

Leu Ile Ser Lys Phe Asn Phe Thr Ile Ser Lys Asn Tyr Arg His Ala
                485                 490                 495

Pro Ile Val Val Leu Thr Ile Lys Pro Lys Tyr Gly Val Gln Val Ile
            500                 505                 510

Leu Lys Pro Leu Val Ser
        515

## Description

### Technical field of the invention

The present invention generally relates to biotechnology engineering, and specifically to a genetically engineered bacterium capable of producing cytokinins with isoprenoid side chains (isoprenoid cytokinins), and the preparation and application thereof.

### Background of the invention

Cytokinins are essential plant hormones that control numerous plant growth and developmental processes from seed germination to plant and leaf senescence, characterized by their ability for induction of cell division (Mok, Martin, and Mok 2000). Naturally occurring cytokinins are adenine derivatives with either an aromatic side chain (aromatic cytokinins) or an isoprene-derived side chain at the *N*⁶-terminus of adenine (isoprenoid cytokinins). The *N*⁶-side-chain structure and configuration determine both the cytokinin type and its activity. The isoprenoid cytokinins are widespread and are produced naturally in plants and algae, and also in many species of bacteria, fungi, nematodes, and parasitic insects (Stirk and van Staden 2010). Natural isoprenoid cytokinins are: *N⁶*-(D2-isopentenyl)adenine (iP), *trans*-zeatin (tZ), *cis*-zeatin (cZ) and dihydrozeatin (DZ) (Figure 1).

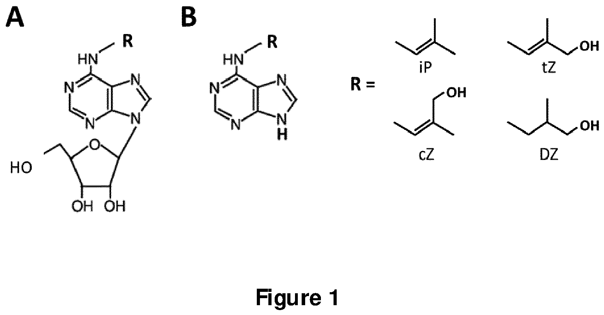

Isoprenoid cytokinins are derivatives of adenine or adenosine in which the exocyclic amino group at 6 has been modified by attachment of a dimethylallyl side chain, resulting in the parent compound *N*⁶-(D2-isopentenyl)adenine (iP) and its riboside, *N*⁶-(D2-isopentenyl)adenosine (iPR). The dimethylallyl side chain can be hydroxylated at 9' to give *trans-zeatin* (tZ) or its riboside, *trans-*ribosylzeatin (tZR). Also, the exocyclic double bond can be reduced to give dihydrozeatin (DZ) or its riboside, ribosyl dihydrozeatin (DZR) (Figure 1). All six compounds, iP, iPR, tZ, tZR, DZ, and DZR are biologically active and occur naturally.

The majority of naturally occurring cytokinins exist in distinctive structural derivatives or forms, such as free bases, ribosides, and nucleotides, or conjugates with glucose, xylose, or amino acid residues. In plants, the cytokinin free bases are considered the most biologically active forms while glucose conjugates are considered to be either permanently inactive or reversible storage forms, depending on the location of glycosylation.

Zeatin is the predominant form of cytokinin in plants. Zeatin is an adenine derivative with a hydroxylated isoprene-derived side chain at the *N*⁶ position of adenine, which can exist in *cis-* or *trans*-configuration (Figure 1). *Trans*-zeatin is one of the most effective naturally occurring cytokinins. In many previous studies, the analytical methods failed to distinguish *cis-* and *trans-*zeatin, thereby obscuring the understanding of the presence and role of both compounds in the observed physiological processes.

Despite the structural similarity, *cis*-zeatin is synthesized through the tRNA pathway, whereas in contrast, *trans*-zeatin, and biosynthetically related compounds iP and DZ are synthesized in plant cells through the *de novo* (or AMP) biosynthesis pathway.

In the tRNA pathway, *cis*-zeatin is a recycled product of degradation of isopentenylated tRNAs. *Cis-*zeatin is synthesized in almost all organisms except Archaea (Schäfer et al. 2015) at extremely low rates by tRNA-isopentenyltransferases (tRNA-IPTs) that catalyze the prenylation of adenine 37 on specific (UNN-)tRNAs leading to the formation of isopentenyl adenine (IP)-containing tRNA. In the *de novo* cytokinin biosynthesis pathway, the first step is N-prenylation of adenosine 5'-phosphates (AMP, ADP, or ATP) with dimethylallyl diphosphate (DMAPP) catalyzed by adenylate isopentenyltransferase (IPT, EC 2.5.1.27), which produces isopentenyladenine nucleotide (iP). iP, produced by IPT in plants, then undergoes hydroxylation at the prenyl side chain to result in tZ-nucleotides. In Arabidopsis, two cytochrome P450 monooxygenases, CYP735A1, and CYP735A2, catalyze the hydroxylation reaction. The CYP735As preferentially utilize iP-nucleotides rather than the iP-nucleoside and iP. Since this reaction is stereo-specific, the CYP735As produce tZ-nucleotides (Takei, Yamaya, and Sakakibara 2004). In the final step of both pathways, the *de novo* and the tRNA pathway, the cytokinin-activating enzyme cytokinin riboside 5'-monophosphate phosphoribohydrolase 'Lonely guy' (LOG, EC 3.2.2.n1) removes the ribosyl moiety and converts cytokinin nucleotides to their active nucleobases (Kurakawa et al. 2007). All four cytokinin nucleoside monophosphates, iPRM P, tZRM P, DZRM P, and cZRM P are utilized by LOG.

In the case of plant infection by phytopathogenic bacteria, such as *Agrobacterium tumefaciens,* biosynthesis of tZ is initiated to facilitate the infection. During the infection process, tZ or iP biosynthesis can occur inside the bacterial cells or bacterial IPT gene homologs can be integrated into the host's nuclear genome and expressed in the infected plant cells.

Importantly, substrate specificities differ between IPTs of bacteria, and higher plants (Kakimoto 2001; Sakakibara 2005). In the first aspect, bacterial IPTs, such asTmr and Tzs from *A. tumefaciens,* use only AM P as an acceptor (whereas plant IPT enzymes preferentially use ADP and ATP) to form *trans*-zeatin riboside 5'-monophosphate (tZRMP) (Sakakibara 2006; Kamada-Nobusada and Sakakibara 2009). Besides, Agrobacterium IPTs Tzs and Tmr are capable of using either DM APP or 1-hydroxy-2-methyl-2-butenyl 4-diphosphate (HMBDP), the hydroxylated precursor of DMAPP in the methylerythritol phosphate (MEP) pathway, as the side chain donor. When DMAPP is used asa substrate, the primary product is iPRMP, whereas tZRMP is formed when IPT utilizes HMBDP. Therefore, the expression of Agrobacterium IPTTmr in the chloroplastsof the plant cells during the infection creates a metabolic bypass for direct synthesis of tZRMP, sequestering the efficient substrate supply of isoprenoid precursors HMBDP in plastids of the plant cells. As in the native pathway, LOG phosphoribohydrolase finally releases the ribose monophosphate moiety from either iP- or tZ-nucleoside monophosphate (iPRMP, tZRMP), creating the biologically active molecule iP and tZ.

The tumor-inducing plant pathogenic bacterium *A. tumefaciens* has 2 IPTs: Tmr and Tzs. Tmr and Tzs are homologous proteins, both are DMAPP: AMP isopentenyltransferases, but their amino acids crucial for substrate recognition differ from those of plant adenylate IPTs (Chu et al., 2010). The *tmr* (*ipt*) gene is located in the T-region of the Ti-plasmid, which mediates infection in host plants. The *tmr* gene is transferred from the bacterium to the plant genome to force the host plantsto produce cytokinins, which results in tumor formation. *In vitro* experiments demonstrated that Tmr transferred both DMAPP and HMBDP to AMP with similar Km values (Sakakibara et al. 2005). Nopaline-producing strains of *A. tumefaciens* possess another gene for DMAPP : AMP isopentenyltransferase, *tzs,* which is present in the *vir* region of Ti-plasmid and is not translocated to the plant cells. Tzs enables the high level of cytokinin production and secretion by these A. *tumefaciens* strains (Morris et al. 1993), and can also use HMBDPor DMAPPto produce iPRMP and tZRMP. Tzs has 51.3% protein sequence identity to Tmr. Genes coding for DMAPP : AMP isopentenyltransferases homologous to tmr/tzs are present in other bacteria of the genus *Agrobacterium, A. vitis* and *A. rhizogenes,* as well as in other plant-pathogenic bacteria, such as *Pseudomonas syringae* pv. *savastanoi, Pseudomonas solanacearum, Pantoea agglomerans,* and *Rhodococcus fascians* (Kakimoto, 2003).

Isopentenyl donor, dimethylallyl diphosphate (DMAPP), and its precursor 1-hydroxy-2-methyl-2-butenyl 4-diphosphate (HM BDP) are produced by the methylerythritol phosphate (M EP) pathway in chloroplasts of plants and bacteria, also in *Bacillus subtilis.* In the MEP pathway, pyruvate and glyceraldehyde-3-phosphate are condensed by the enzyme 1-deoxy-D-xylulose-5-phosphate synthase (Dxs, EC 2.2.1.7) into a metabolic cascade ultimately producing HMBDP, DMAPP, and isoprene or larger terpenoid compounds. The first step in the M EP pathway, mediated by DXS, is the rate-limiting step for isoprenoid production in plants and bacteria (Julsing et al. 2007).

### Summary of the invention

The natural biosynthesis rate of cytokinins in plant-pathogenic bacteria through the *de novo* pathway during infection is very low. It depends on the expression of the IPT enzyme of bacterial origin, the LOG enzyme, and the building blocks, supplied by the metabolism of the host cells. Such an infection-based system is not readily transferable to industrial-scale production. Due to the high activity and potential of using cytokinin hormones in agricultural applications, there is thus a need for efficient and sustainable production of cytokinins such as iP, tZ, and ribosides tZR and iPR in genetically and biotechnologically amenable host strains.

The object of the present invention is to provide means allowing more efficient production of cytokinins with isoprenoid side chains (isoprenoid cytokinins), such astZand iP, and their ribosides tZR and iPR. More particularly, it is an object of the present invention to provide means allowing the production of cytokinins with isoprenoid side chains (isoprenoid cytokinins), such as tZ and iP, and their ribosides tZR and iPR, at higher nominal yield.

This is achieved by the present inventors who have engineered bacterial strains, which a) express a heterologous polypeptide having adenylate isopentenyltransferase activity and b) have been modified to have an increased protein expression of a polypeptide having cytokinin riboside 5'-monophosphate phosphoribohydrolase activity. As shown in the Examples, such engineered bacterial strains surprisingly show unusually high titers of isoprenoid cytokinins of over 10 mg/L in the supernatant. In consequence, this means that the context of plant cell infection is no longer required and the biosynthetic substrates and cofactors for efficient biosynthesis of isoprenoid cytokinins such as tZ, and iP, and their ribosides tZR and iPR are effectively supplied by the engineered bacterial cell.

The present invention thus provides in a first aspect a bacterium expressing a heterologous polypeptide having adenylate isopentenyltransferase activity. More particularly, the present invention provides a bacterium, which a) expresses a heterologous polypeptide having adenylate isopentenyltransferase activity and b) has been modified to have an increased protein expression of a polypeptide having cytokinin riboside 5'-monophosphate phosphoribohydrolase activity compared to an otherwise identical bacterium that does not carry said modification.

The present invention further provides in a second aspect a method for producing a cytokinin or riboside derivative thereof, particularly an isoprenoid cytokinin or riboside derivative thereof, comprising cultivating a bacterium according to the present invention under suitable culture conditions in a suitable culture medium.

The present invention may be summarized by the following items:
1. A bacterium, which a) expresses a heterologous polypeptide having adenylate isopentenyltransferase activity and b) has been modified to have an increased protein expression of a polypeptide having cytokinin riboside 5'-monophosphate phosphoribohydrolase activity compared to an otherwise identical bacterium that does not carry said modification.
2. The bacterium according to item 1, wherein the polypeptide having adenylate isopentenyltransferase activity is selected from the group consisting of: i) a polypeptide comprising an amino acid sequence of any one of SEQ ID NOs: 1 to 33; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 33.
3. The bacterium according to item 1, wherein the polypeptide having adenylate isopentenyltransferase activity is selected from the group consisting of: i) a polypeptide comprising the amino acid sequence of any one of SEQ ID NOs: 1 to 10; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 10.
4. The bacterium according to item 1, wherein the polypeptide having adenylate isopentenyltransferase activity is selected from the group consisting of: i) a polypeptide comprising the amino acid sequence of SEQ ID NO: 1; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 1.
5. The bacterium according to any one of items 1 to 4, wherein the bacterium comprises an exogenous nucleic acid molecule comprising a nucleotide sequence encoding said heterologous polypeptide.
6. The bacterium according to item 5, wherein the exogenous nucleic acid molecule further comprises a promoter that isfunctional in the bacterium to cause the production of an mRNA molecule and that isoperably linked to the nucleotide sequence encoding said heterologous polypeptide.
7. The bacterium according to item 5 or 6, wherein the exogenous nucleic acid molecule is a vector.
8. The bacterium according to item 5 or 6, wherein the exogenous nucleic acid molecule is stably integrated into the genome of the bacterium.
9. The bacterium according to any one of items 1 to 8, wherein the increase in protein expression of the polypeptide having cytokinin riboside 5'-monophosphate phosphoribohydrolase activity is achieved by increasing the number of copies of a gene encoding said polypeptide.
10. The bacterium according to item 9, wherein the increase in the number of copies of the gene is achieved by introducing into the bacterium one or more exogenous nucleic acid molecules (such as one or more vectors) comprising the gene operably linked to a promoter that is functional in the bacterium to cause the production of an mRNA molecule.
11. The bacterium according to any one of items 1 to 10, wherein the bacterium comprises an exogenous nucleic acid molecule (such as a vector) comprising a nucleotide sequence encoding the polypeptide having cytokinin riboside 5'-monophosphate phosphoribohydrolase activity.
12. The bacterium according to item 11, wherein the exogenous nucleic acid molecule further comprises a promoter that isfunctional in the bacterium to cause the production of an mRNA molecule and that isoperably linked to the nucleotide sequence encoding the polypeptide.
13. The bacterium according to any one of items 10 to 12, wherein the exogenous nucleic acid molecule is a vector.
14. The bacterium according to any one of items 10 to 12, wherein the exogenous nucleic acid molecule is stably integrated into the genome of the bacterium.
15. The bacterium according to any one of items 1 to 14, wherein the increase in protein expression of the polypeptide having cytokinin riboside 5'-monophosphate phosphoribohydrolase activity is achieved by modifying the ribosome binding site.
16. The bacterium according to any one of items 1 to 14, wherein the increase in protein expression of the polypeptide having cytokinin riboside 5'-monophosphate phosphoribohydrolase activity is achieved by increasing the strength of the promoter operably linked to the gene encoding the polypeptide.
17. The bacterium according to any one of items 1 to 16, wherein the polypeptide having cytokinin riboside 5'-monophosphate phosphoribohydrolase activity is selected from the group consisting of: i) a polypeptide comprising an amino acid sequence of any one of SEQ ID NOs: 34 to 62 and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 34 to 62.
18. The bacterium according to any one of items 1 to 17, wherein the polypeptide having cytokinin riboside 5'-monophosphate phosphoribohydrolase activity is selected from the group consisting of: i) a polypeptide comprising an amino acid sequence of any one of SEQ ID NOs: 34 to 44; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 34 to 44.
19. The bacterium according to any one of items 1 to 17, wherein the polypeptide having cytokinin riboside 5'-monophosphate phosphoribohydrolase activity is selected from the group consisting of: i) a polypeptide comprising the amino acid sequence of SEQ ID NO: 34; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 34.
20. The bacterium according to any one of items 1 to 19, wherein the polypeptide having cytokinin riboside 5'-monophosphate phosphoribohydrolase activity is a bacterial polypeptide having cytokinin riboside 5'-monophosphate phosphoribohydrolase activity.
21. The bacterium according to anyone of items 1 to 20, wherein the bacterium has been further modified to have an increased protein expression of a polypeptide having 1-deoxy-D-xylulose-5-phosphate synthase activity compared to an otherwise identical bacterium that does not carry said modification.
22. The bacterium according to item 21, wherein the increase in protein expression of the polypeptide having 1-deoxy-D-xylulose-5-phosphate synthase activity is achieved by increasing the number of copies of a gene encoding said polypeptide.
23. The bacterium according to item 22, wherein the increase in the number of copies of the gene is achieved by introducing into the bacterium one or more exogenous nucleic acid molecules (such as one or more vectors) comprising the gene operably linked to a promoter that isfunctional in the bacterium to cause the production of an mRNA molecule.
24. The bacterium according to any one of items 21 to 23, wherein the bacterium comprises an exogenous nucleic acid molecule (such as a vector) comprising a nucleotide sequence encoding the polypeptide having 1-deoxy-D-xylulose-5-phosphate synthase activity.
25. The bacterium according to item 24, wherein the exogenous nucleic acid molecule further comprises a promoter that isfunctional in the bacterium to cause the production of an mRNA molecule and that isoperably linked to the nucleotide sequence encoding the polypeptide.
26. The bacterium according to any one of items 23 to 25, wherein the exogenous nucleic acid molecule is a vector.
27. The bacterium according to any one of items 23 to 25, wherein the exogenous nucleic acid molecule is stably integrated into the genome of the bacterium.
28. The bacterium according to any one of items 21 to 27, wherein the increase in protein expression of the polypeptide having 1-deoxy-D-xylulose-5-phosphate synthase activity is achieved by modifying the ribosome binding site.
29. The bacterium according to any one of items 21 to 28, wherein the increase in protein expression of the polypeptide having 1-deoxy-D-xylulose-5-phosphate synthase activity is achieved by increasing the strength of the promoter operably linked to the gene encoding the polypeptide.
30. The bacterium according to any one of items 21 to 29, wherein the polypeptide having 1-deoxy-D-xylulose-5-phosphate synthase activity is selected from the group consisting of: i) a polypeptide comprising an amino acid sequence of any one of SEQ ID NOs: 63 to 70; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 63 to 70.
31. The bacterium according to any one of items 21 to 30, wherein the polypeptide having 1-deoxy-D-xylulose-5-phosphate synthase activity is selected from the group consisting of: i) a polypeptide comprising an amino acid sequence of any one of SEQ ID NOs: 63 to 65; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 63 to 65.
32. The bacterium according to any one of items 21 to 31, wherein the polypeptide having 1-deoxy-D-xylulose-5-phosphate synthase activity is selected from the group consisting of: i) a polypeptide comprising the amino acid sequence of SEQ ID NO: 63; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 63.
33. The bacterium according to any one of items 21 to 31, wherein the polypeptide having 1-deoxy-D-xylulose-5-phosphate synthase activity is selected from the group consisting of: i) a polypeptide comprising the amino acid sequence of SEQ ID NO: 64; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 64.
34. The bacterium according to any one of items 21 to 33, wherein the polypeptide having 1-deoxy-D-xylulose-5-phosphate synthase activity is a bacterial polypeptide having 1-deoxy-D-xylulose-5-phosphate synthase activity.
35. The bacterium according to any one of items 1 to 34, which has been further modified to have an increased expression and/or activity of at least one enzyme involved in the purine nucleotide biosynthesis pathway (e.g., at least one enzyme involved in the adenosine monophosphate biosynthesis pathway) compared to an otherwise identical bacterium that does not carry said modification.
36. The bacterium according to item 35, wherein the at least one enzyme involved in the purine nucleotide biosynthesis pathway is selected from the group consisting of: an enzyme having ribose-phosphate diphosphokinase activity, an enzyme having amidophosphoribosyltransferase activity, an enzyme having formyltetrahydrofolate deformylase activity, an enzyme having adenylosuccinate lyase activity, an enzyme having phosphoribosylaminoimidazole-carboxamide formyltransferase activity, an enzyme having adenylosuccinate synthase activity and an enzyme having adenosine kinase activity.
37. The bacterium according to any one of items 1 to 36, which has been further modified to have a decreased expression and/or activity of at least one endogenous enzyme involved in the purine nucleotide degradation pathway compared to an otherwise identical bacterium that does not carry said modification.
38. The bacterium according to item 37, wherein the at least one endogenous enzyme involved in the purine nucleotide degradation pathway is selected from the group consisting of: an enzyme having purine nucleoside phosphorylase activity and an enzyme having adenosine-phosphoribosyltransferase activity.
39. The bacterium according to item 37 or 38, wherein the at least one endogenous enzyme involved in the purine nucleotide degradation pathway is an enzyme having purine nucleoside phosphorylase activity.
40. The bacterium according to any one of items 37 to 39, wherein the at least one endogenous enzyme involved in the purine nucleotide degradation pathway is an enzyme having adenosine-phosphoribosyltransf erase activity.
41. The bacterium according to any one of items 1 to 40, which has been further modified to have a decreased expression and/or activity of at least one endogenous enzyme involved in the guanosine monophosphate biosynthesis pathway compared to an otherwise identical bacterium that does not carry said modification.
42. The bacterium according to item 41, wherein the at least one endogenous enzyme involved in the guanosine monophosphate biosynthesis pathway isselected from the group consisting of: an enzyme having IMP dehydrogenase activity and an enzyme having GMP synthetase activity.
43. The bacterium according to item 41 or 42, wherein the at least one endogenous enzyme involved in the guanosine monophosphate biosynthesis pathway is an enzyme having IMP dehydrogenase activity.
44. The bacterium according to any one of items 41 to 43, wherein the at least one endogenous enzyme involved in the guanosine monophosphate biosynthesis pathway is an enzyme having GMP synthetase activity.
45. The bacterium according to any one of items 1 to 44, which has been further modified to have an increased protein expression of a polypeptide having cytochrome P450 monooxygenase (CYP450) activity compared to an otherwise identical bacterium that does not carry said modification.
46. The bacterium according to item 45, wherein the increase in protein expression of the polypeptide having cytochrome P450 monooxygenase (CYP450) activity is achieved by increasing the number of copies of a gene encoding said polypeptide.
47. The bacterium according to item 46, wherein the increase in the number of copies of the gene is achieved by introducing into the bacterium one or more exogenous nucleic acid molecules (such as one or more vectors) comprising the gene operably linked to a promoter that is functional in the bacterium to cause the production of an mRNA molecule.
48. The bacterium according to any one of items 1 to 47, wherein the bacterium comprises an exogenous nucleic acid molecule (such as a vector) comprising a nucleotide sequence encoding the polypeptide having cytochrome P450 monooxygenase (CYP450) activity.
49. The bacterium according to item 48, wherein the exogenous nucleic acid molecule further comprises a promoter that isfunctional in the bacterium to cause the production of an mRNA molecule and that isoperably linked to the nucleotide sequence encoding the polypeptide.
50. The bacterium according to any one of items 47 to 49, wherein the exogenous nucleic acid molecule is a vector.
51. The bacterium according to any one of items 47 to 49, wherein the exogenous nucleic acid molecule is stably integrated into the genome of the bacterium.
52. The bacterium according to any one of items 45 to 51, wherein the polypeptide having cytochrome P450 monooxygenase (CYP450) activity is selected from the group consisting of:
   i) a polypeptide comprising an amino acid sequence of any one of SEQ ID NOs: 93 to 95 and
   ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 93 to 95.
53. The bacterium according to any one of items 1 to 52, wherein the bacterium is of the family selected from the group consisting of *Enterobacteriaceae, Bacillaceae, Lactobacillaceae* and *Corynebacteriaceae.*
54. The bacterium according to any one of items 1 to 52, wherein the bacterium is of the family selected from the group consisting of *Bacillaceae* and *Corynebacteriaceae.*
55. The bacterium according to any one of items 1 to 52, wherein the bacterium is of the family *Bacillaceae.*
56. The bacterium according to any one of items 1 to 52, wherein the bacterium is of the family *Corynebacteriaceae.*
57. The bacterium according to any one of items 1 to 52, wherein the bacterium is of the genus *Bacillus, Lactococcus, Lactobacillus, Clostridium, Corynebacterium, Geobacillus, Thermoanaerobacterium, Streptococcus, Pseudomonas, Streptomyces, Escherichia, Shigella, Acinetobacter, Citrobacter, Salmonella, Klebsiella, Enterobacter, Erwinia, Kluyvera, Serratia, Cedecea, Morganella, Hafnia, Edwardsiella, Providencia, Proteus* or *Yersinia.*
58. The bacterium according to any one of items 1 to 52, wherein the bacterium is of the genus *Bacillus* or *Corynebacterium.*
59. The bacterium according to any one of items 1 to 52, wherein the bacterium is of the genus *Bacillus.*
60. The bacterium according to any one of items 1 to 52, wherein the bacterium is of the genus *Corynebacterium.*
61. The bacterium according to any one of items 1 to 52, wherein the bacterium is *Bacillus subtilis.*
62. The bacterium according to any one of items 1 to 34, wherein the bacterium is *Corynebacterium stationis.*
63. Method for producing an isoprenoid cytokinin or riboside derivative thereof, comprising cultivating a bacterium according to any one of items 1 to 62 under suitable culture conditions in a suitable culture medium.
64. The method according to item 63, wherein the isoprenoid cytokinin or riboside derivative thereof is selected from the group consisting of *trans*-zeatin (tZ), *trans*-zeatin riboside (tZR), *N*⁶-(D2-isopentenyl)adenine (iP), N(6)-(dimethylallyl)adenosine (iPR), dihydrozeatin (DZ), ribosyl dihydrozeatin (DZR), and combinations thereof.
65. The method according to item 64, wherein the isoprenoid cytokinin or riboside derivative thereof is *trans*-zeatin (tZ) and *trans*-zeatin riboside (tZR), respectively.

### Brief description of the figures

**Figure 1****:** Isoprenoid cytokinins: A) Ribosides: N⁶-(D2-isopentenyl)adenine riboside (iPR), *trans-*zeatin riboside (tZR), *cis*-zeatin riboside (cZR) and dihydrozeatin riboside (DZR). B) Free bases: N⁶-(D2-isopentenyl)adenine (iP), *trans-*zeatin (tZ), *cis*-zeatin (cZ) and dihydrozeatin (DZ).

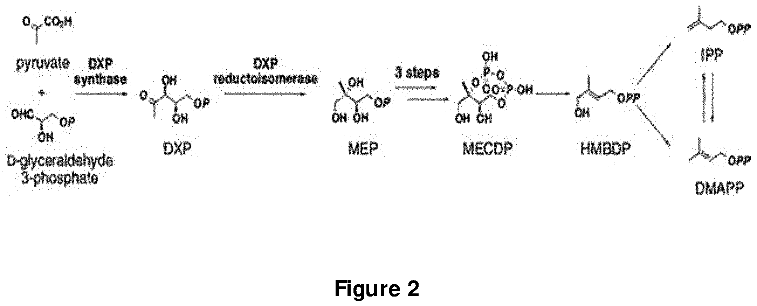

**Figure 2****:** Schematic representation of the MEP metabolic pathway.

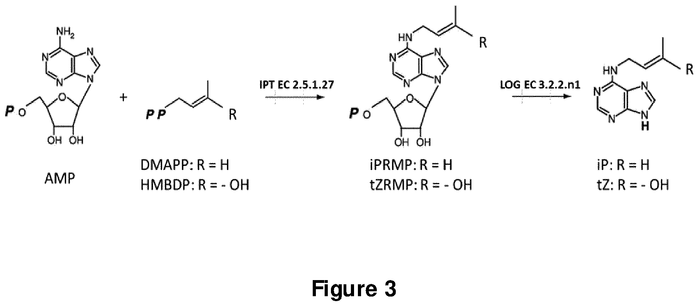

**Figure 3****:** Isoprenoid cytokinin biosynthesis pathway with heterologous expression of IPT (EC 2.5.1.27) and LOG (EC3.2.2.n1).
**Figure 4****:** Production of *trans-zeatin* and related isoprenoid cytokinins of *Bacillus subtilis* strains in experiments in shaker-scale experiments. VKPM B2116 - parent strain, TZAB14, TZAB15 - IPT-LOG.
**Figure 5****:** Production of *trans*-zeatin and related isoprenoid cytokinins of *Bacillus subtilis* strains in experiments in shaker-scale experiments with adenine sulfate. VKPM B2116 - parent strain, TZAB14 - IPT-LOG.
**Figure 6****:** Production of *trans-zeatin* and related isoprenoid cytokinins by *Bacillus subtilis* strains with IPT and LOG genes and DXSgene in shaker-scale experiments. VKPM B2116 - parent strain, TZAB15 - IPT-LOG, TZAB43 - IPT-LOG-DXS.
**Figure 7****:** Production of *trans-zeatin* and related isoprenoid cytokinins by *Bacillus subtilis* strains expressing IPT (SEQ ID NO: 1) and LOG (SEQ ID NO: 34) or IPT (SEQ ID NO: 2) and LOG (SEQ ID NO: 34) after 28h in shaker-scale experiments. VKPM B2116 - parent strain, TZAB1, TZAB2, TZAB3, TZAB4 - strains with IPT (SEQ ID NO: 2) and LOG (SEQ ID NO: 34), TZAB14, TZAB15 - strains with IPT (SEQ ID NO: 1) and LOG (SEQ ID NO: 34).

The present invention is now described in more detail below.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYM OLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.).

### Bacterium of the invention

As indicated above, the present inventors have engineered bacterial strains, which a) express a heterologous polypeptide having adenylate isopentenyltransferase activity and b) have been modified to have an increased protein expression of a polypeptide having cytokinin riboside 5'-monophosphate phosphoribohydrolase activity. As shown in the Examples, such engineered bacterial strains surprisingly show unusually high titers of isoprenoid cytokinins of over 10 mg/L in the supernatant. In consequence, this means that the context of plant cell infection is no longer required and the biosynthetic substrates and cofactors for efficient biosynthesis of isoprenoid cytokinins such as tZ, and iP, and their ribosides tZR and iPR are effectively supplied by the engineered bacterial cell.

The present invention thus provides in a first aspect a bacterium expressing a heterologous polypeptide having adenylate isopentenyltransferase activity. More particularly, the present invention provides a bacterium, which a) expresses a heterologous polypeptide having adenylate isopentenyltransferase activity and b) has been modified to have an increased protein expression of a polypeptide having cytokinin riboside 5'-monophosphate phosphoribohydrolase activity compared to an otherwise identical bacterium that does not carry said modification.

Adenylate isopentenyltransferases (IPTs) are a well-defined class of enzymes catalyzing the first step in the *de novo* cytokinin biosynthesis pathway, the N-prenylation of adenosine 5'-phosphates (AMP, ADP, or ATP) with either dimethylallyl diphosphate (DMAPP) or 1-hydroxy-2-methyl-2-butenyl 4-diphosphate (HMBDP). There are two types of IPTs (EC 2.5.1.27 and EC 2.5.1.112). The IPT enzymes are present in bacteria and plants. IPTs from bacteria, such as from Agrobacterium which contains two IPT homologs, Tzs and Tmr, use AMP as a prenyl acceptor, and HMBDP or DMAPP as the donor (EC 2.5.1.27). The product of the reaction with HMBDP is tZ-nucleotide. Moreover, IPTs from bacteria belong to the Pfam family IPT (PF01745) in the Pfam database of protein families and domains (https://pfam.xfam.org/family/ipt). The IPT Pfam domain genes are phylogenetically scattered and found only in some members of Actinobacteria, Cyanobacteria, • - Proteobacteria, • -Proteobacteria, and •-Proteobacteria and in the eukaryote Dictyostelium discoideum (Nishii et al., 2018). IPTs from higher plants use predominantly ATP or ADP as a prenyl acceptor and DMAPP as the donor (EC 2.5.1.112) and belong to the Pfam family IPPT (PF01715) (https://pfam.xfam.org/family/ippt). The product of the reaction is iP, which is then hydroxylated at the prenyl side chain to result in tZ-nucleotides.

Tzs of *Agrobacterium tumefaciens* (IPT(PF01745); EC2.5.1.27; SEQ ID NO: 1) consists of 2 domains: the N-terminal domain with a five-stranded parallel • -sheet, which is surrounded by 3 • -helices (• 1 - • 3), and the C-terminal domain with 5 • helices (• 4 - • 8). The N-terminal domain contains a nucleotide-binding p loop motif Gly-8-Pro-Thr-Cys-Ser-Gly-Lys-Thr-15, and is structurally related to the *p* loop-containing nucleoside triphosphate hydrolase (pNTPase) superfamily. The C-terminal side of • 8 extends to the N-terminal and is attached to it. The interface between the domains forms a solvent-accessible channel, which binds AM P. The prenylation site of AM P binds to Asp-33 and Ser-45. DMAPP is bound to Asp-173, Tyr-211, and His-214. Thr-10, Asp-33, and Arg-138 are fully conserved among IPTs. In Tzs, the hydrophilic region formed by the side chains contains His-214 and Asp-173, which are two critical amino acid residues within the substrate-binding pocket that distinguish the presence or absence of the hydroxyl group of the prenyl-donor substrate, which allows the use of HM BDP (Sugawara et al. 2008).

Generally, the polypeptide having adenylate isopentenyltransferase activity employed according to the invention will be heterologous to the bacterium, which means that said polypeptide is normally not found in or made (i.e. expressed) by the bacterium, but is derived from a different species. Moreover, the polypeptide having adenylate isopentenyltransferase activity is derived from or corresponds to a member of the Pfam family IPT (PF01745), and preferably is a bacterial polypeptide having adenylate isopentenyltransferase activity. With "bacterial polypeptide having adenylate isopentenyltransferase activity" it is meant that the polypeptide having adenylate isopentenyltransferase activity is derived from a bacterium, such as *Agrobacterium tumefaciens.*

Polypeptides having adenylate isopentenyltransferase activity most suitable for the biosynthesisof isoprenoid cytokinins including tZ, iP, tZR and iPR, are enzymes capable of using both HMBDP and DMAPP as prenyl donors, such as Tzs or Tmr of *Agrobacterium tumefaciens.* Moreover, they belong to the Pfam family IPT (PF01745) and contain an Asp residue in a position equivalent to position 173 of SEQ ID NO:1, a Tyr residue in a position equivalent to position 211 of SEQ ID NO:1 and/or a His residue in a position equivalent to position 214 of SEQ ID NO:1.

A polypeptide having adenylate isopentenyltransferase activity for use according to the invention may for instance be a polypeptide having adenylate isopentenyltransferase activity selected from the group consisting of: i) a polypeptide comprising the amino acid sequence of any one of SEQ ID NOs: 1 to 33; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 33.

A polypeptide having adenylate isopentenyltransferase activity for use according to the invention may for instance be a polypeptide having adenylate isopentenyltransferase activity selected from the group consisting of: i) a polypeptide comprising the amino acid sequence of any one of SEQ ID NOs: 1 to 33; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 33.

A polypeptide having adenylate isopentenyltransferase activity for use according to the invention may for instance be a polypeptide having adenylate isopentenyltransferase activity selected from the group consisting of: i) a polypeptide comprising the amino acid sequence of any one of SEQ ID NOs: 1 to 10; and ii) a polypeptide comprising an amino acid sequence, which has at least about 85%, such as at least about 90%, at least about 93%, at least 95%, at least 96%, at least 97%, at least 98%, or at least 99%, sequence identity to the amino acid sequence of any one of SEQ ID NOs: 1 to 10.

A polypeptide having adenylate isopentenyltransferase activity for use according to the invention may for instance be a polypeptide having adenylate isopentenyltransferase activity selected from the group consisting of: i) a polypeptide comprising the amino acid sequence of any one of SEQ ID NOs: 1 to 10; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 10.

A polypeptide having adenylate isopentenyltransferase activity for use according to the invention may for instance be a polypeptide having adenylate isopentenyltransferase activity selected from the group consisting of: i) a polypeptide comprising the amino acid sequence of any one of SEQ ID NOs: 1 to 10; and ii) a polypeptide comprising an amino acid sequence, which has at least about 85%, such as at least about 90%, at least about 93%, at least 95%, at least 96%, at least 97%, at least 98%, or at least 99%, sequence identity to the amino acid sequence of any one of SEQ ID NOs: 1 to 10.

According to some embodiments, the polypeptide having adenylate isopentenyltransferase activity comprises an amino acid sequence having at least 50%, such as at least 55%, sequence identity with the amino acid sequence SEQ ID NO: 1. According to some embodiments, the polypeptide having adenylate isopentenyltransferase activity comprises an amino acid sequence having at least 60%, such as at least 65%, sequence identity with the amino acid sequence SEQ ID NO: 1. According to some embodiments, the polypeptide having adenylate isopentenyltransferase activity comprises an amino acid sequence having at least 70%, such as at least 75%, sequence identity with the amino acid sequence SEQ ID NO: 1. According to some embodiments, the polypeptide having adenylate isopentenyltransferase activity comprises an amino acid sequence having at least 80%, such as at least 85%, sequence identity with the amino acid sequence SEQ ID NO: 1. According to some embodiments, the polypeptide having adenylate isopentenyltransferase activity comprises an amino acid sequence having at least 90%, such as at least 95%, sequence identity with the amino acid sequence SEQ ID NO: 1. According to some embodiments, the polypeptide having adenylate isopentenyltransferase activity comprises an amino acid sequence having at least 95%, such as at least 97%, sequence identity with the amino acid sequence SEQ ID NO: 1. According to some embodiments, the polypeptide having adenylate isopentenyltransferase activity comprises the amino acid sequence of SEQ ID NO: 1.

According to some embodiments, the polypeptide having adenylate isopentenyltransferase activity comprises an amino acid sequence having at least 50%, such as at least 55%, sequence identity with the amino acid sequence SEQ ID NO: 2. According to some embodiments, the polypeptide having adenylate isopentenyltransferase activity comprises an amino acid sequence having at least 60%, such as at least 65%, sequence identity with the amino acid sequence SEQ ID NO: 2. According to some embodiments, the polypeptide having adenylate isopentenyltransferase activity comprises an amino acid sequence having at least 70%, such as at least 75%, sequence identity with the amino acid sequence SEQ ID NO: 2. According to some embodiments, the polypeptide having adenylate isopentenyltransferase activity comprises an amino acid sequence having at least 80%, such as at least 85%, sequence identity with the amino acid sequence SEQ ID NO: 2. According to some embodiments, the polypeptide having adenylate isopentenyltransferase activity comprises an amino acid sequence having at least 90%, such as at least 95%, sequence identity with the amino acid sequence SEQ ID NO: 2. According to some embodiments, the polypeptide having adenylate isopentenyltransferase activity comprises an amino acid sequence having at least 95%, such as at least 97%, sequence identity with the amino acid sequence SEQ ID NO: 2. According to some embodiments, the polypeptide having adenylate isopentenyltransferase activity comprises the amino acid sequence of SEQ ID NO: 2.

According to some embodiments, the polypeptide having adenylate isopentenyltransferase activity comprises an amino acid sequence having at least 50%, such as at least 55%, sequence identity with the amino acid sequence SEQ ID NO: 3. According to some embodiments, the polypeptide having adenylate isopentenyltransferase activity comprises an amino acid sequence having at least 60%, such as at least 65%, sequence identity with the amino acid sequence SEQ ID NO: 3. According to some embodiments, the polypeptide having adenylate isopentenyltransferase activity comprises an amino acid sequence having at least 70%, such as at least 75%, sequence identity with the amino acid sequence SEQ ID NO: 3. According to some embodiments, the polypeptide having adenylate isopentenyltransferase activity comprises an amino acid sequence having at least 80%, such as at least 85%, sequence identity with the amino acid sequence SEQ ID NO: 3. According to some embodiments, the polypeptide having adenylate isopentenyltransferase activity comprises an amino acid sequence having at least 90%, such as at least 95%, sequence identity with the amino acid sequence SEQ ID NO: 3. According to some embodiments, the polypeptide having adenylate isopentenyltransferase activity comprises an amino acid sequence having at least 95%, such as at least 97%, sequence identity with the amino acid sequence SEQ ID NO: 3. According to some embodiments, the polypeptide having adenylate isopentenyltransferase activity comprises the amino acid sequence of SEQ ID NO: 3.

According to some embodiments, the polypeptide having adenylate isopentenyltransferase activity comprises an amino acid sequence having at least 50%, such as at least 55%, sequence identity with the amino acid sequence SEQ ID NO: 4. According to some embodiments, the polypeptide having adenylate isopentenyltransferase activity comprises an amino acid sequence having at least 60%, such as at least 65%, sequence identity with the amino acid sequence SEQ ID NO: 4. According to some embodiments, the polypeptide having adenylate isopentenyltransferase activity comprises an amino acid sequence having at least 70%, such as at least 75%, sequence identity with the amino acid sequence SEQ ID NO: 4. According to some embodiments, the polypeptide having adenylate isopentenyltransferase activity comprises an amino acid sequence having at least 80%, such as at least 85%, sequence identity with the amino acid sequence SEQ ID NO: 4. According to some embodiments, the polypeptide having adenylate isopentenyltransferase activity comprises an amino acid sequence having at least 90%, such as at least 95%, sequence identity with the amino acid sequence SEQ ID NO: 4. According to some embodiments, the polypeptide having adenylate isopentenyltransferase activity comprises an amino acid sequence having at least 95%, such as at least 97%, sequence identity with the amino acid sequence SEQ ID NO: 4. According to some embodiments, the polypeptide having adenylate isopentenyltransferase activity comprises the amino acid sequence of SEQ ID NO: 4.

According to some embodiments, the polypeptide having adenylate isopentenyltransferase activity comprises an amino acid sequence having at least 50%, such as at least 55%, sequence identity with the amino acid sequence SEQ ID NO: 5. According to some embodiments, the polypeptide having adenylate isopentenyltransferase activity comprises an amino acid sequence having at least 60%, such as at least 65%, sequence identity with the amino acid sequence SEQ ID NO: 5. According to some embodiments, the polypeptide having adenylate isopentenyltransferase activity comprises an amino acid sequence having at least 70%, such as at least 75%, sequence identity with the amino acid sequence SEQ ID NO: 5. According to some embodiments, the polypeptide having adenylate isopentenyltransferase activity comprises an amino acid sequence having at least 80%, such as at least 85%, sequence identity with the amino acid sequence SEQ ID NO: 5. According to some embodiments, the polypeptide having adenylate isopentenyltransferase activity comprises an amino acid sequence having at least 90%, such as at least 95%, sequence identity with the amino acid sequence SEQ ID NO: 5. According to some embodiments, the polypeptide having adenylate isopentenyltransferase activity comprises an amino acid sequence having at least 95%, such as at least 97%, sequence identity with the amino acid sequence SEQ ID NO: 5. According to some embodiments, the polypeptide having adenylate isopentenyltransferase activity comprises the amino acid sequence of SEQ ID NO: 5.

According to some embodiments, the polypeptide having adenylate isopentenyltransferase activity comprises an amino acid sequence having at least 50%, such as at least 55%, sequence identity with the amino acid sequence SEQ ID NO: 6. According to some embodiments, the polypeptide having adenylate isopentenyltransferase activity comprises an amino acid sequence having at least 60%, such as at least 65%, sequence identity with the amino acid sequence SEQ ID NO: 6. According to some embodiments, the polypeptide having adenylate isopentenyltransferase activity comprises an amino acid sequence having at least 70%, such as at least 75%, sequence identity with the amino acid sequence SEQ ID NO: 6. According to some embodiments, the polypeptide having adenylate isopentenyltransferase activity comprises an amino acid sequence having at least 80%, such as at least 85%, sequence identity with the amino acid sequence SEQ ID NO: 6. According to some embodiments, the polypeptide having adenylate isopentenyltransferase activity comprises an amino acid sequence having at least 90%, such as at least 95%, sequence identity with the amino acid sequence SEQ ID NO: 6. According to some embodiments, the polypeptide having adenylate isopentenyltransferase activity comprises an amino acid sequence having at least 95%, such as at least 97%, sequence identity with the amino acid sequence SEQ ID NO: 6. According to some embodiments, the polypeptide having adenylate isopentenyltransferase activity comprises the amino acid sequence of SEQ ID NO: 6.

According to some embodiments, the polypeptide having adenylate isopentenyltransferase activity comprises an amino acid sequence having at least 50%, such as at least 55%, sequence identity with the amino acid sequence SEQ ID NO: 7. According to some embodiments, the polypeptide having adenylate isopentenyltransferase activity comprises an amino acid sequence having at least 60%, such as at least 65%, sequence identity with the amino acid sequence SEQ ID NO: 7. According to some embodiments, the polypeptide having adenylate isopentenyltransferase activity comprises an amino acid sequence having at least 70%, such as at least 75%, sequence identity with the amino acid sequence SEQ ID NO: 7. According to some embodiments, the polypeptide having adenylate isopentenyltransferase activity comprises an amino acid sequence having at least 80%, such as at least 85%, sequence identity with the amino acid sequence SEQ ID NO: 7. According to some embodiments, the polypeptide having adenylate isopentenyltransferase activity comprises an amino acid sequence having at least 90%, such as at least 95%, sequence identity with the amino acid sequence SEQ ID NO: 7. According to some embodiments, the polypeptide having adenylate isopentenyltransferase activity comprises an amino acid sequence having at least 95%, such as at least 97%, sequence identity with the amino acid sequence SEQ ID NO: 7. According to some embodiments, the polypeptide having adenylate isopentenyltransferase activity comprises the amino acid sequence of SEQ ID NO: 7.

According to some embodiments, the polypeptide having adenylate isopentenyltransferase activity comprises an amino acid sequence having at least 50%, such as at least 55%, sequence identity with the amino acid sequence SEQ ID NO: 8. According to some embodiments, the polypeptide having adenylate isopentenyltransferase activity comprises an amino acid sequence having at least 60%, such as at least 65%, sequence identity with the amino acid sequence SEQ ID NO: 8. According to some embodiments, the polypeptide having adenylate isopentenyltransferase activity comprises an amino acid sequence having at least 70%, such as at least 75%, sequence identity with the amino acid sequence SEQ ID NO: 8. According to some embodiments, the polypeptide having adenylate isopentenyltransferase activity comprises an amino acid sequence having at least 80%, such as at least 85%, sequence identity with the amino acid sequence SEQ ID NO: 8. According to some embodiments, the polypeptide having adenylate isopentenyltransferase activity comprises an amino acid sequence having at least 90%, such as at least 95%, sequence identity with the amino acid sequence SEQ ID NO: 8. According to some embodiments, the polypeptide having adenylate isopentenyltransferase activity comprises an amino acid sequence having at least 95%, such as at least 97%, sequence identity with the amino acid sequence SEQ ID NO: 8. According to some embodiments, the polypeptide having adenylate isopentenyltransferase activity comprises the amino acid sequence of SEQ ID NO: 8.

According to some embodiments, the polypeptide having adenylate isopentenyltransferase activity comprises an amino acid sequence having at least 50%, such as at least 55%, sequence identity with the amino acid sequence SEQ ID NO: 9. According to some embodiments, the polypeptide having adenylate isopentenyltransferase activity comprises an amino acid sequence having at least 60%, such as at least 65%, sequence identity with the amino acid sequence SEQ ID NO: 9. According to some embodiments, the polypeptide having adenylate isopentenyltransferase activity comprises an amino acid sequence having at least 70%, such as at least 75%, sequence identity with the amino acid sequence SEQ ID NO: 9. According to some embodiments, the polypeptide having adenylate isopentenyltransferase activity comprises an amino acid sequence having at least 80%, such as at least 85%, sequence identity with the amino acid sequence SEQ ID NO: 9. According to some embodiments, the polypeptide having adenylate isopentenyltransferase activity comprises an amino acid sequence having at least 90%, such as at least 95%, sequence identity with the amino acid sequence SEQ ID NO: 9. According to some embodiments, the polypeptide having adenylate isopentenyltransferase activity comprises an amino acid sequence having at least 95%, such as at least 97%, sequence identity with the amino acid sequence SEQ ID NO: 9. According to some embodiments, the polypeptide having adenylate isopentenyltransferase activity comprises the amino acid sequence of SEQ ID NO: 9.

According to some embodiments, the polypeptide having adenylate isopentenyltransferase activity comprises an amino acid sequence having at least 50%, such as at least 55%, sequence identity with the amino acid sequence SEQ ID NO: 10. According to some embodiments, the polypeptide having adenylate isopentenyltransferase activity comprises an amino acid sequence having at least 60%, such as at least 65%, sequence identity with the amino acid sequence SEQ ID NO: 10. According to some embodiments, the polypeptide having adenylate isopentenyltransferase activity comprises an amino acid sequence having at least 70%, such as at least 75%, sequence identity with the amino acid sequence SEQ ID NO: 10. According to some embodiments, the polypeptide having adenylate isopentenyltransferase activity comprises an amino acid sequence having at least 80%, such as at least 85%, sequence identity with the amino acid sequence SEQ ID NO: 10. According to some embodiments, the polypeptide having adenylate isopentenyltransferase activity comprises an amino acid sequence having at least 90%, such as at least 95%, sequence identity with the amino acid sequence SEQ ID NO: 10. According to some embodiments, the polypeptide having adenylate isopentenyltransferase activity comprises an amino acid sequence having at least 95%, such as at least 97%, sequence identity with the amino acid sequence SEQ ID NO: 10. According to some embodiments, the polypeptide having adenylate isopentenyltransferase activity comprises the amino acid sequence of SEQ ID NO: 10.

Techniques for determining adenylate isopentenyltransferase activity are well known to the skilled person. Exemplary methods have been described, e.g. by Takei, Sakakibara, and Sugiyama (2001) and by Frébortová, Greplová et al, (2015). The adenylate isopentenyltransferase activity may for instance be determined in accordance with any of the following assays:
(1) Enzyme is incubated in a reaction mixture (1M betaine, 20 mM triethanolamine, 50 mM KCl, 10 mM MgCl₂, 1 mM dithiothreitol, 1 mg/ml bovine serum albumin, pH 8.0) with 1 mM AMP and 340 • M DMAPPat 25•°C for 20 min. The reaction is stopped by the addition of a quarter volume of 10% acetate and centrifuged at 18,000 x g for 20 min. The resulting supernatant is subjected to cytokinin analysis. One unit of IPT activity is defined as the amount of enzyme that produced 1 • mol of iPM P/min under the condition of the reaction (Takei, Sakakibara, and Sugiyama 2001).
(2) Activity assay is performed at 25°Covernight in 200 • l of a reaction mixture (100 mM Tris/HCI buffer, pH 7.5, containing 10 mM MgCl₂), with 100 • M AMP and 100 • M DMAPP (Echelon BioSciences, Salt Lake City, UT, USA) as substrates and 100 • l of purified enzyme. To assess substrate preference of IPT, ADP or ATP are used as isoprene chain accepting substrates, whereas isopenthenyl diphosphate or HMBPP (Echelon Bioscences) as isoprene chain donating substrates. The reaction is initiated by adding the isoprenoid substrate and stopped by heating to 95°C for 5 min to inactivate the enzyme. The IPT activity assay isbased on determination of reaction products by HPLC or capillary electrophoresis with UV detection at 268 nm. Cytokinin ribosides and corresponding monophosphates are determined on Symmetry C18 column (2.1 x 150 mm, 5 • m; Waters, Milford, MA, USA) connected to Alliance 2695 high performance liquid chromatograph (Waters). The column waseluted by a linear gradient of15 mM ammonium formate, pH 4.0 (A) and methanol (B) using the following solvent mixture: 0-25 min, 5-60% B, 25-26 min, 60-100% B, 26-27 min 100% B. Linear gradient of15 mM ammonium formate, pH 4.0 (A) and acetonitrile (B) was used for the analysis of oligoribonucleotide hydrolysates (0-30 min, 5-24% B, 30-31 min, 24-100% B). The flow rate is 0.25 ml/min and the column temperature is 30°C. The concentration of the product is determined by a calibration curve method using authentic standard compounds (Olchemim, Olomouc, Czech Republic). Capillary electrophoresis is used for determination of cytokinin di- and triphosphates (Frébortová, Greplová et al, 2015).

Besides expressing a heterologous polypeptide having adenylate isopentenyltransferase activity, the bacterium of the present invention is further characterized in that it has been modified to have an increased protein expression of a polypeptide having cytokinin riboside 5'-monophosphate phosphoribohydrolase activity compared to an otherwise identical bacterium that does not carry said modification.

By "increased protein expression" it is meant that the amount of the polypeptide having cytokinin riboside 5'-monophosphate phosphoribohydrolase activity produced by the thus modified bacterium is increased compared to an otherwise identical bacterium that does not carry said modification. More particularly, by "increased expression" it is meant that the amount of the polypeptide having cytokinin riboside 5'-monophosphate phosphoribohydrolase activity produced by the thus modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical bacterium that does not carry said modification. The amount of protein in a given cell can be determined by any suitable quantification technique known in the art, such as ELISA, Immunohistochemistry, or Western Blotting.

An increase in protein expression may be achieved by any suitable means well-known to those skilled in the art. For example, an increase in protein expression may be achieved by increasing the number of copies of the gene encoding the polypeptide having cytokinin riboside 5'-monophosphate phosphoribohydrolase activity in the bacterium, such as by introducing into the bacterium an exogenous nucleic acid, such as a vector, comprising the gene encoding the polypeptide having cytokinin riboside 5'-monophosphate phosphoribohydrolase activity operably linked to a promoter that is functional in the bacterium to cause the production of an mRNA molecule.

An increase in protein expression may also be achieved by the integration of at least a second copy of the gene encoding the polypeptide having cytokinin riboside 5'-monophosphate phosphoribohydrolase activity into the genome of the bacterium.

An increase in protein expression may also be achieved by increasing the strength of the promoter operably linked to the gene encoding the polypeptide having cytokinin riboside 5'-monophosphate phosphoribohydrolase activity, e.g. by replacing the native promoter with a promoter that enables higher expression and overproduction of polypeptide compared to the native promoter. The promoters that can be used include natural promoters from *Bacillus subtilis, Bacillus amyloliquefaciens* or similar, such as P43, P15, Pveg, Pylb, PgroES, PsigX, PtrnQ, Ppst, PsodA, PrpsF, PlepA, PliaG, PrpsF, Ppst, PfusA, PsodA, Phag as well as artificial promoters active in *Bacillus subtilis* or inducible *Bacillus subtilis* promoters, such as PmtIA, Pspac, PxylA, PsacB, or similar. Further examples include natural promoters from *Corynebacterium,* such as P CP_2454, Ptuf and Psod, natural promoters from *E coli,* such as T7, ParaBAD, Plac, Ptac and Ptrc, and the promoter P F1 derived from the corynephage BFK20.

An increase in protein expression may also be achieved by modifying the ribosome binding site on the mRNA molecule encoding the polypeptide having cytokinin riboside 5'-monophosphate phosphoribohydrolase activity. By modifying the sequence of the ribosome binding site, the translation initiation rate may be increased, thus increasing translation efficiency.

According to some embodiments, the increase in the number of copies of the gene is achieved by introducing into the bacterium one or more (such as two or three) exogenous nucleic acid molecules (such as one or more vectors) comprising the gene operably linked to a promoter that is functional in the host cell to cause the production of an mRNA molecule.

According to some embodiments, a bacterium of the invention comprises an exogenous nucleic acid molecule (such as a vector) comprising one or more (such as two, three or four) nucleotide sequences encoding the polypeptide having cytokinin riboside 5'-monophosphate phosphoribohydrolase activity. Suitably, the exogenous nucleic acid molecule further comprises a promoter that isfunctional in the bacterium to cause the production of an mRNA molecule and that is operably linked to the nucleotide sequence encoding said polypeptide having cytokinin riboside 5'-monophosphate phosphoribohydrolase activity. According to some embodiments, the exogenous nucleic acid molecule is stably integrated into the genome of the bacterium.

A polypeptide having cytokinin riboside 5'-monophosphate phosphoribohydrolase activity is a cytokinin-activating enzyme with the enzymatic function as cytokinin riboside 5'-monophosphate phosphoribohydrolase. Such polypeptides, also referred to as Lonely Guy (LOG) proteins, are encoded in the genomes of a wide range of organisms, and a majority of LOG proteins are prokaryotic. Enzymesfrom several organisms, such as *Oryza sativa, Arabidopsisthaliana, Claviceps purpurea, Mycobacterium tuberculosis* and *Corynebacterium glutamicum* have been characterized as LOGs by biochemical and functional studies. LOG protein was originally characterized as a phytohormone-activating plant enzyme, while bacterial LOG homologs were mistakenly designated as putative lysine decarboxylases (LDCs) without experimental evidence. Their true enzymatic activity was recently confirmed by functional analysisof the *Corynebacterium glutamicum* homolog (Seo et al. 2016). Two types of LOG proteins were identified in bacteria, dimeric type I LOGs and hexameric type II LOGs. Type II LOG proteins have different oligomeric state and residues at the prenyl-binding site. Type I LOGs can be further divided into 2 subgroups, type Ia and type lb. Type Ia includes dimeric LOGs from most organisms, whereas type Ib includes dimeric LOGs from Actinomycetales. Type II LOG are divided into type IIa, which include hexameric LOGs from most organisms, and type lib with LOGs from higher plants (Seo and Kim 2017).

LOG proteins produce active cytokinins via dephosphoribosylation, hydrolysis of the bond between *N⁶*-substituted bases and ribose 5•-monophosphatesin cytokinin precursors such as iPRM Por *trans-*zeatin riboside 5•-monophosphate (tZRMP). *C*. *glutamicum* LOG is composed of two identical monomers with a central • -sheet, formed by seven parallel • -strands, which issurrounded by eight • -helices. LOG proteins contain "PGGXGTXXE" motif that contributes to the formation of an active site. The active site is formed in the pocket of the dimer, and the conserved "PGGXGTXXE" motif is on the surface of the pocket. The "PGGXGTXXE" motif is a nucleotide-binding site, and the conserved residues stabilize bound AM P (Seo et al. 2016). The motif is highly conserved in all LOG enzymes (Seo and Kim 2017).

The polypeptide having cytokinin riboside 5'-monophosphate phosphoribohydrolase activity may be derived from the same species as the bacterium in which it is expressed or may be derived from a species different to the one in which it is expressed (i.e. it is heterologous). According to some embodiments, the polypeptide having cytokinin riboside 5'-monophosphate phosphoribohydrolase activity is derived from the same species as the bacterium in which it is expressed. According to some embodiments, the polypeptide having cytokinin riboside 5'-monophosphate phosphoribohydrolase activity is derived from a species different from the one in which it is expressed (i.e. it is heterologous).

According to some embodiments, the polypeptide having cytokinin riboside 5'-monophosphate phosphoribohydrolase activity is a bacterial polypeptide having cytokinin riboside 5'-monophosphate phosphoribohydrolase activity. With "bacterial polypeptide having cytokinin riboside 5'-monophosphate phosphoribohydrolase activity" it is meant that the polypeptide having cytokinin riboside 5'-monophosphate phosphoribohydrolase activity is naturally derived from a bacterium, such as *Corynebacterium glutamicum.*

A polypeptide having cytokinin riboside 5'-monophosphate phosphoribohydrolase activity for use according to the invention may for instance be a polypeptide having cytokinin riboside 5'-monophosphate phosphoribohydrolase activity selected from the group consisting of: i) a polypeptide comprising an amino acid sequence of any one of SEQ ID NOs: 34 to 62; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 34 to 62.

A polypeptide having cytokinin riboside 5'-monophosphate phosphoribohydrolase activity for use according to the invention may for instance be a polypeptide having cytokinin riboside 5'-monophosphate phosphoribohydrolase activity selected from the group consisting of: i) a polypeptide comprising an amino acid sequence of any one of SEQ ID NOs: 34 to 44; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 34 to 44.

According to some embodiments, the polypeptide having cytokinin riboside 5'-monophosphate phosphoribohydrolase activity comprises the amino acid sequence of SEQ ID NO: 34 or an amino acid sequence having at least 70%, such as at least 75%, sequence identity with SEQ ID NO: 34. According to some embodiments, the polypeptide having cytokinin riboside 5'-monophosphate phosphoribohydrolase activity comprises the amino acid sequence of SEQ ID NO: 34 or an amino acid sequence having at least 80%, such as at least 85%, sequence identity with SEQ ID NO: 34. According to some embodiments, the polypeptide having cytokinin riboside 5'-monophosphate phosphoribohydrolase activity comprises the amino acid sequence of SEQ ID NO: 34 or an amino acid sequence having at least 90%, such as at least 95%, sequence identity with SEQ ID NO: 34. According to some embodiments, the polypeptide having cytokinin riboside 5'-monophosphate phosphoribohydrolase activity comprises the amino acid sequence of SEQ ID NO: 34.

According to some embodiments, the polypeptide having cytokinin riboside 5'-monophosphate phosphoribohydrolase activity comprises the amino acid sequence of SEQ ID NO: 35 or an amino acid sequence having at least 70%, such as at least 75%, sequence identity with SEQ ID NO: 35. According to some embodiments, the polypeptide having cytokinin riboside 5'-monophosphate phosphoribohydrolase activity comprises the amino acid sequence of SEQ ID NO: 35 or an amino acid sequence having at least 80%, such as at least 85%, sequence identity with SEQ ID NO: 35. According to some embodiments, the polypeptide having cytokinin riboside 5'-monophosphate phosphoribohydrolase activity comprises the amino acid sequence of SEQ ID NO: 35 or an amino acid sequence having at least 90%, such as at least 95%, sequence identity with SEQ ID NO: 35. According to some embodiments, the polypeptide having cytokinin riboside 5'-monophosphate phosphoribohydrolase activity comprisesthe amino acid sequence of SEQ ID NO: 35.

According to some embodiments, the polypeptide having cytokinin riboside 5'-monophosphate phosphoribohydrolase activity comprises the amino acid sequence of SEQ ID NO: 36 or an amino acid sequence having at least 70%, such as at least 75%, sequence identity with SEQ ID NO: 36. According to some embodiments, the polypeptide having cytokinin riboside 5'-monophosphate phosphoribohydrolase activity comprises the amino acid sequence of SEQ ID NO: 36 or an amino acid sequence having at least 80%, such as at least 85%, sequence identity with SEQ ID NO: 36. According to some embodiments, the polypeptide having cytokinin riboside 5'-monophosphate phosphoribohydrolase activity comprises the amino acid sequence of SEQ ID NO: 36 or an amino acid sequence having at least 90%, such as at least 95%, sequence identity with SEQ ID NO: 36. According to some embodiments, the polypeptide having cytokinin riboside 5'-monophosphate phosphoribohydrolase activity comprises the amino acid sequence of SEQ ID NO: 36.

According to some embodiments, the polypeptide having cytokinin riboside 5'-monophosphate phosphoribohydrolase activity comprises the amino acid sequence of SEQ ID NO: 37 or an amino acid sequence having at least 70%, such as at least 75%, sequence identity with SEQ ID NO: 37. According to some embodiments, the polypeptide having cytokinin riboside 5'-monophosphate phosphoribohydrolase activity comprises the amino acid sequence of SEQ ID NO: 37 or an amino acid sequence having at least 80%, such as at least 85%, sequence identity with SEQ ID NO: 37. According to some embodiments, the polypeptide having cytokinin riboside 5'-monophosphate phosphoribohydrolase activity comprises the amino acid sequence of SEQ ID NO: 37 or an amino acid sequence having at least 90%, such as at least 95%, sequence identity with SEQ ID NO: 37.

According to some embodiments, the polypeptide having cytokinin riboside 5'-monophosphate phosphoribohydrolase activity comprises the amino acid sequence of SEQ ID NO: 37.

According to some embodiments, the polypeptide having cytokinin riboside 5'-monophosphate phosphoribohydrolase activity comprises the amino acid sequence of SEQ ID NO: 38 or an amino acid sequence having at least 70%, such as at least 75%, sequence identity with SEQ ID NO: 38. According to some embodiments, the polypeptide having cytokinin riboside 5'-monophosphate phosphoribohydrolase activity comprises the amino acid sequence of SEQ ID NO: 38 or an amino acid sequence having at least 80%, such as at least 85%, sequence identity with SEQ ID NO: 38. According to some embodiments, the polypeptide having cytokinin riboside 5'-monophosphate phosphoribohydrolase activity comprises the amino acid sequence of SEQ ID NO: 38 or an amino acid sequence having at least 90%, such as at least 95%, sequence identity with SEQ ID NO: 38. According to some embodiments, the polypeptide having cytokinin riboside 5'-monophosphate phosphoribohydrolase activity comprises the amino acid sequence of SEQ ID NO: 38.

According to some embodiments, the polypeptide having cytokinin riboside 5'-monophosphate phosphoribohydrolase activity comprises the amino acid sequence of SEQ ID NO: 39 or an amino acid sequence having at least 70%, such as at least 75%, sequence identity with SEQ ID NO: 39. According to some embodiments, the polypeptide having cytokinin riboside 5'-monophosphate phosphoribohydrolase activity comprises the amino acid sequence of SEQ ID NO: 39 or an amino acid sequence having at least 80%, such as at least 85%, sequence identity with SEQ ID NO: 39. According to some embodiments, the polypeptide having cytokinin riboside 5'-monophosphate phosphoribohydrolase activity comprises the amino acid sequence of SEQ ID NO: 39 or an amino acid sequence having at least 90%, such as at least 95%, sequence identity with SEQ ID NO: 39. According to some embodiments, the polypeptide having cytokinin riboside 5'-monophosphate phosphoribohydrolase activity comprises the amino acid sequence of SEQ ID NO: 39.

According to some embodiments, the polypeptide having cytokinin riboside 5'-monophosphate phosphoribohydrolase activity comprises the amino acid sequence of SEQ ID NO: 40 or an amino acid sequence having at least 70%, such as at least 75%, sequence identity with SEQ ID NO: 40. According to some embodiments, the polypeptide having cytokinin riboside 5'-monophosphate phosphoribohydrolase activity comprises the amino acid sequence of SEQ ID NO: 40 or an amino acid sequence having at least 80%, such as at least 85%, sequence identity with SEQ ID NO: 40.

According to some embodiments, the polypeptide having cytokinin riboside 5'-monophosphate phosphoribohydrolase activity comprises the amino acid sequence of SEQ ID NO: 40 or an amino acid sequence having at least 90%, such as at least 95%, sequence identity with SEQ ID NO: 40. According to some embodiments, the polypeptide having cytokinin riboside 5'-monophosphate phosphoribohydrolase activity comprises the amino acid sequence of SEQ ID NO: 40.

According to some embodiments, the polypeptide having cytokinin riboside 5'-monophosphate phosphoribohydrolase activity comprises the amino acid sequence of SEQ ID NO: 41 or an amino acid sequence having at least 70%, such as at least 75%, sequence identity with SEQ ID NO: 41. According to some embodiments, the polypeptide having cytokinin riboside 5'-monophosphate phosphoribohydrolase activity comprises the amino acid sequence of SEQ ID NO: 41 or an amino acid sequence having at least 80%, such as at least 85%, sequence identity with SEQ ID NO: 41. According to some embodiments, the polypeptide having cytokinin riboside 5'-monophosphate phosphoribohydrolase activity comprises the amino acid sequence of SEQ ID NO: 41 or an amino acid sequence having at least 90%, such as at least 95%, sequence identity with SEQ ID NO: 41. According to some embodiments, the polypeptide having cytokinin riboside 5'-monophosphate phosphoribohydrolase activity comprises the amino acid sequence of SEQ ID NO: 41.

According to some embodiments, the polypeptide having cytokinin riboside 5'-monophosphate phosphoribohydrolase activity comprises the amino acid sequence of SEQ ID NO: 42 or an amino acid sequence having at least 70%, such as at least 75%, sequence identity with SEQ ID NO: 42. According to some embodiments, the polypeptide having cytokinin riboside 5'-monophosphate phosphoribohydrolase activity comprises the amino acid sequence of SEQ ID NO: 42 or an amino acid sequence having at least 80%, such as at least 85%, sequence identity with SEQ ID NO: 42. According to some embodiments, the polypeptide having cytokinin riboside 5'-monophosphate phosphoribohydrolase activity comprises the amino acid sequence of SEQ ID NO: 42 or an amino acid sequence having at least 90%, such as at least 95%, sequence identity with SEQ ID NO: 42. According to some embodiments, the polypeptide having cytokinin riboside 5'-monophosphate phosphoribohydrolase activity comprises the amino acid sequence of SEQ ID NO: 42.

According to some embodiments, the polypeptide having cytokinin riboside 5'-monophosphate phosphoribohydrolase activity comprises the amino acid sequence of SEQ ID NO: 43 or an amino acid sequence having at least 70%, such as at least 75%, sequence identity with SEQ ID NO: 43.

According to some embodiments, the polypeptide having cytokinin riboside 5'-monophosphate phosphoribohydrolase activity comprises the amino acid sequence of SEQ ID NO: 43 or an amino acid sequence having at least 80%, such as at least 85%, sequence identity with SEQ ID NO: 43. According to some embodiments, the polypeptide having cytokinin riboside 5'-monophosphate phosphoribohydrolase activity comprises the amino acid sequence of SEQ ID NO: 43 or an amino acid sequence having at least 90%, such as at least 95%, sequence identity with SEQ ID NO: 43. According to some embodiments, the polypeptide having cytokinin riboside 5'-monophosphate phosphoribohydrolase activity comprises the amino acid sequence of SEQ ID NO: 43.

According to some embodiments, the polypeptide having cytokinin riboside 5'-monophosphate phosphoribohydrolase activity comprises the amino acid sequence of SEQ ID NO: 44 or an amino acid sequence having at least 70%, such as at least 75%, sequence identity with SEQ ID NO: 44. According to some embodiments, the polypeptide having cytokinin riboside 5'-monophosphate phosphoribohydrolase activity comprises the amino acid sequence of SEQ ID NO: 44 or an amino acid sequence having at least 80%, such as at least 85%, sequence identity with SEQ ID NO: 44. According to some embodiments, the polypeptide having cytokinin riboside 5'-monophosphate phosphoribohydrolase activity comprises the amino acid sequence of SEQ ID NO: 44 or an amino acid sequence having at least 90%, such as at least 95%, sequence identity with SEQ ID NO: 44. According to some embodiments, the polypeptide having cytokinin riboside 5'-monophosphate phosphoribohydrolase activity comprises the amino acid sequence of SEQ ID NO: 44.

Techniques for determining cytokinin riboside 5'-monophosphate phosphoribohydrolase activity are well known to the skilled person. Exemplary methods have been described, e.g. by Seo et al. (2016). The cytokinin riboside 5'-monophosphate phosphoribohydrolase activity may for instance be determined in accordance with the following method:
The phosphoribohydrolase activity is determined by detecting adenine ring compounds separated by thin-layer chromatography (TLC) method. The enzyme reaction is carried out in a mixture of 20 mM AMP, 36 mM Tris-HCl, pH 8.0, and 23 • M purified enzyme at 30 °C, and then the reaction is stopped by heating the mixture at 95 °Cfor 1.5 min. The reaction mixture is then dotted on a PEI-cellulose-F plastic TLC sheet (Merck Millipore). The mobile phase is 1 M sodium chloride. After development in the TLC chamber, the sheet isdried completely. Adenine ring-including compounds are detected by UV lamp (290 nm) (Seo et al. 2016).

In order to increase the supply of the isopentenyl side chain precursor of isoprenoid cytokinins, metabolic flux through the methylerythritol 4-phosphate (M EP) pathway may be increased. This is achieved primarily through overexpression of 1-Deoxy-D-xylulose 5-phosphate (DXP) synthase (DXS).

Hence, according to some embodiments, the bacterium of the present invention is characterized in that it has been further modified to have an increased protein expression of a polypeptide having 1-deoxy-D-xylulose-5-phosphate synthase activity compared to an otherwise identical bacterium that does not carry said modification.

By "increased protein expression" it is meant that the amount of the polypeptide having 1-deoxy-D-xylulose-5-phosphate synthase activity produced by the thus modified bacterium is increased compared to an otherwise identical bacterium that does not carry said modification. More particularly, by "increased expression" it is meant that the amount of the polypeptide having 1-deoxy-D-xylulose-5-phosphate synthase activity produced by the thus modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared an otherwise identical bacterium that does not carry said modification. The amount of protein in a given cell can be determined by any suitable quantification technique known in the art, such as ELISA, Immunohistochemistry, or Western Blotting.

An increase in protein expression may be achieved by any suitable means well-known to those skilled in the art. For example, an increase in protein expression may be achieved by increasing the number of copies of the gene or genes encoding the polypeptide having 1-deoxy-D-xylulose-5-phosphate synthase activity in the bacterium, such as by introducing into the bacterium an exogenous nucleic acid, such as a vector, comprising the gene or genes encoding the polypeptide having 1-deoxy-D-xylulose-5-phosphate synthase activity operably linked to a promoter that is functional in the bacterium to cause the production of an mRNA molecule.

An increase in protein expression may also be achieved by the integration of at least a second copy of the gene encoding the polypeptide having 1-deoxy-D-xylulose-5-phosphate synthase activity into the genome of the bacterium.

An increase in protein expression may also be achieved by increasing the strength of the promoter operably linked to the gene encoding the polypeptide having 1-deoxy-D-xylulose-5-phosphate synthase activity, e.g. by replacing the native promoter with a promoter that enables higher expression and overproduction of polypeptide compared to the native promoter. The promoters that can be used include natural promoters from *Bacillus subtilis, Bacillus amyloliquefaciens* or similar, such as P43, P15, Pveg, Pylb, PgroES, PsigX, PtrnQ, Ppst, PsodA, PrpsF, PlepA, PliaG, PrpsF, Ppst, PfusA, PsodA, Phag as well as artificial promoters active in *Bacillus subtilis* or inducible *Bacillus subtilis* promoters, such as PmtIA, Pspac, PxylA, PsacB, or similar. Further examples include natural promoters from *Corynebacterium,* such as P CP_2454, Ptuf and Psod, natural promoters from *E. coli,* such as T7, ParaBAD, Plac, Ptac and Ptrc, and the promoter PF1 derived from the corynephage BFK20.

An increase in protein expression may also be achieved by modifying the ribosome binding site on the mRNA molecule encoding the polypeptide having 1-deoxy-D-xylulose-5-phosphate synthase activity. By modifying the sequence of the ribosome binding site, the translation initiation rate may be increased, thus increasing translation efficiency.

According to some embodiments, the increase in the number of copies of the gene is achieved by introducing into the bacterium one or more (such as two or three) exogenous nucleic acid molecules (such as one or more vectors) comprising the gene operably linked to a promoter that is functional in the bacterium to cause the production of an mRNA molecule.

According to some embodiments, a bacterium of the invention comprises an exogenous nucleic acid molecule (such as a vector) comprising one or more (such as two, three, or four) nucleotide sequences encoding the polypeptide having 1-deoxy-D-xylulose-5-phosphate synthase activity. Suitably, the exogenous nucleic acid molecule further comprises a promoter that is functional in the bacterium to cause the production of an mRNA molecule and that is operably linked to the nucleotide sequence encoding said polypeptide having 1-deoxy-D-xylulose-5-phosphate synthase activity. According to some embodiments, the exogenous nucleic acid molecule isstably integrated into the genome of the bacterium.

Apolypeptide having 1-deoxy-D-xylulose-5-phosphate synthase activity is an enzyme that catalyzes the condensation between D-glyceraldehyde 3-phosphate and pyruvate to produce 1-deoxy-D-xylulose 5-phosphate (DXP) (DXS; E.C. 2.2.1.7). DXS catalyzes the first enzymatic step of the isoprenoid biosynthesis of HMBDP and DMAPP in the methylerythritol phosphate (MEP) pathway, whose kcat/Km value is substantially lower than for other enzymes in this pathway (Kuzuyama et al. 2000). DXSis present in a single copy in eubacteria, whereas green algae and higher plants have two or more genes encoding DXS, which form three distinguishable groups. In higher plants, the expression of the distinct DXS isoenzymes depends on the type of tissue and developmental stage.

DXS is highly conserved in bacteria and plants. Its protein sequence has about 20% identity with transketolase and pyruvate dehydrogenase E1 subunit. All three enzymes catalyze similar reactions and require coenzyme thiamine pyrophosphate (TPP). DXSfrom *E coli* contains three domains (I, II, and III), which are homologous to the equivalent domains in transketolase and the E1 subunit of pyruvate dehydrogenase. Two DXS monomers are arranged side-by-side in a dimer. Domain I (residues 1-319) is above domains II (residues 320-495) and III (residues 496-629) of the same monomer. All three domains have the • / • fold with a central, mostly parallel • -sheet between • helices. Domain I contains a five-stranded parallel • -sheet, domain II contains a six-stranded parallel • -sheet, and domain III contains a five-stranded • -sheet with the first strand anti-parallel to the other four strands. Domain I has several extended surface segments, at the N-terminus (residues 1-49), after the first strand (residues 81-122), and in the connection between the fourth and fifth strands (residues 184-250).

The active site of DXS is located at the interface of domains I and II in the same monomer. The C-terminal ends of the central parallel • -sheets of the two domains are pointed towards each other, and the TPP coenzyme is located at the bottom of a pocket in this interface. The amino-pyrimidine ring of TPP interacts with domain II, while the pyrophosphate group interacts with domain I. The C2 atom of the thiazolium ring is exposed to the substrate-binding site. The C2 atom participates in the reaction. The pyrophosphate group of TPP has numerous polar interactions with the enzyme. The active site is composed of a magnesium ion bound between the two phosphate groups, and the side chainsof Asp154, Asn183 and Met185. The Gly153-Asp-Gly155-Asn183 sequence in DXSis consistent with the TPP binding motif »GDG-X(25-30)-N«. The C2 constitutes pyruvate-binding site. GAP is located in the pocket (Xiang et al., 2007).

Increasing DXS activity is recognized as the most effective strategy for increased terpenoid biosynthesis in many species, also in *B. subtilis* (Yang et al. 2019), as several studies indicate that the formation of DXP is the limiting step of the M EP pathway. A single amino acid mutation in Dxs of *E coli and Deinococcus radiodurans* increased their catalytic activities. The mutation Y392Fof *E. coli* Dxs increased the relative catalytic activity by more than 2.5-fold compared to the wild type (Xiang et al. 2012). DXS is regulated by a negative feedback mechanism by the end products of the MEP pathway, IPP, and DMAPP (Banerjee et al. 2013). DXS has been a subject of site-directed mutagenesis for alleviating the negative feedback inhibition of IPP and DMAPP. Mutation at A147G/A352G of *Populus trichocarpa* DXS reduced its IPP binding affinity slightly, but it increased Km for TPP and pyruvate, and it decreased the catalytic efficiency of the enzyme about 15-fold (Banerjee et al., 2016). Overexpression of several other enzymes of the pathway besides DXS has also been tested with mixed results in different bacteria. Also, the overexpression of the entire MEP pathway as artificial operons (operon dxs-ispD-ispF-ispH and ispC/dxr-ispE-ispG-ispA) has been tested in *B. subtilis* (Xue et al. 2015).

The polypeptide having 1-deoxy-D-xylulose-5-phosphate synthase activity may be derived from the same species as the bacterium in which it is expressed or may be derived from a species different from the one in which it is expressed (i.e. it is heterologous). According to some embodiments, the polypeptide having 1-deoxy-D-xylulose-5-phosphate synthase activity is derived from the same species as the bacterium in which it is expressed. According to some embodiments, the polypeptide having 1-deoxy-D-xylulose-5-phosphate synthase activity is derived from a species different from the one in which it is expressed (i.e. it is heterologous).

Preferably, the polypeptide having 1-deoxy-D-xylulose-5-phosphate synthase activity is a bacterial polypeptide having 1-deoxy-D-xylulose-5-phosphate synthase activity. With "bacterial polypeptide having 1-deoxy-D-xylulose-5-phosphate synthase activity" it is meant that the polypeptide having 1-deoxy-D-xylulose-5-phosphate synthase activity is naturally derived from a bacterium, such as *Bacillus subtilis.*

A polypeptide having 1-deoxy-D-xylulose-5-phosphate synthase activity for use according to the invention may for instance be a polypeptide having 1-deoxy-D-xylulose-5-phosphate synthase activity selected from the group consisting of: i) a polypeptide comprising an amino acid sequence of any one of SEQ ID NOs: 63 to 70; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 63 to 70.

According to some embodiments, the polypeptide having 1-deoxy-D-xylulose-5-phosphate synthase activity comprises the amino acid sequence of SEQ ID NO: 63 or an amino acid sequence having at least 70%, such as at least 75%, sequence identity with SEQ ID NO: 63. According to some embodiments, the polypeptide having 1-deoxy-D-xylulose-5-phosphate synthase activity comprises the amino acid sequence of SEQ ID NO: 63 or an amino acid sequence having at least 80%, such as at least 85%, sequence identity with SEQ ID NO: 63. According to some embodiments, the polypeptide 1-deoxy-D-xylulose-5-phosphate synthase activity comprises the amino acid sequence of SEQ ID NO: 63 or an amino acid sequence having at least 90%, such as at least 95%, sequence identity with SEQ ID NO: 63. According to some embodiments, the polypeptide having 1-deoxy-D-xylulose-5-phosphate synthase activity comprises the amino acid sequence of SEQ ID NO: 63.

According to some embodiments, the polypeptide having 1-deoxy-D-xylulose-5-phosphate synthase activity comprises the amino acid sequence of SEQ ID NO: 64 or an amino acid sequence having at least 70%, such as at least 75%, sequence identity with SEQ ID NO: 64. According to some embodiments, the polypeptide having 1-deoxy-D-xylulose-5-phosphate synthase activity comprises the amino acid sequence of SEQ ID NO: 64 or an amino acid sequence having at least 80%, such as at least 85%, sequence identity with SEQ ID NO: 64. According to some embodiments, the polypeptide 1-deoxy-D-xylulose-5-phosphate synthase activity comprises the amino acid sequence of SEQ ID NO: 64 or an amino acid sequence having at least 90%, such as at least 95%, sequence identity with SEQ ID NO: 64. According to some embodiments, the polypeptide having 1-deoxy-D-xylulose-5-phosphate synthase activity comprises the amino acid sequence of SEQ ID NO: 64.

According to some embodiments, the polypeptide having 1-deoxy-D-xylulose-5-phosphate synthase activity comprises the amino acid sequence of SEQ ID NO: 65 or an amino acid sequence having at least 70%, such as at least 75%, sequence identity with SEQ ID NO: 65. According to some embodiments, the polypeptide having 1-deoxy-D-xylulose-5-phosphate synthase activity comprises the amino acid sequence of SEQ ID NO: 65 or an amino acid sequence having at least 80%, such as at least 85%, sequence identity with SEQ ID NO: 65. According to some embodiments, the polypeptide 1-deoxy-D-xylulose-5-phosphate synthase activity comprises the amino acid sequence of SEQ ID NO: 65 or an amino acid sequence having at least 90%, such as at least 95%, sequence identity with SEQ ID NO: 65. According to some embodiments, the polypeptide having 1-deoxy-D-xylulose-5-phosphate synthase activity comprises the amino acid sequence of SEQ ID NO: 65.

According to some embodiments, the polypeptide having 1-deoxy-D-xylulose-5-phosphate synthase activity comprises the amino acid sequence of SEQ ID NO: 66 or an amino acid sequence having at least 70%, such as at least 75%, sequence identity with SEQ ID NO: 66. According to some embodiments, the polypeptide having 1-deoxy-D-xylulose-5-phosphate synthase activity comprises the amino acid sequence of SEQ ID NO: 66 or an amino acid sequence having at least 80%, such as at least 85%, sequence identity with SEQ ID NO: 66. According to some embodiments, the polypeptide 1-deoxy-D-xylulose-5-phosphate synthase activity comprises the amino acid sequence of SEQ ID NO: 66 or an amino acid sequence having at least 90%, such as at least 95%, sequence identity with SEQ ID NO: 66. According to some embodiments, the polypeptide having 1-deoxy-D-xylulose-5-phosphate synthase activity comprises the amino acid sequence of SEQ ID NO: 66.

According to some embodiments, the polypeptide having 1-deoxy-D-xylulose-5-phosphate synthase activity comprises the amino acid sequence of SEQ ID NO: 67 or an amino acid sequence having at least 70%, such as at least 75%, sequence identity with SEQ ID NO: 67. According to some embodiments, the polypeptide having 1-deoxy-D-xylulose-5-phosphate synthase activity comprises the amino acid sequence of SEQ ID NO: 67 or an amino acid sequence having at least 80%, such as at least 85%, sequence identity with SEQ ID NO: 67. According to some embodiments, the polypeptide 1-deoxy-D-xylulose-5-phosphate synthase activity comprises the amino acid sequence of SEQ ID NO: 67 or an amino acid sequence having at least 90%, such as at least 95%, sequence identity with SEQ ID NO: 67. According to some embodiments, the polypeptide having 1-deoxy-D-xylulose-5-phosphate synthase activity comprises the amino acid sequence of SEQ ID NO: 67.

According to some embodiments, the polypeptide having 1-deoxy-D-xylulose-5-phosphate synthase activity comprises the amino acid sequence of SEQ ID NO: 68 or an amino acid sequence having at least 70%, such as at least 75%, sequence identity with SEQ ID NO: 68. According to some embodiments, the polypeptide having 1-deoxy-D-xylulose-5-phosphate synthase activity comprises the amino acid sequence of SEQ ID NO: 68 or an amino acid sequence having at least 80%, such as at least 85%, sequence identity with SEQ ID NO: 68. According to some embodiments, the polypeptide 1-deoxy-D-xylulose-5-phosphate synthase activity comprises the amino acid sequence of SEQ ID NO: 68 or an amino acid sequence having at least 90%, such as at least 95%, sequence identity with SEQ ID NO: 68. According to some embodiments, the polypeptide having 1-deoxy-D-xylulose-5-phosphate synthase activity comprises the amino acid sequence of SEQ ID NO: 68.

As noted above, modification of the wild type DXS sequence can increase the catalytic activities. The present invention thus particularly contemplates the use of mutant DXS having increased activity or an inactivated negative feedback mechanism compared to the wild type DXS enzyme from which it is derived. Non-limiting examples of such mutant DXSenzymes are those set out in SEQ ID NOs. 69 and 70.

According to some embodiments, the polypeptide having 1-deoxy-D-xylulose-5-phosphate synthase activity comprises the amino acid sequence of SEQ ID NO: 69 or an amino acid sequence having at least 70%, such as at least 75%, sequence identity with SEQ ID NO: 69, with the proviso that the amino acid at position 392 is not Y, preferably with the proviso that the amino acid at position 392 is F. According to some embodiments, the polypeptide having 1-deoxy-D-xylulose-5-phosphate synthase activity comprisesthe amino acid sequence of SEQ ID NO: 69 or an amino acid sequence having at least 80%, such as at least 85%, sequence identity with SEQ ID NO: 69, with the proviso that the amino acid at position 392 is not Y, preferably with the proviso that the amino acid at position 392 is F. According to some embodiments, the polypeptide 1-deoxy-D-xylulose-5-phosphate synthase activity comprises the amino acid sequence of SEQ ID NO: 69 or an amino acid sequence having at least 90%, such as at least 95%, sequence identity with SEQ ID NO: 69, , with the proviso that the amino acid at position 392 is not Y, preferably with the proviso that the amino acid at position 392 is F. According to some embodiments, the polypeptide having 1-deoxy-D-xylulose-5-phosphate synthase activity comprises the amino acid sequence of SEQ ID NO: 69.

According to some embodiments, the polypeptide having 1-deoxy-D-xylulose-5-phosphate synthase activity comprises the amino acid sequence of SEQ ID NO: 70 or an amino acid sequence having at least 70%, such as at least 75%, sequence identity with SEQ ID NO: 70, with the proviso that the amino acid at position 389 is not Y, preferably with the proviso that the amino acid at position 389 is F. According to some embodiments, the polypeptide having 1-deoxy-D-xylulose-5-phosphate synthase activity comprisesthe amino acid sequence of SEQ ID NO: 70 or an amino acid sequence having at least 80%, such as at least 85%, sequence identity with SEQ ID NO: 70, with the proviso that the amino acid at position 389 is not Y, preferably with the proviso that the amino acid at position 389 is F. According to some embodiments, the polypeptide 1-deoxy-D-xylulose-5-phosphate synthase activity comprises the amino acid sequence of SEQ ID NO: 70 or an amino acid sequence having at least 90%, such as at least 95%, sequence identity with SEQ ID NO: 70, with the proviso that the amino acid at position 389 is not Y, preferably with the proviso that the amino acid at position 389 is F. According to some embodiments, the polypeptide having 1-deoxy-D-xylulose-5-phosphate synthase activity comprises the amino acid sequence of SEQ ID NO: 70.

Techniques for determining 1-deoxy-D-xylulose-5-phosphate synthase activity are well known to the skilled person. Exemplary methods have been described, e.g. by Kuzuyama et al (2000) and by Kudoh et al (2017). The 1-deoxy-D-xylulose-5-phosphate synthase activity may for instance be determined in accordance with the following methods:
(1) The 1-deoxy-D-xylulose-5-phosphate synthase activity is determined by DXS assay (Kuzuyama et al., 2000): The standard assay system consists of 100 mM Tris-HCl (pH 8.0) containing 1 mM MgCl₂, 2 mM dl-dithiothreitol, 1 mM sodium pyruvate, 2 mM dl-glyceraldehyde 3-phosphate, and 150 • M thiamine diphosphate in a final volume of 0.5 ml. The reaction is initiated by adding the enzyme solution to the complete assay mixture at 37°C, and after a 10 min-incubation, the reaction is halted by incubation at 100°C for 1 min. Next, the reaction mixture is treated with alkaline phosphatase at 56°C for 60 min to dephosphorylate completely the reaction product, DXP. Production of the resulting dephosphorylated compound, 1-deoxyxylulose (DX), is monitored by a refractive index spectrometer (model RI-71; Showa Denko, Tokyo, Japan) with a Shodex KS-801 (8-by 300-mm) column (Showa Denko), eluted with H₂O at a flow rate of 1 ml/min at 80°C. DX is eluted at 8.6 min under this condition. The amount of DX production is precisely estimated by using chemically synthesized DX as the standard. One unit of DXS activity isdefined as the amount of the enzyme that caused the production of 1 • mol of DXP per min at 37°C. Production of DXP by the DXS is monitored at 195 nm by high-performance liquid chromatography with a Senshu Pak NH2-1251-N (4.6- by 250-mm) column (Senshu Science, Tokyo, Japan) eluted with 100 mM KH₂PO₄ (pH 3.5) at a flow rate of 1 ml/min. DXP is eluted at 8.1 min under this condition.
(2) A coupled enzyme assay of DXS (Kudoh et al., 2017): DXS activity is measured using a coupled enzyme assay with DXR from *E coli* as the coupling enzyme. In this assay, DXP generated by the DXS activity is further converted to M EP. As this step consumes NADPH, the overall reaction can be measured spectrophotometrically at 340 nm. Assay mixtures contains 100 mM Tris/HCl (pH 7.8), 10 mM MgCl₂, 0.3 mM thiamine pyrophosphate (TPP), 1 mM dithiothreitol (DTT), 0.3 mM nicotinamide adenine dinucleotide phosphate (NADPH), various concentrations of sodium pyruvate (0.05 - 5 mM) and D,L-GAP (0.2 - 2.0 mM), and DXR (100 or 50 mg/ ml). The mixtures are incubated at 30°Cfor 2 min inside a temperature-controlled spectrophotometer (model UV-1800, Shimadzu, Kyoto, Japan) and added to the DXSsample (final concentration of 50 or 25 mg/ml) to start the reaction. The reaction is traced by monitoring the absorption at 340 nm at 30°C.

Purine nucleotides are structural components of DNA and RNA, energy carriers (i.e. ATP and GTP), enzyme cofactors (i.e. NAD⁺ and NADP⁺), and as such are essential metabolites for cellular physiology. The synthesis of purine nucleotides starts with the synthesis of 5• -phosphoribosyl-pyrophosphate (PRPP) from D-ribose 5-phosphate and ATP. The enzyme PRPP synthase (Ribose-phosphate diphosphokinase; EC2.7.6.1) catalyzes the transfer of the diphosphoryl group of ATP to the D-ribose 5-phosphate, with the simultaneous formation of AM P. PRPP synthase is ubiquitous among free-living organisms. Most bacteria have one gene encoding PRPP synthase, whereas more than one gene is present in eukaryotes. The next step is the synthesis of 5-phospho-• -D-ribosylamine from PRPP and glutamine catalyzed by glutamine 5-phosphoribosyl-1-pyrophosphate (PRPP) amidotransferase (amidophosphoribosyltransferase; EC2.4.2.14), which is encoded by *purF* in *B. subtilis.* This is the rate-limiting reaction of purine *de novo* synthesis. The further 10 steps leading from 5-phospho-•-D-ribosylamine to the synthesis of inosine-5-phosphate (IMP) are catalyzed by the enzymes encoded in the *pur* operon. The *pur* operon is negatively regulated by PurR repressor at transcriptional level encoded by *purR,* and by PurBoxes, specific DNA sequences in the upstream control regions of affected genes. IMP is the branching point for the synthesis of AM P and GM P. AM P is synthesized from IMP in two enzymatic steps, catalyzed by PurA and PurB, whereas GM P is synthesized from IMP by GuaB and GuaA. The expression of genes or operons encoding enzymes of the purine synthesis is regulated by purine bases and nucleosides in the growth medium. The enzymes PRPP synthetase, PRPP amidotransferase, adenylosuccinate synthetase and IMP dehydrogenase are regulated by the feedback inhibition of the end products of the pathway. PurR repressor inhibits the initiation of transcription. The salvage pathways also participate to generate the corresponding mononucleotides AMP and GMP by utilization of hypoxanthine, guanine, and adenine.

The purine nucleotide biosynthesis pathway is well-studied because of the role of purine nucleotides in the primary metabolism. It includes both the *de novo* synthesis pathway and the salvage pathway. Deregulation of the purine nucleotide biosynthesis pathway at transcription and metabolic levels enhanced the metabolic flow through the purine nucleotide biosynthesis pathway and consequently increased the yield of products directly stemming from the purine nucleotide biosynthesis pathway: inosine, guanosine, adenosine, and riboflavin. Various modifications including gene overexpression, gene deletions, and enzyme deregulation by mutations had been used successfully for increasing the yield of purine nucleotide biosynthesis pathway products. Exemplary modifications in *B. subtilis* enzymes with a positive effect on the increase in purine flux are listed in Table 1 below.

**Table 1: Modifications in B. subtilis enzymes with a positive effect on the increase in purine flux.**

| **Enzyme** | **Modifications and mutations** | **Effect of mutation** | **Reference** |
|---|---|---|---|
| PRPP synthetase (*prs*) | N120Sand L135I, overexpressio n | Deregulated enzyme: release of negative feedback inhibition from ADP and GDP, significant increase of sensitivity to inorganic phosphate (Pi) activation, increased purine production | (Zakataeva et al., 2012) |
| PRPP amidotransferase) (*purF*) | S283A, K305Q, R307Q and S347A, overexpressio n | Deregulated enzyme: release of the negative feedback inhibition, increased purine production | (Chen et al., 1997) |
| PRPP amidotransferase) (*purF*) | D293V, K316Q, S400W, overexpressio n | Deregulated enzyme: release of the negative feedback inhibition, increased purine production | (Shi et al., 2009) |
| 5'-UTR of *pur* operon | Disruption of guanine riboswitch | Upregulation of pur operon | (Shi et al., 2014) |
| Promoter Ppur | Deletion of attenuator region, changes in -10 sequence | Increased expression of pur operon genes (genes of purine biosynthesis pathway except *prs* and *purF)* | (Asahara et al., 2010) |
| PurR | Gene inactivation (deletion) | Deletion of the repressor of *pur* operon - increased expression of pur operon genes (genes of purine biosynthesis pathway except prs and purF) | (Shi et al., 2009) |
| Adenylosuccinate synthetase (*purA*) | P242N | Significantly reduced enzyme activity (decreased flux to AM P, increased flux to GM P) | (Wang et al., 2016) |
| Adenylosuccinate synthetase (*purA*) | Overexpression | Increased flux to AM P | (Li et al. 2019) |
| IM P dehydrogenase (*guaB*) | A226V, resistance to mycophenolic acid (M PA) | Significantly reduced enzyme activity (decreased flux to GM P) | (Asahara et al., 2010) |
| IM P dehydrogenase (*guaB*) | deletion | Auxotrophy for guanosine, increased production of AM P/adenosine | Patent US3730836A |
| GMP synthetase (*guaA*) | deletion | Auxotrophy for guanosine, increased production of AM P/adenosine | Patent US3730836A |
| YvrH, two-component response regulator | R222Q | Deregulation of purine biosynthesis, increase in purine metabolites | (Wang et al., 2011) |
| Purine nucleoside phosphorylase (*deoD*) | Deletion, inactivation | Deletion of degradation of adenosine to adenine | (Asahara et al., 2010) |
| Purine nucleoside phosphorylase *(pupG)* | Deletion, inactivation | Prevention of adenosine and inosine degradation, increased accumulation of inosine | (Wang et al., 2016) |
| hypoxanthine-guanine-phosphoribosyltransferase (HGPRTase; gene *hpt*) | deletion | Inactivation of purine-depleting reactions | (Peifer et al., 2012) |
| adenosine-phosphoribosyltransferase (APRTase; gene *apt*) | Resistance to 2-fluoroadenine | Decreased degradation of IMP, AM P synthesis via the salvage pathway is enhanced | (Ch rist iansen, Schou and Nygaard, 1997) |
| hypoxanthine-guanine-phosphoribosyltransferase (HGPRTase; gene *hpt)* | Resistance to 8-azaguanine | Increased xanthosine and guanosine production (shift from inosine production) | (Konishi and Shiro, 1968) |
| hypoxanthine-guanine-phosphoribosyltransferase (HGPRTase; gene *hpt*) | Resistance to 8-thioguanine | Changed purine flow | (Ch rist iansen, Schou and Nygaard, 1997) |
| xanthine phosphoribosyltransferase (XPRTase; gene *xpt*) | Resistance to 8-azaxanthine | Changed purine flow | (Ch rist iansen, Schou and Nygaard, 1997) |

Hence, according to some embodiments, the bacterium of the present invention is characterized in that it has been further modified to have an increased expression and/or activity of at least one enzyme involved in the purine nucleotide biosynthesis pathway compared to an otherwise identical bacterium that does not carry said modification.

According to some embodiments, the bacterium of the present invention is characterized in that it has been further modified to have an increased expression and/or activity of at least one enzyme involved in the adenosine monophosphate biosynthesis pathway compared to an otherwise identical bacterium that does not carry said modification.

The at least one enzyme involved in the purine nucleotide biosynthesis pathway (e.g., the at least enzyme involved in the adenosine monophosphate biosynthesis pathway) may be derived from the same species as the bacterium in which it is expressed or may be derived from a species different to the one in which it is expressed (i.e. it is heterologous). According to some embodiments, at least one enzyme involved in the purine nucleotide biosynthesis pathway (e.g., the at least enzyme involved in the adenosine monophosphate biosynthesis pathway) is derived from the same species as the bacterium in which it is expressed. According to some embodiments, the at least one enzyme involved in the purine nucleotide biosynthesis pathway (e.g., the at least enzyme involved in the adenosine monophosphate biosynthesis pathway) is derived from a species different from the one in which it is expressed (i.e. it is heterologous).

By "increased protein expression" it is meant that the amount of the enzyme involved in the purine nucleotide biosynthesis pathway (e.g., the enzyme involved in the adenosine monophosphate biosynthesis pathway) produced by the thus modified bacterium is increased compared to an otherwise identical bacterium that does not carry said modification. More particularly, by "increased expression" it is meant that the amount of the enzyme involved in the purine nucleotide biosynthesis pathway (e.g., the enzyme involved in the adenosine monophosphate biosynthesis pathway) produced by the thus modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700%at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical bacterium that does not carry said modification. The amount of protein in a given cell can be determined by any suitable quantification technique known in the art, such as ELISA, Immunohistochemistry, or Western Blotting.

An increase in protein expression may be achieved by any suitable means well-known to those skilled in the art. For example, an increase in protein expression may be achieved by increasing the number of copies of the gene encoding the enzyme involved in the purine nucleotide biosynthesis pathway (e.g., the enzyme involved in the adenosine monophosphate biosynthesis pathway) in the bacterium, such as by introducing into the bacterium an exogenous nucleic acid, such as a vector, comprising the gene encoding the enzyme involved in the purine nucleotide biosynthesis pathway (e.g., the enzyme involved in the adenosine biosynthesis pathway) operably linked to a promoter that isfunctional in the bacterium to cause the production of an mRNA molecule.

An increase in protein expression may also be achieved by the integration of at least a second copy of the gene encoding the enzyme involved in the purine nucleotide biosynthesis pathway (e.g., the enzyme involved in the adenosine monophosphate biosynthesis pathway) into the genome of the bacterium.

An increase in protein expression may also be achieved by increasing the strength of the promoter operably linked to the gene encoding the enzyme involved in the purine nucleotide biosynthesis pathway (e.g., the enzyme involved in the adenosine monophosphate biosynthesis pathway), e.g. by replacing the native promoter with a promoter that enables higher expression and overproduction of the enzyme compared to the native promoter. The promoters that can be used include natural promoters from *Bacillus subtilis, Bacillus amyloliquefaciensor* similar, such as P43, P15, Pveg, Pylb, PgroES, PsigX, PtrnQ,, Ppst, PsodA, PrpsF, PlepA, PliaG, PrpsF, Ppst, PfusA, PsodA, Phag aswell as artificial promoters active in *Bacillus subtilis* or inducible *Bacillus subtilis* promoters, such as PmtIA, Pspac, PxylA, PsacB, or similar. Further examples include natural promoters from *Corynebacterium,* such as P CP_2454, Ptuf and Psod, natural promoters from *E coli,* such as T7, ParaBAD, Plac, Ptac and Ptrc, and the promoter P F1 derived from the corynephage BFK20.

An increase in protein expression may also be achieved by modifying the ribosome binding site on the mRNA molecule encoding the enzyme involved in the purine nucleotide biosynthesis pathway. By modifying the sequence of the ribosome binding site, the translation initiation rate may be increased, thus increasing translation efficiency.

According to some embodiments, the increase in the number of copies of the gene is achieved by introducing into the bacterium one or more (such as two or three) exogenous nucleic acid molecules (such as one or more vectors) comprising the gene operably linked to a promoter that is functional in the host cell to cause the production of an mRNA molecule.

According to some embodiments, a bacterium of the invention comprises an exogenous nucleic acid molecule (such as a vector) comprising one or more (such as two, three, or four) nucleotide sequences encoding the enzyme involved in the purine nucleotide biosynthesis pathway (e.g., the enzyme involved in the adenosine monophosphate biosynthesis pathway). Suitably, the exogenous nucleic acid molecule further comprises a promoter that isfunctional in the bacterium to cause the production of an mRNA molecule and that is operably linked to the nucleotide sequence encoding said the enzyme involved in the purine nucleotide biosynthesis pathway (e.g., said enzyme involved in the adenosine biosynthesis monophosphate pathway). According to some embodiments, the exogenous nucleic acid molecule is stably integrated into the genome of the bacterium.

The at least one enzyme involved in the purine nucleotide biosynthesis pathway may be an enzyme selected from the group consisting of: an enzyme having ribose-phosphate diphosphokinase activity, an enzyme having amidophosphoribosyltransferase activity, an enzyme having formyltetrahydrofolate deformylase activity, an enzyme having phosphoribosylamine-glycine ligase activity, an enzyme having phosphoribosylglycineamide formyltransferase activity, an enzyme having phosphoribosylformylglycinamidine synthase activity, an enzyme having phosphoribosylformylglycineamidine cyclo-ligase activity, an enzyme having N5-carboxyaminoimidazole ribonucleotide synthetase activity, an enzyme having N5-carboxyaminoimidazole ribonucleotide mutase activity, an enzyme having phosphoribosylaminoimidazolesuccinocarboxamide synthase activity, an enzyme having adenylosuccinate lyase activity, an enzyme having phosphoribosylaminoimidazole-carboxamide formyltransferase activity, an enzyme having IMP cyclohydolase activity, an enzyme having adenylosuccinate synthase activity, an enzyme having adenylate kinase activity, an enzyme having ATP synthase activity, an enzyme having adenosine kinase activity, an enzyme having IMP dehydrogenase activity and an enzyme having GMP synthase activity.

The at least one enzyme involved in the adenosine monophosphate biosynthesis pathway may be an enzyme selected from the group consisting of: an enzyme having ribose-phosphate diphosphokinase activity, an enzyme having amidophosphoribosyltransferase activity, an enzyme having formyltetrahydrofolate deformylase activity, an enzyme having phosphoribosylamine-glycine ligase activity, an enzyme having phosphoribosylglycineamide formyltransferase activity, an enzyme having phosphoribosylformylglycinamidine synthase activity, an enzyme having phosphoribosylformylglycineamidine cyclo-ligase activity, an enzyme having N5-carboxyaminoimidazole ribonucleotide synthetase activity, an enzyme having N5-carboxyaminoimidazole ribonucleotide mutase activity, an enzyme having phosphoribosylaminoimidazolesuccinocarboxamide synthase activity, an enzyme having adenylosuccinate lyase activity, an enzyme having phosphoribosylaminoimidazole-carboxamide formyltransferase activity, an enzyme having IMP cyclohydolase activity, an enzyme having adenylosuccinate synthase activity, an enzyme having adenylate kinase activity, an enzyme having ATP synthase activity and an enzyme having adenosine kinase activity.

According to some embodiments, the at least one enzyme involved in the purine nucleotide biosynthesis pathway (such as in the adenosine monophosphate biosynthesis pathway) is selected from the group consisting of: an enzyme having ribose-phosphate diphosphokinase activity, an enzyme having amidophosphoribosyltransferase activity, an enzyme having formyltetrahydrofolate deformylase activity, an enzyme having adenylosuccinate lyase activity, an enzyme having phosphoribosylaminoimidazole-carboxamide formyltransferase activity, an enzyme having adenylosuccinate synthase activity and an enzyme having adenosine kinase activity.

According to some embodiments, the bacterium of the present invention has been further modified to have any one of the modifications as disclosed in Table 1 in relation to one or more of its endogenous enzymes involved in the purine nucleotide biosynthesis pathway. Particularly, the bacterium may be the result of random mutagenesis making it resistant to an inhibitor of the enzyme in question.

According to some embodiments, the bacterium of the present invention is characterized in that it has been further modified to have a decreased expression and/or activity of at least one endogenous enzyme involved in the purine nucleotide degradation pathway compared to an otherwise identical bacterium that does not carry said modification.

According to some embodiments, the bacterium of the invention may be modified to have a decreased expression of at least one endogenous enzyme involved in the purine nucleotide degradation pathway compared to an otherwise identical bacterium that does not carry said modification.

According to some embodiments, the bacterium of the invention may be modified to have a decreased expression level of the endogenous gene encoding said at least one endogenous enzyme involved in the purine nucleotide degradation pathway compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95%or at least 100%compared to the otherwise identical bacterium.

According to some embodiments, the endogenous gene encoding said enzyme has been inactivated, such as by deletion of part of or the entire gene sequence.

According to some embodiments, the endogenous gene encoding said enzyme has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said enzyme is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said enzyme is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said enzyme.

According to some embodiments, the expression of said at least one endogenous enzyme involved in the purine nucleotide degradation pathway is decreased by way of inhibition.

Inhibition of the expression of the said endogenous enzyme may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said at least one endogenous enzyme involved in the purine nucleotide degradation pathway is decreased (e.g., inhibited) by transcriptional and/or translational repression of the endogenous gene encoding said polypeptide.

According to some embodiments, the expression of said at least one endogenous enzyme involved in the purine nucleotide degradation pathway is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous enzyme. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific mRNA. Exemplary types of RNAi molecules include microRNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of at least one endogenous enzyme involved in the purine nucleotide degradation pathway compared to an otherwise identical bacterium that does not carry said modification.

A decrease of the activity of the endogenous enzyme involved in the purine nucleotide degradation pathway may be achieved by any suitable means known in the art. For example, the activity may be decreased by introducing one or more mutations in the active site of the enzyme resulting in the reduction or loss of activity. Thus, according to some embodiments, the activity of the endogenous enzyme involved in the purine nucleotide degradation pathway is decreased by at least one active-site mutation resulting in the reduction or lossof activity. At least one active-site mutation may, for example, be at least one non-conservative amino acid substitution.

According to some embodiments, the at least one enzyme involved in the purine nucleotide degradation pathway is selected from the group consisting of: a purine nucleoside phosphorylase and adenosine-phosphoribosyltransferase. According to some embodiments, the at least one endogenous enzyme involved in the purine nucleotide degradation pathway is a purine nucleoside phosphorylase. According to some embodiments, the at least one endogenous enzyme involved in the purine nucleotide degradation pathway is adenosine-phosphoribosyltransferase.

According to some embodiments, the bacterium of the invention hasbeen further modified to have a decreased expression and/or activity of at least one endogenous enzyme involved in the guanosine monophosphate biosynthesis pathway compared to an otherwise identical bacterium that does not carry said modification.

According to some embodiments, the bacterium of the invention may be modified to have a decreased expression level of the endogenous gene encoding said at least one endogenous enzyme involved in the guanosine monophosphate biosynthesis pathway compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the endogenous gene encoding said enzyme has been inactivated, such as by deletion of part of or the entire gene sequence.

According to some embodiments, the endogenousgene encoding said enzyme has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said enzyme is to use the CRISPRi system. The CRISPRi system was developed as a tool for targeted repression of gene expression or for blocking targeted locationson the genome. The CRISPRi system consistsof the catalytically inactive, "dead" Cas9 protein (dCas9) and a guide RNA that defines the binding site for the dCas9 to DNA.

Thus, according to some embodiments, the endogenous gene encoding said enzyme is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said enzyme.

According to some embodiments, the expression of said at least one endogenous enzyme involved in the guanosine monophosphate biosynthesis pathway is decreased by way of inhibition.

Inhibition of the expression of the said endogenous enzyme may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said at least one endogenous enzyme involved in the guanosine monophosphate biosynthesis pathway is decreased (e.g., inhibited) by transcriptional and/or translational repression of the endogenous gene encoding said polypeptide.

According to some embodiments, the expression of said at least one endogenous enzyme involved in the guanosine monophosphate biosynthesis pathway is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous enzyme. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific m RNA. Exemplary types of RNAi molecules include micro RNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of at least one endogenous enzyme involved in the guanosine monophosphate biosynthesis pathway compared to an otherwise identical bacterium that does not carry said modification.

A decrease of the activity of the endogenous enzyme involved in the guanosine monophosphate biosynthesis pathway may be achieved by any suitable means known in the art. For example, the activity may be decreased by introducing one or more mutations in the active site of the enzyme resulting in the reduction or loss of activity. Thus, according to some embodiments, the activity of the endogenous enzyme involved in the guanosine monophosphate biosynthesis pathway is decreased by at least one active-site mutation resulting in the reduction or loss of activity. At least one active-site mutation may, for example, be at least one non-conservative amino acid substitution.

According to some embodiments, the at least one enzyme involved in the guanosine monophosphate biosynthesis pathway is selected from the group consisting of: IMP dehydrogenase and GMP synthetase.

According to some embodiments, the at least one enzyme involved in the guanosine monophosphate biosynthesis pathway is IM P dehydrogenase.

According to some embodiments, the at least one enzyme involved in the guanosine monophosphate biosynthesis pathway is GM P synthetase.

CYP450s are a diverse group of heme-containing enzymes, which catalyze a wide range of oxidative reactions. Cytochrome P450 monooxygenases (CYP450) also catalyze the hydroxylation of isopentenyladenine-type cytokinins. In Arabidopsis, 2 cytochrome P450 monooxygenases for hydroxylation of isopentenyladenine-type cytokinins are present, CYP735A1 and CYP735A2. The CYP735As catalyze a stereo-specific reaction of hydroxylation of iP-nucleotides, and with lower affinity the hydroxylation of the iP-nucleoside or iP to synthesize tZ (Takei et al., 2004b). Most CYP450s of plant origin are membrane-anchored to the endoplasmic reticulum (ER). Cytochrome P450 monooxygenases are also present in bacteria, such as *Rhodococcus fascians.* The cytochromes P450 generally fall into two broad classes, depending on the nature of the auxiliary protein(s). Class I P450sare found on the membranes of mitochondria and in bacteria, and class II cytochromes P450 are typified by the liver microsomal enzymes in mammalian cells. Class I P450s are three-component systems comprising a flavin adenine dinucleotide (FAD)-containing reductase, an iron-sulfur protein (ferredoxin), and the P450. Class II cytochromes P450 are composed of an FAD-containing, flavin mononucleotide (FMN)-containing NADPH-dependent cytochrome P450 reductase and a P450. Class III and class IV CYP450s have also been described in bacteria, but class I are the most common CYP450s in bacteria. The best-characterized bacterial cytochrome P450 monooxygenase system from *Pseudomonas putida* P450cam consists of three soluble proteins: putidaredoxin reductase; putidaredoxin, an intermediary iron-sulfur protein; and the cytochrome P450cam. The CYP450 Fas1 is encoded in the *fas* reagion of the linear plasmid and is thought to hydroxylate cytokinins produced by *R. fascians* (Frebort et al., 2011).

Cytochrome P450 monooxygenase (CYP450) can hydroxylate iP-nucleotides to produce trans-zeatin in three enzymatic steps from AM P and DM APP. The enzyme can stereo-specifically hydroxylate the prenyl side chain of iPRM Pand can thus make a bypassto tZRM P, which can be further activated to produce trans-zeatin.

Hence, according to some embodiments, the bacterium of the present invention is characterized in that it has been further modified to have increased protein expression of a polypeptide having cytochrome P450 monooxygenase (CYP450) activity compared to an otherwise identical bacterium that does not carry said modification.

By "increased protein expression" it is meant that the amount of the polypeptide having cytochrome P450 monooxygenase (CYP450) activity produced by the thus modified bacterium is increased compared to an otherwise identical bacterium that does not carry said modification. More particularly, by "increased expression" it is meant that the amount of the polypeptide having cytochrome P450 monooxygenase (CYP450) activity produced by the thus modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical bacterium that does not carry said modification. The amount of protein in a given cell can be determined by any suitable quantification technique known in the art, such as ELISA, Immunohistochemistry, or Western Blotting.

An increase in protein expression may be achieved by any suitable means well-known to those skilled in the art. For example, an increase in protein expression may be achieved by increasing the number of copies of the gene encoding the polypeptide having cytochrome P450 monooxygenase (CYP450) activity in the bacterium, such as by introducing into the bacterium an exogenous nucleic acid, such as a vector, comprising the gene encoding the polypeptide having cytochrome P450 monooxygenase (CYP450) activity operably linked to a promoter that is functional in the bacterium to cause the production of an m RNA molecule.

An increase in protein expression may also be achieved by the integration of at least a second copy of the gene encoding the polypeptide having cytochrome P450 monooxygenase (CYP450) activity into the genome of the bacterium.

An increase in protein expression may also be achieved by increasing the strength of the promoter operably linked to the gene encoding the polypeptide having cytochrome P450 monooxygenase (CYP450) activity. The promoters that can be used include natural promoters from *Bacillussubtilis, Bacillus amyloliquefaciens* or similar, such as P43, P15, Pveg, Pylb, PgroES, PsigX, PtrnQ,, Ppst, PsodA, PrpsF, PlepA, PliaG, PrpsF, Ppst, PfusA, PsodA, Phag as well as artificial promoters active in *Bacillus subtilis* or inducible *Bacillus subtilis* promoters, such as PmtlA, Pspac, PxylA, PsacB, or similar. Further examples include natural promoters from *Corynebacterium,* such as P CP_2454, Ptuf and Psod, natural promoters from *E coli,* such as T7, ParaBAD, Plac, Ptac and Ptrc, and the promoter P F1 derived from the corynephage BFK20.

An increase in protein expression may also be achieved by modifying the ribosome binding site on the mRNA molecule encoding the polypeptide having cytochrome P450 monooxygenase (CYP450) activity. By modifying the sequence of the ribosome binding site the translation initiation rate may be increased, thus increasing translation efficiency.

According to some embodiments, the increase in the number of copies of the gene is achieved by introducing into the bacterium one or more (such as two or three) exogenous nucleic acid molecules (such as one or more vectors) comprising the gene operably linked to a promoter that is functional in the host cell to cause the production of an mRNA molecule.

According to some embodiments, a bacterium of the invention comprises an exogenous nucleic acid molecule (such as a vector) comprising one or more (such as two, three, or four) nucleotide sequences encoding the polypeptide having cytochrome P450 monooxygenase (CYP450) activity. Suitably, the exogenous nucleic acid molecule further comprises a promoter that is functional in the bacterium to cause the production of an mRNA molecule and that is operably linked to the nucleotide sequence encoding said polypeptide having cytochrome P450 monooxygenase (CYP450) activity. According to some embodiments, the exogenous nucleic acid molecule isstably integrated into the genome of the bacterium.

The polypeptide having cytochrome P450 monooxygenase (CYP450) activity may be derived from the same species as the bacterium in which it is expressed or may be derived from a species different to the one in which it is expressed (i.e. it is heterologous). According to some embodiments, the polypeptide having cytochrome P450 monooxygenase (CYP450) activity is derived from the same species as the bacterium in which it is expressed. According to some embodiments, the polypeptide having cytochrome P450 monooxygenase (CYP450) activity is derived from a species different from the one in which it is expressed (i.e. it is heterologous). By way of example, in case that a bacterium does not have an endogenous gene encoding a polypeptide having cytochrome P450 monooxygenase (CYP450) activity, then the polypeptide having cytochrome P450 monooxygenase (CYP450) activity expressed in said bacterium will be heterologous to said bacterium.

According to some embodiments, the polypeptide having cytochrome P450 monooxygenase (CYP450) activity is a bacterial polypeptide having cytochrome P450 monooxygenase (CYP450) activity. With "bacterial polypeptide having cytochrome P450 monooxygenase (CYP450) activity" it is meant that the polypeptide having cytochrome P450 monooxygenase (CYP450) activity is naturally derived from a bacterium, such as *Rhodococcus fascians.* A non-limiting example of a bacterial polypeptide having cytochrome P450 monooxygenase (CYP450) activity is set out in SEQ ID NO: 93.

According to some embodiments, the polypeptide having cytochrome P450 monooxygenase (CYP450) activity is a plant polypeptide having cytochrome P450 monooxygenase (CYP450) activity. With "plant polypeptide having cytochrome P450 monooxygenase (CYP450) activity" it is meant that the polypeptide having cytochrome P450 monooxygenase (CYP450) activity is naturally derived from a plant, such as *Arabidopsis thaliana.* Non-limiting examples of a plant polypeptide having cytochrome P450 monooxygenase (CYP450) activity are set out in SEQ ID NOs: 94 and 95.

A polypeptide having cytochrome P450 monooxygenase (CYP450) activity for use according to the invention may for instance be a polypeptide having cytochrome P450 monooxygenase (CYP450) activity selected from the group consisting of: i) a polypeptide comprising an amino acid sequence of any one of SEQ ID NOs: 93 to 95; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 93 to 95.

According to some embodiments, the polypeptide having cytochrome P450 monooxygenase (CYP450) activity comprises the amino acid sequence of SEQ ID NO: 93 or an amino acid sequence having at least 70%, such as at least 75%, sequence identity with SEQ ID NO: 93. According to some embodiments, the polypeptide having cytochrome P450 monooxygenase (CYP450) activity comprises the amino acid sequence of SEQ ID NO: 93 or an amino acid sequence having at least 80%, such as at least 85%, sequence identity with SEQ ID NO: 93. According to some embodiments, the polypeptide having cytochrome P450 monooxygenase (CYP450) activity comprises the amino acid sequence of SEQ ID NO: 93 or an amino acid sequence having at least 90%, such as at least 95%, sequence identity with SEQ ID NO: 93. According to some embodiments, the polypeptide having cytochrome P450 monooxygenase (CYP450) activity comprises the amino acid sequence of SEQ ID NO: 93.

According to some embodiments, the polypeptide having cytochrome P450 monooxygenase (CYP450) activity comprises the amino acid sequence of SEQ ID NO: 94 or an amino acid sequence having at least 70%, such as at least 75%, sequence identity with SEQ ID NO: 94. According to some embodiments, the polypeptide having cytochrome P450 monooxygenase (CYP450) activity comprises the amino acid sequence of SEQ ID NO: 94 or an amino acid sequence having at least 80%, such as at least 85%, sequence identity with SEQ ID NO: 94. According to some embodiments, the polypeptide having cytochrome P450 monooxygenase (CYP450) activity comprises the amino acid sequence of SEQ ID NO: 94 or an amino acid sequence having at least 90%, such as at least 95%, sequence identity with SEQ ID NO: 94. According to some embodiments, the polypeptide having cytochrome P450 monooxygenase (CYP450) activity comprises the amino acid sequence of SEQ ID NO: 94.

According to some embodiments, the polypeptide having cytochrome P450 monooxygenase (CYP450) activity comprises the amino acid sequence of SEQ ID NO: 95 or an amino acid sequence having at least 70%, such as at least 75%, sequence identity with SEQ ID NO: 95. According to some embodiments, the polypeptide having cytochrome P450 monooxygenase (CYP450) activity comprises the amino acid sequence of SEQ ID NO: 95 or an amino acid sequence having at least 80%, such as at least 85%, sequence identity with SEQ ID NO: 95. According to some embodiments, the polypeptide having cytochrome P450 monooxygenase (CYP450) activity comprises the amino acid sequence of SEQ ID NO: 95 or an amino acid sequence having at least 90%, such as at least 95%, sequence identity with SEQ ID NO: 95. According to some embodiments, the polypeptide having cytochrome P450 monooxygenase (CYP450) activity comprises the amino acid sequence of SEQ ID NO: 95.

A bacterium according to the present invention can be produced from any suitable bacterium. The bacterium may be Gram-positive or Gram-negative. Non-limiting examples for Gram-negative bacterial host cells include species from the genera *Escherichia, Erwinia, Klebsiella,* and *Otrobacter.* Non-limiting examples of Gram-positive bacterial host cells include species from the genera *Bacillus, Lactococcus, Lactobacillus, Clostridium, Corynebacterium, Sreptomyces, Streptococcus,* and *Cellulomonas.* According to some embodiments, the bacterium of the present invention is Gram-positive. According to some embodiments, the bacterium of the present invention is Gram-negative.

According to some embodiments, the bacterium of the present invention is a bacterium of the family selected from the group consisting of *Enterobacteriaceae, Bacillaceae, Lactobacillaceae, and Corynebacteriaceae.* According to some embodiments, the recombinant host cell is a bacterium of the family *Enterobacteriaceae.* According to some embodiments, the recombinant host cell is a bacterium of the family *Bacillaceae.* According to some embodiments, the recombinant host cell is a bacterium of the family *Corynebacteriaceae.*

According to some embodiments, the bacterium of the present invention is a bacterium, which may be a bacterium of the genus *Bacillus, Lactococcus, Lactobacillus, Clostridium, Corynebacterium, Geobacillus, Thermoanaerobacterium, Streptococcus, Pseudomonas, Sreptomyces, Escherichia, Shigella, Acinetobacter, Otrobacter, Salmonella, Klebsiella, Enterobacter, Erwinia, Kluyvera, Serratia, Cedecea, Morganella, Hafnia, Edwardsiella, Providencia, Proteus,* or *Yersinia.*

According to some embodiments, the bacterium of the present invention is a bacterium of the genus *Bacillus.* Non-limiting examples of a bacterium of the genus *Bacillus* are *Bacillus subtilis, Bacillus amyloliquefaciens, Bacillus licheniformis,* and *Bacillus mojavensis.* According to some embodiments, the bacterium of the present invention is *Bacillus subtilis.*

According to some embodiments, the bacterium of the present invention is a bacterium of the genus *Corynebacterium.* Non-limiting examples of a bacterium of the genus *Corynebacterium* are *Corynebacterium glutamicum* and *Corynebacterium stationis.* According to some, the bacterium of the present invention is *Corynebacterium glutamicum.* According to some, the bacterium of the present invention is *Corynebacterium stationis.* Within the context of the present invention, *Corynebacterium stationis and Corynebacterium ammoniagenes* refer to the same species and may be used interchangeably.

According to some embodiments, the bacterium of the present invention is a bacterium of the genus *Escherichia.* A non-limiting example of a bacterium of the genus *Escherichia* is *Escherichia coli.* According to some, the bacterium of the present invention is *Escherichia coli.*

As noted above, a bacterium of the invention is modified to express one or more polypeptides as detailed herein, which may mean that an exogenous nucleic acid molecule, such as a DNA molecule, which comprises a nucleotide sequence encoding said polypeptide has been introduced in the bacterium. Thus, a bacterium of the present invention may comprise an exogenous nucleic acid molecule, such as a DNA molecule, which comprises a nucleotide sequence encoding the polypeptide in question. Techniques for introducing an exogenous nucleic acid molecule, such as a DNA molecule, into a bacterial cell are well-known to those of skill in the art and include transformation (e.g., heat shock or natural transformation) among others.

In order to facilitate (over)expression of a polypeptide in the bacterium, the exogenous nucleic acid molecule may comprise suitable regulatory elements such as a promoter that is functional in the bacterial cell to cause the production of an mRNA molecule and that is operably linked to the nucleotide sequence encoding said polypeptide.

Promoters useful in accordance with the invention are any known promoters that are functional in a given host cell to cause the production of an m RNA molecule. Many such promoters are known to the skilled person. Such promoters include promoters normally associated with other genes, and/or promoters isolated from any bacteria. The use of promoters for protein expression is generally known to those skilled in the art of molecular biology, for example, see Sambrook et al., Molecular cloning: A Laboratory Manual, Cold Spring Harbor Laboratory, Cold Spring Harbor, N.Y., 1989. The promoter employed may be inducible, such as a temperature-inducible promoter (e.g., a pLor pRphage lambda promoters, each of which can be controlled by the temperature-sensitive lambda repressor c1857). The term "inducible" used in the context of a promoter means that the promoter only directs transcription of an operably linked nucleotide sequence if a stimulus is present, such as a change in temperature or the presence of a chemical substance ("chemical inducer"). As used herein, "chemical induction" according to the present invention refers to the physical application of an exogenousor endogenous substance (incl. macromolecules, e.g., proteins or nucleic acids) to a host cell. This has the effect of causing the target promoter present in the host cell to increase the rate of transcription. Alternatively, the promoter employed may be constitutive. The term "constitutive" used in the context of a promoter means that the promoter is capable of directing transcription of an operably linked nucleotide sequence in the absence of a stimulus (such as heat shock, chemicals, etc.).

Temperature induction systems work, for example, by employing promoters that are repressed by thermolabile repressors. These repressors are active at lower temperatures for example at 30•C, while unable to fold correctly at 37 •C and are therefore inactive. Such circuits therefore can be used to directly regulate the genes of interest also by genome integration of the genes along with the repressors. Examples of such a temperature-inducible expression system are based on the pL and/or pR• phage promoters, which are regulated by the thermolabile cl857 repressor. Similar to the genome integrated DE3 system, the expression of the T7 RNA polymerase gene may also be controlled using a temperature-controlled promoter system, while the expression of the genes of interest can be controlled using a T7 promoter.

Non-limiting examples of promoters functional in bacteria include both constitutive and inducible promoters such as T7 promoter, the beta-lactamase and lactose promoter systems; alkaline phosphatase (phoA) promoter, a tryptophan (trp) promoter system, tetracycline promoter, lambda-phage promoter, ribosomal protein promoters; and hybrid promoters such as the tac promoter. Other bacterial and synthetic promoters are also suitable.

Besides a promoter, the exogenous nucleic acid molecule may further comprise at least one regulatory element selected from a 5' untranslated region (5'UTR) and 3' untranslated region (3' UTR). Many such 5' UTRs and 3' UTRs derived from prokaryotes and eukaryotes are well known to the skilled person. Such regulatory elements include 5' UTRs and 3' UTRs normally associated with other genes, and/or 5' UTRs and 3' UTRs isolated from any bacteria.

Usually, the 5' UTR contains a ribosome binding site (RBS), also known as the Shine-Dalgarno sequence, which is usually 3-10 base pairs upstream from the initiation codon.

The exogenous nucleic acid molecule may be a vector or part of a vector, such as an expression vector. Normally, such a vector remains extrachromosomal within the bacterial cell, which means that it is found outside of the nucleus or nucleoid region of the bacterium.

It is also contemplated by the present invention that the exogenous nucleic acid molecule is stably integrated into the genome of the bacterium. Means for stable integration into the genome of a host cell, e.g., by homologous recombination, are well known to the skilled person.

### Method of the invention

The present invention also provides methods for producing an isoprenoid cytokinin or riboside derivative thereof, comprising cultivating a bacterium according to the present invention under suitable culture conditions in a suitable culture medium.

The method may further comprise collecting the isoprenoid cytokinin or riboside derivative thereof from the culture medium.

According to some embodiments, the isoprenoid cytokinin or riboside derivative thereof is selected from the group consisting of *trans-zeatin* (tZ), *trans-zeatin* riboside (tZR), *N⁶*-(D2-isopentenyl)adenine (iP), *N⁶*-(dimethylallyl)adenosine (iPR), dihydrozeatin (DZ), ribosyl dihydrozeatin (DZR), and combinations thereof.

According to some embodiments, the isoprenoid cytokinin or riboside derivative thereof is *trans-*zeatin (tZ) and trans-zeatin riboside (tZR), respectively.

The culture medium employed may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers or may be prepared according to published receipts, e.g. the American Type Culture Collection (ATCC) Catalogue of strains. Non-limiting standard media well known to the skilled person include Luria Bertani (LB) broth, Sabouraud Dextrose (SD) broth, MSbroth, Yeast Peptone Dextrose, BMMY, GMMY, or Yeast Malt Extract (YM) broth, which are all commercially available. A non-limiting example of suitable media for culturing bacterial cells, such as *B. subtilis, L. lactisor E coli* cells, including minimal media and rich media such as Luria Broth (LB), M9 media, M17 media, SA media, MOPS media, Terrific Broth, YT and others.

The carbon source may be any suitable carbon substrate know in the art, and particularly any carbon substrate commonly used in the cultivation of microorganisms and/ or fermentation. Non-limiting examplesof suitable fermentable carbon substrates include carbohydrates (e.g., C5 sugars such as arabinose or xylose, or C6 sugars such as glucose), glycerol, glycerine, acetate, dihydroxyacetone, one-carbon source, methanol, methane, oils, animal fats, animal oils, plant oils, fatty acids, lipids, phospholipids, glycerolipids, monoglycerides, diglycerides, triglycerides, renewable carbon sources, polypeptides (e.g., a microbial or plant protein or peptide), yeast extract, component from a yeast extract, peptone, casaminoacids or any combination of two or more of the foregoing.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferroussulfate, manganese sulfate, calcium chloride, and the like can be used.

In order to further improve the production of the isoprenoid cytokinin or riboside derivative thereof, such as is *trans-zeatin* (tZ) and trans-zeatin riboside (tZR), respectively, the culture medium may be supplemented with a source of adenine or adenosine, such as adenine sulfate. Thus, according to some embodiments, the culture medium comprises adenine sulfate. The concentration of adenine sulfate in the culture medium may generally be in the range from about 0.1 g/Lto about 4 g/L, such asfrom about 1 g/Lto about 3.5 g/L. According to some embodiments, the concentration of adenine sulfate in the culture medium is in the range from about 2.5 g/L to about 3.5 g/L. However, other sources of adenine or adenosine, such as yeast extract, are also contemplated for use in accordance with the invention.

The cultivation can be preferably performed under aerobic conditions, such as by a shaking culture, and by a stirring culture with aeration, at a temperature of about 20 to about 45 °C, such as about 30 to 38 °C, such as at about 37°C. According to some embodiments, the cultivation is performed at a temperature of about 30 to 38 °C, such as at about 37°C. The pH of the culture is usually above 5, such as in a range from about 6 to about 8, preferably from about 6.5 to about 7.5, more preferably from about 6.8 to about 7.2. According to some embodiments, the cultivation is performed at a pH from about 6 to about 8. The pH of the culture can be adjusted with ammonia, calcium carbonate, various acids, various bases, and buffers. The cultivation may be carried out for a period in the range from 10 to 70 h, such as in the range of 10 to 24 h or 10 to 48 h.

After cultivation, solids such as cells can be removed from the culture medium by centrifugation or membrane filtration. The isoprenoid cytokinin or riboside derivative thereof can be collected by a conventional method for the isolation and purification of chemical compounds from a medium. Well-known purification procedures include, but are not limited to, centrifugation or filtration, precipitation, ion exchange, chromatographic methods such as e.g. ion-exchange chromatography or gel filtration chromatography, and crystallization methods.

The present invention thus provides an isoprenoid cytokinin or riboside derivative thereof obtainable by a method asdetailed herein.

### Abbreviations

iP - *N⁶*-(D2-isopentenyl)adenine
iPR - *N⁶*-(D2 -isopentenyl)adenine riboside alias *N⁶* -(D2 -isopentenyl)adenosine
tZ - *trans-zeatin*
tZR - *trans*-ribosylzeatin alias trans-zeatin riboside
DZ - dihydrozeatin
DZR - ribosyl dihydrozeatin alias dihydrozeatin riboside
cZ - *cis-ze*atin
MVA pathway - mevalonate biosynthesis pathway
M EP pathway - methylerythritol phosphate biosynthesis pathway
DM APP - dimethylallyl diphosphate
HM BDP - 1-hydroxy-2-methyl-2-butenyl 4-diphosphate
DXP - 1-deoxy-D-xylulose-5-phosphate
DXS - 1-deoxy-D-xylulose-5-phosphate synthase; DXP-synthase (EC2.2.1.7)
tZRM P - *trans-zeatin* riboside 5'-monophosphate
iPRM P - *N⁶*-(D2 -isopentenyl)adenine riboside 5'-monophosphate
DZRM P - dihydrozeatin riboside 5'-monophosphate
cZRM P - cis-zeatin riboside 5'-monophosphate
IPT - Adenylate isopentenyltransferase (EC2.5.1.27)
LOG - cytokinin riboside 5'-monophosphate phosphoribohydrolase 'Lonely guy' (EC3.2.2.n1)
CYP450 - cytochrome P450 monooxygenase

### Certain other definitions

As used herein, a"polypeptide having adenylate isopentenyltransferase activity" or a "polypeptide, which has adenylate isopentenyltransferase activity" means a polypeptide that catalyzes the reactions: Dimethylallyl diphosphate + AM P <=> diphosphate + N(6)-(dimethylallyl)adenosine 5'-phosphate (EC 2.5.1.27) and optionally 1-hydroxy-2-methyl-2-butenyl 4-diphosphate + AM P <=> diphosphate + *trans*-zeatin riboside 5'-phosphate. Non-limiting examples of such polypeptides are provided in SEQ ID NOs: 1 to 33.

As used herein, a"polypeptide having cytokinin riboside 5'-monophosphate phosphoribohydrolase activity" or a "polypeptide which has cytokinin riboside 5'-monophosphate phosphoribohydrolase activity" means a polypeptide that catalyzes the reaction: N(6)-(Delta(2)-isopentenyl)-adenosine 5'-phosphate + H(2)O <=> N(6)-(dimethylallyl)adenine + D-ribose 5'-phosphate (EC 3.2.2.n1). Non-limiting examples of such polypeptides are provided in SEQ ID NOs: 34 to 62.

As used herein, a "polypeptide having 1-deoxy-D-xylulose-5-phosphate synthase activity" or a "polypeptide, which has 1-deoxy-D-xylulose-5-phosphate synthase activity" means a polypeptide that catalyzes the reaction: Pyruvate + D-glyceraldehyde 3-phosphate <=> 1-deoxy-D-xylulose 5-phosphate + CO(2) (EC2.2.1.7). Non-limiting examples of such polypeptides are provided in SEQ ID NOs: 63 to 70.

As used herein, an "enzyme having ribose-phosphate diphosphokinase activity" or an "enzyme, which has ribose-phosphate diphosphokinase activity" means an enzyme that catalyzes the reaction: ATP + D-ribose 5-phosphate <=> AMP + 5-phospho-alpha-D-ribose 1-diphosphate (EC 2.7.6.1). An enzyme having ribose-phosphate diphosphokinase activity is encoded by the bacterial gene *prs* or an ortholog thereof.

Asused herein, an "enzyme having amidophosphoribosyltransferase activity" or an "enzyme,which has amidophosphoribosyltransferase activity" means an enzyme that catalyzes the reaction: 5-phospho-beta-D-ribosylamine + diphosphate + L-glutamate <=> L-glutamine + 5-phospho-alpha-D-ribose 1-diphosphate + H₂O (EC 2.4.2.14). An enzyme having amidophosphoribosyltransferase activity is encoded by the bacterial gene *purF* or an ortholog thereof.

As used herein, an "enzyme having formyltetrahydrofolate deformylase activity" or an "enzyme, which has formyltetrahydrofolate deformylase activity" means an enzyme that catalyzes the reaction: 10-formyltetrahydrofolate + H₂O <=> formate + tetrahydrofolate (EC3.5.1.10). An enzyme having amidophosphoribosyltransferase activity is encoded by the bacterial gene *purU* or an ortholog thereof.

As used herein, an "enzyme having phosphoribosylamine-glycine ligase activity" or an "enzyme, which has phosphoribosylamine-glycine ligase activity" means an enzyme that catalyzes the reaction: ATP + 5-phospho-beta-D-ribosylamine + glycine <=> ADP + phosphate + N¹-(5-phospho-beta-D-ribosyl)glycinamide (EC 6.3.4.13). An enzyme having phosphoribosylamine-glycine ligase activity is encoded by the bacterial gene *pur D* or an ortholog thereof.

As used herein, an "enzyme having phosphoribosylglycineamide formyltransferase activity" or an "enzyme, which has phosphoribosylglycineamide formyltransferase activity" means an enzyme that catalyzes the reaction: 10-formyltetrahydrofolate + N¹-(5-phospho-beta-D-ribosyl)glycinamide <=> tetrahydrofolate + N²-formyl-N¹-(5-phospho-beta-D-ribosyl)glycinamide (EC 2.1.2.2). An enzyme having phosphoribosylglycineamide formyltransferase activity is encoded by the bacterial gene *purTor* an ortholog thereof.

As used herein, an "enzyme having phosphoribosylformylglycinamidine synthase activity" or an "enzyme, which has phosphoribosylformylglycinamidine synthase activity" means an enzyme that catalyzes the reaction: ATP + N²-formyl-N¹-(5-phospho-beta-D-ribosyl)glycinamide + L-glutamine + H₂O <=> ADP + phosphate + 2-(formamido)-N¹-(5-phospho-beta- D-ribosyl)acetamidine + L-glutamate (EC 6.3.5.3). An enzyme having phosphoribosylformylglycinamidine synthase activity is encoded by the bacterial gene *purL* or an ortholog thereof.

As used herein, an "enzyme having phosphoribosylformylglycineamidine cyclo-ligase activity" or an "enzyme, which has phosphoribosylformylglycineamidine cyclo-ligase activity" means an enzyme that catalyzes the reaction: ATP + 2-(formamido)-N¹-(5-phospho-beta-D-ribosyl)acetamidine <=> ADP + phosphate + 5-amino-1-(5-phospho-beta-D-ribosyl)imidazole (EC 6.3.3.1). An enzyme having phosphoribosylformylglycineamidine cyclo-ligase activity is encoded by the bacterial gene *purM* or an ortholog thereof.

As used herein, an "enzyme having N5-carboxyaminoimidazole ribonucleotide synthetase activity" or an "enzyme, which has N5-carboxyaminoimidazole ribonucleotide synthetase activity" means an enzyme that catalyzes the reaction: ATP + 5-amino-1-(5-phospho-D-ribosyl)imidazole + HCO₃⁻ <=> ADP + phosphate + 5-carboxyamino-1-(5-phospho- D-ribosyl)imidazole (EC 6.3.4.18). An enzyme having N5-carboxyaminoimidazole ribonucleotide synthetase activity is encoded by the bacterial gene *pur K* or an ortholog thereof.

As used herein, an "enzyme having N5-carboxyaminoimidazole ribonucleotide mutase activity" or an "enzyme, which has N5-carboxyaminoimidazole ribonucleotide mutase activity" means an enzyme that catalyzes the reaction: 5-carboxyamino-1-(5-phospho-D-ribosyl)imidazole <=> 5-amino-1-(5-phospho-D-ribosyl)imidazole-4-carboxylate (EC 5.4.99.18). An enzyme having N5-carboxyaminoimidazole ribonucleotide mutase activity is encoded by the bacterial gene *purE* or an ortholog thereof.

As used herein, an "enzyme having phosphoribosylaminoimidazolesuccinocarboxamide synthase activity" or an "enzyme, which has phosphoribosylaminoimidazolesuccinocarboxamide synthase activity" means an enzyme that catalyzes the reaction: ATP + 5-amino-1-(5-phospho-D-ribosyl)imidazole-4-carboxylate + L-aspartate <=> ADP + phosphate + (S)-2-(5-amino-1-(5-phospho-D-ribosyl)imidazole-4-carboxamido)succinate (EC 6.3.2.6). An enzyme having phosphoribosylaminoimidazolesuccinocarboxamide synthase activity is encoded by the bacterial gene *purC* or an ortholog thereof.

As used herein, an "enzyme having adenylosuccinate lyase activity" or an "enzyme, which has adenylosuccinate lyase activity" meansan enzyme that catalyzes the reaction: (S)-2-(5-amino-1-(5-phospho-D-ribosyl)imidazole-4-carboxamido)succinate <=> fumarate + 5-amino-1-(5-phospho-D-ribosyl)imidazole-4-carboxamide (EC4.3.2.2). An enzyme having adenylosuccinate lyase activity is encoded by the bacterial gene *pur B* or an ortholog thereof.

As used herein, an "enzyme having phosphoribosylaminoimidazole-carboxamide formyltransferase activity" or an "enzyme, which has phosphoribosylaminoimidazole-carboxamide formyltransferase activity" means an enzyme that catalyzes the reaction: 10-formyltetrahydrofolate + 5-amino-1-(5-phospho-D-ribosyl)imidazole-4-carboxamide <=> tetrahydrofolate + 5-formamido-1-(5-phospho-D-ribosyl)imidazole-4-carboxamide (EC 2.1.2.3). An enzyme having phosphoribosylaminoimidazole-carboxamide formyltransferase activity is encoded by the bacterial gene *purH* or an ortholog thereof.

As used herein, an "enzyme having IM P cyclohydrolase activity" or an "enzyme, which has IM P cyclohydrolase activity" means an enzyme that catalyzes the reaction: IM P + H₂O <=> 5-formamido-1-(5-phospho-D-ribosyl)imidazole-4-carboxamide (EC 3.5.4.10). An enzyme having IM P cyclohydrolase activity is, for example, encoded by the bacterial gene *pur H* or an ortholog thereof.

As used herein, an "enzyme having adenylosuccinate synthase activity" or an "enzyme, which has adenylosuccinate synthase activity" means an enzyme that catalyzes the reaction: GTP + IM P + L-aspartate <=> GDP + phosphate + N⁶-(1,2-dicarboxyethyl)-AM P (EC 6.3.4.4). An enzyme having adenylosuccinate synthase activity is encoded by the bacterial gene *pur A* or an ortholog thereof.

As used herein, an "enzyme having adenylate kinase activity" or an "enzyme, which has adenylate kinase activity" means an enzyme that catalyzes the reaction: ATP + AM P <=> 2 ADP (EC2.7.4.3). An enzyme having adenylate kinase activity is encoded by the bacterial gene *adk* or an ortholog thereof.

As used herein, an "enzyme having ATP synthase activity" or an "enzyme, which has ATP synthase activity" means an enzyme that catalyzes the reaction: ATP + H₂O + H⁺ (cytosol) <=> ADP + phosphate + H⁺ (periplasm) (EC3.6.3.14). An enzyme having ATPsynthase activity is, for example, the ATP synthase F₀ or F₁ complex encoded by the bacterial *atp* operon (including the genes *atpB, atpF, atpE, atpD, atpG, atpA, atpH* and *atpC*) or orthologs thereof.

As used herein, an "enzyme having adenosine kinase activity" or an "enzyme, which has adenosine kinase activity" means an enzyme that catalyzes the reaction: ATP + adenosine <=> ADP + AM P (EC 2.7.1.20). An enzyme having adenosine kinase activity is encoded by the bacterial gene *adk* or orthologs thereof.

As used herein, an "enzyme having IM P dehydrogenase activity" or an "enzyme, which has IM P dehydrogenase activity" means an enzyme that catalyzes the reaction: Inosine 5'-phosphate + NAD⁺ + H₂O <=> xanthosine 5'-phosphate + NADH (EC 1.1.1.205). An enzyme having IM P dehydrogenase activity is encoded by the bacterial gene *guaB* or an ortholog thereof.

As used herein, an "enzyme having GM P synthase activity" or an "enzyme, which has GM Psynthase activity" means an enzyme that catalyzes the reaction: ATP + XM P + L-glutamine + H₂O <=> AM P + diphosphate + GM P+ L-glutamate (EC6.3.5.2). An enzyme having GM P synthase activity is encoded by the bacterial gene *gua A* or an ortholog thereof.

As used herein, an "enzyme having purine nucleoside phosphorylase activity" or an "enzyme, which has purine nucleoside phosphorylase activity" means an enzyme that catalyzes the reaction: Purine nucleoside + phosphate <=> purine + alpha-D-ribose 1-phosphate (EC 2.4.2.1). An enzyme having purine nucleoside phosphorylase activity is, for example, encoded by the bacterial gene *deo D* or an ortholog thereof.

As used herein, an "enzyme having adenosine phosphoribosyltransferase activity" or an "enzyme, which has adenosine phosphoribosyltransferase activity" means an enzyme that catalyzes the reaction: AM P + diphosphate <=> adenine + 5-phospho-alpha-D-ribose 1-diphosphate (EC2.4.2.7). An enzyme having adenosine phosphoribosyltransferase activity is, for example, encoded by the bacterial gene *apt* or an ortholog thereof.

Asused herein, "purine nucleotide biosynthesis pathway" is understood to include both the *de novo* biosynthesis pathway and the salvage pathway by which nucleotides are synthesized.

As used herein, "adenosine monophosphate biosynthesis pathway" is understood to include both the *de novo* biosynthesis pathway and the salvage pathway by which adenosine monophosphate is synthesized.

As used herein, "guanosine monophosphate biosynthesis pathway" is understood to include both the *de novo* biosynthesis pathway and the salvage pathway by which guanosine monophosphate is synthesized.

As used herein, a "polypeptide having cytochrome P450 monooxygenase (CYP450) activity" or a "polypeptide, which hascytochrome P450 monooxygenase (CYP450) activity" means a polypeptide that catalyzes the trans-hydroxylation reactions: 1) N6-(• 2-isopentenyl)-adenosine 5'-monophosphate + a reduced [NADPH-hemoprotein reductase] + oxygen • trans-zeatin riboside monophosphate + an oxidized [NADPH-hemoprotein reductase] + H₂O; 2) N6-(• 2-isopentenyl)-adenosine 5'-triphosphate + a reduced [NADPH-hemoprotein reductase] + oxygen • trans-zeatin riboside triphosphate + an oxidized [NADPH-hemoprotein reductase] + H₂O; 3) N6-(• 2-isopentenyl)-adenosine 5'-diphosphate + a reduced [NADPH-hemoprotein reductase] + oxygen • trans-zeatin riboside diphosphate + an oxidized [NADPH-hemoprotein reductase] + H₂O (EC 1.14.14.-). A polypeptide having cytochrome P450 monooxygenase (CYP450) activity is, for example, encoded by the bacterial gene FAS1, or *Arabidopsis thaliana* (plant) genes CYP735A1 and CYP735A2, or an ortholog thereof. Non-limiting examples include SEQ ID 93 to 95.

"Polypeptide" and "protein" are used interchangeably herein to denote a polymer of at least two amino acids covalently linked by an amide bond, regardless of length or post-translational modification (e.g., glycosylation, phosphorylation, lipidation, myristoylation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a bacterium, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genesthat are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it ispresent or which isfound in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus, or a different kingdom, as the host DNA.

"Heterologous" as used herein in the context of apolypeptide means that apolypeptide is normally not found in or made (i.e. expressed) by the host microorganism, but derived from a different organism, a different species, a different genus, or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression level" of a gene it is meant that the amount of the transcription product, respectively the amount of the polypeptide encoded by said gene produced by the modified bacterium is decreased compared to an otherwise identical bacterium that does not carry said modification. More particularly, by "decreased expression level" of a gene it is meant that the amount of the transcription product, respectively the amount of the polypeptide encoded by said gene produced by the modified bacterium is decreased by at least 10%, such as at least 20%, at least 30%, at least 40%, at least 50% at least 60%, at least 70%, at least 80%, at least 90% or at least 100%, compared to an otherwise identical bacterium that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry, or Western Blotting, and the like.

Expression of a gene can be decreased by introducing a mutation into the gene in the genome of the modified bacterium so that the intracellular activity of the polypeptide encoded by the gene is decreased as compared to an otherwise identical bacterium that does not carry the said mutation. Mutations, which result in a decreased expression of the gene include the replacement of one nucleotide or more to cause an amino acid substitution in the polypeptide encoded by the gene (missense mutation), the introduction of a stop codon (nonsense mutation), deletion, or insertion of nucleotides to cause a frameshift, insertion of a drug-resistance gene, or deletion of a part of the gene or the entire gene (Qiu and Goodman, 1997; Kwon et al., 2000). The expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The lambda-red mediated gene replacement is a particularly suitable method to inactive one or more genes as described herein.

"Inactivating", "inactivation" and "inactivated", when used in the context of a gene, means that the gene in question no longer expresses a functional protein. It is possible that the modified DNA region is unable to naturally express the gene due to the deletion of a part of or the entire gene sequence, the shifting of the reading frame of the gene, the introduction of missense/nonsense mutation(s), or the modification of an adjacent region of the gene, including sequences controlling gene expression, such as a promoter, enhancer, attenuator, ribosome- binding site, etc. Preferably, a gene of interest is inactivated by the deletion of a part of or the entire gene sequence, such as by gene replacement. Inactivation may also be accomplished by introducing or expressing a rare-cutting endonuclease able to selectively inactivate by DNA cleavage, preferably by the double-strand break, the gene of interest. A "rare-cutting endonuclease" within the context of the present invention includes transcription activator-like effector (TALE) nucleases, meganucleases, zinc-finger nucleases (ZFN), and RNA-guided endonucleases.

The presence or absence of a gene in the genome of a bacterium can be detected by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the protein encoded by the gene can be measured by well-known methods, including SDS-PAGE followed by an immunoblotting assay (Western blotting analysis), and the like.

By "increased expression level" of a gene it is meant that the amount of the transcription product, respectively the amount of the polypeptide encoded by said gene produced by the modified bacterium is increased compared to an otherwise identical bacterium that does not carry said modification. More particularly, by "increased expression level" of a gene it is meant that the amount of the transcription product, respectively the amount of the polypeptide encoded by said gene produced by the modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000% or at least about 10000%, compared to an otherwise identical bacterium that does not carry said modification. The level of expression of a gene can be determined by well-known methods, including PCR, Southern blotting, and the like. In addition, the level of gene expression can be estimated by measuring the amount of m RNA transcribed from the gene using various well-known methods, including Northern blotting, quantitative RT-PCR, and the like. The amount of the polypeptide encoded by the gene can be measured by well-known methods, including ELISA, Immunohistochemistry, or Western Blotting, and the like.

By "increased expression level" of a polypeptide it is meant that the amount of the polypeptide in question produced by the modified microorganism is increased compared to an otherwise identical bacterium that does not carry said modification. More particularly, by "increased expression level" of a polypeptide it is meant that the amount of the polypeptide in question produced by the modified bacterium is increased by at least 10%, such as at least 20%, at least 30%, at least 40%, at least 50% at least 60%, at least 70%, at least 80%, at least 90%, at least 100%, at least 150%, at least 200%, at least 300%, at least 400%, at least 500%, at least 600%, at least 700% at least 800%, at least about 900%, at least about 1000%, at least about 2000%, at least about 3000%, at least about 4000%, at least about 5000%, at least about 6000%, at least about 7000%, at least about 8000% at least about 9000%or at least about 10000%, compared an otherwise identical bacterium that does not carry said modification. The amount of a polypeptide produced in a given cell can be determined by any suitable quantification technique known in the art, such as ELISA, Immunohistochemistry, or Western Blotting.

An increase in polypeptide expression may be achieved by any suitable meanswell-known to those skilled in the art. For example, an increase in polypeptide expression may be achieved by increasing the number of copies of the gene or genes encoding the polypeptide in the microorganism, such as by introducing into the microorganism an exogenous nucleic acid, such as a vector, comprising the gene or genes encoding the polypeptide operably linked to a promoter that is functional in the microorganism to cause the production of an mRNA molecule. An increase in polypeptide expression may also be achieved by the integration of at least a second copy of the gene or genes encoding the polypeptide into the genome of the microorganism. An increase in polypeptide expression may also be achieved by increasing the strength of the promoter(s) operably linked to the gene or genes encoding the polypeptide. An increase in polypeptide expression may also be achieved by modifying the ribosome binding site on the m RNA molecule encoding the polypeptide. By modifying the sequence of the ribosome binding site, the translation initiation rate may be increased, thus increasing translation efficiency.

As used herein, "decreased", "decreasing" or "decrease of" expression of a polypeptide (e.g. an enzyme involved in the purine nucleotide degradation pathway) means that the expression of said polypeptide in a modified bacterium is reduced compared to the expression of said polypeptide in an otherwise identical bacterium that does not carry said modification (control). The expression of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). More particularly, "decreased", "decreasing" or "decrease of" expression of a polypeptide means that the amount of the polypeptide in the modified bacterium is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical bacterium that does not carry said modification (control). The expression or amount of a polypeptide in a microorganism can be determined by any suitable means known in the art, including techniques such as ELISA, Immunohistochemistry, Western Blotting, or Flow Cytometry.

As used herein, "decreased", "decreasing" or "decrease of" activity of a polypeptide (e.g. an enzyme involved in the purine nucleotide degradation pathway) means that the catalytic activity of said polypeptide in a modified bacterium is reduced compared to the catalytic activity of said polypeptide in an otherwise identical bacterium that does not carry said modification (control). The activity of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). The activity of a polypeptide in a microorganism can be determined by any suitable protein and enzyme activity assay.

As used herein, "inhibitor of the enzyme" refers to any chemical compound, natural or synthetic, that inhibits the catalytic activity of the enzyme. An inhibitor of the enzyme does not necessarily need to achieve 100%or complete inhibition. In this regard, an inhibitor of the enzyme can induce any level of inhibition.

"Substitution" or "substituted" refers to the modification of the polypeptide by replacing one amino acid residue with another, for instance, the replacement of a Serine residue with a Glycine or Alanine residue in a polypeptide sequence is an amino acid substitution. When used with reference to a polynucleotide, "substitution" or "substituted" refers to a modification of the polynucleotide by replacing one nucleotide with another, for instance, the replacement of cytosine with thymine in a polynucleotide sequence is a nucleotide substitution.

"Conservative substitution", when used with reference to a polypeptide, refers to a substitution of an amino acid residue with adifferent residue having asimilar side chain, and thus typically involves the substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with a hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid, and a hydrophilic amino acid substituted with a hydrophobic amino acid.

"Expression" includes any step involved in the production of a polypeptide (e.g., encoded enzyme) including, but not limited to, transcription, post-transcriptional modification, translation, post-translational modification, and secretion.

As used herein, the "regulatory region" of a gene refers to a nucleic acid sequence that affects the expression of a coding sequence. Regulatory regions are known in the art and include, but are not limited to, promoters, enhancers, transcription terminators, polyadenylation sites, matrix attachment regions, and/or other elements that regulate the expression of a coding sequence.

As used herein, "vector" refers to a nucleic acid molecule capable of transporting another nucleic acid molecule to which it has been linked. One type of vector is a "plasmid", which refers to a circular double-stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refersto a sequence of DNA, usually upstream (5') of the coding region of a structural gene, which controls the expression of the coding region by providing recognition and binding sites for RNA polymerase and other factors, which may be required for initiation of transcription. The selection of the promoter will depend upon the nucleic acid sequence of interest. A suitable "promoter" is generally one, which is capable of supporting the initiation of transcription in a bacterium of the invention, causing the production of an mRNA molecule. "Strong" promoters include, for example, natural promotersfrom *Bacillussubtilis, Bacillus amyloliquefaciens or* similar, such as P43, P15, Pveg, Pylb, Pgro ES, PsigX, PtrnQ, Ppst, PsodA, PrpsF, PlepA, PliaG, PrpsF, Ppst, PfusA, PsodA, Phag as well as artificial promoters active in *Bacillus subtilis* or inducible *Bacillus* subtilis promoters, such as PmtlA, Pspac, PxylA, PsacB, or similar, that enable efficient expression and overproduction of proteins. Further examplesof "strong" promoters include natural promoters from *Corynebacterium,* such as P CP_2454, Ptuf and Psod, natural promoters from *E coli,* such as T7, and the promoter P F1 derived from the corynephage BFK20.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "%sequence identity" and "percent identity" are used herein to refer to comparisons between an amino acid sequence and a reference amino acid sequence. The "%sequence identity", as used herein, is calculated from the two amino acid sequences as follows: The sequences are aligned using Version 9 of the Genetic Computing Group's GAP (global alignment program), using the default BLOSUM 62 matrix with a gap open penalty of -12 (for the first null of a gap) and a gap extension penalty of -4 (for each additional null in the gap). After alignment, percentage identity is calculated by expressing the number of matches as a percentage of the number of amino acids in the reference amino acid sequence.

"Reference sequence" or "reference amino acid sequence" refers to a defined sequence to which another sequence is compared. In the context of the present invention, a reference amino acid sequence may, for example, be an amino acid sequence set forth in SEQ ID NO: 1.

As used herein, the term "about" means plus or minus 10% of the numerical value of the number with which it is being used.

Where a numerical limit or range is stated herein, the endpoints are included. Also, all values and sub-ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps, or components but do not preclude the addition of one or more additional features, steps, components or groups thereof.

Having generally described this invention, a further understanding can be obtained by reference to certain specific examples, which are provided herein for purposes of illustration only, and are not intended to be limiting unless otherwise specified.

### Examples

### Example 1: Selection of a starting strain

Different Bacillus strains can be used as starting strains for the engineering of isoprenoid cytokinin production (Table 2). *Bacillus* spp. strains can be isolated from nature or obtained from culture collections. Among others, starting strains for isoprenoid cytokinin production can be selected among *Bacillus subtilis* strains that have already been subjected to classical methods of mutagenesis and selection to overproduce metabolites related to the purine nucleotide biosynthetic pathway. For example, strains overproducing riboflavin, inosine, guanosine or adenosine may be selected. Strains subjected to random mutagenesis and toxic metabolic inhibitors from purine nucleotide and riboflavin pathway are preferred and are included in Table 3.

**Table 2. Potential non-GM O starting strains that could be used for the development of isoprenoid-cytokinins-producing strain.**

| **Species** | **Strain** | **Alternative strain name** | **Product** | **Availability** | **Remarks** |
|---|---|---|---|---|---|
| *B. subtilis* | 168 | ATCC6051 | none | yes | Type strain |
| *B*. *subtilis* subsp*. spizizenii* | W23 | ATCC 23059/ NRRL B-14472 | none | yes | Type strain |
| *B. subtilis* | RB50 | NRRL B18502 | riboflavin | yes | Developed by Roche/ DSM |
| *B. subtilis* | RB58 | ATCC55053 | riboflavin | yes | Containing additional copy of rib operon |
| *B. subtilis* | VNII Genetika 304 | VKPM B2116, VBB38 | riboflavin | yes | Developed by VNII Genetika |
| *B. subtilis* | FERM-P1657 | | riboflavin | no | Ajinomoto |
| *B. subtilis* | FERM -P 2292 | | riboflavin | no | Ajinomoto |
| *B. subtilis* | AJ1 2644 | FERM BP-3855 | riboflavin | no | Ajinomoto |
| *B. subtilis* | AJ1 2643 | FERM BP-3856 | riboflavin | no | Ajinomoto |
| *B. subtilis* | ATCC13952 | | inosine | yes | |
| *B. subtilis* | ATCC19221 | IFO 14123 | guanosine | yes | |
| *B. subtilis* | ATCC13956 | IFO 14124 | inosine | yes | |
| *Bacillus* sp. | ATCC21615 | ASB-5741 | none | yes | Asahi Chemical Industry |
| *Bacillus* sp. | ATCC21616 | ASB-5741-2059A | adenosine | yes | Asahi Chemical Industry |
| *Corynebacterium stationis (ammoniagenes)* | ATCC6872 | DSM 20305, IAM 1645, NCTC2399 | adenosine | yes | |

**Table 3. M etabolic inhibitors interfering with the purine nucleotide or the riboflavin biosynthesis pathway.**

| **M etabolic inhibitor** | **Type of inhibitor** |
|---|---|
| 8-azaguanine | purine analogue |
| thioguanine | purine analogue |
| 8-azaxanthine | purine analogue |
| decoyinine | purine analogue |
| roseoflavin | structural analogue of riboflavin |

*Bacillus subtilisVKPM* B2116 is a hybrid strain of *B. subtilis* 168 (most common *B. subtilis* host strain with approx. 4 Mbp genome) with a 6.4 kbp island of DNA from the strain *B. subtilis* W23. Such architecture is common for most *B. subtilis* industrial strains and was obtained by transforming B. *subtilis* 168 (tryptophan auxotroph *trpC*) with W23 (prototrophic TrpC+) DNA. The 6.4 kbp W23 island in the genome of VKPM B21216 is the same as in *B. subtilis* SM Y*,* which is one of the B. *subtilis* legacy strains with genome publicly available (Zeigler et al. 2008). *B. subtilisVKPM* B2116 is a direct descendant strain of *B. subtilisSMY,* obtained by classical mutagenesis and selection. Another name for this strain is B. *subtilis* VNII Genetika 304. The description of the construction of the strain is in Soviet Union patent SU908092, filed in 1980. The mutations were obtained by subsequent mutagenesis and selection of metabolic inhibitors. The strain VKPM B2116 is resistant to roseoflavin, a toxic analogue of vitamin B2, due to a mutation in the *ribCgene,* encoding a flavin kinase. This strain is also resistant to 8-azaguanine, a toxic purine analogue.

### Example 2: Synthesis of synthetic genes IPT SEQ ID 1 and LOG SEQ ID 34 for isoprenoid cytokinin biosynthesis, optimized for Bacillus subtilis

The amino acid sequences of adenylate dimethylallyltransferase (IPT) Tzs from *Agrobacterium tumefaciens* (syn. *Agrobacterium fabrum* (strain C58 / ATCC33970), gene *tzs,* E.C.2.5.1.27, UniProt: P58758) (SEQ ID NO: 1) and of cytokinin riboside 5'-monophosphate phosphoribohydrolase (LOG) from *Corynebacterium glutamicum* (strain ATCC 13032 / DSM 20300 / NCIM B 10025, gene *Cgl2379,* E.C. 3.2.2.n1, UniProt: Q8NN34) (SEQ ID NO: 34) were used for generating codon-optimized nucleotide sequences for gene expression in *B. subtilis* by using GENEius (Eurofins). The synthetic DNA fragment IPT-LOG (SEQ ID NO: 71) was designed with the addition of an RBS sequence, and with short adapter sequences at both ends of the synthetic fragment for further assembly of the synthetic isoprenoid cytokinin operon expression cassette.

### Example 3: Synthesis of synthetic genes IPT SEQ ID 2 and LOG SEQ ID 34 for isoprenoid cytokinin biosynthesis, optimized for Bacillus subtilis

The amino acid sequences of adenylate dimethylallyltransferase (IPT) Tmr from *Agrobacterium tumefaciens(syn. Agrobacterium fabrum* (strain C58 / ATCC33970), gene *izt,* E.C.2.5.1.27, Uniprot: P0A3L5) (SEQ ID NO: 2) and of cytokinin riboside 5'-monophosphate phosphoribohydrolase (LOG) from *Corynebacterium glutamicum* (gene *Cgl2379,* E.C. 3.2.2.n1, UniProt: Q8NN34) (SEQ ID NO: 34) were used for generating codon-optimized nucleotide sequences for gene expression in *B. subtilis* using GENEius (Eurofins). The synthetic DNA fragment IPT-LOG (SEQ ID NO: 72) was designed with the addition of an RBS sequence, and with short adapter sequences at both ends of the synthetic fragment for further assembly of the synthetic isoprenoid cytokinin operon expression cassette.

### Example 4: Assembly of synthetic isoprenoid cytokinin operons containing IPT and LOG, and transformation into B. subtilis

The synthetic fragments containing synthetic genes IPT-LOG (SEQ ID NO: 71 and SEQ ID NO: 72) for isoprenoid cytokinin biosynthesis were assembled in artificial isoprenoid cytokinin operons. The initial and end fragments containing gene integration homology, the promoter sequence, and the erythromycin selectable marker (SEQ ID NO: 73) were designed and synthesized for stable genome integration into the *amyE*locus in the genome of *B. subtilis.*

The first step of the artificial operon assembly was PCR amplification of separate DNA fragments performed using primer pair SEQ ID NO: 74 and SEQ ID 75 for the initial fragment SEQ ID NO: 75, and for synthetic fragments IPT-LOG SEQ ID NO: 71 and SEQ ID NO: 72 containing genes for isoprenoid-cytokinin biosynthesis. The primer set SEQ ID NO: 77 and SEQ ID NO: 78 was used for amplification of the end fragment SEQ ID NO: 79.

The fragments were amplified using Eppendorf cycler and Phusion polymerase (Thermo Fisher) in the buffer provided by the manufacturer and with the addition of 200 µM dNTPs, 0.5 µM of each primer, and approximately 10 ng of template in a final volume of 50 µl for 30 cycles using the PCR cycling conditions: 98 °C30s, 30 cycles of (98°C30s, 68.5°C25s, 72°C23/25s), 72 °C5 min, 10 °C hold.

PCR reaction products of each fragment were run on 0.8 % agarose gel, excised from the gel, and extracted from the gel by GeneJET Gel Extraction Kit (Thermo Fisher) according to the protocol provided by the manufacturer. The fragmentswere assembled into an artificial operon by repetitive steps of restriction and ligation. A combination of *Spel (Bcul)* and *Xbal* restriction sites was used to provide compatible restriction ends for a successful ligation. After each step of ligation, the combined fragments were used as a new template for the next PCR amplification. The restriction was done in 50 µl volume with the addition of 5 µl FD green buffer (Thermo Fisher Scientific), 2 - 3 µl of the selected enzyme (*Spel (Bcul),* and *Xbal,* Thermo Fisher), and up to app. 1500 ng of PCR fragment. After restriction digest, the digested DNA fragments were cleaned with Wizard SV Gel and PCR Clean-up system according to the protocol provided by the manufacturer. The first two fragments were used in the ligation reaction with 2.5 U of T4 DNA ligase (Thermo Fisher) in the buffer provided by the manufacturer and the addition of 5 % PEG 4000 and both fragments in a 1:1 molar ratio to the final volume of 15 µl. In the next step, 1 µl of inactivated ligation was used as a template in a new 50 µL PCRwith a primer set SEQ ID NO: 80 and SEQ ID NO: 81, and the same PCR mix and PCR cycling conditions as previously but with longer elongation time. The restriction digest, cleaning, and ligation steps were repeated for ligation of the end fragment. PCR was run on 0.8 % agarose gel, the fragment was excised from the gel, digested and cleaned as before, and ligated as before. The final operon containing the amy Ehomology, promoter with RBSsequence, IPT and LOG genes, and erythromycin resistance cassette was amplified using the primer pair SEQ ID NO: 76 and SEQ ID NO: 77, cleaned and ligated as described before. The constructed synthetic trans-zeatin operons containing IPT-LOG SEQ ID NO: 82 and SEQ ID NO: 83 were used for the transformation of *Bacillus subtilis* VKPM B2116. The transformation with IPT-LOG operon SEQ ID NO: 83 resulted in the transformed strains TZAB1, TZAB2, TZAB3, and TZAB4. The transformation with IPT-LOGoperon SEQ ID NO: 82 resulted in the transformed strains TZAB14 and TZAB15. The correct integration of the artificial operons at *amy*Eintegration site was confirmed by cPCR.

**Table 4. Bacillus subtilis strains obtained by transformation of IPT-LOG operons.**

| | **IPT-LOG SEQ ID 83** | **IPT-LOG SEQ ID 82** |
|---|---|---|
| **VKPM B2116** | / | / |
| **TZAB1** | **+** | / |
| **TZAB2** | **+** | / |
| **TZAB3** | **+** | / |
| **TZAB4** | **+** | / |
| **TZAB14** | / | **+** |
| **TZAB15** | / | **+** |

### Example 5: Increased isoprenoid cytokinin biosynthesis by increasing of the isoprenoid precursor supply with the overexpression of the dxs

The isoprenoid side chain for the biosynthesis of isoprenoid cytokinins is synthesized by the M EP pathway in *B. subtilis.* The protein sequence of 1-deoxyxylulose-5-phosphate synthase (DXS, E.C. 2.2.1.7) from *B. subtilis* was used for generation of the synthetic nucleotide sequence of *dxs by* using a codon-optimizing feature GENEius (Eurofins) for gene expression in *B. subtilis.* The synthetic DNA fragments of *dxs* SEQ ID NO: 84 and SEQ ID NO: 85 were designed in two overlapping parts, which were joined by overlap PCR. The entire joined synthetic gene *dxs* SEQ ID NO: 86 was assembled in an artificial operon. The initial and end fragments containing *lacA* homology, the promoter sequence, and the spectinomycin selectable marker SEQ ID NO: 87 were designed and synthesized for stable genome integration into the genome of *B. subtilis.* The fragments were assembled as described in Example 4.

The first step of the artificial operon assembly was PCR amplification of separate DNA fragments performed with a set of primers SEQ ID NO: 74 and SEQ ID NO: 75 for the initial fragment SEQ ID NO: 88, the end fragment SEQ ID 89, and the joined synthetic gene *dxs* SEQ ID NO: 86.

The fragments were amplified using Eppendorf cycler and Phusion polymerase (Thermo Fisher) in the buffer provided by the manufacturer and with the addition of 200 µM dNTPs, 0.5 µM of each primer, and approximately 10 ng of template in a final volume of 50 µl for 30 cycles. The PCRcycling conditions used were 98 °C30s, 30 cycles of (98°C30s, 71°C25s, 72°C23/25s), 72 °C5 min, 10 °C hold.

PCR reaction products of each fragment were run on 0.8 % agarose gel, excised from the gel, and extracted from the gel by GeneJET Gel Extraction Kit (Thermo Fisher) according to the protocol provided by the manufacturer. The fragmentswere assembled into an artificial operon by repetitive steps of restriction and ligation. A combination of *Spel (Bcul)* and *Xbal* restriction sites was used to provide compatible restriction ends for a successful ligation. After each step of ligation, the combined fragments were used as a new template for the next PCR amplification. The restriction was done in 50 µl volume with the addition of 5 µl FD green buffer (Thermo Fisher Scientific), 2 - 3 µl of the selected enzyme (*SpeI (Bcu*I*),* and *X*baI, Thermo Fisher), and up to app. 1500 ng of PCR fragment. After restriction digest, the digested DNA fragments were cleaned with Wizard SV Gel and PCR Clean-up system according to the protocol provided by the manufacturer. The first two fragments were used in the ligation reaction with 2.5 U of T4 DNA ligase (Thermo Fisher) in the buffer provided by the manufacturer and both fragments in a 1:1 molar ratio to the final volume of 15 µl. In the next step, 1 µl of inactivated ligation was used as a template in a new 50 µL PCRwith a primer set SEQ ID NO: 74 and SEQ ID NO: 75, and the same PCR mix and PCR cycling conditions as previously but with longer elongation time. The restriction digest, cleaning and ligation stepswere repeated for ligation of the end fragment. In the last step, 1 µl of inactivated ligation was used as a template for complete synthetic dxs operon SEQ ID NO: 90 in a new 50 µL PCR using the primers SEQ ID NO: 91 and SEQ ID NO: 92, and the same PCR mix and PCR cycling conditions as previously but with longer elongation time. PCR was run on 0.8 % agarose gel, the fragment was excised from the gel, cleaned as before, and ligated as before. The constructed synthetic dxs operon was used for the transformation of *Bacillus subtilis*TZAB15 with IPT-LOG operon at *amyE(SEQ* ID NO: 80). The transformation with the artificial DXSoperon (SEQ ID NO: 90) resulted in the transformed strain TZAB43. The correct integration of the artificial operon at *lacA* integration site was confirmed by cPCR.

**Table 5. Bacillus subtilis strains obtained by transformation of the strain B. subtilis TZAB15 with DXSoperon.**

| | **IPT-LOG SEQ ID 81** | **DXS SEQ ID 88** |
|---|---|---|
| **VKPM B2116** | / | / |
| **TZAB15** | + | / |
| **TZAB43** | + | + |

### Example 6: Cultivation of Bacillus subtilis strains for production of isoprenoid cytokinins

All of the constructed strains and control strain have been cultivated following this procedure. The frozen stocks of the strains VKPM B2116, TZAB1, TZAB2, TZAB3, TZAB4, TZAB14, TZAB15 and TZAB43, preserved in 20% glycerol at -80°C were spread onto solid seed medium containing erythromycin and lincomycin in the appropriate concentration and incubated for approximately 1 day at 37°C. For further testing, a vegetative-stage medium was inoculated with 1 to 5 plugs of the culture on solid seed plates per baffled 250-mL Erlenmeyer flask containing 25 mL of vegetative medium and appropriate amounts of antibiotics. The cultures were incubated at 37°Cfor 18-20 h at 220 RPM. The culture in the vegetative medium was used as seed culture, and it was inoculated into the production medium. A 10-% inoculum was used (2.5 mL per 25 mLof production medium in 250-mL Erlenmeyer flask). The production medium was used as described or it was amended with adenine sulfate (end concentration 3 g/L). The cultures were incubated at 30°Cor 37°Cfor up to 48 h at 220 RPM. The fermented cultureswere sampled and analyzed asdescribed in the Example 7. The titer of trans-zeatin, trans-zeatin riboside, and isopentenyl adenine was measured using the LC/ M S as described in the Example 7.

**Table 6. Composition of the solid seed medium**

| **Compound** | **Per 1L** |
|---|---|
| Tryptone | 10 g |
| Yeast extract | 5 g |
| NaCl | 5 g |
| Maltose | 20 g |
| Agar | 20 g |
| pH 7.2-7.4 | |

**Table 7. Composition of the vegetative medium**

| **Compound** | **Per 1L** |
|---|---|
| Molasses | 20 g |
| CSL | 20 g |
| Yeast extract | 5 g |
| M gSO₄* 7H₂O | 0.5 g |
| NH₄)₂SO₄ | 5 g |

The ingredients of the vegetative medium (Table 7) were mixed and the pH was set to 7.2-7.4. KH₂PO₄- K₂HPO₄ solution was then added in the final concentration for KH₂PO₄ 1.5 g/L and K₂HPO₄ 3.5 g/L. The medium was distributed into Erlenmeyer flasks (25 ml/ 250 ml-baffled Erlenmeyer flask) and autoclaved 30 min, 121°C. Sterile glucose was added after autoclaving in the final concentration of 7.5 g/L. Antibiotics were added before inoculation.

**Table 8. Composition of the production medium**

| **Compound** | **Per 1L** |
|---|---|
| Yeast | 20 g |
| CSL | 5 g |
| M gSO₄* 7H₂O | 0.5 g |

The ingredients of the production medium (Table 8) were mixed and the pH was set to 7.2-7.4. KH₂PO₄- K₂HPO₄ solution was then added in the final concentration for KH₂PO₄ 1.5 g/L and K₂HPO₄ 3.5 g/L. The medium was autoclaved at 121°C for 30 min. Sterile urea solution (20 ml of stock solution, final concentration is 6 g/L), sterile glucose solution (500 ml of stock solution, final concentration is 100 g/L glucose)were added after autoclaving to obtain 1 Lof production medium. Appropriate antibiotics were added before inoculation. The medium was then distributed into sterile Erlenmeyer flasks (25 ml/ 250 ml-baffled Erlenmeyer flask).

### Example 7: Analysis of isoprenoid cytokinins

All of the strainsthat had been cultivated following the procedure described in the Example 6 were analysed according to this procedure. The fermented production medium was sampled and immediately frozen at -20°C. For extraction of the metabolites, the fermented production medium was diluted 1:1 with the extraction buffer composed of a 1:1 mixture of methanol and 100 mM ammonium acetate pH 4. The samples were extracted for 1 h at room temperature with constant agitation, centrifuged at 4000 - 4500 RPM for 15 min, and filtered (0.22 um). The samples were immediately analyzed by LC/ MS or stored at -20°Cuntil analysis.

The samples were analyzed on Thermo Accela 1250 HPLC instrument coupled to Thermo TSQ Quantum Access MAX, MS/ MS capable mass spectrometer. The method is based on Thermo Accucore C30, 150x4,6 mm, 2.6 um particle size column, kept at 60°C, with mobile phase A - 0.1% formic acid in water and mobile phase B- methanol, in gradient program, with starting conditions: 95%A, linear gradient increase in B%to 50%at 10 min, and 5 min stabilization to initial conditions, at 1 ml / min flow rate. Mass spectrometer was equipped with hESI ion source, operated in positive (+) mode, with spray voltage set at 4600 V, vaporizer temperature at 350 °C, collision pressure at 1.0 torr and 10 V collision energy. Trans-zeatin was observed in M RM mode with transitions from parent 219.9 m/z to daughter ions: 185.2, 148.0 and 136.0.

### Example 8: Production of trans-zeatin and related isoprenoid cytokinins by Bacillus subtilis strains with heterologous expression of IPT and LOG

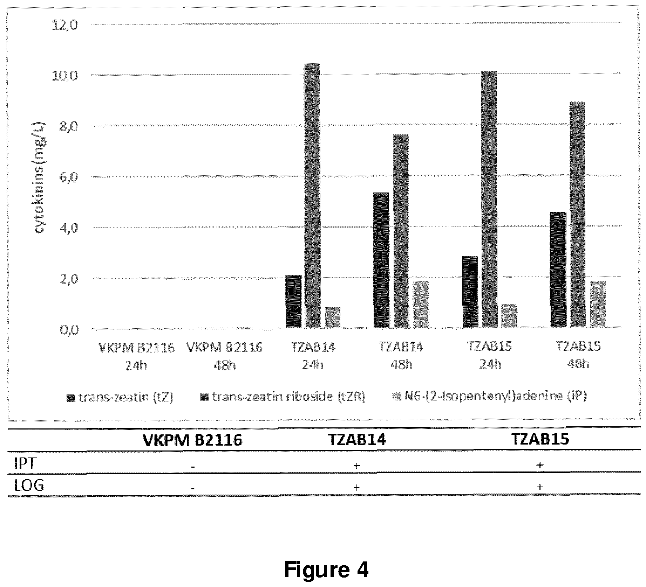

The cultivation was performed as described in the Example 6. The extraction and analysis were performed as described in Example 7. The yields of isoprenoid cytokinins detected are shown in Figure 4. The strains expressing IPT-LOG SEQ ID NO: 82 produce isoprenoid cytokinins in the amounts up to 10 mg/L (see Figure 4).

### Example 9: Production of trans-zeatin and related isoprenoid cytokinins by Bacillus subtilis strains with heterologous expression of IPT and LOG, and with adenine sulfate in the growth medium

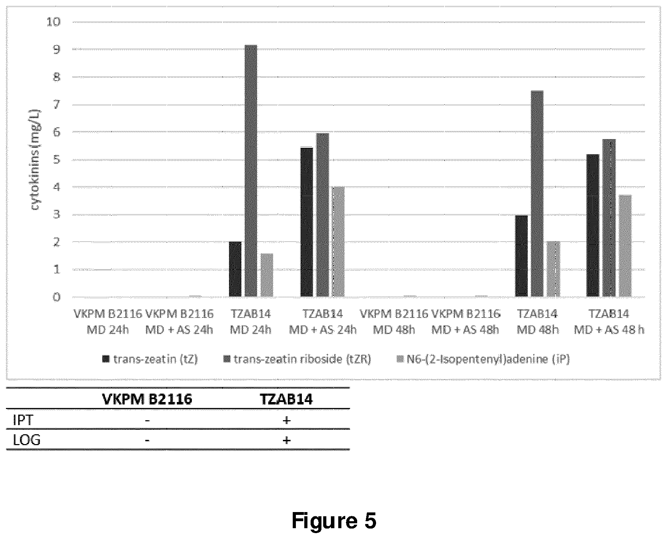

The cultivation was performed as described in the Example 6. The production medium was composed as described in the Example 6, or it was amended with adenine sulfate in the final concentration 3 g/L. The fermentation broth was extracted and analyzed as described in the Example 7. The results are shown in Figure 5. The production of isoprenoid cytokinins in the strain TZAB14 with IPT-LOG SEQ ID NO: 82 is increased in the medium containing adenine sulfate compared to the medium without adenine sulfate.

### Example 10: Production of trans-zeatin and related isoprenoid cytokinins by Bacillus subtilis strains with heterologous expression of IPT-LOG SEQ ID 82 and overexpression of DXS

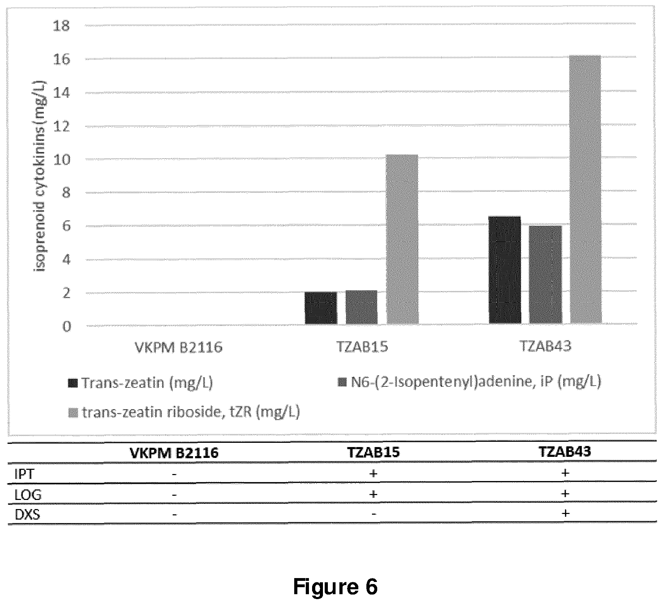

The cultivation was performed as described in the Example 6. The extraction and analysis were performed as described in the Example 7. The yields are shown in Figure 6. The production of isoprenoid cytokinins is increased in the strains with IPT-LOG operon SEQ ID NO: 82 and the DXS operon SEQ ID NO: 90.

### Example 11: Analysis of trans-zeatin and related isoprenoid cytokinins production by Bacillus subtilis strains with IPT-LOG (SEQ ID NO: 83)

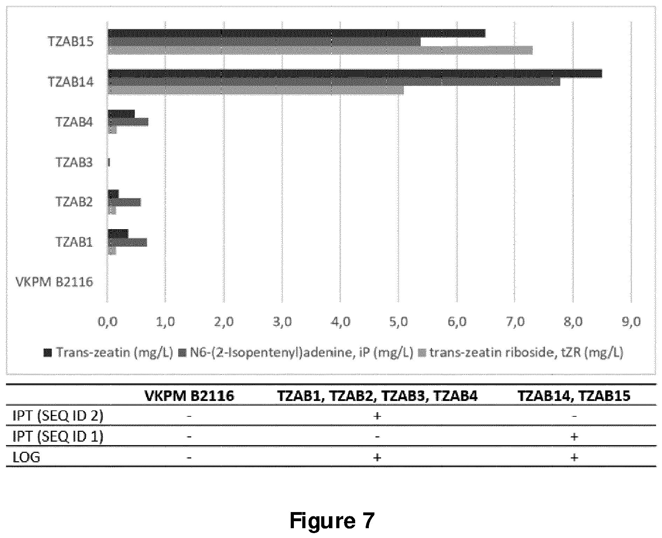

The cultivation was performed as described in the Example 6. The extraction and analysis were performed as described in the Example 7. The strains TZAB1, TZAB2, TZAB3 and TZAB4 with IPT-LOG SEQ ID NO: 83 and the strains TZAB14 and TZAB 15 with IPT-LOG SEQ ID NO: 82 produced isoprenoid cytokinins. The results are shown in Figure 7.

### List of references cited in the description

Akiyoshi, D. E., D. A. Regier, G. Jen, and M. P. Gordon. 1985. "Cloning and Nucleotide Sequence of the Tzs Gene from Agrobacterium Tumefaciens Strain T37." Nucleic Acids Research 13 (8): 2773-88. https://doi.org/10.1093/nar/13.8.2773.
Akiyoshi, Donna E., Dean A. Regier, and Milton P. Gordon. 1987. "Cytokinin Production by Agrobacterium and Pseudomonas Spp." Journal of Bacteriology 169 (9): 4242-48. https://doi.org/10.1128/ jb.169.9.4242-4248.1987.
Arkhipova, T. N., S. U. Veselov, A. I. Melentiev, E. V. Martynenko, and G. R. Kudoyarova. 2005. "Ability of Bacterium Bacillus Subtilis to Produce Cytokinins and to Influence the Growth and Endogenous Hormone Content of Lettuce Plants." Plant and Soil 272 (1-2): 201-9. https://doi.org/10.1007/s11104-004-5047-x.
Asahara, T. et al. (2010) 'Accumulation of gene-targeted Bacillus subtilis mutations that enhance fermentative inosine production', Applied Microbiology and Biotechnology. Springer-Verlag, 87(6), pp. 2195-2207. doi: 10.1007/s00253-010-2646-8.
Banerjee, A. et al. (2013) 'Feedback inhibition of deoxy-D-xylulose-5-phosphate synthase regulates the methylerythritol 4-phosphate pathway', Journal of Biological Chemistry. doi: 10.1074/jbc.M 113.464636.
Chen, S. et al. (1997) 'Mechanism of the synergistic end-product regulation of Bacillus subtilis glutamine phosphoribosylpyrophosphate amidotransferase by nucleotides', Biochemistry, 36(35), pp. 10718-10726. doi: 10.1021/bi9711893.
Christiansen, L. C., Schou, S. and Nygaard, P. E. R. (1997) Xanthine Metabolism in Bacillus subtilis•: Characterization of the xpt-pbuX Operon and Evidence for Purine- and Nitrogen-Controlled Expression of Genes Involved in Xanthine Salvage and Catabolism', 179(8), pp. 2540-2550.
Datsenko KA, Wanner BL: One-step inactivation of chromosomal genesin Escherichiacoli K-12 using PCR products. Proc Natl Acad Sci U SA 2000, 97:6640-6645.
Frebort, I., M. Kowalska, T. Hluska, J. Frebortova, P. Galuszka, Marta Kowalska1 Ivo Fre' bort1,*, Toma' s• Hluska1, et al. 2011. "Evolution of Cytokinin Biosynthesis and Degradation." Journal of Experimental Botany 62 (8): 2431-52. https://doi.org/10.1093/jxb/err004.
Frébortová, Jitka, Marta Greplová, Michael F. Seidl, Alexander Heyl, and Ivo Frébort. 2015. "Biochemical Characterization of Putative Adenylate Dimethylallyltransferase and Cytokinin Dehydrogenase from Nostoc Sp. PCC 7120." PLoS ONE 10 (9). https://doi.org/10.1371/journal.pone.0138468.
Julsing, M attijs K., Michael Rijpkema, Herman J. Woerdenbag, Wim J. Quax, and Oliver Kayser. 2007. "Functional Analysis of Genes Involved in the Biosynthesis of Isoprene in Bacillus Subtilis." Applied Microbiology and Biotechnology 75 (6): 1377-84. https://doi.org/10.1007/s00253-007-0953-5.
Kakimoto, T. 2001. "Identification of Plant Cytokinin Biosynthetic Enzymes as Dimethylallyl Diphosphate:ATP/ADP Isopentenyltransferases." Plant and Cell Physiology. https://doi.org/10.1093/pcp/pce112.
Kamada-Nobusada, Tomoe, and Hitoshi Sakakibara. 2009. "Molecular Basis for Cytokinin Biosynthesis." Phytochemistry 70 (4): 444-49. https://doi.org/10.1016/j.phytochem.2009.02.007.
Konishi, S. and Shiro, T. (1968) 'Fermentative Production of Guanosine by 8-Azaguanine Resistant of Bacillus subtilis', Agricultural and Biological Chemistry, 32(3), pp. 396-398. doi: 10.1080/00021369.1968.10859067.
Kurakawa, Takashi, Nanae Ueda, Masahiko Maekawa, Kaoru Kobayashi, Mikiko Kojima, Yasuo Nagato, Hitoshi Sakakibara, and Junko Kyozuka. 2007. "Direct Control of Shoot Meristem Activity by a Cytokinin-Activating Enzyme." Nature 445 (7128): 652-55. https://doi.org/10.1038/nature05504.
Kuzuyama, T. et al. (2000) 'Cloning and characterization of 1-deoxy-D-xylulose 5-phosphate synthase from Streptomycessp. Strain CL190,which uses both the mevalonate and nonmevalonate pathways for isopentenyl diphosphate biosynthesis.', Journal of bacteriology. American Society for Microbiology (ASM), 182(4), pp. 891-7. doi: 10.1128/jb.182.4.891-897.2000.
Kwon DH, Peña JA, Osato M S, Fox JG, Graham DY, Versalovic J: Frameshift mutations in rdxA and metronidazole resistance in North American Helicobacter pylori isolates. JAntimicrob Chemother 2000, 46(5): 793-796.
Li, Biao, Zhi-Ying Ying Yan, Xiao-Na Na Liu, Jun Zhou, Xia-Yuan Yuan Wu, Ping Wei, Hong-Hua Hua Ja, and Xiao-Yu Yu Yong. 2019. "Increased Fermentative Adenosine Production by Gene-Targeted Bacillus Subtilis Mutation." Journal of Biotechnology 298 (June): 1-4. https://doi.org/10.1016/j.jbiotec.2019.04.007.
Mok, Machteld C., Ruth C. Martin, and David W. S. Mok. 2000. "Cytokinins: Biosynthesis Metabolism and Perception." In Vitro Cellular & Developmental Biology - Plant 36 (2): 102-7. https://doi.org/10.1007/s11627-000-0021-7.
Nishii K, Wright F, Chen Y-Y, Möller M (2018) Tangled history of a multigene family: The evolution of ISOPENTENYLTRANSFERASE genes. PLoS ONE 13(8): e0201198. https://doi.org/10.1371/journal.pone.0201198
Patel, Pooja P, Purvi M Rakhashiya, Kiran S Chudasama, and Vrinda S Thaker. 2012. "Isolation , Purification and Estimation of Zeatin from Corynebacterium Aurimucosum." European Journal of Experimental Biology 2 (1): 1-8.
Peifer, S. et al. (2012) 'Metabolic engineering of the purine biosynthetic pathway in Corynebacterium glutamicum resultsin increased intracellular pool sizesof IM Pand hypoxanthine', Microbial Cell Factories. BioM ed Central, 11(1), p. 138. doi: 10.1186/1475-2859-11-138.
Powell, G. K., and R. O. Morris. 1986. "Nucleotide Sequence and Expression of a Pseudomonas Savastanoi Cytokinin Biosynthetic Gene: Homology with Agrobacterium Tumefaciens Tmr and Tzs Loci." Nucleic Acids Research 14 (6): 2555-65. https://doi.org/10.1093/nar/14.6.2555.
Qui Z and Goodman M F: The Escherichia coli polB locus is identical to dinA, the structural gene for DNA polymerase II. Characterization of Pol II purified from a polB mutant. J Biol Chem. 1997, 272(13): 8611-8617.
Regier, Dean A., and Roy O. Morris. 1982. "Secretion of Trans-Zeatin by Agrobacterium Tumefaciens: A Function Determined by the Nopaline Ti Plasmid." Biochemical and Biophysical Research Communications 104 (4): 1560-66. https://doi.org/10.1016/0006-291X(82)91429-2.
Sakakibara, Hitoshi. 2005. "Cytokinin Biosynthesis and Regulation." Vitamins and Hormones. Vitam Horm. https://doi.org/10.1016/S0083-6729(05)72008-2.
Sakakibara, Hitoshi. 2006. "Cytokinins: Activity, Biosynthesis, and Translocation." Annual Review of Plant Biology 57 (1): 431-49. https://doi.org/10.1146/annurev.arplant.57.032905.105231.
Sakakibara, Hitoshi, Hiroyuki Kasahara, Nanae Ueda, Mikiko Kojima, Kentaro Takei, Shojiro Hishiyama, Tadao Asami, et al. 2005. "Agrobacterium Tumefaciens Increases Cytokinin Production in Plastids by Modifying the Biosynthetic Pathway in the Host Plant." Proceedings of the National Academy of Sciences of the United States of America 102 (28): 9972-77. https://doi.org/10.1073/pnas.0500793102.
Scarbrough, E., D. J. Armstrong, and F. Skoog. 1973. "Isolation of Cis Zeatin from Corynebacterium Fascians Cultures." Proceedings of the National Academy of Sciences of the United States of America 70 (12 II): 3825-29. https://doi.org/10.1073/pnas.70.12.3825.
Schäfer, Martin, Christoph Brütting, Ivan David Meza-Canales, Dominik K Großkinsky, Radomira Vankova, Ian T. Baldwin, and Stefan M eldau. 2015. "The Role of Cis -Zeatin-Type Cytokinins in Plant Growth Regulation and Mediating Responses to Environmental Interactions." Journal of Experimental Botany 66 (16): 4873-84. https://doi.org/10.1093/jxb/erv214.
Seo, Hogyun, and Kyung-Jin Jin Kim. 2017. "Structural Basis for a Novel Type of Cytokinin-Activating Protein." Scientific Reports 7 (1): 45985. https://doi.org/10.1038/srep45985.
Seo, Hogyun, Sangwoo Kim, Hye-Young Sagong, Hyeoncheol Francis Son, Kyeong Sik Jn, II-Kwon Kim, and Kyung-Jin Kim. 2016. "Structural Basis for Cytokinin Production by LOG from Corynebacterium Glutamicum" 6 (1): 31390. https://doi.org/10.1038/srep31390.
Shi, S. et al. (2009) 'Transcriptome analysis guided metabolic engineering of Bacillus subtilis for riboflavin production', Metabolic Engineering. Academic Press, 11(4-5), pp. 243-252. doi: 10.1016/j.ymben.2009.05.002.
Shi, T. et al. (2014) 'Deregulation of purine pathway in Bacillus subtilis and its use in riboflavin biosynthesis', Microbial Cell Factories, 13(1), pp. 1-16. doi: 10.1186/s12934-014-0101-8.
Stirk, Wendy A., and J. van Staden. 2010. "Flow of Cytokinins through the Environment." Plant Growth Regulation 62 (November 2010): 101-16. https://doi.org/10.1007/s10725-010-9481-x.
Streletskii, Rostislav A, Aleksey V Kachalkin, Anna M Glushakova, Andrey M Yurkov, and Vladimir V Demin. 2019. "Yeasts Producing Zeatin." PeerJ7: e6474. https://doi.org/10.7717/peerj.6474.
Sugawara, Hajime, Nanae Ueda, Mikiko Kojima, Nobue Makita, Tomoyuki Yamaya, and Hitoshi Sakakibara. 2008. "Structural Insight into the Reaction Mechanism and Evolution of Cytokinin Biosynthesis." Proceedings of the National Academy of Sciences 105 (7): 2734-39. https://doi.org/10.1073/pnas.0707374105.
Takei, Kentaro, Hitoshi Sakakibara, and Tatsuo Sugiyama. 2001. "Identification of Genes Encoding Adenylate Isopentenyltransferase, a Cytokinin Biosynthesis Enzyme, in Arabidopsis Thaliana." Journal of Biological Chemistry 276 (28): 26405-10. https://doi.org/10.1074/jbc.M102130200.
Takei, Kentaro, Tomoyuki Yamaya, and Hitoshi Sakakibara. 2004. "Arabidopsis CYP735A1 and CYP735A2 Encode Cytokinin Hydroxylases That Catalyse the Biosynthesis of Trans-Zeatin." Journal of Biological Chemistry 279 (40): 41866-72. https://doi.org/10.1074/jbc.M406337200.
Wang, X. et al. (2016) 'Directed evolution of adenylosuccinate synthetase from Bacillus subtilis and its application in metabolic engineering', Journal of Biotechnology. Elsevier B.V., 231(May), pp. 115-121. doi: 10.1016/j.jbiotec.2016.05.032.
Wang, Z. et al. (2011) 'Enhancement of riboflavin production with Bacillus subtilis by expression and site-directed mutagenesis of zwf and gnd gene from Corynebacterium glutamicum.', Bioresource technology, 102(4), pp. 3934-40. doi: 10.1016/j.biortech.2010.11.120.
Xiang, S. et al. (2007) 'Crystal Structure of 1-Deoxy-d-xylulose 5-Phosphate Synthase, a Crucial Enzyme for Isoprenoids Biosynthesis', Journal of Biological Chemistry, 282(4), pp. 2676-2682. doi: 10.1074/jbc.M610235200.
Xiang, S. et al. (2012) '1-Deoxy-d-Xylulose 5-Phosphate Synthase (DXS), a Crucial Enzyme for Isoprenoids Biosynthesis', in Isoprenoid Synthesis in Plants and Microorganisms. New York, NY: Springer New York, pp. 17-28. doi: 10.1007/978-1-4614-4063-5_2.
Xue, D. et al. (2015) 'Enhanced C30 carotenoid production in Bacillus subtilis by systematic overexpression of M EP pathway genes', Applied Microbiology and Biotechnology, 99(14), pp. 5907-5915. doi: 10.1007/s00253-015-6531-3.
Yang, S. et al. (2019) 'Modular Pathway Engineering of Bacillus subtilis to promote de novo biosynthesis of menaquinone-7', ACS Synthetic Biology. American Chemical Society, 8(1), pp. 70-81. doi: 10.1021/acssynbio.8b00258.
Zakataeva, N. P. et al. (2012) 'Wild-type and feedback-resistant phosphoribosyl pyrophosphate synthetases from Bacillus amyloliquefaciens: Purification, characterization, and application to increase purine nucleoside production', Applied Microbiology and Biotechnology, 93(5), pp. 2023-2033. doi: 10.1007/s00253-011-3687-3.
Zeigler, Daniel R., Zoltán Prágai, Sabrina Rodriguez, Bastien Chevreux, Andrea Muffler, Thomas Albert, Renyuan Bai, Markus Wyss, and John B. Perkins. 2008. "The Origins of 168, W23, and Other Bacillus Subtilis Legacy Strains." Journal of Bacteriology 190 (21): 6983-95. https://doi.org/10.1128/ JB.00722-08.

## Claims

1. A bacterium, which a) expresses a heterologous polypeptide having adenylate isopentenyltransferase activity and b) has been modified to have an increased protein expression of a polypeptide having cytokinin riboside 5'-monophosphate phosphoribohydrolase activity compared to an otherwise identical bacterium that does not carry said modification.

2. The bacterium according to claim 1, wherein the polypeptide having adenylate isopentenyltransferase activity is selected from the group consisting of: i) a polypeptide comprising an amino acid sequence of any one of SEQ ID NOs: 1 to 33; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 1 to 33.

3. The bacterium according to claim 1 or 2, wherein the polypeptide having adenylate isopentenyltransferase activity is selected from the group consisting of: i) a polypeptide comprising an amino acid sequence of SEQ ID NO: 1; and ii) a polypeptide comprising an amino acid sequence, which has at least about 50%, such as at least about 55%, at least about 60%, at least about 65%, at least about 70%, at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 1.

4. The bacterium according to any one of claims 1 to 3, wherein the polypeptide having cytokinin riboside 5'-monophosphate phosphoribohydrolase activity is selected from the group consisting of: i) a polypeptide comprising an amino acid sequence of any one of SEQ ID NOs: 34 to 62 and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 34 to 62.

5. The bacterium according to any one of claims 1 to 4, wherein the polypeptide having cytokinin riboside 5'-monophosphate phosphoribohydrolase activity is selected from the group consisting of: i) a polypeptide comprising the amino acid sequence of SEQ ID NO: 34; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of SEQ ID NO: 34.

6. The bacterium according to any one of claims 1 to 5, wherein the bacterium has been further modified to have an increased protein expression of a polypeptide having 1-deoxy-D-xylulose-5-phosphate synthase activity compared to an otherwise identical bacterium that does not carry said modification.

7. The bacterium according to claim 6, wherein the polypeptide having 1-deoxy-D-xylulose-5-phosphate synthase activity is selected from the group consisting of: i) a polypeptide comprising an amino acid sequence of any one of SEQ ID NOs: 63 to 70; and ii) a polypeptide comprising an amino acid sequence, which has at least about 70%, such as at least about 75%, at least about 80%, at least about 85%, at least about 90%, at least about 93%, at least 95%, at least 96%, at least 97%, at least 98%, or at least 99%, sequence identity to the amino acid sequence of any one of SEQ ID NOs: 63 to 70.

8. The bacterium according to any one of claims 1 to 7, which has been further modified to have an increased expression and/or activity of at least one enzyme involved in the adenosine monophosphate biosynthesis pathway compared to an otherwise identical bacterium that does not carry said modification.

9. The bacterium according to claim 8, wherein the at least one enzyme involved in the adenosine monophosphate biosynthesis pathway is selected from the group consisting of: an enzyme having ribose-phosphate diphosphokinase activity, an enzyme having amidophosphoribosyltransferase activity, an enzyme having formyltetrahydrofolate deformylase activity, an enzyme having adenylosuccinate lyase activity, an enzyme having phosphoribosylaminoimidazole-carboxamide formyltransferase activity, an enzyme having adenylosuccinate synthase activity and an enzyme having adenosine kinase activity.

10. The bacterium according to any one of claims 1 to 9, which has been further modified to have a decreased expression and/or activity of at least one endogenous enzyme involved in the purine nucleotide degradation pathway compared to an otherwise identical bacterium that does not carry said modification.

11. The bacterium according to claim 10, wherein the at least one endogenous enzyme involved in the purine nucleotide degradation pathway is selected from the group consisting of: an enzyme having purine nucleoside phosphorylase activity and an enzyme having adenosine-phosphoribosyltransferase activity.

12. The bacterium according to any one of claims 1 to 11, which has been further modified to have a decreased expression and/or activity of at least one endogenous enzyme involved in the guanosine monophosphate biosynthesis pathway compared to an otherwise identical bacterium that does not carry said modification.

13. The bacterium according to claim 12, wherein the at least one endogenous enzyme involved in the guanosine monophosphate biosynthesis pathway isselected from the group consisting of: an enzyme having IM P dehydrogenase activity and an enzyme having GM P synthetase activity.

14. The bacterium according to any one of claims 1 to 13, wherein the bacterium isof the family selected from the group consisting of *Bacillaceae* and *Corynebacteriaceae.*

15. Method for producing an isoprenoid cytokinin or riboside derivative thereof, comprising cultivating a bacterium according to any one of claims 1 to 14 under suitable culture conditions in a suitable culture medium.
